(19) Europäisches Patentamt
European Patent Office
Office européen des brevets

(11) EP 1 473 365 A1

(12) **EUROPEAN PATENT APPLICATION**
published in accordance with Art. 158(3) EPC

(43) Date of publication:
**03.11.2004 Bulletin 2004/45**

(21) Application number: **03737460.0**

(22) Date of filing: **03.02.2003**

(51) Int Cl.[7]: **C12N 15/12**, C07K 14/47, C07K 16/18, C12N 5/10, C12P 21/02, C12Q 1/68, A61K 31/7088, A61K 39/395, A61K 45/00, A61K 48/00, A61P 15/08, A61P 35/00, A61P 43/00

(86) International application number:
**PCT/JP2003/001057**

(87) International publication number:
**WO 2003/066860 (14.08.2003 Gazette 2003/33)**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(30) Priority: **04.02.2002 JP 2002027299**

(71) Applicant: **Takeda Chemical Industries, Ltd.**
**Osaka-shi, Osaka 541-0045 (JP)**

(72) Inventors:
- **OHTAKI, Tetsuya**
  **Tsukuba-shi, Ibaraki 305-0031 (JP)**
- **MASUDA, Yasushi**
  **Tsukuba-shi, Ibaraki 305-0032 (JP)**
- **TAKATSU, Yoshihiro**
  **Tsukuba-shi, Ibaraki 305-0032 (JP)**

(74) Representative: **Rickard, Timothy Mark Adrian**
**Takeda Euro IP Department,**
**11-12 Charles II Street**
**London SW1Y 4QU (GB)**

# (54) ANGIOGENESIS INHIBITORS

(57) The present invention provides a method of screening a compound or its salt promoting or inhibiting the activity of a novel protein, etc; a screening kit therefor; compounds or salt thereof obtained by the screening; drugs containing these compounds or salts thereof; and so on. These drugs are usable as, for example, angiogenesis inhibitors in prevention /remedies for cancer, ovary diseases, inflammatory diseases, diabetic retinopathy, etc.

EP 1 473 365 A1

**Description**

TECHNICAL FIELD

The present invention relates to (1) (i) a peptide or its salt characterized by containing the same or substantially the same amino acid sequence as the amino acid sequence represented by SEQ ID NO: 21, SEQ ID NO: 81, SEQ ID NO: 82, SEQ ID NO: 109, SEQ ID NO: 138, SEQ ID NO: 19, SEQ ID NO: 39 or SEQ ID NO: 37; and (ii) a method / kit of screening a compound or its salt, etc. useful as a angiogenesis inhibitors, which comprises using a protein or its salt containing the same or substantially the same amino acid sequence as the amino acid sequence represented by SEQ ID NO: 1, SEQ ID NO: 44, SEQ ID NO: 51, SEQ ID NO: 52, SEQ ID NO: 53, SEQ ID NO: 54, SEQ ID NO: 171 or SEQ ID NO: 173, which is an orphan receptor protein; (2) an angiogenesis inhibitors which inhibit an activity of a protein or its salt containing the same or substantially the same amino acid sequence as the amino acid sequence represented by SEQ ID NO: 1, SEQ ID NO: 44, SEQ ID NO: 51, SEQ ID NO: 52, SEQ ID NO: 53, SEQ ID NO: 54, SEQ ID NO: 171 or SEQ ID NO: 173; (3)a protein, its salt, its preparation method or its use, etc. characterized by containing the same or substantially the same amino acid sequence as the amino acid sequence represented by SEQ ID NO: 171 or SEQ ID NO: 173.

BACKGROUND ART

A novel growth factor for a bovine adrenal capillary vessel endothelial cell from a library which was gathered various secretory proteins (Nature, 412 p.877-884, 2001). The above substance is a growth factor which is specific for endothelial cell derived from endocrine tissue and is different from a known vascular endothelial cell growth factor, VEGF, named EG-VEGF by this property. EG-VEGF is suggested to participate in the formation of fenestrate which is high permeable and physiologically characterizing an endocrine tissue. A translation modulating region of the gene has recognizing site of hypoxia-inducible factor-1 (HIF-1) which relates to a n expression induction under low-oxygen condition. Actually, in a cancer cell derived from adrenal, an expression induction of EG-VEGF gene under low-oxygen condition is confirmed. Further, in a human polycystic ovary syndrome, it is described that a strong expression of EG-VEGF is confirmed.
Meanwhile, EG-VEGF (hereinafter sometimes referred to as ZAQ ligand peptide) binds to ZAQ which is an orphan receptor and activated ZAQ (WO 01/16309). ZAQ ligand peptide which has activity of an ileum contraction has highly similar sequence to MIT1 which was isolated from (FEBS Letters 461, p. 183-188, 1999) snake and is its mammalian type peptide. Also, peptide of snake poison MIT1 and its human homologue other than human type ZAQ ligand peptide binds to ZAQ and activates ZAQ.
Recent days, a compound which inhibits an action of a VEGF receptor has been developed as a anticancer agent on the basis of action of its angiogenesis inhibitor, however, since the foundation of EG-VEGF, it has been considered to be cases which does not sufficiently inhibit an angiogenesis with only blocking the action of VEGF. Further, it is possible to find cases which are operated by EG-VEGF rather than VEGF as a virulence factor of a disease, depending on future development of medical research. In that case, an EG-VEGF inhibitor is expected to be used as an elemental substance. In the past research, it was not absolutely clarified that in the process of inducing a growth of endothelial cell by EG-VEGF, whether a receptor such as ZAQ relate to or not, or whether a quite different receptor relate to or not. However, in the development of EG-VEGF antagonist, in terms of simplifying a method of searching an antagonist and description of action mechanism, the receptor is necessary to be clarified. Further, safe, superior angiogenesis inhibitor of cancer is desired.

DISCLOSURE OF THE INVENTION

The present inventors carried out a research using a bovine adrenal capillary vessel endothelial cell (BACE) and found that ZAQ and its relative receptor, I1E were expressed in BACE. Further, they also found that BACE responded to human type ZAQ ligand peptide, snake poison MIT1 and its human homologue (human Bv8 peptide) and rising intercellular calcium ion, activation of MAP kinase, increase of uptake quantity of thymidine or increase of proliferating ability were induced. According to the result of the studies, it is concluded that an endothelial cell growth factor activity of human type ZAQ ligand peptide was induced through ZAQ and (or) I5E. in accordance with these knowledge, the present inventors found that the compound which blocks activity of ZAQ or I5E is inhibitors of peptide of EG-VEGF or Bv8 and the compound provides a convenient research method of the inhibitors of EG-VEGF or Bv8, and have thus come to accomplish the present invention.
That is, the present invention provides the following features:

(1) An angiogenesis inhibitor, which comprises a compound or salt thereof inhibiting the activity of a peptide or a salt thereof containing the same or substantially the same amino acid sequence shown in SEQ ID NO: 81, SEQ ID NO:82, SEQ ID NO: 109, SEQ ID NO:138, SEQ ID NO:19 or SEQ ID NO: 39.

(1a) An angiogenesis inhibitor, which comprises a compound or salt thereof inhibiting the activity of a peptide or a salt thereof containing the same or substantially the same amino acid sequence shown in SEQ ID NO: 81, SEQ ID NO:82, SEQ ID NO: 109 or SEQ ID NO: 138.

(1b) An angiogenesis inhibitor, which comprises a compound or salt thereof inhibiting the activity of a peptide or a salt thereof containing the same or substantially the same amino acid sequence shown in SEQ ID NO: 19 or SEQ ID NO: 39,

(2) An angiogenesis inhibitor, which comprises a compound or salt thereof inhibiting the activity of a protein or a salt thereof containing the same or substantially the same the amino acid sequence shown in SEQ ID NO: 1, SEQ ID NO: 44, SEQ ID NO:51, SEQ ID NO:52, SEQ ID NO:53, SEQ ID NO:54, SEQ ID NO: 171 or SEQ ID NO:173,

(3) An angiogenesis inhibitor, which comprises a compound or salt thereof inhibiting the binding of (a) a peptide or salt thereof containing the same or substantially the same amino acid sequence shown in SEQ ID NO: 21, SEQ ID NO:81, SEQ ID NO:82, SEQ ID NO: 109, SEQ ID NO: 138, SEQ ID NO:19 or SEQ ID NO:39, with (b) a protein or salt thereof having an amino acid sequence identical to or substantially identical to the amino acid sequence shown in SEQ ID NO: 1, SEQ ID NO:44, SEQ ID NO:51, SEQ ID NO:52, SEQ ID NO:53, SEQ ID NO:54, SEQ ID NO: 171 or SEQ ID NO: 173,

(3a) An angiogenesis inhibitor, which comprises a compound or salt thereof inhibiting the binding of (a) a peptide or salt thereof containing the same or substantially the same amino acid sequence shown in SEQ ID NO: 21with (b) a protein or salt thereof having an amino acid sequence identical to or substantially identical to the amino acid sequence shown in SEQ ID NO: 1, SEQ ID NO:44, SEQ ID NO:51, SEQ ID NO:52, SEQ ID NO:53, SEQ ID NO: 54, SEQ ID NO:171 or SEQ ID NO: 173,

(3b) An angiogenesis inhibitor, which comprises a compound or salt thereof inhibiting the binding of (a) a peptide or salt thereof containing the same or substantially the same amino acid sequence shown in SEQ ID NO:81, SEQ ID NO:82, SEQ ID NO: 109, SEQ ID NO: 138, with (b) a protein or salt thereof having an amino acid sequence identical to or substantially identical to the amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO: 173,

(3c) An angiogenesis inhibitor, which comprises a compound or salt thereof inhibiting the binding of (a) a peptide or salt thereof containing the same or substantially the same amino acid sequence shown in SEQ ID NO:19 or SEQ ID NO:39, with (b) a protein or salt thereof having an amino acid sequence identical to or substantially identical to the amino acid sequence shown in SEQ ID NO: 1, SEQ ID NO:44, SEQ ID NO:51, SEQ ID NO:52, SEQ ID NO: 53, SEQ ID NO:54, SEQ ID NO:171 or SEQ ID NO:173,

(4) An angiogenesis inhibitor, which comprises a compound or salt thereof inhibiting the action of a peptide or salt thereof containing the same or substantially the same amino acid sequence shown in SEQ ID NO: 21, SEQ ID NO:81, SEQ ID NO:82, SEQ ID NO: 109, SEQ ID NO: 138, SEQ ID NO:19 or SEQ ID NO:39, to activate a protein or salt thereof having an amino acid sequence identical to or substantially identical to the amino acid sequence shown in SEQ ID NO: 1, SEQ ID NO:44, SEQ ID NO:51, SEQ ID NO:52, SEQ ID NO:53, SEQ ID NO:54, SEQ ID NO: 171 or SEQ ID NO: 173,

(4a) An angiogenesis inhibitor, which comprises a compound or salt thereof inhibiting the action of a peptide or salt thereof containing the same or substantially the same amino acid sequence shown in SEQ ID NO: 21 to activate a protein or salt thereof having an amino acid sequence identical to or substantially identical to the amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO: 173,

(4b) An angiogenesis inhibitor, which comprises a compound or salt thereof inhibiting the action of a peptide or salt thereof containing the same or substantially the same amino acid sequence shown in SEQ ID NO:81, SEQ ID NO:82, SEQ ID NO:109, SEQ ID NO:138, to activate a protein or salt thereof having an amino acid sequence identical to or substantially identical to the amino acid sequence shown in SEQ ID NO: 1, SEQ ID NO:44, SEQ ID NO:51, SEQ ID NO:52, SEQ ID NO:53, SEQ ID NO:54, SEQ ID NO: 171 or SEQ ID NO: 173,

(4c) An angiogenesis inhibitor, which comprises a compound or salt thereof inhibiting the action of a peptide or salt thereof containing the same or substantially the same amino acid sequence shown in SEQ ID NO:19 or SEQ ID NO:39, to activate a protein or salt thereof having an amino acid sequence identical to or substantially identical to the amino acid sequence shown in SEQ ID NO: 1, SEQ ID NO:44, SEQ ID NO:51, SEQ ID NO:52, SEQ ID NO: 53, SEQ ID NO:54, SEQ ID NO:171 or SEQ ID NO: 173,

(5) An angiogenesis inhibitor, which comprises an anti-sense nucleotide having a nucleotide sequence or portion thereof complementary to or substantially complementary to the nucleotide sequence of a DNA encoding a peptide or partial peptide thereof containing the same or substantially the same amino acid sequence shown in SEQ ID NO: 81, SEQ ID NO:82, SEQ ID NO: 109, SEQ ID NO:138, SEQ ID NO: 19 or SEQ ID NO:39,

(6) An angiogenesis inhibitor, which comprises an antibody against a peptide, partial peptide thereof or salt thereof containing the same or substantially the same amino acid sequence shown in SEQ ID NO: 81, SEQ ID NO:82, SEQ ID NO: 109, SEQ ID NO: 138, SEQ ID NO:19 or SEQ ID NO:39,

(7) An angiogenesis inhibitor, which comprises an anti-sense nucleotide having a nucleotide sequence or portion thereof complementary to or substantially complementary to the nucleotide sequence of a DNA encoding a protein or partial peptide thereof containing the same or substantially the same amino acid sequence shown in SEQ ID NO: 1, SEQ ID NO: 44, SEQ ID NO:51, SEQ ID NO:52, SEQ ID NO:53, SEQ ID NO:54, SEQ ID NO: 171 or SEQ ID NO:173,

(8) An angiogenesis inhibitor, which comprises an antibody against a protein, partial peptide thereof or salt thereof containing the same or substantially the same amino acid sequence shown in SEQ ID NO: 1, SEQ ID NO:44, SEQ ID NO:51, SEQ ID NO: 52, SEQ ID NO:53, SEQ ID NO:54, SEQ ID NO: 171 or SEQ ID NO: 173,

(9) The angiogenesis inhibitor according to any one of (1) to (8), which is an agent for preventing or treating cancer, polycystic ovary syndrome or ovarian hyperstimulation syndrome,

(9a) The angiogenesis inhibitor according to above (9), wherein the cancer is a cancer of endocrine tissue,

(9b) The angiogenesis inhibitor according to any one of (1) to (8), which is an agent for preventing or treating polycystic ovary syndrome.

(10) An agent for diagnosing cancer, polycystic ovary syndrome or ovarian hyperstimulation syndrome, which comprises (a) a polynucleotide encoding a peptide or salt thereof containing the same or substantially the same amino acid sequence shown in SEQ ID NO: 81, SEQ ID NO:82, SEQ ID NO: 109, SEQ ID NO: 138, SEQ ID NO: 19 or SEQ ID NO:39, or (b) a polynucleotide encoding a protein or salt thereof having an amino acid sequence identical to or substantially identical to the amino acid sequence shown in SEQ ID NO: 1, SEQ ID NO: 44, SEQ ID NO: 51, SEQ ID NO: 52, SEQ ID NO: 53, SEQ ID NO: 54, SEQ ID NO: 171 or SEQ ID NO: 173,

(11) A method for screening a compound or salt thereof inhibiting the activity of a protein or salt thereof containing the same or substantially the same amino acid sequence shown in SEQ ID NO: 1, SEQ ID NO: 44, SEQ ID NO: 51, SEQ ID NO: 52, SEQ ID NO: 53, SEQ ID NO: 54, SEQ ID NO: 171 or SEQ ID NO: 173, wherein endothelial cells are used in the screening test,

(12) An angiogenesis inhibitor, which comprises a compound or salt thereof selected by the screening method according to (11),

(13) A method for screening an angiogenesis inhibitor, characterized in that said method uses (a) a peptide or salt thereof containing the same or substantially the same amino acid sequence shown in SEQ ID NO: 21, SEQ ID NO:81, SEQ ID NO:82, SEQ ID NO: 109, SEQ ID NO:138, SEQ ID NO:19 or SEQ ID NO:39, and/or (b) a protein or salt thereof containing the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO: 173,

(13a) A method for screening an angiogenesis inhibitor, characterized in that said method uses (a) a peptide or salt thereof containing the same or substantially the same amino acid sequence shown in SEQ ID NO: 21 and/or (b) a protein or salt thereof containing the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO: 173,

(13b) A method for screening an angiogenesis inhibitor, characterized in that said method uses (a) a peptide or salt thereof containing the same or substantially the same amino acid sequence shown in SEQ ID NO:81, SEQ ID NO:82, SEQ ID NO:109, SEQ ID NO:138, and/or (b) a protein or salt thereof containing the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO: 173,

(13c) A method for screening an angiogenesis inhibitor, characterized in that said method uses (a) a peptide or salt thereof containing the same or substantially the same amino acid sequence shown in SEQ ID NO:19 or SEQ ID NO:39, and/or (b) a protein or salt thereof containing the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO: 173,

(14) A kit for screening an angiogenesis inhibitor, characterized in that said method comprises (a) a peptide or salt thereof containing the same or substantially the same amino acid sequence shown in SEQ ID NO: 21, SEQ ID NO:81, SEQ ID NO:82, SEQ ID NO: 109, SEQ ID NO:138, SEQ ID NO:19 or SEQ ID NO:39, and/or (b) a protein or salt thereof containing the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO: 173,

(15) A method for screening an angiogenesis inhibitor, characterized in that said method uses (a) a polynucleotide encoding a peptide or salt thereof containing the same or substantially the same amino acid sequence shown in SEQ ID NO: 81, SEQ ID NO:82, SEQ ID NO: 109, SEQ ID NO: 138, SEQ ID NO: 19 or SEQ ID NO:39, and/or (b) a polynucleotide encoding a protein or salt thereof containing the same or substantially the same amino acid sequence shown in SEQ ID NO: 1, SEQ ID NO:44, SEQ ID NO:51, SEQ ID NO:52, SEQ ID NO:53, SEQ ID NO: 54, SEQ ID NO:171 or SEQ ID NO:173,

(15a) A method for screening an angiogenesis inhibitor, characterized in that said method uses (a) a polynucleotide encoding a peptide or salt thereof containing the same or substantially the same amino acid sequence shown in SEQ ID NO: 81, SEQ ID NO:82, SEQ ID NO:109, SEQ ID NO:138, and/or (b) a polynucleotide encoding a protein or salt thereof containing the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO: 173,
(15b) A method for screening an angiogenesis inhibitor, characterized in that said method uses (a) a polynucleotide encoding a peptide or salt thereof containing the same or substantially the same amino acid sequence shown in SEQ ID NO:19 or SEQ ID NO:39, and/or (b) a polynucleotide encoding a protein or salt thereof containing the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO:173,
(16) A method for screening an angiogenesis inhibitor, characterized in that said method uses (a) an antibody against a peptide, partial peptide thereof or salt thereof containing the same or substantially the same amino acid sequence shown in SEQ ID NO: 21, SEQ ID NO:81, SEQ ID NO:82, SEQ ID NO:109, SEQ ID NO:138, SEQ ID NO:19 or SEQ ID NO:39, and/or (b) an antibody against a protein, partial peptide thereof or salt thereof containing the same or substantially the same amino acid sequence shown in SEQ ID NO: 1, SEQ ID NO:44, SEQ ID NO: 51, SEQ ID NO:52, SEQ ID NO:53, SEQ ID NO:54, SEQ ID NO:171 or SEQ ID NO:173,
(17) An angiogenesis inhibitor, which is obtained using the screening method according to (13), (15) or (16), or the screening kit according to (14),
(18) An angiogenesis inhibitor, which comprises a compound or salt thereof having an action to inhibit the growth of endothelial cells,
(19) A protein or salt thereof, characterized in that said protein contains the same or substantially the same amino acid sequence shown in SEQ ID NO: 171 or SEQ ID NO:137,
(20) A protein or salt thereof consisting of the amino acid sequence shown in SEQ ID NO: 171,
(21) A protein or salt thereof consisting of the amino acid sequence shown in SEQ ID NO: 137,
(22) A partial peptide or salt thereof of the protein according to (19),
(23) A polynucleotide, which comprises a polynucleotide encoding the protein according to (19),
(24) The polynucleotide according to (23), wherein said polynucleotide is DNA,
(25) A polynucleotide consisting of a nucleotide sequence shown in SEQ ID NO: 172 or SEQ ID NO: 174,
(26) A recombinant vector comprising the polynucleotide according to (24),
(27) A transformant transformed by the recombinant vector according to (26),
(28) A method for producing the protein or salt thereof according to (19), characterized in that said method comprises culturing the transformant according to (27), and producing and accumulating the protein according to (19),
(29) An antibody against the protein, partial peptide thereof or salt thereof according to (19),
(30) A drug comprising the antibody according to (29),
(31) A diagnostic agent comprising the antibody according to (29),
(32) A method for screening a compound or salt thereof promoting or inhibiting the activity of the protein or salt thereof according to (19), characterized in that said method uses the protein, partial peptide thereof or salt thereof according to (19),
(33) A kit for screening a compound or salt thereof promoting or inhibiting the activity of the protein or salt thereof according to (19), characterized in that said method uses the protein, partial peptide thereof or salt thereof according to (19),
(34) A compound or salt thereof promoting or inhibiting the activity of the protein or salt thereof according to (19), which is selected using the screening method according to (32) or the screening kit according to (33),
(35) A drug comprising the compound or salt thereof according to (34),
(36) A method for inhibiting angiogenesis in a mammal, characterized in that said method comprises administration of an effective dose of a compound or salt thereof inhibiting the activity of a protein or salt thereof comprising the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO:173, to said mammal,
(37) Use of a compound or salt thereof inhibiting the activity of a protein or salt thereof comprising the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO:173 to produce an angiogenesis inhibitor,
(38) A method for preventing or treating cancer in a mammal, polycystic ovary syndrome or ovarian hyperstimulation syndrome, characterized in that said method comprises administration of an effective dose of a compound or salt thereof inhibiting the activity of a protein or salt thereof comprising the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO:173, to said mammal,
(39) Use of a compound or salt thereof inhibiting the activity of a protein or salt thereof comprising the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO:173 to produce an agent for preventing or treating cancer, polycystic ovary syndrome or ovarian hyperstimulation syndrome.

BRIEF DESCRIPTION OF THE DRAWINGS

FIG. 1 shows the measurement of ZAQ receptor activation action of human type Bv8 and MIT1 obtained in Reference Example 4. In the figure, —○— shows human type Bv8 peptide and —●— shows MIT1.

FIG. 2 shows the measurement of I5E receptor activation action of human type Bv8 and MIT1 obtained in Reference Example 4. In the figure, —○— shows human type Bv8 peptide and —●— shows MIT1.

FIG. 3 shows the measurement of ZAQ activation action of purified ZAQ ligand peptide obtained in Reference Example 11 (3).

FIG. 4 shows the measurement of ZAQ receptor activation action of human type ZAQ ligand peptide and MIT1 obtained in Reference Example 13 (3). In the figure, —○— shows human type Bv8 peptide and —●— shows MIT1.

FIG. 5 shows the measurement of I5E receptor activation action of human type ZAQ ligand peptide and MIT1 obtained in Reference Example 13 (3). In the figure, —○— shows human type Bv8 peptide and —●— shows MIT1.

FIG. 6 shows the result of the quantity of bovine type ZAQ and bovine type I5E mRNA in bovine type adrenal capillary endothelial cell (BACE) and bovine type vascular endothelial cell (BAE) by TaqMan PCR method obtained in Example 2. In the figure, □ shows copy numbers of bovine type ZAQ mRNA per 1ng of total RNA, ■ also shows copy numbers of bovine type I5E mRNA .

FIG. 7 shows measurement of rise activity of intercellular calcium ion concentration by FLIPR using BACE obtained in Example 3. In the figure, —●— shows human type ZAQ ligand peptide, —▲— shows human type Bv8 peptide and —■— shows MIT1.

FIG. 8 shows the results of dose reactivity of ZAQ related peptide affecting activation of p42 MAP kinase and p44 MAP kinase in BACE obtained in Example 3. In the figure, C shows a control which the ligand (liquid) is not administrated, M shows that MIT1 is added, L1 shows that human type ZAQ ligand peptide is added, L2 shows that human type Bv8 peptide is added, respectively. Further, p44 MAPK-P shows a band of phosphorylated p44 MAP kinase, p42 MAPK-P shows a band of phosphorylated p42 MAP kinase.

FIG.9 shows results of dose reactivity of ZAQ related peptide affecting activation of p38 MAP kinase in BACE obtained in Example 3. In the figure, C shows a control which the ligand (liquid) is not administrated, M shows that MIT1 is added, L1 shows that human type ZAQ ligand peptide is added, L2 shows that human type Bv8 peptide is added, respectively. Further, p38 MAPK-P shows a band of phosphorylated p38 MAP kinase.

FIG. 10 shows results of dose reactivity of peptide affecting uptake of [$^3$H] thymidine in BACE obtained in Example 3. In the figure, —●— shows human type ZAQ ligand peptide, —▲— shows human type Bv8 peptide and —■— shows MIT1.

FIG. 11 shows the affection of human type ZAQ ligand peptide and human typw Bv8 peptide to proliferation of BACE. Vertical axis shows cell numbers of BACE per 1 well. ZAQL-1 of lateral axis shows human type ZAQ ligand and ZAQL-2 of that shows human type Bv8 peptide.* shows that P value obtained from Student's t test is less than 0.05, ** also shows that P value is less than 0.001.

BEST MODE FOR CARRYING OUT THE INVENTION

The peptide characterized by containing the same or substantially the same amino acid sequence as the amino acid sequence represented by SEQ ID NO: 81, SEQ ID NO:82, SEQ ID NO:109, SEQ ID NO:138, SEQ ID NO: 19 or SEQ ID NO: 39, or its salt (hereinafter sometimes merely referred to as "the peptide of the present invention") is a peptide capable of binding to a protein containing the same or substantially the same amino acid sequence as the amino acid sequence represented by SEQ ID NO: 1, SEQ ID NO:44, SEQ ID NO:51, SEQ ID NO:52, SEQ ID NO:53, SEQ ID NO:54, SEQ ID NO:171 or SEQ ID NO:173, or its salt (hereinafter sometimes merely referred to as "the protein of the present invention"), and is a peptide or its salt capable of binding to the protein of the present invention or its salt to activate the same. The capabilities of the peptide of the present invention to bind to the protein of the present invention and to activate the protein of the present invention can be determined by the methods, which will be later described.

The peptide of the present invention may be any peptide derived from any cell of human or non-human mammals (e.g., guinea pig, rat, mouse, rabbit, swine, sheep, bovine, monkey, etc.) (preferably rat, mouse, etc.) (e.g., splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.); hematocyte cell (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG, etc.) or any tissues where such cells are present, such as brain or any of brain regions (e.g., olfactory bulb, amygdaloid nucleus, basal ganglia, hippocampus, thalamus, hypothalamus, subthalamic nucleus, cerebral cortex, medulla oblongata, cerebellum, occipital lobes, frontal lobe, lateral lobe, putamen, caudate nucleus, corpus callosum, substantia nigra), spinal cord, pituitary gland, stomach, pancreas, kidneys, liver, gonads, thyroid gland, gallbladder, bone marrow, adrenal glands, skin, muscle, lung, digestive tracts (e.g., colon, small intestine), vascular vessels, heart, thymus, spleen, submandibular gland, peripheral blood, peripheral blood cells, prostate, testes, testicles, ovaries, placenta, uterus, bones, joints, skeletal muscles, etc. (especially, brain and each region of the brain). The peptide may also be a synthetic peptide.

When the peptide of the present invention has a signal sequence, the peptide can be efficiently secreted extracellularly.

The amino acid sequence which has substantially the same amino acid sequence as that represented by SEQ ID NO:81 or SEQ ID NO:82 includes an amino acid sequence having at least about 60% homology( preferably at least about 70% homology, and more preferably at least about 80% homology, much more preferably at least 85% homology, particularly preferably at least 90% homology, the most preferably at least 95% homology) to the amino acid sequence represented by SEQ ID NO:81 or SEQ ID NO:82.

The peptide of the present invention is a peptide or its salt characterized by containing the same or substantially the same amino acid sequence as the amino acid sequence represented by SEQ ID NO: 81 or SEQ ID NO: 82 and having an activity substantially equivalent to that of the amino acid sequence represented by SEQ ID NO: 81or SEQ ID NO: 82, etc.

Hereinafter, the peptide containing the amino acid sequence represented by SEQ ID NO: 81 or SEQ ID NO: 82 is sometimes referred to as human type ZAQ ligand mature peptide.

Examples of the peptide which has substantially the same amino acid sequence as the amino acid sequence represented by SEQ ID NO: 81 or SEQ ID NO: 82 include a peptide containing substantially the same amino acid sequence as the amino acid sequence represented by SEQ ID NO: 81 or SEQ ID NO: 82 and having an activity substantially equivalent to that of the amino acid sequence represented by SEQ ID NO: 81 or SEQ ID NO: 82, etc. Specifically, the peptide which has substantially the same amino acid sequence as the amino acid sequence represented by SEQ ID NO: 81, SEQ ID NO:82, SEQ ID NO:83, SEQ ID NO:84.

Hereinafter, the peptide containing the amino acid sequence represented by SEQ ID NO: 83 or SEQ ID NO: 84 is sometimes referred to as human type ZAQ ligand precursor peptide.

The amino acid sequence which is substantially the same amino acid sequence as the amino acid sequence represented by SEQ ID NO: 109 includes an amino acid sequence having at least about 95% homology (preferably at least about 97% homology, and more preferably at least about 99% homology) to the amino acid sequence represented by SEQ ID NO: 109.

Examples of the peptide which has substantially the same amino acid sequence as the amino acid sequence represented by SEQ ID NO: 109 include a peptide containing substantially the same amino acid sequence as the amino acid sequence represented by SEQ ID NO: 109 and having an activity substantially equivalent to that of the amino acid sequence represented by SEQ ID NO: 109, etc.

Hereinafter, the peptide having the amino acid sequence represented by SEQ ID NO: 109 is sometimes referred to as mouse type ZAQ ligand maturation peptide.

Preferred examples of the peptides, which contain the same or substantially the same amino acid sequence as the amino acid sequence represented by SEQ ID NO: 109, are peptides containing the same or substantially the same amino acid sequence as the amino acid sequence represented by SEQ ID NO: 109 and having an activity substantially equivalent to that of the amino acid sequence represented by SEQ ID NO: 109. Specifically, the peptides include peptides having the amino acid sequence represented by SEQ ID NO: 109 or SEQ ID NO: 107, and the like.

Hereinafter, the peptide having the amino acid sequence represented by SEQ ID NO: 107 is sometimes referred to as mouse type ZAQ ligand precursor peptide.

The amino acid sequence which is substantially the same amino acid sequence as the amino acid sequence represented by SEQ ID NO: 138 includes an amino acid sequence having at least about 95% homology (preferably at least about 97% homology, and more preferably at least about 99% homology) to the amino acid sequence represented by SEQ ID NO: 138.

Examples of the peptide which has substantially the same amino acid sequence as the amino acid sequence represented by SEQ ID NO: 138 include a peptide containing substantially the same amino acid sequence as the amino acid sequence represented by SEQ ID NO: 138 and having an activity substantially equivalent to that of the amino acid sequence represented by SEQ ID NO: 138, etc. Specifically, the peptide which has substantially the same amino acid sequence as the amino acid sequence represented by SEQ ID NO: 138 include a peptide containing as the amino acid sequence represented by SEQ ID NO: 140, SEQ ID NO: 142, and the like.

Hereinafter, the peptide having the amino acid sequence represented by SEQ ID NO: 138 is sometimes referred to as rat type ZAQ ligand maturation peptide.

Specifically, the peptide which has substantially the same amino acid sequence as the amino acid sequence represented by SEQ ID NO: 138 include a peptide having as the amino acid sequence represented by SEQ ID NO: 132, SEQ ID NO: 134 or SEQ ID NO: 136, and the like.

Hereinafter, the peptide having the amino acid sequence represented by SEQ ID NO: 107 is sometimes referred to as rat type ZAQ ligand precursor peptide.

The amino acid sequence which has substantially the same amino acid sequence as that represented by SEQ ID NO:19 or SEQ ID NO:39 includes an amino acid sequence having at least about 60% homology( preferably at least about 70% homology, and more preferably at least about 80% homology, much more preferably at least 85% homology, particularly preferably at least 90% homology, the most preferably at least 95% homology) to the amino acid sequence represented by SEQ ID NO: 19 or SEQ ID NO:39.

Examples of the peptide which has substantially the same amino acid sequence as the amino acid sequence represented by SEQ ID NO: 19 or SEQ ID NO: 39 include a peptide containing substantially the same amino acid sequence as the amino acid sequence represented by SEQ ID NO: 19 or SEQ ID NO: 39 and having an activity substantially equivalent to that of the amino acid sequence represented by SEQ ID NO: 19 or SEQ ID NO: 39, and the like.

Hereinafter, the peptide having the amino acid sequence represented by SEQ ID NO: 19 is sometimes referred to as human type Bv8 maturation peptide, the peptide having the amino acid sequence represented by SEQ ID NO: 39 is sometimes referred to as rat type Bv8 maturation peptide or mouse type Bv8 maturation peptide.

Preferred examples of the peptides, which contain the same or substantially the same amino acid sequence as the amino acid sequence represented by SEQ ID NO: 19, are peptides containing the same or substantially the same amino acid sequence as the amino acid sequence represented by SEQ ID NO: 19 and having an activity substantially equivalent to that of the amino acid sequence represented by SEQ ID NO: 19. Specifically, the peptides include peptides having the amino acid sequence represented by SEQ ID NO: 19 or SEQ ID NO: 17, and the like.

Preferred examples of the peptides, which contain the same or substantially the same amino acid sequence as the amino acid sequence represented by SEQ ID NO: 39, are peptides containing the same or substantially the same amino acid sequence as the amino acid sequence represented by SEQ ID NO: 39 and having an activity substantially equivalent to that of the amino acid sequence represented by SEQ ID NO: 39. Specifically, the peptides include peptides having the amino acid sequence represented by SEQ ID NO: 37, SEQ ID NO: 39 or SEQ ID NO: 55, and the like.

Hereinafter, the peptide having the amino acid sequence represented by SEQ ID NO: 17 is sometimes referred to as human type Bv8 precursor peptide. The peptide having the amino acid sequence represented by SEQ ID NO: 37 is sometimes referred to as rat type Bv8 precursor peptide. The peptide having the amino acid sequence represented by SEQ ID NO: 55 is sometimes referred to as mouse type Bv8 precursor peptide.

The amino acid sequence which has substantially the same amino acid sequence as that represented by SEQ ID NO: 21 includes an amino acid sequence having at least about 60% homology( preferably at least about 70% homology, and more preferably at least about 80% homology, much more preferably at least 85% homology, particularly preferably at least 90% homology, the most preferably at least 95% homology) to the amino acid sequence represented by SEQ ID NO:21.

Examples of the peptide which has substantially the same amino acid sequence as the amino acid sequence represented by SEQ ID NO: 21 include a peptide containing substantially the same amino acid sequence as the amino acid sequence represented by SEQ ID NO: 21 and having an activity substantially equivalent to that of the amino acid sequence represented by SEQ ID NO: 21, and the like.

Hereinafter, the peptide having the amino acid sequence represented by SEQ ID NO: 21 is sometimes referred to as MIT1.

The substantially equivalent activity refers to, for example, a binding activity to the protein of the present invention, a signal transduction activity mediated by the protein of the present invention, etc. The term substantially equivalent is used to mean that these activities are equivalent in nature. It is thus preferred that these activities such as a binding activity to the protein of the present invention, a signal transduction activity mediated by the protein of the present invention, etc. are equivalent in potency (e.g., about 0.5 to about 2 times), and it is allowable that even differences among grades such as the strength of these activities and molecular weight of the peptide are present.

These activities can be assayed by publicly known methods with a modification, and may also be assayed by, for example, the screening methods which will be later described.

As the peptide of the present invention, there are also employed peptides containing (i) an amino acid sequence wherein at least 1 or 2 (preferably approximately 1 to 30, more preferably approximately 1 to 20) amino acids are deleted in the amino acid sequence represented by SEQ ID NO: 81 SEQ ID NO: 82, SEQ ID NO: 109, SEQ ID NO:138, SEQ ID NO:19 or SEQ ID NO: 39, (ii) an amino acid sequence wherein at least 1 or 2 (preferably approximately 1 to 40, more preferably approximately 1 to 30, particularly preferably approximately 1 to 20) amino acids are added to the amino acid sequence represented by SEQ ID NO: 81 SEQ ID NO: 82, SEQ ID NO: 109, SEQ ID NO: 138, SEQ ID NO: 19 or SEQ ID NO: 39, (iii) an amino acid sequence wherein at least 1 or 2 (preferably approximately 1 to 40, more preferably approximately 1 to 30, particularly preferably approximately 1 to 20) amino acids in the amino acid sequence represented by SEQ ID NO: 81 SEQ ID NO: 82, SEQ ID NO: 109, SEQ ID NO: 138, SEQ ID NO:19 or SEQ ID NO: 39 are substituted with other amino acids, or (iv) an amino acid sequence in combination thereof; or the like.

Specific examples of the peptide of the present invention include human-derived peptides containing the amino acid sequence represented by SEQ ID NO: 81, human-derived peptides containing the amino acid sequence represented by SEQ ID NO: 82, mouse-derived peptides containing the amino acid sequence represented by SEQ ID NO: 109, rat-derived peptides containing the amino acid sequence represented by SEQ ID NO: 138, rat-derived peptides containing the amino acid sequence represented by SEQ ID NO: 140, rat-derived peptides containing the amino acid sequence represented by SEQ ID NO: 142, human-derived peptides containing the amino acid sequence represented by SEQ ID NO: 19, rat-derived or mouse-derived peptides containing the amino acid sequence represented by SEQ ID NO: 39, and the like.

The partial peptides of the peptide of the present invention may be any partial peptides so long as they can be employed for the screening methods for pharmaceuticals, etc., which will be later described, and may have an activity substantially equivalent to the activity of the peptide of the present invention. In the partial peptides, the number of amino acids is at least 10 and preferably at least 20, in the amino acid sequence which constitutes the peptide of the present invention.

Herein, the term "substantially equivalent activity" is intended to mean the same significance as defined above. The "substantially equivalent activity" can be assayed in the same way as described above.

The partial peptides may be those (i) wherein at least 1 or 2 (preferably several (1 to 4)) amino acids are deleted in the amino acid sequences described above, (ii) wherein at least 1 or 2 (preferably approximately 1 to 20, more preferably approximately 1 to 10 and most preferably several (1 to 5)) amino acids are added to the amino acid sequences described above, or (iii) wherein at least 1 or 2 (preferably several (1 to 4)) amino acids in the amino acid sequences described above are substituted with other amino acids.

Hereinafter the peptide of the present invention and the partial peptides of the peptide of the present invention are sometimes collectively referred to as the peptide of the present invention.

The protein of the present invention may be any protein derived from any cells of human or mammals (e.g., guinea pig, rat, mouse, rabbit, swine, sheep, bovine, monkey, etc.) (e.g., splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.); or hemocyte type cells (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.); or any tissues where such cells are present, such as brain or any of brain regions (e.g., olfactory bulb, amygdaloid nucleus, basal ganglia, hippocampus, thalamus, hypothalamus, subthalamic nucleus, cerebral cortex, medulla oblongata, cerebellum, occipital lobes, frontal lobe, lateral lobe, putamen, caudate nucleus, corpus callosum, substantia nigra), spinal cord, pituitary gland, stomach, pancreas, kidneys, liver, gonads, thyroid gland, gallbladder, bone marrow, adrenal glands, skin, muscle, lung, digestive tracts (e.g., colon, small intestine), vascular vessels, heart, thymus, spleen, submandibular gland, peripheral blood, peripheral blood cells, prostate, testes, orchis, ovaries, placenta, uterus, bones, joints, skeletal muscles, etc. (especially, brain and each region of the brain); the proteins may also be synthetic proteins.

When the protein of the present invention has a signal sequence, the peptide or protein can be efficiently secreted extracellularly.

The amino acid sequence which is substantially the same amino acid sequence as the amino acid sequence represented by SEQ ID NO: 1 includes, e.g., an amino acid sequence having at least about 90% homology, preferably at least about 95% homology and more preferably at least about 98% homology to the amino acid sequence represented by SEQ ID NO: 1, and the like.

As the protein having substantially the same amino acid sequence as the amino acid sequence represented by SEQ ID NO: 1, preferred are, for example, proteins containing substantially the same amino acid sequence as the amino acid sequence represented by SEQ ID NO: 1 and having an activity substantially equivalent to that of the protein containing substantially the same amino acid sequence as the amino acid sequence represented by SEQ ID NO: 1, and the like.

As the protein having the same or substantially the same amino acid sequence as the amino acid sequence represented by SEQ ID NO: 1, preferred are, for example, proteins containing the same or substantially the same amino acid sequence as the amino acid sequence represented by SEQ ID NO: 1 and having an activity substantially equivalent to that of the amino acid sequence represented by SEQ ID NO: 1, and the like. Specifically, there are proteins described in WO 01/16309, and the like.

Hereinafter, the protein having the amino acid sequence represented by SEQ ID NO: 1 is sometimes referred to as ZAQ or human type ZAQ.

The amino acid sequence which is substantially the same amino acid sequence as the amino acid sequence represented by SEQ ID NO: 44 includes, e.g., an amino acid sequence having at least about 90% homology, preferably at least about 95% homology and more preferably at least about 98% homology to the amino acid sequence represented by SEQ ID NO: 44, and the like.

As the protein having substantially the same amino acid sequence as the amino acid sequence represented by SEQ ID NO: 44, preferred are, for example, proteins containing substantially the same amino acid sequence as the amino acid sequence represented by SEQ ID NO: 44 and having an activity substantially equivalent to that of the protein containing substantially the same amino acid sequence as the amino acid sequence represented by SEQ ID NO: 44, and the like.

As the protein having the same or substantially the same amino acid sequence as the amino acid sequence represented by SEQ ID NO: 44, preferred are, for example, proteins containing the same or substantially the same amino acid sequence as the amino acid sequence represented by SEQ ID NO: 44 and having an activity substantially equivalent to that of the amino acid sequence represented by SEQ ID NO: 44, and the like.

The amino acid sequence which is substantially the same amino acid sequence as the amino acid sequence represented by SEQ ID NO: 51 includes, e.g., an amino acid sequence having at least about 90% homology, preferably at least about 95% homology and more preferably at least about 98% homology to the amino acid sequence represented by SEQ ID NO: 51, and the like.

As the protein having substantially the same amino acid sequence as the amino acid sequence represented by SEQ ID NO: 51, preferred are, for example, proteins containing substantially the same amino acid sequence as the amino acid sequence represented by SEQ ID NO: 51 and having an activity substantially equivalent to that of the protein containing substantially the same amino acid sequence as the amino acid sequence represented by SEQ ID NO: 51, and the like.

As the protein having the same or substantially the same amino acid sequence as the amino acid sequence represented by SEQ ID NO: 51, preferred are, for example, proteins containing the same or substantially the same amino acid sequence as the amino acid sequence represented by SEQ ID NO: 51 and having an activity substantially equivalent to that of the amino acid sequence represented by SEQ ID NO: 51, and the like.

The amino acid sequence which is substantially the same amino acid sequence as the amino acid sequence represented by SEQ ID NO: 52 includes, e.g., an amino acid sequence having at least about 90% homology, preferably at least about 95% homology and more preferably at least about 98% homology, to the amino acid sequence represented by SEQ ID NO: 52, and the like.

As the protein having substantially the same amino acid sequence as the amino acid sequence represented by SEQ ID NO: 52, preferred are, for example, proteins containing substantially the same amino acid sequence as the amino acid sequence represented by SEQ ID NO: 52 and having an activity substantially equivalent to that of the protein containing substantially the same amino acid sequence as the amino acid sequence represented by SEQ ID NO: 52, and the like.

As the protein having the same or substantially the same amino acid sequence as the amino acid sequence represented by SEQ ID NO: 52, preferred are, for example, proteins containing the same or substantially the same amino acid sequence as the amino acid sequence represented by SEQ ID NO: 52 and having an activity substantially equivalent to that of the amino acid sequence represented by SEQ ID NO: 52, and the like. Specifically, there are proteins described in WO 98/46620, and the like.

Hereinafter, the protein having the amino acid sequence represented by SEQ ID NO: 52 is sometimes referred to as I5E or human type I5E.

The amino acid sequence which is substantially the same amino acid sequence as the amino acid sequence represented by SEQ ID NO: 53 includes, e.g., an amino acid sequence having at least about 95% homology, preferably at least about 96% homology, more preferably at least about 97% and most preferably at least about 98% homology, to the amino acid sequence represented by SEQ ID NO: 53, and the like.

As the protein having substantially the same amino acid sequence as the amino acid sequence represented by SEQ ID NO: 53, preferred are, for example, proteins containing substantially the same amino acid sequence as the amino acid sequence represented by SEQ ID NO: 53 and having an activity substantially equivalent to that of the protein containing substantially the same amino acid sequence as the amino acid sequence represented by SEQ ID NO: 53, and the like.

As the protein having the same or substantially the same amino acid sequence as the amino acid sequence represented by SEQ ID NO: 53, preferred are, for example, proteins containing the same or substantially the same amino acid sequence as the amino acid sequence represented by SEQ ID NO: 53 and having an activity substantially equivalent to that of the amino acid sequence represented by SEQ ID NO: 53, and the like. Specifically, there are proteins described in Biochem. Biophys. Acta, 1491, 369-375, 2000, and the like.

The amino acid sequence which is substantially the same amino acid sequence as the amino acid sequence represented by SEQ ID NO: 54 includes, e.g., an amino acid sequence having at least about 95% homology, preferably at least about 96% homology, more preferably at least about 97% and most preferably at least about 98% homology, to the amino acid sequence represented by SEQ ID NO: 54, and the like.

As the protein having substantially the same amino acid sequence as the amino acid sequence represented by SEQ ID NO: 54, preferred are, for example, proteins containing substantially the same amino acid sequence as the amino acid sequence represented by SEQ ID NO: 54 and having an activity substantially equivalent to that of the protein containing substantially the same amino acid sequence as the amino acid sequence represented by SEQ ID NO: 54, and the like.

As the protein having the same or substantially the same amino acid sequence as the amino acid sequence represented by SEQ ID NO: 54, preferred are, for example, proteins containing the same or substantially the same amino acid sequence as the amino acid sequence represented by SEQ ID NO: 54 and having an activity substantially equivalent to that of the amino acid sequence represented by SEQ ID NO: 54, and the like. Specifically, there are proteins described in WO 98/46620, and the like.

The amino acid sequence which is substantially the same amino acid sequence as the amino acid sequence represented by SEQ ID NO: 171 includes, e.g., an amino acid sequence having at least about 90% homology, preferably at least about 95% homology and more preferably at least about 98% homology to the amino acid sequence represented by SEQ ID NO: 171, and the like.

As the protein having substantially the same amino acid sequence as the amino acid sequence represented by SEQ ID NO: 171, preferred are, for example, proteins containing substantially the same amino acid sequence as the amino acid sequence represented by SEQ ID NO: 171 and having an activity substantially equivalent to that of the protein containing substantially the same amino acid sequence as the amino acid sequence represented by SEQ ID NO: 171, and the like.

As the protein having the same or substantially the same amino acid sequence as the amino acid sequence represented by SEQ ID NO: 171, preferred are, for example, proteins containing the same or substantially the same amino acid sequence as the amino acid sequence represented by SEQ ID NO: 171 and having an activity substantially equivalent to that of the amino acid sequence represented by SEQ ID NO: 171, and the like.

Hereinafter, the protein having the amino acid sequence represented by SEQ ID NO: 171 is sometimes referred to as ZAQ or bovine type ZAQ.

The amino acid sequence which is substantially the same amino acid sequence as the amino acid sequence represented by SEQ ID NO: 173 includes, e.g., an amino acid sequence having at least about 90% homology, preferably at least about 95% homology and more preferably at least about 98% homology, to the amino acid sequence represented by SEQ ID NO: 173, and the like.

As the protein having substantially the same amino acid sequence as the amino acid sequence represented by SEQ ID NO: 173, preferred are, for example, proteins containing substantially the same amino acid sequence as the amino acid sequence represented by SEQ ID NO: 173 and having an activity substantially equivalent to that of the protein containing substantially the same amino acid sequence as the amino acid sequence represented by SEQ ID NO: 173, and the like.

As the protein having the same or substantially the same amino acid sequence as the amino acid sequence represented by SEQ ID NO: 173, preferred are, for example, proteins containing the same or substantially the same amino acid sequence as the amino acid sequence represented by SEQ ID NO: 173 and having an activity substantially equivalent to that of the amino acid sequence represented by SEQ ID NO: 173, and the like.

Hereinafter, the protein having the amino acid sequence represented by SEQ ID NO: 173 is sometimes referred to as I5E or bovine type I5E.

The substantially equivalent activity refers to, for example, a binding activity to the peptide of the present invention, a signal transduction activity, etc.

The term substantially equivalent is used to mean that these activities are equivalent in nature. It is thus preferred that these activities such as a binding activity to the peptide of the present invention, a signal transduction activity, etc. are equivalent in potency (e.g., about 0.5 to about 2 times), and it is allowable that even differences among grades such as the strength of these activities and molecular weight of the protein are present.

These activities such as a binding activity to the peptide of the present invention, a signal transduction activity, etc. can be assayed by publicly known methods with a modification.

As the protein of the present invention, there are also employed proteins containing (i) an amino acid sequence wherein at least 1 or 2 (preferably approximately 1 to 30, more preferably approximately 1 to 10 and most preferably several (1 or 2)) amino acids are deleted in the amino acid sequence represented by SEQ ID NO: 1, SEQ ID NO: 44, SEQ ID NO: 51, SEQ ID NO: 52, SEQ ID NO: 53, SEQ ID NO: 54, SEQ ID NO: 171 or SEQ ID NO: 173, (ii) an amino acid sequence wherein at least 1 or 2 (preferably approximately 1 to 30, more preferably approximately 1 to 10 and most preferably approximately several (1 or 2)) amino acids are added to the amino acid sequence represented by SEQ ID NO: 1, SEQ ID NO: 44, SEQ ID NO: 51, SEQ ID NO: 52, SEQ ID NO: 53, SEQ ID NO: 54, SEQ ID NO: 171 or SEQ ID NO: 173, (iii) an amino acid sequence wherein at least 1 or 2 (preferably approximately 1 to 30, more preferably approximately 1 to 10 and most preferably approximately several (1 or 2)) amino acids in the amino acid sequence represented by SEQ ID NO: 1, SEQ ID NO: 44, SEQ ID NO: 51, SEQ ID NO: 52, SEQ ID NO: 53, SEQ ID NO: 54, SEQ ID NO: 171 or SEQ ID NO: 173 are substituted with other amino acids, or (iv) an amino acid sequence in combination thereof; or the like.

Specific examples of the protein of the present invention include human-derived (more preferably human brain-derived) proteins containing the amino acid sequence represented by SEQ ID NO: 1 or SEQ ID NO: 52, rat-derived proteins containing the amino acid sequence represented by SEQ ID NO: 44 or SEQ ID NO: 51, mouse-derived proteins containing the amino acid sequence represented by SEQ ID NO: 53 or SEQ ID NO: 54, bovine-derived proteins containing the amino acid sequence represented by SEQ ID NO: 171 or SEQ ID NO: 173, and the like.

The partial peptides of the protein of the present invention (hereinafter sometimes merely referred to as "the partial peptide of the present invention") may be any partial peptides of the protein of the present invention; in the protein molecules of the present invention, there may be employed, e.g., the site exposed outside cell membranes and having a substantially equivalent ligand binding activity, and the like are employed.

Specific examples of the partial peptides of the protein are peptides containing the part which has been analyzed to be an extracellular domain (hydrophilic domain) in the hydrophobic plotting analysis. A peptide containing a hydrophobic domain part can be used as well. In addition, a peptide containing each domain separately as well as a peptide containing plural domains together may also be employed.

In the partial peptides of the present invention, the number of amino acids is at least 20, preferably at least 50 and more preferably at least 100, in the amino acid sequence, which constitutes the protein of the present invention.

The term substantially the same amino acid sequence is used to mean an amino acid sequence having at least about 50% homology, preferably at least about 70% homology, more preferably at least about 80% homology, much more preferably at least about 90% homology, and most preferably at least about 95% homology, to these amino acid sequences.

Herein, the term "substantially equivalent ligand binding activity" is intended to mean the same significance as defined above. The "substantially equivalent ligand binding activity" can be assayed by publicly known methods with a modification.

The partial peptides of the present invention may be those wherein at least 1 or 2 (preferably approximately 1 to 10, more preferably several (1 or 2)) amino acids are deleted in the amino acid sequence represented by SEQ ID NO: 1, SEQ ID NO: 44, SEQ ID NO: 51, SEQ ID NO: 52, SEQ ID NO: 53, SEQ ID NO: 54, SEQ ID NO: 171 or SEQ ID NO: 173; those wherein at least 1 or 2 (preferably approximately 1 to 20, more preferably approximately 1 to 10 and most preferably several (1 or 2)) amino acids are added to the amino acid sequence; or those wherein at least 1 or 2 (preferably approximately 1 to 10, more preferably approximately 1 to 5 and most preferably several (1 or 2)) amino acids are substituted with other amino acids.

As the salts of the peptide of the present invention, the protein of the present invention or its partial peptides, particularly preferred are physiologically acceptable acid addition salts. Examples of such salts employed are salts with, for example, inorganic acids (e.g., hydrochloric acid, phosphoric acid, hydrobromic acid or sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid or benzenesulfonic acid), and the like.

Throughout the present specification, the peptides and proteins are represented in accordance with the conventional way of describing peptides, that is, the N-terminus (amino terminus) at the left hand and the C-terminus (carboxyl terminus) at the right hand. In the proteins of the present invention including the protein containing the amino acid sequence represented by SEQ ID NO: 47, the C-terminus may be in the form of a carboxyl group (-COOH), a carboxylate (-COO-), an amide (-CONH2) or an ester (-COOR).

Herein, examples of the ester group represented by R include a C1-6 alkyl group such as methyl, ethyl, n-propyl, isopropyl, n-butyl, etc.; a C3-8 cycloalkyl group such as cyclopentyl, cyclohexyl, etc.; a C6-12 aryl group such as phenyl, α-naphthyl, etc.; a C7-14 aralkyl such as a phenyl-C1-2 alkyl group, e.g., benzyl, phenethyl, etc.; an α-naphthyl-C1-2 alkyl group such as α-naphthylmethyl, etc.; and the like. In addition, pivaloyloxymethyl or the like, which is used widely as an ester for oral administration, may also be used.

Where the peptide of the present invention and the protein of the present invention (hereinafter sometimes simply referred to as "the peptide/protein of the present invention") contain a carboxyl group (or a carboxylate) at a position other than the C-terminus, it may be amidated or esterified and such an amide or ester is also included within the peptide/protein of the present invention. The ester group in this case may be the same ester group as that described with respect to the above C-terminal group; etc.

Furthermore, examples of the peptide/protein of the present invention include variants of the peptide/protein of the present invention described above, wherein the amino group at the N-terminal methionine residue is protected with a protecting group (e.g., a C1-b acyl group such as a C1-6 alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C1-6 acyl group such as a C2-6 alkanoyl group, e.g., formyl group, acetyl group, etc.), conjugated peptides/proteins such as glycopeptide/ glycoproteins having sugar chains; and the like.

In the partial peptide of the present invention, the C-terminal may be a carboxyl group (-COOH), a carboxylate (-COO-), an amide (-CONH2) or an ester (-COOR). Herein, R in the ester has the same significance as defined above. When the partial peptide of the present invention contains a carboxyl group (or a carboxylate) at the site other than the C-terminus, the carboxyl group may be amidated or esterified and those amides or esters are also included in the partial peptide of the present invention. As the esters in this case, for example, the C-terminal esters described above may be employed.

As in the protein of the present invention described above, the partial peptide of the present invention further includes those in which the amino group of the N-terminal methionine residue is protected by a protecting group, those in which the N-terminal residue is cleaved in vivo and the produced Gln is pyroglutamated, those in which substituents on the side chains of amino acids in the molecule are protected by appropriate protecting groups, conjugated peptides such as those, to which sugar chains are coupled, i.e., so-called glycopeptides, and the like.

The peptide of the present invention and the protein of the present invention may be manufactured by publicly known methods used to purify peptides/proteins from the human or mammal cells or tissues described above, or may also be manufactured by culturing transformants containing DNAs encoding, e.g., the peptide of the present invention later described or the protein of the present invention containing the amino acid sequence represented by SEQ ID NO: 81, SEQ ID NO: 82, SEQ ID NO: 109, SEQ ID NO: 138, SEQ ID NO: 140, SEQ ID NO: 142, SEQ ID NO: 19, SEQ ID NO: 39, SEQ ID NO: 1, SEQ ID NO: 44, SEQ ID NO: 51, SEQ ID NO: 52, SEQ ID NO: 53, SEQ ID NO: 54, SEQ ID NO: 171 or SEQ ID NO: 173. Alternatively, the peptide/protein of the present invention may also be manufactured by the peptide/protein synthesis methods later described. Furthermore, the protein or its salt having the amino acid sequence represented by SEQ ID NO: 53 may be manufactured by the method described in Biochem. Biophys. Acta, 1491, 369-375, 2000. The protein or its salt having the amino acid sequence represented by SEQ ID NO: 52 or SEQ ID NO: 54 may be manufactured by the method described in WO 98/46620.

Where the peptide/protein of the present invention is/are manufactured from human or mammal tissues or cells, the human or mammal tissues or cells are homogenized, then the peptide/protein are extracted with an acid, etc., isolated and purified from the extract obtained by a combination of chromatography techniques such as reversed phase chromatography, ion exchange chromatography, and the like.

To synthesize the peptide of the present invention or the protein of the present invention or its partial peptides, or amides or salts thereof, commercially available resins that are normally used for the peptide/protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids in which $\alpha$-amino groups and functional groups on the side chains are appropriately protected are condensed on the resin in the order of the sequence of the objective peptide/protein according to various condensation methods publicly known. At the end of the reaction, the peptide/protein is excised from the resin and at the same time, the protecting groups are removed. Then, intramolecular disulfide bond-forming reaction is performed in a highly diluted solution to obtain the objective peptide/protein or amides thereof.

For condensation of the protected amino acids described above, a variety of activation reagents available for the peptide/protein synthesis may be used, and carbodiimides are particularly preferably employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents used to activate the protected amino acids or condense with the resin may be chosen from solvents that are known to be usable for peptide/protein condensation reactions. For example, there may be employed acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxan, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower alkanoyl group, such as acetyl group, an aroyl group such as benzoyl group, and a group derived from carbonic acid such as benzyloxycarbonyl group and ethoxycarbonyl group. Examples of a group appropriately used for the etherification include benzyl group, tetrahydropyranyl group, t-butyl group, etc.

Examples of groups for protecting the phenolic hydroxyl group of tyrosine include Bzl, Cl2-Bzl, 2-nitrobenzyl, Br-Z, tertiary butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting amino acids include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the activated amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia, etc.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the peptide/protein, for example, the α-carboxyl group of the carboxy terminal amino acid is first protected by amidation; the peptide chain is then extended to amino group for a desired length. Thereafter, a peptide/protein in which only the protecting group of the N-terminal α-amino group has been eliminated from the peptide chain and a peptide/protein in which only the protecting group of the C-terminal carboxyl group has been eliminated are manufactured. The two peptides/proteins are condensed in a mixture of the solvents described above. The details of the condensation reaction are the same as described above. After the protected peptide/protein obtained by the condensation is purified, all the protecting groups are eliminated by the method described above to give the desired crude peptide/crude protein. This crude peptide/crude protein are purified by various known purification means. Lyophilization of the major fraction gives the amide of the desired peptide/protein.

To prepare the esterified peptide/protein, for example, the α-carboxyl group of the carboxy terminal amino acid is condensed with a desired alcohol to prepare the amino acid ester, which is followed by procedure similar to the preparation of the amidated peptide/protein above to give the desired esterified peptide/protein.

The peptide of the present invention and the protein of the present invention can be manufactured by publicly known methods for peptide synthesis. Also, the particle peptides of the protein of the present invention or salts thereof can be manufactured by publicly known methods for peptide synthesis or by cleaving the protein of the present invention with an appropriate peptidase.

For the methods of peptide synthesis, for example, either solid phase synthesis or liquid phase synthesis may be used. That is, the partial peptide or amino acids that can constitute the peptide of the present invention or the protein of the present invention can be condensed with the remaining part of the partial peptide of the present invention. Where the product contains protecting groups, these protecting groups are removed to give the desired peptide. Publicly known methods for condensation and elimination of the protecting groups are described in i) to v) below.

i) M. Bodanszky & M.A. Ondetti: Peptide Synthesis, Interscience Publishers, New York (1966)
ii) Schroeder & Luebke: The Peptide, Academic Press, New York (1965)
iii) Nobuo Izumiya, et al.: Peptide Gosei-no-Kiso to Jikken (Basics and experiments of peptide synthesis), published by Maruzen Co. (1975)
iv) Haruaki Yajima & Shunpei Sakakibara: Seikagaku Jikken Koza (Biochemical Experiment) 1, Tanpakushitsu no Kagaku (Chemistry of Proteins) IV, 205 (1977)
v) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the peptide of the present invention, the protein of the present invention or the partial peptide thereof can be purified and isolated by a combination of conventional purification methods such as solvent extraction, distillation, column chromatography, liquid chromatography and recrystallization. When the peptide, protein or partial peptide obtained by the above methods is in a free form, the peptide, protein or partial peptide can be converted into an appropriate salt by a publicly known method; when the peptide, protein or partial peptide is obtained in a salt form, it can be converted into a free form by a publicly known method.

The polynucleotide encoding the peptide of the present invention or the protein of the present invention may be any polynucleotide, so long as it contains the base sequence encoding the peptide of the present invention or the protein of the present invention described above, and preferably the polynucleotide is a DNA. Such a DNA may be any one of genomic DNA, genomic DNA library, cDNA derived from the cells or tissues described above, cDNA library derived from the cells or tissues described above and synthetic DNA. The vector to be used for the library may be any of bacteriophage, plasmid, cosmid, phagemid, and the like. In addition, the DNA can be amplified by reverse transcriptase polymerase chain reaction (hereinafter abbreviated as RT-PCR) with total RNA or mRNA fraction prepared from the cells or tissues described above.

Specifically, the DNA encoding the peptide containing the amino acid sequence represented by SEQ ID NO: 81 may be any one of, for example, a DNA containing the base sequence represented by SEQ ID NO: 87, or a DNA having a base sequence hybridizable to the base sequence represented by SEQ ID NO: 87 under high stringent conditions and encoding a peptide which has an activity substantially equivalent to the activity of the peptide of the present invention (e.g., a binding activity to the protein of the present invention, a signal transduction activity mediated by the protein of the present invention, etc.).

The DNA containing the base sequence represented by SEQ ID NO: 87 includes a DNA containing the base sequence represented by SEQ ID NO: 87 or SEQ ID NO: 89, and the like.

Examples of the DNA that is hybridizable to the base sequence represented by SEQ ID NO: 87 under high stringent conditions include a DNA containing the base sequence having at least about 95% homology, preferably at least about 97% homology and more preferably at least about 99% homology, to the base sequence represented by SEQ ID NO: 87.

Specifically, the DNA encoding the peptide containing the amino acid sequence represented by SEQ ID NO: 82 may be any DNA, so long as it is a DNA containing the base sequence represented by SEQ ID NO: 88 or a DNA having a DNA hybridizable to a DNA having the base sequence represented by SEQ ID NO: 88 under high stringent conditions and encoding a peptide which has an activity substantially equivalent to the activity of the peptide containing the amino acid sequence represented by SEQ ID NO: 82 (a binding activity to the protein of the present invention, a signal transduction activity mediated by the protein of the present invention, etc.).

Examples of the DNA containing the base sequence represented by SEQ ID NO: 88 include DNA containing the base sequence represented by SEQ ID NO: 90, and the like.

As the DNA hybridizable to a DNA having the base sequence represented by SEQ ID NO: 88 under high stringent conditions, there may be employed a DNA containing the base sequence having at least about 95% homology, preferably at least about 97% homology and more preferably at least about 99% homology, to the base sequence represented by SEQ ID NO: 88, and the like.

The DNA encoding the protein containing the amino acid sequence represented by SEQ ID NO: 109 may be any DNA, so long as it is, e.g., a DNA containing the base sequence represented by SEQ ID NO: 110, or a DNA having a DNA hybridizable to a DNA having the base sequence represented by SEQ ID NO: 110 under high stringent conditions and encoding a protein which has an activity substantially equivalent to the activity of the protein containing the amino acid sequence represented by SEQ ID NO: 109 (a binding activity to the peptide of the present invention, a signal transduction activity, etc.).

As the DNA hybridizable to a DNA containing the base sequence represented by SEQ ID NO: 110 under high stringent conditions, there may be employed a DNA containing the base sequence having at least about 90% homology, preferably at least about 95% homology and more preferably at least about 98% homology, to the base sequence represented by SEQ ID NO: 110, and the like.

The DNA encoding the protein containing the amino acid sequence represented by SEQ ID NO: 138, SEQ ID NO: 140 or SEQ ID NO:142 may be any DNA, so long as it is, e.g., a DNA containing the base sequence represented by SEQ ID NO: 139, SEQ ID NO: 141, SEQ ID NO: 143, or a DNA having a DNA hybridizable to a DNA containing the base sequence represented by SEQ ID NO: 139, SEQ ID NO: 141, SEQ ID NO: 143 under high stringent conditions and encoding a protein which has an activity substantially equivalent to the activity of the protein containing the amino acid sequence represented by SEQ ID NO: 138, SEQ ID NO: 140 or SEQ ID NO: 142 (a binding activity to the peptide of the present invention, a signal transduction activity, etc.).

As the DNA hybridizable to a DNA containing the base sequence represented by SEQ ID NO: 139, SEQ ID NO: 141, SEQ ID NO: 143 under high stringent conditions, there may be employed a DNA containing the base sequence having at least about 97% homology, preferably at least about 98% homology and more preferably at least about 99% homology, to the base sequence represented by SEQ ID NO: 139, SEQ ID NO: 141, SEQ ID NO: 143, and the like.

The DNA encoding the protein containing the amino acid sequence represented by SEQ ID NO: 19 may be any DNA, so long as it is, e.g., a DNA containing the base sequence represented by SEQ ID NO: 20, or a DNA having a DNA hybridizable to a DNA containing the base sequence represented by SEQ ID NO: 20 under high stringent conditions and encoding a protein which has an activity substantially equivalent to the activity of the protein containing the amino acid sequence represented by SEQ ID NO: 19 (a binding activity to the peptide of the present invention, a signal transduction activity, etc.).

Examples of the DNA containing the base sequence represented by SEQ ID NO: 20 include DNA containing the base sequence represented by SEQ ID NO: 20 or SEQ ID NO: 18, and the like.

As the DNA hybridizable to a DNA containing the base sequence represented by SEQ ID NO: 20 under high stringent conditions, there may be employed a DNA containing the base sequence having at least about 95% homology, preferably at least about 97% homology and more preferably at least about 98% homology, to the base sequence represented by SEQ ID NO: 20, and the like.

The DNA encoding the protein containing the amino acid sequence represented by SEQ ID NO: 39 may be any DNA, so long as it is, e.g., a DNA containing the base sequence represented by SEQ ID NO: 40 or SEQ ID NO: 57, or a DNA having a DNA hybridizable to a DNA containing the base sequence represented by SEQ ID NO: 40 or SEQ ID NO: 57 under high stringent conditions and encoding a protein which has an activity substantially equivalent to the activity of the protein containing the amino acid sequence represented by SEQ ID NO: 39 (a binding activity to the peptide of the present invention, a signal transduction activity, etc.).

Examples of the DNA containing the base sequence represented by SEQ ID NO: 40 or SEQ ID NO: 57 include DNA containing the base sequence represented by SEQ ID NO: 38 or SEQ ID NO: 40 SEQ ID NO: 56, SEQ ID NO: 57, and the like.

As the DNA hybridizable to a DNA containing the base sequence represented by SEQ ID NO: 40 or SEQ ID NO: 57 under high stringent conditions, there may be employed a DNA containing the base sequence having at least about 90% homology, preferably at least about 95% homology and more preferably at least about 98% homology, to the base sequence represented by SEQ ID NO: 40 or SEQ ID NO: 57, and the like.

The DNA encoding the protein containing the amino acid sequence represented by SEQ ID NO: 1 may be any DNA, so long as it is, e.g., a DNA containing the base sequence represented by SEQ ID NO: 2 or SEQ ID NO: 3, or a DNA having a DNA hybridizable to a DNA containing the base sequence represented by SEQ ID NO: 2 or SEQ ID NO: 3 under high stringent conditions and encoding a protein which has an activity substantially equivalent to the activity of the protein containing the amino acid sequence represented by SEQ ID NO: 1 (a binding activity to the peptide of the present invention, a signal transduction activity, etc.).

As the DNA hybridizable to a DNA containing the base sequence represented by SEQ ID NO: 2 or SEQ ID NO: 3 under high stringent conditions, there may be employed a DNA containing the base sequence having at least about 95% homology, preferably at least about 97% homology and more preferably at least about 98% homology, to the base sequence represented by SEQ ID NO: 2 or SEQ ID NO: 3, and the like.

The DNA encoding the protein containing the amino acid sequence represented by SEQ ID NO: 44 may be any DNA, so long as it is, e.g., a DNA containing the base sequence represented by SEQ ID NO: 43, or a DNA having a DNA hybridizable to a DNA containing the base sequence represented by SEQ ID NO: 43 under high stringent conditions and encoding a protein which has an activity substantially equivalent to the activity of the protein containing the amino acid sequence represented by SEQ ID NO: 44 (a binding activity to the peptide of the present invention, a signal transduction activity, etc.).

As the DNA hybridizable to a DNA containing the base sequence represented by SEQ ID NO: 43 under high stringent conditions, there may be employed a DNA containing the base sequence having at least about 95% homology,

preferably at least about 97% homology and more preferably at least about 98% homology, to the base sequence represented by SEQ ID NO: 43, and the like.

The DNA encoding the protein containing the amino acid sequence represented by SEQ ID NO: 51 may be any DNA, so long as it is, e.g., a DNA containing the base sequence represented by SEQ ID NO: 50, or a DNA having a DNA hybridizable to a DNA containing the base sequence represented by SEQ ID NO: 50 under high stringent conditions and encoding a protein which has an activity substantially equivalent to the activity of the protein containing the amino acid sequence represented by SEQ ID NO: 51 (a binding activity to the peptide of the present invention, a signal transduction activity, etc.).

As the DNA hybridizable to a DNA containing the base sequence represented by SEQ ID NO: 50 under high stringent conditions, there may be employed a DNA containing the base sequence having at least about 95% homology, preferably at least about 97% homology and more preferably at least about 98% homology, to the base sequence represented by SEQ ID NO: 50, and the like.

The DNA encoding the protein containing the amino acid sequence represented by SEQ ID NO: 52 may be any DNA, so long as it is, e.g., a DNA containing the base sequence represented by SEQ ID NO: 6, or a DNA having a DNA hybridizable to a DNA containing the base sequence represented by SEQ ID NO: 6 under high stringent conditions and encoding a protein which has an activity substantially equivalent to the activity of the protein containing the amino acid sequence represented by SEQ ID NO: 52 (a binding activity to the peptide of the present invention, a signal transduction activity, etc.).

As the DNA hybridizable to a DNA containing the base sequence represented by SEQ ID NO: 6 under high stringent conditions, there may be employed a DNA containing the base sequence having at least about 95% homology, preferably at least about 97% homology and more preferably at least about 98% homology, to the base sequence represented by SEQ ID NO: 6, and the like.

The DNA encoding the protein containing the amino acid sequence represented by SEQ ID NO: 53 may be any DNA, so long as it is, e.g., a DNA containing the base sequence represented by SEQ ID NO: 63, or a DNA having a DNA hybridizable to a DNA containing the base sequence represented by SEQ ID NO: 63 under high stringent conditions and encoding a protein which has an activity substantially equivalent to the activity of the protein containing the amino acid sequence represented by SEQ ID NO: 53 (a binding activity to the peptide of the present invention, a signal transduction activity, etc.).

As the DNA hybridizable to a DNA containing the base sequence represented by SEQ ID NO: 63 under high stringent conditions, there may be employed a DNA containing the base sequence having at least about 95% homology, preferably at least about 97% homology and more preferably at least about 98% homology, to the base sequence represented by SEQ ID NO: 63, and the like.

The DNA encoding the protein containing the amino acid sequence represented by SEQ ID NO: 54 may be any DNA, so long as it is, e.g., a DNA containing the base sequence represented by SEQ ID NO: 64, or a DNA having a DNA hybridizable to a DNA containing the base sequence represented by SEQ ID NO: 64 under high stringent conditions and encoding a protein which has an activity substantially equivalent to the activity of the protein containing the amino acid sequence represented by SEQ ID NO: 54 (a binding activity to the peptide of the present invention, a signal transduction activity, etc.).

As the DNA hybridizable to a DNA containing the base sequence represented by SEQ ID NO: 64 under high stringent conditions, there may be employed a DNA containing the base sequence having at least about 95% homology, preferably at least about 97% homology and more preferably at least about 98% homology, to the base sequence represented by SEQ ID NO: 64, and the like.

The DNA encoding the protein containing the amino acid sequence represented by SEQ ID NO: 171 may be any DNA, so long as it is, e.g., a DNA containing the base sequence represented by SEQ ID NO: 172, or a DNA having a DNA hybridizable to a DNA containing the base sequence represented by SEQ ID NO: 172 under high stringent conditions and encoding a protein which has an activity substantially equivalent to the activity of the protein containing the amino acid sequence represented by SEQ ID NO: 171 (a binding activity to the peptide of the present invention, a signal transduction activity, etc.).

As the DNA hybridizable to a DNA containing the base sequence represented by SEQ ID NO: 172 under high stringent conditions, there may be employed a DNA containing the base sequence having at least about 95% homology, preferably at least about 97% homology and more preferably at least about 98% homology, to the base sequence represented by SEQ ID NO: 172, and the like.

The DNA encoding the protein containing the amino acid sequence represented by SEQ ID NO: 173 may be any DNA, so long as it is, e.g., a DNA containing the base sequence represented by SEQ ID NO: 174, or a DNA having a DNA hybridizable to a DNA containing the base sequence represented by SEQ ID NO: 174 under high stringent conditions and encoding a protein which has an activity substantially equivalent to the activity of the protein containing the amino acid sequence represented by SEQ ID NO: 173 (a binding activity to the peptide of the present invention, a signal transduction activity, etc.).

As the DNA hybridizable to a DNA containing the base sequence represented by SEQ ID NO: 174 under high stringent conditions, there may be employed a DNA containing the base sequence having at least about 95% homology, preferably at least about 97% homology and more preferably at least about 98% homology, to the base sequence represented by SEQ ID NO: 174, and the like.

The hybridization can be carried out by publicly known methods or by a modification thereof, for example, according to the method described in Molecular Cloning, 2nd. (J. Sambrook et al., Cold Spring Harbor Lab. Press, 1989), etc. When a commercially available library is used, hybridization may be carried out according to the instructions of the attached manufacturer's protocol. The hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.

More specifically, the DNA encoding the peptide containing the amino acid sequence represented by SEQ ID NO: 81 includes a DNA containing the base sequence represented by SEQ ID NO: 87; the DNA encoding the peptide containing the amino acid sequence represented by SEQ ID NO: 82 includes a DNA containing the base sequence represented by SEQ ID NO: 88; the DNA encoding the peptide containing the amino acid sequence represented by SEQ ID NO: 83 includes a DNA containing the base sequence represented by SEQ ID NO: 89; the DNA encoding the peptide containing the amino acid sequence represented by SEQ ID NO: 84 includes a DNA containing the base sequence represented by SEQ ID NO: 90; the DNA encoding the peptide containing the amino acid sequence represented by SEQ ID NO: 109 includes a DNA containing the base sequence represented by SEQ ID NO: 110; the DNA encoding the peptide containing the amino acid sequence represented by SEQ ID NO: 107 includes a DNA containing the base sequence represented by SEQ ID NO: 108; the DNA encoding the peptide containing the amino acid sequence represented by SEQ ID NO: 138 includes a DNA containing the base sequence represented by SEQ ID NO: 139; and the DNA encoding the peptide containing the amino acid sequence represented by SEQ ID NO: 140 includes a DNA containing the base sequence represented by SEQ ID NO: 141, and the DNA encoding the peptide containing the amino acid sequence represented by SEQ ID NO: 142 includes a DNA containing the base sequence represented by SEQ ID NO: 143, and the DNA encoding the peptide containing the amino acid sequence represented by SEQ ID NO: 132 includes a DNA containing the base sequence represented by SEQ ID NO: 133, and the DNA encoding the peptide containing the amino acid sequence represented by SEQ ID NO: 134 includes a DNA containing the base sequence represented by SEQ ID NO: 135, and the DNA encoding the peptide containing the amino acid sequence represented by SEQ ID NO: 136 includes a DNA containing the base sequence represented by SEQ ID NO: 137, and the DNA encoding the peptide containing the amino acid sequence represented by SEQ ID NO: 19 includes a DNA containing the base sequence represented by SEQ ID NO: 20, and the DNA encoding the peptide containing the amino acid sequence represented by SEQ ID NO: 17 includes a DNA containing the base sequence represented by SEQ ID NO: 18, and the DNA encoding the peptide containing the amino acid sequence represented by SEQ ID NO: 39 includes a DNA containing the base sequence represented by SEQ ID NO: 40 or SEQ ID NO: 57, and the DNA encoding the peptide containing the amino acid sequence represented by SEQ ID NO: 37 includes a DNA containing the base sequence represented by SEQ ID NO: 38, and the DNA encoding the peptide containing the amino acid sequence represented by SEQ ID NO: 55 includes a DNA containing the base sequence represented by SEQ ID NO: 56.

Also, the DNA encoding the protein containing the amino acid sequence represented by SEQ ID NO: 1 includes a DNA containing the base sequence represented by SEQ ID NO: 2 or SEQ ID NO: 3; the DNA encoding the protein containing the amino acid sequence represented by SEQ ID NO: 44 includes a DNA containing the base sequence represented by SEQ ID NO: 43; the DNA encoding the protein containing the amino acid sequence represented by SEQ ID NO: 51 includes a DNA containing the base sequence represented by SEQ ID NO: 50; the DNA encoding the protein containing the amino acid sequence represented by SEQ ID NO: 52 includes a DNA containing the base sequence represented by SEQ ID NO: 6; the DNA encoding the protein containing the amino acid sequence represented by SEQ ID NO: 53 includes a DNA containing the base sequence represented by SEQ ID NO: 63; and the DNA encoding the protein containing the amino acid sequence represented by SEQ ID NO: 54 includes a DNA containing the base sequence represented by SEQ ID NO: 64; and the DNA encoding the protein containing the amino acid sequence represented by SEQ ID NO: 171 includes a DNA containing the base sequence represented by SEQ ID NO: 172; and the DNA encoding the protein containing the amino acid sequence represented by SEQ ID NO: 173 includes a DNA containing the base sequence represented by SEQ ID NO: 174.

The nucleotide (oligonucleotide) comprising a base sequence complementary to the base sequence of a DNA encoding the peptide of the present invention or a part thereof is intended to include not only a DNA encoding the peptide of the present invention but also an RNA.

According to the present invention, anti-sense (oligo)nucleotides (nucleic acids) that can inhibit replication or expression of the gene of the peptide of the present invention can be designed and synthesized, on the basis of base sequence information of a DNA encoding the cloned or sequenced peptide. Such (oligo)nucleotides (nucleic acids) can hybridize to an RNA of the gene of the peptide of the present invention and inhibit the synthesis or function of the RNA, or can regulate/control the expression of the gene of the peptide of the present invention via interaction with RNAs associated with the peptide of the present invention. (Oligo)nucleotides complementary to the specified sequences of RNAs associated with the peptide of the present invention and (oligo)nucleotides that can specifically hybridize to RNAs associated with the peptide of the present invention are useful for regulating/controlling expression of the gene of the peptide of the present invention in vivo and in vitro, and are also useful for the treatment or diagnosis of diseases.

The term "correspond" is used to mean homologous or complementary to a specific sequence of nucleotides including genes, base sequences or nucleic acids. As between nucleotides, base sequences or nucleic acids and peptides, the term "corresponding" usually refers to amino acids of a peptide that is instructed to be derived from the sequence of nucleotides (nucleic acids) or its complements. The 5' end hairpin loop, 5' end 6-base-pair repeats, 5' end untranslated region, polypeptide translation initiation codon, protein coding region, ORF translation initiation codon, 3' untranslated region, 3' end palindrome region, and 3' end hairpin loop of the G protein-coupled receptor protein gene may be selected as preferred target regions, though any region may be a target within genes for the peptide of the present invention.

The relationship between the targeted nucleic acids and the (oligo) nucleotides complementary to at least a portion of the target region, specifically the relationship between the target and the (oligo) nucleotides hybridizable to the target, is denoted to be in "an anti-sense". The anti-sense (oligo) nucleotides may be polydeoxynucleotides containing 2-deoxy-D-ribose, polydeoxynucleotides containing D-ribose, any other type of (oligo) nucleotides which are N-glycosides of a purine or pyrimidine base, other polymers containing non-nucleotide backbones (e.g., protein nucleic acids and synthetic sequence-specific nucleic acid polymers that are commercially available), other polymers containing nonstandard linkages (provided that the polymers contain nucleotides with such a configuration that allows base pairing or base stacking, as is found in DNAs or RNAs), etc. The anti-sense (oligo) nucleotides may be a double-stranded DNA, a single-stranded DNA, a double-stranded RNA, a single-stranded RNA and also a DNA:RNA hybrid, and further includes unmodified polynucleotides or unmodified oligonucleotides, those with publicly known types of modifications, for example, those with labels known in the art, those with caps, methylated (oligo)nucleotides, those with substitution of one or more naturally occurring nucleotides with their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (including nucleases, nuclease inhibitors, toxins, antibodies, signal peptides, poly-L-lysine, etc.), saccharides (e.g., monosaccharides, etc.), etc., those with intercalators (e.g., acridine, psoralen, etc.), those containing chelators (e.g., metals, radioactive metals, boron, oxidative metals, etc.), those containing alkylating agents, those with modified linkages (e.g., $\alpha$ anomeric nucleic acids, etc.). Herein, the terms "nucleoside", "nucleotide" and "nucleic acid" are used to refer to moieties that contain not only the purine and pyrimidine bases, but also other heterocyclic bases, which have been modified Such modifications include methylated purines and pyrimidines, acylated purines and pyrimidines and other heterocyclic rings. Modified nucleotides and modified nucleotides also include modifications on the sugar moiety, for example, wherein one or more hydroxyl groups may optionally be replaced with a halogen, aliphatic groups, or may be converted into the corresponding functional groups such as ethers, amines, or the like.

The anti-sense nucleic acid of the present invention is RNA, DNA or a modified nucleic acid. Specific examples of the modified nucleic acid are, but not limited to, sulfurized and thiophosphate derivatives of nucleic acids and those resistant to degradation of polynucleoside amides or oligonucleoside amides The anti-sense nucleic acids of the present invention can be modified preferably based on the following design, that is, by increasing the intracellular stability of the anti-sense nucleic acid, increasing the cellular permeability of the anti-sense nucleic acid, increasing the affinity of the nucleic acid to the target sense strand to a higher level, or minimizing the toxicity, if any, of the anti-sense nucleic acid.

Many such modifications are known in the art, as disclosed in J. Kawakami, et al., Pharm. Tech. Japan, Vol. 8, pp. 247, 1992; Vol. 8, pp. 395, 1992; S. T. Crooke et al. ed., Anti-sense Research and Applications, CRC Press, 1993; etc.

The anti-sense nucleic acid of the present invention may contain altered or modified sugars, bases or linkages. The anti-sense nucleic acid may also be provided in a specialized form such as liposomes, microspheres or may be applied to gene therapy or may be provided in combination with attached moieties. Such attached moieties include polycations such as polylysine that act as charge neutralizers of the phosphate backbone, or hydrophobic moieties such as lipids (e.g., phospholipids, cholesterols, etc.) that potentiate the interaction with cell membranes or increase uptake of the nucleic acid. Preferred examples of the lipids to be attached are cholesterols or derivatives thereof (e. g., cholesteryl chloroformate, cholic acid, etc.). These moieties may be attached at the 3' or 5' ends of the nucleic acid and may be also attached through a base, sugar, or intramolecular nucleoside linkage Other moieties may be capping groups specifically placed at the 3' or 5' ends of the nucleic acid to prevent degradation by nuclease such as exonuclease, RNase, etc Such capping groups include, but are not limited to, hydroxyl protecting groups known in the art, including glycols such as polyethylene glycol, tetraethylene glycol, and the like.

The inhibitory activity of the anti-sense nucleic acid can be examined using the transformant of the present invention, the gene expression system of the present invention in vitro or in vivo, or the translation system of proteins in vitro and in vivo. The nucleic acid can be applied to cells by a variety of publicly known methods.

The DNA encoding the partial peptide of the present invention may be any DNA, so long as it contains the base sequence encoding the partial peptide of the present invention described above. The DNA may also be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid, phagemid and the like. In addition, the DNA can be directly amplified by reverse transcriptase polymerase chain reaction with the mRNA fraction prepared from the cells or tissues described above.

Specifically, as the DNA encoding the partial peptide of the present invention there are employed, for example, a DNA that has a part of the base sequence of the DNA containing the base sequence represented by SEQ ID NO: 2 or SEQ ID NO: 3, SEQ ID NO: 43, SEQ ID NO: 50, SEQ ID NO: 6, SEQ ID NO: 63, SEQ ID NO: 64, SEQ ID NO:172, SEQ ID NO: 174 or (ii) a DNA having a base sequence hybridizable to the base sequence represented by SEQ ID NO: 2 or SEQ ID NO: 3, SEQ ID NO: 43, SEQ ID NO: 50, SEQ ID NO: 6, SEQ ID NO: 63, SEQ ID NO: 64, SEQ ID NO: 172, SEQ ID NO: 174 under high stringent conditions and containing a part of the base sequence of a DNA encoding a protein having substantially the same activity (e.g., a ligand binding activity, a signal transduction activity, etc.) as that of the protein of the present invention.

Examples of the DNA that is hybridizable to the base sequence represented by SEQ ID NO: 2 or SEQ ID NO: 3, SEQ ID NO: 43, SEQ ID NO: 50, SEQ ID NO: 6, SEQ ID NO: 63, SEQ ID NO: 64, SEQ ID NO:172, SEQ ID NO: 174 include a DNA containing the base sequence having at least about 90% homology, preferably at least about 95% homology, and more preferably at least about 98% homology, to the base sequence represented by SEQ ID NO: 2 or SEQ ID NO: 3, SEQ ID NO: 43, SEQ ID NO: 50, SEQ ID NO: 6, SEQ ID NO: 63, SEQ ID NO: 64, SEQ ID NO: 172, SEQ ID NO: 174.

For cloning of the DNA that completely encodes the peptide of the present invention, the DNA may be either amplified by PCR using synthetic DNA primers containing a part of the base sequence of the peptide of the present invention, or the DNA inserted into an appropriate vector can be selected by hybridization with a labeled DNA fragment or synthetic DNA that encodes a part or the entire region of the peptide of the present invention. The hybridization can be carried out, for example, according to the method described in Molecular Cloning, 2nd. (J. Sambrook et al., Cold Spring Harbor Lab. Press, 1989). Where commercially available library is used, the hybridization may also be performed in accordance with the protocol described in the attached instructions.

Cloning of the DNA that completely encodes the protein of the present invention or its partial peptides (hereinafter merely referred to as the protein of the present invention) may be carried out as in the cloning of the DNA that completely encodes the peptide of the present invention.

Conversion of the DNA base sequence can be effected by PCR or publicly known methods such as the ODA-LA PCR method, the Gupped duplex method or the Kunkel method, or modifications thereof, by using publicly known kits available as Mutan™-super Express Km (TaKaRa Shuzo Co., Ltd.) or Mutan™-K (TaKaRa Shuzo Co., Ltd.), etc.

The cloned DNA encoding the peptide/protein can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the peptide of the present invention and the protein of the present invention can be manufactured, for example, by (a) excising the desired DNA fragment from the DNA encoding the peptide of the present invention and the protein of the present invention, (b) and then ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, pcDNA3.1, pRc/CMV2, pRc/RSV (Invitrogen, Inc.), etc.

The promoter used in the present invention may be any promoter if it matches well with a host to be used for gene expression. In the case of using animal cells as the host, examples of the promoter include SRα promoter, SV40 promoter, HIV-LTR promoter, CMV promoter, HSV-TK promoter, etc.

Among them, CMV promoter, SRα promoter or the like is preferably used. Where the host is bacteria of the genus Escherichia, preferred examples of the promoter include trp promoter, lac promoter, recA promoter, λPL promoter, lpp promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin promoter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as Neor, G418 resistance), etc. In particular, when dhfr gene is used as the selection marker together with CHO (dhfr-) cell, the objective gene may also be selected on thymidine free media.

If necessary, a signal sequence that matches with a host is added to the N-terminal side of the protein of the present invention. Examples of the signal sequence that can be used are Pho A signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing the DNA encoding the peptide/protein of the present invention thus constructed, transformants can be manufactured.

Examples of the host, which may be employed, are bacteria belonging to the genus Escherichia, bacteria belonging to the genus Bacillus, yeast, insect cells, insects, animal cells, and the like.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R-, NA87-11A, DKD-5D, 20B-12, Schizosaccharomyces pombe NCYC1913, NCYC2036, Pichia pastoris, etc.

Examples of insect cells include, for the virus AcNPV, Spodoptera frugiperda cell (Sf cell), MG1 cell derived from mid-intestine of Trichoplusia ni, High FiveTM cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711) and Sf21 cell (both cells are described in Vaughn, J. L. et al., In vivo, 13, 213-217 (1977).

As the insect, for example, a larva of Bombyx mori can be used (Maeda et al., Nature, 315, 592 (1985)).

Examples of animal cells include monkey cell COS-7, Vero, Chinese hamster cell CHO (hereinafter referred to as CHO cell), dhfr gene deficient Chinese hamster cell CHO (hereinafter simply referred to as CHO(dhfr-) cell), mouse L cell, mouse AtT-20, mouse myeloma cell, rat GH 3, human FL cell, etc.

Bacteria belonging to the genus Escherichia can be transformed, for example, by the method described in Proc. Natl. Acad. Sci. U.S.A., 69, 2110 (1972), Gene, 17,107 (1982), etc. Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995), published by Shujunsha, or Virology, 52, 456 (1973).

Thus, the transformant transformed with the expression vector containing the DNA encoding the G protein-coupled protein can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, etc. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently. Where the bacteria belonging to the genus Escherichia are used as the host, the transformant is usually cultivated at approximately 15 to 43°C for about 3 hours to about 24 hours. If necessary, the culture may further be aerated or agitated.

Where the bacteria belonging to the genus Bacillus are used as the host, the transformant is cultivated generally at approximately 30 to 40°C for about 6 hours to about 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77,4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to about 8. In general, the transformant is cultivated at about 20°C to about 35°C for about 24 hours to about 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture may be aerated or agitated.

Where animal cells are employed as the host, the transformant is cultivated in, for example, MEM medium containing about 5% to about 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 hours to about 60 hours and, if necessary, the culture may be aerated or agitated.

As described above, the peptide/protein of the present invention can be produced in the cell or cell membrane, or outside the cell, of the transformant.

The peptide/protein of the present invention can be separated and purified from the culture described above, e.g., by the following procedures.

When the peptide/protein of the present invention is extracted from the culture or cells, after cultivation, the transformants or cells are collected by a publicly known method and suspended in an appropriate buffer. The transformants or cells are then disrupted by publicly known methods such as ultrasonication, a treatment with lysozyme and/or freeze-thaw cycling, followed by centrifugation, filtration, etc. Thus, the crude extract of the peptide/protein of the present invention can be obtained. The buffer used for the procedures may contain a protein denaturant such as urea or guanidine hydrochloride, or a surfactant such as Triton X-100TM, etc. When the peptide/protein of the present invention is secreted in the culture broth, after completion of the cultivation, the supernatant can be separated from the transformants or cells and collected by publicly known methods.

The peptide/protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the peptide/protein thus obtained is in a free form, it can be converted into the salt by publicly known methods or modifications thereof. On the other hand, when the peptide/protein is obtained in the form of a salt, it can be converted into the free form or in the form of a different salt by publicly known methods or modifications thereof.

The peptide/protein produced by the recombinant can be treated, prior to or after the purification, with an appropriate protein modifying enzyme so that the peptide/protein can be appropriately modified to partially remove a peptide/polypeptide. Examples of the protein-modifying enzyme include trypsin, chymotrypsin, arginyl endopeptidase, protein kinase, glycosidase and the like.

The activity of the thus produced peptide of the present invention can be determined by a binding test to the protein of the present invention, an enzyme immunoassay using a specific antibody, or the like.

Also, the activity of the protein of the present invention can be determined by a binding test to the peptide of the present invention, an enzyme immunoassay using a specific antibody, or the like.

The peptide of the present invention may also be manufactured in the form of an active peptide by genetically expressing in host prokaryotic cells and then refolding in a redox buffer. The details are described below.

Where the peptide of the present invention forms an insoluble component (inclusion body) in host prokaryotic cells, after the cells are cultured, collected by centrifugation, etc. and disrupted, the peptide of the present invention can be extracted by solubilizing the inclusion body with a denaturant.

The cell disruption can be carried out in a conventional manner, e.g., by ultrasonication. It is preferred to use as a suspending medium a suitable buffer (e.g., a phosphate buffer, etc.), which pH is adjusted to a value near neutral range (pH of 6.5 to 7.5). In this case, EDTA may be added to promote the cell disruption. After the cells are disrupted as above, the insoluble component (inclusion body) is fractionated by centrifugation or filtration in an optional fashion. For removal of prokaryotic cell-derived proteins as much as possible, washing with, e.g., water or a phosphate buffer is preferred, but urea of about 4M may be used for washing, as the case may be.

The precipitates (pellets) obtained are solubilized using denaturants. As such denaturants there may be used publicly known denaturants, especially guanidine, urea, etc. The denaturant is used normally in a solution thereof. The concentration of the denaturant in an aqueous solution is 4 to 8 mols/l, preferably approximately 6 to 7 mols/l for guanidine, and for urea, 5 to 9 mols/l, preferably about 8 mols/l. Guanidine is used usually as guanidine acid addition salts such as guanidine hydrochloride, etc.

Where the peptide of the present invention forms no insoluble component (inclusion body) in host prokaryotic cells, after the cells are cultured and collected by centrifugation, etc., the peptide of the present invention can be extracted either by solubilizing the cells inclusion body with a denaturant or by cell disruption followed by solubilization with a denaturant.

Examples of the denaturant used to solubilized the collected cells include guanidine, urea, etc. The denaturant is used usually in the form of an aqueous solution. The concentration of the denaturant in an aqueous solution is generally 4 to 8 mols/l, preferably approximately 6 to 7 mols/l for guanidine, and for urea, 5 to 9 mols/l, preferably about 8 mols/l. Guanidine is used usually as guanidine acid addition salts such as guanidine hydrochloride, etc.

The cell disruption may be carried out in a conventional manner, e.g., by ultrasonication or using a French press. Denaturants used to solubilize the disrupted cells may be those publicly known, preferably guanidine, urea, or the like. These denaturants are used usually in the form of an aqueous solution thereof. The concentration of the denaturant in an aqueous solution is generally 4 to 8 mols/l, preferably approximately 6 to 7 mols/l for guanidine, and for urea, 5 to 9 mols/l, preferably about 8 mols/l. Guanidine is used usually as guanidine acid addition salts such as guanidine hydrochloride, etc.

As described above, the inclusion body is solubilized, the cells are directly solubilized with a denaturant, or the cells are disrupted and solubilized with a denaturant to remove impurities by centrifugation, etc. and, if necessary, the resulting supernatant is applied to a purification step. Thereafter, the peptide of the present invention is subjected to refolding (activation, renaturation).

Refolding is effected by adding a redox buffer to the purified peptide of the present invention, or by diluting the supernatant containing the peptide of the present invention with a redox buffer.

As the redox buffer, there are a buffer containing oxidized glutathione (GSSG) and reduced glutathione (GSH), a buffer containing cysteine and cysteine, a buffer containing cysteamine and cystamine, etc. Among them, preferred is a buffer containing GSSG and GSH. Examples of the buffer described above include a Tris buffer, a phosphate buffer, an acetate buffer, a citrate buffer, etc. and among others, a Tris buffer or the like is preferred.

The concentrations of an oxidizing agent (oxidized substance) and a reducing agent (reduced substance) in the redox buffer are, e.g., 0.01 to 100 mmols/l and 0.01 to 100 mmols/l, respectively. More specifically, when GSSG and GSH are employed, the concentration of GSSG used in a redox buffer is 0.01 to 100 mmols/l, preferably 0.1 to 10 mmols/l and preferably 0.1 to 1.0 mmol/l; the concentration of GSH used is 0.01 to 100 mmols/l, preferably 0.1 to 10 mmols/l and preferably 0.2 to 2.0 mmols/l.

In this case, it is preferred to additionally incorporate, e.g., a mercapto-free amino acid, etc. into the redox buffer. In case that the supernatant containing the peptide of the present invention is diluted with the redox buffer, it is preferred to dilute the denaturant to such a concentration that the denaturant will be inert in a neutral pH range suitable for the activation. When using, e.g., guanidine as a denaturant, the concentration of guanidine in the dilution is desirably diluted to 0 to 2.0 mols/l, preferably to about 1 mol/l or less; when using urea as a denaturant, the concentration of urea in the dilution is desirably diluted to 0 to 4.0 mols/l, preferably to about 2 mols/l or less.

The mercapto-free amino acid described above includes, e.g., arginine, aspartic acid, valine, lysine, alanine, citrulline, etc. Arginine or the like is preferred in view of yield. The concentration of the amino acid added to the redox buffer is 0.1 to 1.0 mol/l, preferably 0.2 to 0.8 mol/l.

In the refolding described above, the temperature is from 0 to 30°C, preferably 4 to 15°C and the pH is 7 to 9, preferably 7.5 to 8.5. The time required for the refolding is generally 0.5 to 7 days, preferably 1 to 3 days, more preferably 1 to 2 days.

After the solubilization and prior to the refolding, a conventional and publicly known purification step including, e.g., extraction, salting out, dialysis, distribution, crystallization, recrystallization, gel filtration, chromatography, etc. can be implemented. Preferably, the peptide can be purified, for example, by passing through Sephadex G-25 (Amersham Pharmacia Biotech, Inc.) in 0.1 mo/l phosphate buffer. The denaturant can be separated by dialysis to 0.1 mol/l phosphate buffer, as the case may be.

The purification step may also be carried out, subsequently to the refolding. In general, such purification includes, e.g., extraction, salting out, dialysis, distribution, crystallization, recrystallization, gel filtration, chromatography, etc. Preferred examples of the purification include dialysis, purification methods by ion exchange chromatography using, e.g., SP-Sepharose (Amersham Pharmachia Biotech, Inc.), CM-5PW (Toso Co., Ltd.) or DEAE-5PW (Toso Co., Ltd.), reversed phase chromatography using, e.g., C4P-50 (Showa Denko Co., Ltd.), etc.

Antibodies to the peptide of the present invention, the protein of the present invention or its partial peptides, or salts thereof may be any of polyclonal and monoclonal antibodies, so long as they can recognize the peptide of the present invention, the protein of the present invention or its partial peptides, or salts thereof.

The antibodies to the peptide of the present invention, the protein of the present invention or its partial peptides, or salts thereof (in the specification sometimes simply referred to as the peptide/protein of the present invention) can be manufactured according to publicly known methods for producing antibodies or antisera, using the peptide of the present invention as antigens.

[Preparation of monoclonal antibody]

(a) Preparation of monoclonal antibody-producing cells

The peptide of the present invention is administered to mammals either solely or together with carriers or diluents to the site where the antibody can be produced by the administration. In order to potentiate the antibody productivity upon the administration, complete Freund's adjuvants or incomplete Freund's adjuvants may be administered. The administration is usually carried out once every 2 to 6 weeks approximately 2 to 10 times in total. Examples of the applicable mammals are monkeys, rabbits, dogs, guinea pigs, mice, rats, sheep and goats, with the use of mice and rats being preferred.

In the preparation of monoclonal antibody-producing cells, a warm-blooded animal such as mouse, immunized with an antigen is selected, then spleen or lymph nodes are collected after 2 to 5 days from the final immunization and antibody-producing cells contained therein are fused with myeloma cells to give monoclonal antibody-producing hybridomas. Measurement of the antibody titer in antisera may be carried out, for example, by reacting a labeled form of the peptide of the present invention described later with the antiserum followed by assaying the binding activity of the labeling agent bound to the antibody. The fusion operation may be carried out, for example, using the method known by Koehler and Milstein [Nature, 256, 495 (1975)]. Examples of the fusion promoter are polyethylene glycol (PEG), Sendai virus, etc., among which PEG is preferably employed.

Examples of the myeloma cells are NS-1, P3U1, SP2/0, etc. In particular, P3U1 is preferably employed. A preferred ratio of the count of the antibody-producing cells (spleen cells) used to the count of myeloma cells is within a range of approximately 1:1 to 20:1. When PEG (preferably, PEG 1000 to PEG 6000) is added in a concentration of approximately 10 to 80% followed by culturing at about 20 to 40°C, preferably at about 30 to 37°C for about 1 to 10 minutes, efficient cell fusion can be carried out.

Various methods can be used for screening of a monoclonal antibody-producing hybridoma. Examples of such methods include a method which comprises adding the hybridoma supernatant to a solid phase (e.g., a microplate) adsorbed with the peptide of the present invention as an antigen directly or together with a carrier, adding an anti-immunoglobulin antibody (where mouse cells are used for the cell fusion, anti-mouse immunoglobulin antibody is used) labeled with a radioactive substance or an enzyme, or protein A and detecting the monoclonal antibody bound to the solid phase; a method which comprises adding the hybridoma culture supernatant to a solid phase adsorbed with an anti-immunoglobulin antibody or protein A, adding the peptide of the present invention labeled with a radioactive substance or an enzyme and detecting the monoclonal antibody bound to the solid phase; and the like.

The monoclonal antibody can be selected in accordance with publicly known methods or modifications thereof. In general, the selection can be effected in a medium for animal cells supplemented with HAT (hypoxanthine, aminopterin and thymidine). Any selection and growth media can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.), etc. can be used for the selection and growth medium. The cultivation is carried out generally at 20 to 40°C, preferably at about 37°C. The time for cultivation is normally for 5 days to 3 weeks, preferably 1 to 2 weeks. The cultivation is carried out generally in 5% CO2. The antibody titer of the hybridoma culture supernatant can be determined as in the assay for the antibody titer in antisera described above.

(b) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or the specific purification method which comprises collecting an antibody alone with an activated adsorbent such as an antigen-binding solid phase, protein A or protein G and dissociating the binding to obtain the antibody].

[Preparation of polyclonal antibody]

The polyclonal antibody of the present invention can be manufactured by publicly known methods or the quasi thereof. For example, it can be manufactured by preparing a complex of an immune antigen (antigen of the peptide/ protein of the present invention) and carrier protein, immunizing to mammalian animals as well as above method of a monoclonal antibody, collecting an antibody- containing compound against the peptide/ protein of the present invention from the animal and isolating and purifying the antibody.

According to the complex of immune antigen and carrier protein to immunize to a mammalian animal, kinds of carrier proteins and a mixing ratio of carrier and hapten can be cross-linked by any kinds of compound and any ratio, if antibody is effectively manufactured against the hapten which is immunized by cross-linking to the carrier. For example, bovine serum albumin, bovine thyroglobulin, keyhole limpet, hemocyanin, or the like is coupled to hapten in a weight ratio of approximately 0.1 to 20, preferably about 1 to about 5.

A variety of condensation agents can be used for the coupling of carrier to hapten. Glutaraldehyde, carbodiimide, maleimide activated ester and activated ester reagents containing thiol group or dithiopyridyl group, and the like are used for the coupling.

The condensation product is administered to warm-blooded animals either solely or together with carriers or diluents to the site wherein the antibody can be produced. In order to potentiate the antibody productivity upon the administration, complete Freund's adjuvant or incomplete Freund's adjuvant may be administered. The administration is usually made once every about 2 to 6 weeks approximately 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as used for the determination of serum antibody titer described above. The separation and purification of the polyclonal antibody can be carried out, following the method for the separation and purification of immunoglobulins performed as in the separation and purification of monoclonal antibodies described above.

The peptide of the present invention, the DNA encoding the peptide of the present invention containing the same or the substantially same amino acid as the amino acid sequence represented by SEQ ID NO: 21or salt thereof (hereinafter sometimes referred to as MIT1) or the antibody against the protein of the present invention, polynucleotide encoding them or them is useful for a convenient method (screening) to obtain angiogenesis inhibitor. Specifically, antagonist of the protein of the present invention is useful of for excellent angiogenesis inhibitor.

Further, protein of the present invention, DNA encoding the protein of the present invention,(hereinafter, sometimes referred to as DNA of the present invention) and the antibody to the anti-sense DNA of the present invention and the peptide of the present invention (hereinafter sometimes referred to as the antibody of the present invention) are useful (i) as agents for the treatment/prevention of various diseases with which the peptide of the present invention is associated; (ii) for screening a compound or its salt that alters the binding property between the peptide of the present invention and the protein of the present invention; (iii) for quantification of the peptide of the present invention or a salt thereof; (iv) as a gene diagnostic agent; (v) as a pharmaceutical comprising the anti-sense DNA; (vi) as a pharmaceutical and diagnostic agent comprising the antibody of the present invention; (vii) for preparation of non-human animals bearing the DNA of the present invention; (viii) for drug design based on comparison with structurally similar ligand receptors; etc. In particular, by applying the receptor binding assay system using the expression system of the recombinant protein of the present invention, compounds (e.g., agonists, antagonists, etc.) that alter the binding property of human- or mammal-specific ligands to the protein of the present invention can be screened, and the agonists or antagonists can be used as agents for the prevention/treatment of various diseases.

Hereinafter, the peptide of the present invention, the DNA of the present invention and the antibody of the present invention will be specifically described, with reference to their use.

(1)(i) The compound or the salt thereof inhibiting the activity of the peptide of the present invention, (ii) the compound or the salt thereof inhibiting the activity of the protein of the present invention, (iii) the compound or the salt thereof inhibiting the binding the peptide or MIT1 of the present invention with the protein of the present invention, (iv) angiogenesis inhibitor of the peptide or MT1 of the present invention containing the compound or the salt thereof inhibiting an activated action of the protein of the present invention.

(i) The compound inhibiting the activity of the peptide of the present invention may be any compounds which inhibit an activity of the peptide of the present invention, for example, a compound which inhibits growth activity of the capillary endothelial cell, control activity of the intestinal tract contraction, binding activity of the peptide of the present invention, signal transduction mediating the protein of the present invention.

(ii) The compound inhibiting the activity of the protein of the present invention may be any compounds which inhibit activity of the protein of the present invention, for example, a compound which inhibits the binding activity of the peptide of the present invention or the signal transduction of the peptide of the present invention.

(iii) The compound inhibiting the binding the peptide or MIT1 of the present invention with the protein of the present invention may be any compounds which inhibit the binding he peptide or MIT1 of the present invention with the protein of the present invention.

(iv) The compound inhibiting an activated action of the protein of the present invention of the peptide or MIT1 of the present invention may be any compounds which inhibit an activated action of the protein of the present invention of the peptide or MIT1 of the present invention, for example, a compound which inhibits the binding activity or signal transduction of the protein of the present invention to the present peptide of the present invention by the peptide or MIT1 of the present invention.

(i) to (iv) of a compound or salt thereof described above may be obtained by using the screening method the present invention or the screening kit of the present invention.

As the salts of compound obtained by using the screening methods or screening kits described above, there may be employed, for example, pharmaceutically acceptable salts. Examples of the salts include salts with inorganic bases, salts with organic bases, salts with inorganic acids, salts with organic acids, salts with basic or acidic amino acids, and the like.

Preferred examples of the salts with inorganic bases include alkali metal salts such as sodium salts, potassium salts, etc.; alkaline earth metal salts such as calcium salts, magnesium salts, etc.; aluminum salts, ammonium salts, and the like.

Preferred examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc.

Preferred examples of the salts with inorganic acids include salts with hydrochloric acid, hydrobromic acid, sulfuric acid, phosphoric acid, etc.

Preferred examples of the salts with organic acids include salts with formic acid, acetic acid, propionic acid, fumaric acid, oxalic acid, tartaric acid, maleic acid, citric acid, succinic acid, malic acid, methanesulfonic acid, benzenesulfonic acid, benzoic acid, etc.

Preferred examples of the salts with basic amino acids include salts with arginine, lysine, omithine, etc. Preferred examples of the salts with acidic amino acids include salts with aspartic acid, glutamic acid, etc.

The compound or the salt thereof described above is used as an angiogenesis inhibitor, for example, is safely used as pharmaceuticals for the treatment/prevention of diseases associated with angiogenesis (e.g., cancers (e.g., pancreas cancer, lung cancer, kidney cancer, liver cancer, non-small-cell lung cancer, ovarian cancer, prostate cancer, stomach cancer, urinary bladder cancer, breast cancer, endocervix cancer, colon cancer, rectum cancer, Kaposi's sarcoma, or the like), ovarian diseases (e.g., polycystic ovarian syndrome, ovarial hyperstimulation syndrome, or the like), inflammatory diseases (e.g., articular rheumatism, or the like), diabatic retinopathy, or the like).

Where the compound or its salts obtained by the screening methods or kits of the present invention are used as the pharmaceuticals described above, such can be performed as in the case where the aforesaid peptide of the present invention is provided.

(2) Angiogenesis inhibitor, which comprises an anti-sense nucleotide having a nucleotide sequence or portion thereof complementary to or substantially complementary to the nucleotide sequence of a DNA encoding a peptide of the present invention

Since anti-sense nucleotide described above (anti-sense DNA, or the like) can inhibit the function of the peptide or DNA thereof of the present invention in vivo, it is safely used as a pharmaceuticals for treatment/prevention of diseases associated with angiogenesis, for example, (e.g., cancers (e.g., pancreas cancer, lung cancer, kidney cancer, liver cancer, non-small-cell lung cancer, ovarian cancer, prostate cancer, stomach cancer, urinary bladder cancer, breast cancer, endocervix cancer, colon cancer, rectum cancer, Kaposi's sarcoma, or the like), ovarian diseases (e.g., polycystic ovarian syndrome, ovarial hyperstimulation syndrome, or the like), inflammatory diseases (e.g., articular rheumatism, or the like), diabatic retinopathy, or the like).

Specifically, when the anti-sense DNA itself is administered; or the DNA is inserted into an appropriate vector such as retrovirus vector, adenovirus vector, adenovirus-associated virus vector, etc. and then administered in a conventional manner. The anti-sense DNA may also be administered as it is; or the DNA may be prepared into a pharmaceutical composition with physiologically acceptable carriers such as adjuvants, etc. to accelerate its uptake and the composition may be administered by gene gun or through a catheter such as a catheter with a hydrogel.

(3) Angiogenesis, which comprises an antibody against a protein or a partial peptide of the present invention

The antibody described above is safely used as pharmaceuticals for treatment/prevention of the diseases which is caused by overexpression of the peptide or the protein of the present invention, for example, as an angiogenesis inhibitor, for example, diseases associated with angiogenesis, (e.g., cancers (e.g., pancreas cancer, lung cancer, kidney cancer, liver cancer, non-small-cell lung cancer, ovarian cancer, prostate cancer, stomach cancer, urinary bladder cancer, breast cancer, endocervix cancer, colon cancer, rectum cancer, Kaposi's sarcoma, or the like), ovarian diseases (e.g., polycystic ovarian syndrome, ovarial hyperstimulation syndrome, or the like), inflammatory diseases (e.g., articular rheumatism, or the like), diabatic retinopathy, or the like)

Where the compound or its salts of the present invention are used as the pharmaceuticals described above, such can be performed as in the case where the aforesaid pharmaceuticals containing the antibody of the present invention is provided.

(4) An agent for diagnosing cancer, polycystic ovary syndrome or ovarian hyperstimulation syndrome, which comprises a polynucleotide encoding a peptide or a protein of the present invention

By using the DNA of the present invention, e.g., as a probe, an abnormality (gene abnormality) of the DNA or mRNA encoding the peptide of the present invention in human or other warm-blooded animal (e.g., rat, mouse, guinea pig, rabbit, chicken, sheep, swine, bovine, horse, cat, dog, monkey, etc.) can be detected. Therefore, the DNA of the present invention is useful as a gene diagnostic agent for damages to the DNA or mRNA, its mutation or decreased expression, or increased expression or overexpression of the DNA or mRNA.

The gene diagnosis described above using the DNA of the present invention can be performed by, for example, publicly known Northern hybridization or PCR-SSCP assay (Genomics, 5, 874-879 (1989); Proceedings of the National Academy of Sciences of the United States of America, 86, 2766-2770 (1989)), DNA microarray, and the like.

For example, when reduced expression is detected by northern hybridization or DNA microarray, or when DNA mutation is detected by PCR-SS or DNA microarray, it can be diagnosed that, for example, diseases associated with the proliferation of the capillary vessel endothelial cell, for example, cancers (e.g., pancreas cancer, lung cancer, kidney cancer, liver cancer, non-small-cell lung cancer, ovarian cancer, prostate cancer, stomach cancer, urinary bladder cancer, breast cancer, endocervix cancer, colon cancer, rectum cancer, Kaposi's sarcoma, or the like), ovarian diseases (e.g., polycystic ovarian syndrome, ovarial hyperstimulation syndrome, or the like), inflammatory diseases (e.g., articular rheumatism, or the like), diabatic retinopathy, or the like. are involved or it is highly likely to suffer in the future from these diseases.

(5) Screening using a peptide or MIT1 of the present invention and a protein of the present invention

By a screening method characterized in that said method uses a peptide (containing a partial peptide) or MIT1 of the present invention and the protein (containing a partial protein) of the present invention, or a kit for screening characterized in that said method uses a peptide or MIT1 of the present invention and the protein of the present invention (hereinafter, merely referred to as the screening method of the present invention, or the screening kit of the present invention), for example, compound that alters the binding property between the peptide or MIT1 of the present invention and the protein of the present invention, preferably, is obtained below:

(i) a compound or the salt thereof which inhibits the activity of the peptide of the present invention,
(ii) a compound or the salt thereof which inhibits the activity of the protein of the present invention,
(iii) a compound or the salt thereof which inhibits binding of the peptide or MIT1 of the present invention to the protein of the present invention,
(iv) a compound or the salt thereof which inhibits an activated action of the peptide or MIT1 of the present invention the protein of the present invention.

The compounds or the salts thereof of above (i) to (iv) have an action of inhibiting angiogenesis and is useful as an angiogenesis inhibitor.

According to a screening method or a kit for screening of the present invention, it is described in detail below.

By using the protein of the present invention, or by constructing the expression system of a recombinant form of the protein of the present invention and using the binding assay system to the peptide or MIT1 of the present invention through the expression system (ligand-receptor assay system), the compound or its salt that alters the binding property between the peptide or MIT1 of the present invention and the protein of the present invention (e.g., peptide, protein, a non-peptide compound, a synthetic compound, fermentation product, etc.) can be screened.

Such a compound includes a compound (agonist) having the cell stimulating activity mediated by the protein of the present invention (e.g., the activity that promotes or inhibits arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.), a compound having no cell stimulating activity (antagonist), and the like. The term "alters the binding property between the peptide of the present invention and the protein of the present invention" is used to include both cases where binding of the peptide of the present invention to the protein of the present invention is inhibited and promoted.

Thus, the present invention provides the method of screening a compound or its salt that alters the binding property of the peptide of the present invention to the peptide of the present invention, which comprises comparing (i) the case wherein the peptide of the present invention is brought in contact with the protein of the present invention and (ii) the case wherein the peptide of the present invention and a test compound are brought in contact with the protein of the present invention.

The MIT1 may be employed in the screening of the present invention similarly to the peptide of the present invention.

According to the screening method of the present invention, the method comprises assaying, for example, the binding amount of the peptide of the present invention to the protein of the present invention, the cell stimulating activity, or the like, (i) when the peptide of the present invention is brought in contact with the protein of the present invention described above and (ii) when the peptide of the present invention and a test compound are brought in contact with the protein of the present invention described above, and comparing (i) and (ii).

Specific examples of the screening method of the present invention include:

(a) A method of screening a compound or its salt that alters the binding property between the peptide of the present invention and the protein of the present invention, which comprises measuring the binding amounts of the peptide of the present invention to the protein of the present invention when the peptide of the present invention is brought in contact with the protein of the present invention and when the peptide of the present invention and a test compound are brought in contact with the protein of the present invention; and comparing the binding amounts;

(b) A method of screening a compound or its salt that alters the binding property between the peptide of the present invention and the protein of the present invention, which comprises, when the peptide of the present invention is brought in contact with a cell or its cell membrane fraction containing the protein of the present invention and when the peptide of the present invention and a test compound are brought in contact with a cell or its cell membrane fraction containing the protein of the present invention, assaying binding amounts of the peptide of the present invention to the cell or the membrane fraction; and comparing the binding amounts; and,

(c) The screening method according to (b) described above, wherein the peptide of the present invention is the protein of the present invention expressed on the cell membrane by culturing a transformant bearing a DNA encoding the protein of the present invention;

(d) The receptor-binding assay system such as the screening method described in (a) to (c) above, wherein the peptide of the present invention is a labeled ligand;

(e) A method of screening a compound or its salt that alters the binding property between the peptide of the present invention and the protein of the present invention, which comprises assaying cell stimulating activities mediated by the protein of the present invention, when the peptide of the present invention is brought in contact with a cell or its cell membrane fraction containing the protein of the present invention and when the peptide of the present invention and a test compound are brought in contact with a cell or its cell membrane fraction containing the protein of the present invention; and comparing the activities;

(f) A method of screening a compound or its salt that alters the binding property between the peptide of the present invention and the protein of the present invention, which comprises assaying cell stimulating activities mediated by the protein of the present invention, when the peptide of the present invention is brought in contact with a cell or its cell membrane fraction containing the protein of the present invention and when the peptide of the present invention and a test compound are brought in contact with a cell or its cell membrane fraction containing the protein of the present invention; and comparing the activities; and,

(g) The screening method according to (f) described above, wherein the protein of the present invention is the protein of the present invention expressed on the cell membrane by culturing a transformant bearing a DNA encoding the protein of the present invention; etc.

The screening method of the present invention will be specifically described below.

First, the protein of the present invention used for the screening methods of the present invention may be any of those containing the protein of the present invention described above. However, since human-derived organs in particular are obtained only with extreme difficulty, the protein of the present invention, etc. expressed in large quantities by use of recombinants are suitable. Endothelial cell which is derived from mammalian can be used.

To produce the protein of the present invention, the aforesaid methods, and the like are applied.

When cells containing the protein of the present invention or membrane fractions of these cells are employed in the screening methods of the present invention, these cells or membrane fractions may be prepared following the procedures later described.

Where cells containing the protein of the present invention are employed, the cells may be fixed using glutaraldehyde, formalin, or the like. The fixation can be made by publicly known methods.

The cells containing the protein of the present invention refer to host cells wherein the protein of the present invention is expressed, and such host cells include Escherichia coli, Bacillus subtilis, yeast, insect cells, animal cells, etc. described above.

The cell membrane fraction is used to mean a fraction abundant in cell membrane obtained by cell disruption and subsequent fractionation by publicly known methods. The cell disruption methods include cell squashing using a Potter-Elvehjem homogenizer, disruption using a Waring blender or Polytron (manufactured by Kinematica Inc.), and disruption by ultrasonication, disruption by cell spraying through thin nozzles under an increased pressure using a French press, and the like. Cell membrane fractionation is effected mainly by fractionation using a centrifugal force, such as fractional centrifugation, density gradient centrifugation, etc. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the protein of the present invention expressed and membrane components such as cell-derived phospholipids, membrane proteins, etc.

The amount of the protein of the present invention in the cells or cell membrane fractions containing the protein of the present invention is preferably $10^3$ to $10^8$ molecules, more preferably $10^5$ to $10^7$ molecules, per cell. As the amount of expression increases, the ligand binding activity per unit of the membrane fraction (specific activity) increases so that not only the highly sensitive screening system can be constructed but also large quantities of samples can be assayed on the same lot.

The endothelial cells which is derived from mammalian refer to, for example, the endothelial cell of human, monkey, bovine, swine, rat, mouse or the like (e.g., arterial vessel, capillary vessel).

Specifically, bovine capillary vessel which is one of the mammalian capillary vessels can be prepared described below. Further, by using the same method, capillary vessel which is derived from the brain or ovary of the mammalian can be prepared.

First, after lipid attached on the surface of a bovine adrenal is removed by cutting it out with scissors, the adrenal is sterilize by immersing in physiological saline containing 20% isodine in 5 minutes. Then, the adrenal is washed by physiological saline, is cut into halves and adrenal medulla is removed. Retained adrenal cortex is collected with surgeon knife and scissors and cut off finely. Consequently, the following operation is carried out by using the half of the adrenal cortex. Specifically, the pieces are added 20ml of Minimum essential medium (MEM) containing 0.5% collagenase (Wako Pure Chemical Industries, Ltd) and 0.1% bovine serum albumin (BSA) and incubated 37°C in 30 minutes. Then, the mass of tissue is broken by pipetting, is centrifuged at 1 to 600 rpm in 5 minutes. The obtained pellet is added 25ml of MEM containing 25% BSA and is centrifuged again at 2700rpm in 20 minutes. The obtained pellet is added MEM containing collagenase above described and is incubated at 37°C in 15 minutes. Then, the collagenase digestion is filtrated with nylon gauze (100 micron) and the permeate is collected. The permeate is filtrated with nylon gauze (50 micron) again and bits of tissue which is collected with gauze is washed into 6-well plate which is coated with gelatin by using MEM containing 10% fetal bovine serum (BSA). After 4 hours, the plate is mildly washed with 10% BSA / MEM, then is added MEM containing 2 ng/ml basic fibroblast growth factor and 10 % BSA and is incubated at 37°C under 5% $CO_2$. After that, total volume of the medium is replaced every 3 days. After 5 to 6 days, unnecessary cells are removed under microscope by using Pasteur pipette leaving colonies of the desired cell. After 9 to 10 days, by the desired colonies being attached cloning ring, cells are collected by treatment of trypsin and are removed to 24-well plate. The obtained cells are cultured until being the adequate scale and are used for experiments.

To perform the screening methods such as the receptor-binding assay system, the cell stimulating assay system and the like, for example, a fraction of the protein of the present invention and a labeled form of the peptide of the present invention (e.g., a labeled form of the peptide of the present invention), etc. are employed. For the fraction of the protein of the present invention, a fraction from naturally occurring type of the protein of the present invention or a fraction from recombinant type of the protein of the present invention having an activity equivalent thereto, or the like, are desirable. Herein, the equivalent activity is used to mean an equivalent ligand binding activity, etc. As the labeled peptide of the present invention, for example, there may be used peptide of the present invention labeled with, radioisotope (e.g., [$^3$H], [$^{125}$I], [$^{14}$C], [$^{32}$P], [$^{33}$P], [$^{35}$S], or the like), fluorescent substance (e.g., cyanin fluorescent dye (e.g., Cy 2, Cy3, Cy5.5, Cy7 (manufactured by Amersham Pharmacia Biotech, Inc.), or the like), fluorescamine, fluorescenisotyanate, or the like), enzyme (e.g., β-galactosidase, β-glucosidase, alkaline phosphatase, peroxydase, malate dehydrogenase,or the like), light-emitting substance (e.g., luminol, luminol derivatives, luciferin, lucigenin, or the like) the peptide of the present invention labeled with biotin, lanthanide element, or the like, preferably, the peptide of the present invention labeled with radioisotope. In particular, a labeled form of the peptide of the present invention prepared by publicly known methods using Bolton-Hunter reagent are available as well.

Specifically, screening of the compound that alters the binding property between the peptide of the present invention and the protein of the present invention can be performed by the following procedures. First, a receptor preparation is prepared by suspending cells containing the protein of the present invention or their membrane fractions in a buffer appropriate for screening. Any buffer can be used so long as it does not interfere with ligand-protein binding, such buffer including a phosphate buffer, a Tris-HCl buffer, etc. having pH of 4 to 10 (desirably pH of 6 to 8). For the purpose of minimizing non-specific binding, a surfactant such as CHAPS, Tween-80TM (manufactured by Kao-Atlas Inc.), digitonin, deoxycholate, etc. may be added to the buffer. Further for the purpose of suppressing degradation of the protein of the present invention or the peptide of the present invention by a protease, a protease inhibitor such as PMSF, leupeptin, E-64 (manufactured by Peptide Institute, Inc.), pepstatin, etc. may also be added. A given quantity (5,000 cpm to 500,000 cpm) of a labeled form of the peptide of the present invention is added to 0.01 ml to 10 ml of the receptor solution, and at the same time, 10-4 to 10-1 μM of a test compound is allowed to be co-present. To determine the amount of non-specific binding (NSB), a reaction tube containing a large excess of the peptide of the present invention in an unlabeled form is also provided. The reaction is carried out at 0°C to 50°C, preferably about 4°C to 37°C for 20 minutes to 24 hours, preferably 30 minutes to 3 hours. After completion of the reaction, the reaction mixture is filtrated through glass fiber filter paper, etc. and washed with an appropriate volume of the same buffer. The residual radioactivity in the glass fiber filter paper is then measured by means of a liquid scintillation counter or a γ-counter. When the nonspecific binding (NSB) is subtracted from the count ($B_0$) when any antagonizing compound is absent and the thus obtained count ($B_0$ - NSB) is made 100%, a test compound having the specific binding (B - NSB) of, e.g., 50% or less, can be selected as a candidate substance capable of competitive inhibition.

For assaying the binding of the protein of the present invention to the peptide of the present invention, BIAcore (manufactured by Amersham Pharmacia Biotech, Inc.) may also be employed. According to this technique, the peptide of the present invention is immobilized onto a sensor chip by the amino coupling method following the protocol attached to the device. A buffer such as phosphate buffer, Tris buffer, etc., which contains the protein of the present invention purified from cells containing the protein of the present invention or from transformants containing a DNA encoding the protein of the present invention or a membrane fraction containing the protein of the present invention, or the purified protein of the present invention or a membrane fraction containing the protein of the present invention and a test compound, is passed over the sensor chip at a flow rate of 2 to 20 μl/min. By monitoring that the test compound co-present alters the change in surface plasmon resonance caused by binding of the peptide of the present invention to the protein of the present invention on the sensor chip, the compound that alters the binding of the protein of the present invention to the peptide of the present invention can be screened. According to this method, the alteration can be measured as well, by the procedure which involves immobilizing the protein of the present invention onto a sensor chip and passing over the sensor chip a buffer solution such as phosphate buffer, Tris buffer, etc., which contains the peptide of the present invention or the peptide of the present invention and a test compound. Examples of the test compound are the same as those described above.

To perform the screening methods of the cell stimulating assay system described above, the cell-stimulating activities mediated by the protein of the present invention (e.g., the activity that promotes or suppresses arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, GTPγS-binding activity, etc.) may be assayed by publicly known methods, or using assay kits commercially available. Specifically, the cells containing the protein of the present invention are first cultured on a multi-well plate, etc. Prior to screening, the medium is replaced with a fresh medium or with an appropriate non-cytotoxic buffer, and a test compound or the like is added thereto, followed by culturing for a given period of time. Subsequently, the cells are extracted or the supernatant is recovered and the resulting product is quantified by the respective methods. Where it is difficult to detect the production of an indicator substance for the cell stimulating activity (e.g., arachidonic acid, etc.) due to a degrading enzyme contained in the cells, an inhibitor against such a degrading enzyme may be added prior to the assay. For detecting activities such as the cAMP production suppressing activity, the baseline production in the cells is increased by forskolin or the like and the suppressing effect on the increased baseline production can be detected.

To perform the screening through assaying the cell stimulating activity, cells in which an appropriate form of the protein of the present invention is expressed are required. As the cells wherein the protein of the present invention is expressed, a recombinant type of the aforesaid cell line wherein the protein of the present invention is expressed, etc. are desirable. The cells or transformants wherein the protein of the present invention is expressed may be either stably expressed cells or temporarily expressed cells. The kind of animal cells used is the same as described above.

Examples of the test compound include peptides, proteins, non-peptide compounds, synthetic compounds, fermentation products, cell extracts, plant extracts, animal tissue extracts, and the like.

In more detail, the screening methods of the cell stimulating assay system described above are described in [1] to [12] below.

[1] When receptor-expressed cells are stimulated by a receptor agonist, G protein in the cells is activated and GTP is bound thereto. This phenomenon is observed as well in a membrane fraction of the receptor-expression cells. Usually, GTP is hydrolyzed and changes to GDP. When GTPγS is previously added to the reaction solution, GTPγS is bound to G protein as in GTP, but is not hydrolyzed so that the state of GTPγS bound to the G protein-containing cell membrane is maintained. When labeled GTPγS is used, the labeled GTPγS remained on the cell membrane is measured, whereby the stimulating activity of the receptor agonist in the receptor-expressed cells can be assayed.

Utilizing this reaction, the compound that alters the binding property between the peptide of the present invention and the protein of the present invention can be screened by assaying the stimulating activity of the peptide of the present invention on the cells wherein the protein of the present invention is expressed.

This method is carried out using the membrane fraction containing the protein of the present invention. In this assay method, the substance showing the activity of promoting the binding of GTPγS to the membrane fraction containing the protein of the present invention is an agonist.

Specifically, the compound that alters the binding property between the peptide of the present invention and the protein of the present invention can be screened by assaying the GTPγS binding promoting activities on the membrane fraction containing the protein of the present invention in the presence of labeled GTPγS, when the peptide of the present invention is brought in contact with the membrane fraction containing the protein of the present invention and when the peptide of the present invention and a test compound are brought in contact with the membrane fraction containing the protein of the present invention; and comparing the activities therebetween.

In this method, the test compound showing the activity of suppressing the GTPγS binding promoting activity by the peptide of the present invention against the membrane fraction containing the protein of the present invention can be selected as a candidate substance capable of competitive inhibition.

On the other hand, an agonist can be screened as well by contacting a test compound alone with the membrane fraction containing the protein of the present invention and assaying the GTPγS binding promoting activity on the membrane fraction containing the protein of the present invention.

An example of the screening method is specifically described below.

The membrane fraction containing the protein of the present invention, which is prepared by publicly known methods with a modification, is diluted with a buffer for membrane dilution (50 mM Tris, 5 mM $MgCl_2$, 150 mM NaCl, 1 μM GDP, 0.1% BSA, pH 7.4). A degree of dilution varies depending upon the amount of a receptor expressed. The dilution is dispensed by 0.2 ml each in Falcon 2053, to which the peptide of the present invention or the peptide of the present invention and a test compound are added, and [35S]GTPγS is further added to the mixture in a final concentration of 200 pM. After maintaining at 25°C for an hour, 1.5 ml of ice-cooled wash buffer (50 mM Tris, 5 mM $MgCl_2$, 150 mM NaCl, 0.1% BSA, 0.05% CHAPS, pH 7.4) is added to the mixture followed by filtration through a glass fiber filter paper GF/F. After keeping at 65°C for 30 minutes, the mixture is dried and the radioactivity of [35S] GTPγS bound to the membrane fraction remained on the filter paper is measured with a liquid scintillation counter. When the radioactivity in the experimental zone added with the peptide of the present invention alone is defined as 100% and the radioactivity in the experimental zone not added with the peptide of the present invention is defined as 0%, an effect of the test compound on the GTPγS binding promoting activity by the peptide of the present invention is worked out. The test compound showing the GTPγS binding promoting activity of, for example, 50% or less, can be selected as a candidate compound capable of competitive inhibition.

[2] In the cells wherein the protein of the present invention is expressed, the intracellular cAMP production is suppressed by stimulation of the peptide of the present invention. Utilizing this reaction, the compound that alters the binding property between the peptide of the present invention and the protein of the present invention can be screened by assaying the stimulating activity of the peptide of the present invention on the cells wherein the protein of the present invention is expressed.

Specifically, the compound that alters the binding property between the peptide of the present invention and the protein of the present invention can be screened by assaying the intracellular cAMP production suppressing activities on the cells in the presence of a substance capable of increasing the amount of intracellular cAMP, when the peptide of the present invention is brought in contact with the cells wherein the protein of the present invention is expressed and when the peptide of the present invention and a test compound are brought in contact with the cells wherein the protein of the present invention is expressed; and comparing the activities therebetween.

As the substance capable of increasing the amount of intracellular cAMP, there are employed, e.g., forskolin, calcitonin, etc.

The amount of cAMP produced in the cells wherein the protein of the present invention is expressed can be assayed by the RIA system using an anti-cAMP antibody, whose antibody is obtained from immunized mouse, rat, rabbit, goat, bovine, etc., and [125I]-labeled cAMP (both commercially available) or by the EIA system using an anti-cAMP antibody and labeled cAMP in combination. Quantification by the SPA (Scintillation Proximity Assay) method is also available, using beads, which contain scintillants bearing anti-cAMP antibodies immobilized using protein A or antibodies to IgG, etc. of animal used to produce the anti-cAMP antibodies, and 125I-labeled cAMP (the kit manufactured by Amersham Pharmacia Biotech, Inc. is used).

In this method, the test compound showing the activity of inhibiting the cAMP production suppressing activity by the peptide of the present invention against the cells wherein the protein of the present invention is expressed can be selected as a candidate substance capable of competitive inhibition.

On the other hand, the compound showing the agonist activity can be screened by monitoring the cAMP production suppressing activity when a test compound alone is brought in contact with the cells wherein the protein of the present invention is expressed.

A specific example of the screening method is described below.

The cells wherein the protein of the present invention is expressed (e.g., animal cells such as CHO cells, etc.) ((ZAQC-B1 cells; EXAMPLE 7 later described)) are inoculated on a 24-well plate in 5 × 104 cells/well followed by cultivation for 48 hours. The cells are washed with Hanks' balanced salt solution (pH 7.4) containing 0.2 mM 3-isobutyl-methylxanthine, 0.05% BSA and 20 mM HEPES (hereinafter simply referred to as a reaction buffer). Thereafter, 0.5 ml of the reaction buffer is added to the cells and the mixture is kept warm in the medium for 30 minutes. The reaction buffer is removed and 0.25 ml of a fresh reaction buffer is added to the cells. Then, 0.25 ml of a 2 μM forskolin-containing reaction buffer, in which 1 μM of the peptide of the present invention or 1 μM of the peptide of the present invention and a test compound is/are incorporated, is added to the cells, followed by reacting at 37°C for 24 minutes. The reaction is terminated by adding 100 μl of 20% perchloric acid. The reaction mixture is then put on ice for an hour to extract intracellular cAMP. The amount of cAMP in the extract is measured using a cAMP EIA kit (Amersham Pharmacia Biotech). Taking the amount of cAMP produced by forskolin stimulation as 100% and the amount of cAMP inhibited by addition of 1 μM of the peptide of the present invention as 0%, an effect of the test compound on the cAMP production suppressing activity by the peptide of the present invention is calculated. The test compound that inhibits the activity of the peptide of the present invention to increase the cAMP producing activity, e.g., to 50% or more, can be selected as a candidate substance capable of competitive inhibition.

Further in the case of using the cells wherein the protein of the present invention is expressed and which show the property of increasing the amount of intracellular cAMP through stimulation by the peptide of the present invention, the compound that alters the binding property between the peptide of the present invention and the protein of the present invention can be screened by assaying the intracellular cAMP production promoting activities in the cells, when the peptide of the present invention is brought in contact with the cells wherein the protein of the present invention is expressed and when the peptide of the present invention and a test compound are brought in contact with the cells wherein the protein of the present invention is expressed; and comparing the activities therebetween.

In this method, the test compound showing the activity of inhibiting the cAMP production promoting activity by the peptide of the present invention against the cells wherein the protein of the present invention is expressed can be selected as a candidate substance capable of competitive inhibition.

On the other hand, the compound showing the agonist activity can be screened by monitoring the cAMP production promoting activity when a test compound alone is brought in contact with the cells wherein the protein of the present invention is expressed.

To determine the cAMP production promoting activity, the amount of cAMP produced by adding the peptide of the present invention or the peptide of the present invention and a test compound to the cells (e.g., animal cells such as CHO cells, etc.) wherein the protein of the present invention is expressed, without adding forskolin in the screening method described above, is quantified by the procedure described above.

[3] The compound that alters the binding property between the peptide of the present invention and the protein of the present invention can be screened by assaying the stimulating activity of the peptide of the present invention on the cells, in which the protein of the present invention is expressed, using CRE-reporter gene vector.

A DNA containing CRE (cAMP response element) is inserted into an upstream of the reporter gene in a vector, to acquire CRE-reporter gene vector. In the CRE-reporter gene vector-transfected cells wherein the protein of the present invention is expressed, stimulation accompanied by increased cAMP induces expression of the reporter gene mediated by CRE and production of the gene product (protein) of the reporter gene subsequent thereto. That is, by assaying the enzyme activity of the reporter gene protein, a change in the amount of cAMP in the CRE-reporter gene vector-transfected cells can be detected.

Specifically, the compound that alters the binding property between the peptide of the present invention and the protein of the present invention can be screened by assaying the enzyme activity of the protein encoded by the reporter gene in the presence of a substance capable of increasing the amount of intracellular cAMP, when the peptide of the present invention is brought in contact with the CRE-reporter gene vector-transfected cells wherein the protein of the present invention is expressed and when the peptide of the present invention and a test compound are brought in contact with the CRE-reporter gene vector-transfected cells wherein the protein of the present invention is expressed; and comparing the activities therebetween.

As the substance capable of increasing the amount of intracellular cAMP, there are employed, e.g., forskolin, calcitonin, etc.

As the vector, there may be employed, e.g., PicaGene Basic Vector, PicaGene Enhancer Vector (Toyo Ink Mfg. Co., Ltd.), and the like. A DNA containing CRE is inserted into the multicloning site upstream the reporter gene, e.g., luciferase gene in the vector described above, which is made a CRE-reporter gene vector.

In this method, the test compound that restores the enzyme activity suppression of the reporter gene protein by the peptide of the present invention can be selected as a candidate substance capable of competitive inhibition.

On the other hand, an agonist can be screened by assaying the suppression of a luminescence level increased by forskolin stimulation when a test compound alone is brought in contact with the cells wherein the protein of the present invention is expressed.

Taking as an example in which luciferase is used as a reporter gene, a specific example of this screening method is described below.

The CRE-reporter gene (luciferase)-transfected cells wherein the protein of the present invention is expressed are inoculated on a 24-well plate in 5 × 103 cells/well followed by cultivation for 48 hours. The cells are washed with Hanks' balanced salt solution (pH 7.4) containing 0.2 mM 3isobutyl-methylxanthine, 0.05% BSA and 20 mM HEPES (hereinafter merely referred to as a reaction buffer). Thereafter, 0.5 ml of the reaction buffer is added to the cells and the mixture is kept warm in the medium for 30 minutes. The reaction buffer is removed and 0.25 ml of a fresh reaction buffer is added to the cells. Then, 1 μM of the peptide of the present invention or 1 μM of the peptide of the present invention is/are added to 0.25 ml of the reaction buffer containing 2 μM forskolin, which is added to the cells. The reaction is then carried out at 37°C for 24 minutes. The cells are dissolved in a cell lysis agent for PicaGene (Toyo Ink Mfg. Co., Ltd.) and a luminescent substrate (Toyo Ink Mfg. Co., Ltd.) is added to the lysate. Luminescence by luciferase is measured with a luminometer, a liquid scintillation counter or a top counter. The levels of luminescence by luciferase are measured when only the peptide of the present invention is added and when 1 μM of the peptide of the present invention and a test compound are added, and compared therebetween.

The peptide of the present invention suppresses an increase in luminescence by luciferase associated with forskolin stimulation. The compound that restores the suppression can be selected as a candidate substance capable of competitive inhibition.

As the reporter gene, there may be employed genes, e.g., alkaline phosphatase, chloramphenicol acetyltransferase, β-galactosidase, etc. The enzyme activities of these reporter gene proteins are assayed in accordance with methods publicly know, or using commercially available assay kits. The alkaline phosphatase activity can be assayed by using, e.g., Lumi-Phos 530 manufactured by Wako Pure Chemical Industries, Ltd.; the chloramphenicol acetyltransferase activity by using, e.g., FAST CAT chloramphenicol Acetyltransferase Assay Kit manufactured by Wako Pure Chemical Industries, Ltd.; and the β-galactosidase activity by using, e.g., Aurora Gal-XE manufactured by Wako Pure Chemical Industries, Ltd.

[4] The cells wherein the protein of the present invention is expressed extracellularly release arachidonic acid metabolites by stimulation of the peptide of the present invention. Utilizing this reaction, the compound that alters the binding property between the peptide of the present invention and the protein of the present invention can be screened by assaying the stimulating activity of the peptide of the present invention on the cells wherein the protein of the present invention is expressed.

By previously incorporating labeled arachidonic acid into the cells wherein the protein of the present invention is expressed, the arachidonic acid metabolite-releasing activity can be assayed by measuring the labeled arachidonic acid metabolites released outside the cells.

Specifically, the compound that alters the binding property between the peptide of the present invention and the protein of the present invention can be screened by assaying the arachidonic acid metabolite-releasing activities, when the peptide of the present invention is brought in contact with the labeled arachidonic acid-containing cells wherein the protein of the present invention is expressed and when the peptide of the present invention and a test compound are brought in contact with the labeled arachidonic acid-containing cells wherein the protein of the present invention is expressed; and comparing the activities therebetween.

In this method, the test compound that inhibits the arachidonic acid metabolite-releasing activity by the peptide of the present invention can be selected as a candidate substance capable of competitive inhibition.

Furthermore, the compound showing the agonist activity can be screened as well by contacting a test compound alone with the cells wherein the protein of the present invention is expressed and monitoring the arachidonic acid metabolite-releasing activity of the cells wherein the protein of the present invention is expressed, in accordance with publicly known methods.

A specific example of this screening method is described below.

The cells wherein the protein of the present invention is expressed are inoculated on a 24-well plate in 5 × 104 cells/well. After cultivation for 24 hours, [3H] arachidonic acid is added to the cells in 0.25 μCi/well. Sixteen hours later, the cells are washed with Hanks' balanced salt solution (pH 7.4) containing 0.05% BSA and 20 mM HEPES (hereinafter simply referred to as a reaction buffer). To each well is added 500 μl of the reaction buffer containing the peptide of the present invention in the final concentration of 10 μM, or the peptide of the present invention in the final concentration of 10 μM and a test compound. After incubation at 37°C for 60 minutes, 400 μl of the reaction solution is charged in a scintillator and the amount of [3H] arachidonic acid metabolites released in the reaction solution is measured using a scintillation counter.

When the amount of [3H] arachidonic acid metabolites when 500 µl of the reaction buffer alone is added (neither the peptide of the present invention nor the test compound is added) is taken as 0% and the amount of [3H] arachidonic acid metabolites when the reaction buffer containing 10 µM of the peptide of the present invention is added (no test compound is added) is taken as 100%, the amount of [3H] arachidonic acid metabolites released where the test compound is added is calculated.

The compound showing the arachidonic acid metabolite-releasing activity of, e.g., 50% or less, can be selected as a candidate substance capable of competitive inhibition.

[5] When the cells wherein the protein of the present invention is expressed are stimulated by the peptide of the present invention, an intracellular Ca level increases. The compound that alters the binding property between the peptide of the present invention and the protein of the present invention can be screened by assaying the stimulating activity of the peptide of the present invention, utilizing this reaction.

Specifically, the compound that alters the binding property between the peptide of the present invention and the protein of the present invention is screened by assaying the intracellular calcium level increasing activities when the peptide of the present invention is brought in contact with the cells wherein the protein of the present invention is expressed and when the peptide of the present invention and a test compound are brought in contact with the cells wherein the protein of the present invention is expressed; and comparing the activities therebetween. The assay is carried out in accordance with methods publicly known.

In this method, the test compound that suppresses the intracellular calcium level increasing activity by the peptide of the present invention can be selected as a candidate substance capable of competitive inhibition.

On the other hand, an agonist can be screened as well, by assaying an increase in fluorescence intensity when a test compound alone is added.

A specific example of the screening method is described below.

The cells wherein the protein of the present invention is expressed are inoculated on a sterilized cover glass for microscopy. Two days after, the culture medium is replaced by HBSS in which 4 mM Fura-2 AM (Dojin Kagaku Kenkyusho) is suspended, followed by allowing to stand at room temperature for 2 hours and 30 minutes. After washing with HBSS, the cover glass is set on a cuvette and the peptide of the present invention or the peptide of the present invention and a test compound is/are added thereto. Using a fluorescence spectrophotometer, an increase in the ratio of fluorescence intensities at 505 nm is measured at the excited wavelengths of 340 nm and 380 nm, which are compared.

FLIPR (manufactured by Molecular Device Co.) may also be used. Fluo-3 AM (manufactured by Dojin Kagaku Kenkyusho) is added to a suspension of the cells wherein the protein of the present invention is expressed, thereby to take Fluo-3 AM into the cells. After the supernatant is washed several times through centrifugation and the cells are inoculated on a 96-well plate. After setting in the FLIPR device, the peptide of the present invention or the peptide of the present invention and a test compound is/are added thereto. Using a fluorescence spectrophotometer, an increase in the ratio of fluorescence intensities is measured and compared therebetween, as in the case of Fura-2.

Further in the cells wherein the protein of the present invention is expressed, when such a protein gene (e.g., aequorin, etc.) that emits light in association with an increase of intracellular Ca ions is previously co-expressed, the gene protein (e.g., aequorin, etc.) changes to Ca-bound aequorin by increasing the intracellular Ca ion level to emit light. Utilizing the light emission, the compound that alters the binding property between the peptide of the present invention and the protein of the present invention can be screened.

The cells wherein the protein of the present invention is expressed and the gene of protein capable of emitting light by increasing the intracellular Ca ions is co-expressed, are inoculated on a 96-well plate. The peptide of the present invention or the peptide of the present invention and a test compound is/are added thereto and using a fluorescence spectrophotometer, an increase in the ratio of fluorescence intensities is measured and compared therebetween, as described above.

The test compound that suppresses the increase in fluorescence intensity by the peptide of the present invention can be selected as a candidate substance capable of competitive inhibition.

A specific example of the screening method is described below.

The 24th clone of ETA (endothelin A receptor)-expressed CHO cells (hereinafter abbreviated as ETA24; see Journal of Pharmacology and Experimental Therapeutics, vol. 279, pp. 675-685, 1996) is used for control, and on an assay sample, the intracellular Ca ion level increasing activity is assayed in ZAQC-B1 cells (EXAMPLE 7 later described) and ETA24 cells using FLIPR (manufactured by Molecular Device, Inc.). Both ZAQC-B 1 cells and ETA24 cells are used after subculturing these cells in DMEM supplemented with ZAQC-B1 cells and ETA24 cells as well as 10% dialyzed fetal bovine serum (hereinafter abbreviated as d FBS). ZAQC-B1 and ETA24 cells are suspended in a medium (10% d FBS-DMEM), respectively, in 15 x 104 cells/ml. Using a dispenser, a 200 µl aliquot (3.0 x 104 cells/200 µl/well) of the suspension is inoculated on a 96-well plate for FLIPR (black plate clear bottom, Coster, Inc.), followed by incubation at 37°C overnight in a 5% CO2 incubator. The cells thus incubated were used (hereinafter referred to as the cell plate). Then, 20 ml of H/HBSS (Nissui Hanks 2 (9.8 g of Nissui Pharmaceutical Co., Ltd.), 0.35 g of sodium hydrogencarbonate, 4.77 g of HEPES; after adjusting the pH to 7.4 with a sodium hydroxide solution, the mixture is sterilized through a filter), 200 μl of 250 mM Probenecid and 200 μl of fetal bovine serum (FBS) are mixed. Furthermore, 2 vials (50 μg/vial) of Fluo 3-AM (Dojin Kagaku Kenkyusho) are dissolved in 40 μl of dimethylsulfoxide and 40 μl of 20% Pluronic acid (Molecular Probes, Inc.). The resulting solution is added to H/HBSS-Probenecid-FBS described above. After mixing them, the culture solution is removed and a 100 μl aliquot of the mixture is dispensed in each well of the cell plate using an eight-stranded pipette followed by incubation at 37°C for an hour in a 5% CO2 incubator (dye loading). Then, 150 μl of H/HBSS containing 2.5 mM Probenecid and 0.1% CHAPS is added to the assay sample (each fraction) for dilution, and the dilution is transferred to a 96-well plate (V-Bottom plate, Coster Inc.) (hereinafter referred to as the sample plate). After completion of the dye loading onto the cell plate, the cell plate is washed 4 times with a wash buffer, which is obtained by adding 2.5 mM Probenecid to H/HBSS, using a plate washer (Molecular Devices, Inc.) to leave 100 μl of the wash buffer after washing. The cell plate and the sample plate are set in FLIPR to perform assay (50 μl of a sample is transferred from the sample plate to the cell plate with FLIPR).

[6] When a receptor agonist is added to receptor-expressing cells, the level of intracellular inositol triphosphate increases. By utilizing the intracellular inositol triphosphate producing activity in the cells wherein the protein of the present invention is expressed, the compound that alters the binding property between the peptide of the present invention and the protein of the present invention can be screened.

Specifically, the compound that alters the binding property between the peptide of the present invention and the protein of the present invention is screened by assaying the inositol triphosphate producing activities in the presence of labeled inositol, when the peptide of the present invention is brought in contact with the cells wherein the protein of the present invention is expressed and when the peptide of the present invention and a test compound are brought in contact with the cells wherein the protein of the present invention is expressed; and comparing the activities therebetween. The assay is carried out in accordance with methods publicly known.

In this method, the test compound that suppresses the inositol triphosphate producing activity can be selected as a candidate substance capable of competitive inhibition.

On the other hand, an agonist may also be screened by contacting a test compound alone with the cells wherein the protein of the present invention is expressed and measuring the increase of inositol triphosphate production.

A specific example of the screening method is described below.

The cells wherein the protein of the present invention is expressed are inoculated on a 24-well plate and cultured for a day. Then, the cells are cultured for a day in medium supplemented with myo-[2-3H]inositol (2.5 μCi/well). The cells are thoroughly washed with radioactive inositol-free medium. After the peptide of the present invention or the peptide of the present invention and a test compound is/are added to the cells, 10% perchloric acid is added to terminate the reaction. The reaction mixture is neutralized with 1.5 M KOH and 60 mM HEPES solution and then passed through a column packed with 0.5 ml of AG1 x 8 resin (Bio-Rad). After washing with 5 mM sodium tetraborate (Na2B4O7) and 60 mM ammonium formate, the radioactivity eluted with 1M ammonium formate and 0.1M formic acid is assayed with a liquid scintillation counter. When the radioactivity without adding the peptide of the present invention is made 0% and the radioactivity when the peptide of the present invention is added, is made 100%, an effect of the test compound on the binding of the peptide of the present invention to the protein of the present invention is calculated.

The test compound that reduces the inositol triphosphate producing activity, e.g., to 50% or less, can be selected as a candidate substance capable of competitive inhibition.

[7] The compound that alters the binding property between the peptide of the present invention and the protein of the present invention can be screened by assaying the stimulating activity of the peptide of the present invention on the cells wherein the protein of the present invention is expressed, using a TRE-reporter gene vector.

A DNA containing TRE (TPA response element) is inserted into an upstream of the reporter gene in a vector to acquire a TRE-reporter gene vector. In the TRE-reporter gene vector-transfected cells wherein the protein of the present invention is expressed, stimulation accompanied by an increase of the intracellular Ca level induces expression of TRE-mediated reporter gene and production of the reporter gene product (protein) subsequent thereto. That is, by assaying the enzyme activity of the reporter gene protein, a change in the amount of calcium in the TRE-reporter gene vector-transfected cells can be detected.

Specifically, the compound that alters the binding property between the peptide of the present invention and the protein of the present invention is screened by assaying the activities of the reporter gene protein in the presence of labeled inositol, when the peptide of the present invention is brought in contact with the TRE-reporter gene vector-transfected cells wherein the protein of the present invention is expressed and when the peptide of the present invention and a test compound are brought in contact with the TRE-reporter gene vector-transfected cells wherein the protein of the present invention is expressed; and comparing the activities therebetween.

As the vector, there may be employed, e.g., PicaGene Basic Vector, PicaGene Enhancer Vector (Toyo Ink Mfg. Co., Ltd.), and the like. A DNA containing TRE is inserted into the multicloning site upstream the reporter gene, e.g., luciferase gene in the vector described above, which is made a TRE-reporter gene vector.

In this method, the test compound that suppresses the enzyme activity of reporter gene protein by the peptide of the present invention can be selected as a candidate substance capable of competitive inhibition.

On the other hand, an agonist can also be screened by contacting a test compound alone with the TRE-reporter gene vector-transfected cells wherein the protein of the present invention is expressed and measuring the increased amount of luminescence as in the peptide of the present invention.

Taking as an example the embodiment wherein luciferase is used as the reporter gene, a specific example of this screening method is described below.

The TRE-reporter gene (luciferase)-transfected cells wherein the protein of the present invention is expressed are inoculated on a 24-well plate in 5 × 103 cells/well followed by cultivation for 48 hours. After the cells are washed with Hanks' balanced salt solution (pH 7.4) containing 0.05% BSA and 20 mM HEPES, 10 nM of the peptide of the present invention or 10 nM of the peptide of the present invention and a test compound is/are added to the cells, followed by reacting at 37°C for 60 minutes. The cells are dissolved in a cell lysis agent for PicaGene (Toyo Ink Mfg. Co., Ltd.) and a luminescence substrate (Toyo Ink Mfg. Co., Ltd.) is added to the lysate. The luminescence by luciferase is measured by a luminometer, a liquid scintillation counter or a top counter. The amounts of luminescence by luciferase are measured when the peptide of the present invention is added and when 10 nM of the peptide of the present invention and a test compound are added, and compared therebetween.

The amount of luminescence by luciferase increases with elevation of intracellular calcium by the peptide of the present invention. The compound that suppresses the increase can be selected as a candidate substance capable of competitive inhibition.

As the reporter gene, there may be employed genes, e.g., alkaline phosphatase, chloramphenicol acetyltransferase, β-galactosidase, etc. The enzyme activities of these reporter gene proteins are assayed in accordance with methods publicly known, or by using assay kits commercially available. The alkaline phosphatase activity can be assayed by using, e.g., Lumi-Phos 530 manufactured by Wako Pure Chemical Industries, Ltd.; the chloramphenicol acetyltransferase activity using, e.g., FAST CAT chloramphenicol Acetyltransferase Assay Kit manufactured by Wako Pure Chemical Industries, Ltd.; and the β-galactosidase activity using, e.g., Aurora Gal-XE manufactured by Wako Pure Chemical Industries, Ltd.

[8] The cells wherein the protein of the present invention is expressed are stimulated by the peptide of the present invention to activate MAP kinase, which leads to cell growth. Utilizing this reaction, the compound that alters the binding property between the peptide of the present invention and the protein of the present invention can be screened by assaying the stimulating activity of the peptide of the present invention on the cells wherein the protein of the present invention is expressed.

Specifically, the compound that alters the binding property between the peptide of the present invention and the protein of the present invention is screened by assaying the cell growth, when the peptide of the present invention is brought in contact with the cells wherein the protein of the present invention is expressed and when the peptide of the present invention and a test compound are brought in contact with the cells wherein the protein of the present invention is expressed; and comparing the cell proliferation therebetween.

The growth of the cells wherein the protein of the present invention is expressed may be determined by assaying, e.g., MAP kinase activity, thymidine uptake activity, cell counts, etc.

As a specific example with respect to the MAP kinase activity, the peptide of the present invention or the peptide of the present invention and a test compound is/are added to the cells wherein the protein of the present invention is expressed, MAP kinase fractions are then obtained from cell lysates by immunoprecipitation using an anti-MAP kinase antibody, and MAP kinase activity is assayed by methods publicly known using, e.g., MAP Kinase Assay Kit manufactured by Wako Pure Chemical Industries, Ltd. and γ-[32P]-ATP. Comparison is made in the activity.

With respect to the thymidine uptake activity, the activity is assayed by inoculating the cells wherein the protein of the present invention is expressed on a 24-well plate, culturing, adding the peptide of the present invention or the peptide of the present invention and a test compound to the cells, further adding radioactively labeled thymidine (e.g., [methyl-3H]-thymidine, etc.), causing cell lysis and then counting the radioactivity of the thymidine taken up into the cells with a liquid scintillation counter. Comparison is made in the activity.

With respect to the cell counting, the cells wherein the protein of the present invention is expressed are inoculated on a 24-well plate and cultured, the peptide of the present invention or the peptide of the present invention and a test compound is/are added to the cells, and MTT (3-(4,5-dimethyl-2-thiazolyl)-2,5-diphenyl-2H-tetrazolium bromide) is further added thereto. After the cells are lysed with an aqueous isopropanol solution rendered acidic with hydrochloric acid, MTT formazan changed from MTT taken up into the cells is assayed by the absorption at 570 nm.

In this method, the test compound that suppresses the growth of the cells wherein the protein of the present invention is expressed can be selected as a candidate substance capable of competitive inhibition.

On the other hand, an agonist may also be screened by contacting a test compound alone with the cells wherein the protein of the present invention is expressed and assaying the cell growth activity as in the peptide of the present invention.

A specific example of the screening method utilizing the thymidine uptake activity is described below.

The cells wherein the protein of the present invention is expressed are inoculated on a 24-well plate in 5000 cells/well followed by incubation for one day. Next, the cells are incubated in a serum-free medium for 2 days to bring the cells under starvation. The peptide of the present invention or the peptide of the present invention and a test compound is/are added to the cells. After incubation for 24 hours, [methyl-3H] thymidine is added in 0.015 MBq/well, followed by incubation for 6 hours. After the cells are washed with PBS, methanol is added to the cells. The mixture is allowed to stand for 10 minutes. Next, 5% trichloroacetic acid is added and the mixture is allowed to stand for 15 minutes. The immobilized cells are washed 4 times with distilled water. After cell lysis with 0.3 N sodium hydroxide solution, the radioactivity in the lysate is assayed with a liquid scintillation counter.

The compound that suppresses an increase of the radioactivity when the peptide of the present invention is added, can be selected as a candidate substance capable of competitive inhibition.

[9] When the cells wherein the protein of the present invention is expressed are stimulated by the peptide of the present invention, the potassium channel is activated so that K ions present within the cells are effluxed extracellularly. Utilizing this reaction, the compound that alters the binding property between the peptide of the present invention and the protein of the present invention can be screened by assaying the stimulating activity of the peptide of the present invention on the cells wherein the protein of the present invention is expressed.

Rb ions (rubidium ions) in the related elements to K ions flow out of the cells through the potassium channel without being distinguished from K ions. Thus, radioactive isotope Rb ([$^{86}$Rb]) is previously incorporated in the cells wherein the protein of the present invention is expressed, and the efflux of $^{86}$Rb that flows out in response to stimulation by the peptide of the present invention (efflux activity) is determined thereby to assay the stimulating activity of the peptide of the present invention on the cells wherein the protein of the present invention is expressed.

Specifically, the compound that alters the binding property between the peptide of the present invention and the protein of the present invention is screened by assaying the $^{86}$Rb efflux activities in the presence of $^{86}$Rb, when the peptide of the present invention is brought in contact with the cells wherein the protein of the present invention is expressed and when the peptide of the present invention and a test compound are brought in contact with the cells wherein the protein of the present invention is expressed; and comparing the activities therebetween.

In this method, the test compound that suppresses the increase of the $^{86}$Rb efflux activity associated with stimulation by the peptide of the present invention can be selected as a candidate substance capable of competitive inhibition.

On the other hand, an agonist may also be screened by contacting a test compound alone with the cells wherein the protein of the present invention is expressed and measuring the increase of the $^{86}$Rb efflux activity as in the peptide of the present invention.

A specific example of the screening method is described below.

The cells wherein the protein of the present invention is expressed are inoculated on a 24-well plate and cultured for 2 days. Thereafter, the cells are kept warm for 2 hours in a medium containing 1 mCi/ml of $^{86}$RbCl. The medium is thoroughly washed to completely remove $^{86}$RbCl in the outer liquid. The peptide of the present invention or the peptide of the present invention and a test compound is/are added to the cells. After the outer liquid is recovered 30 minutes later, the radioactivity is measured with a γ counter and compared.

The test compound that suppresses the [$^{86}$Rb] efflux activity in association with stimulation by the peptide of the present invention can be selected as a candidate substance capable of competitive inhibition.

[10] In the cells wherein the protein of the present invention is expressed, the extracellular pH changes in response to the peptide of the present invention. Utilizing this reaction, the compound that alters the binding property between the peptide of the present invention and the protein of the present invention can be screened by assaying the stimulating activity of the peptide of the present invention on the cells wherein the protein of the present invention is expressed.

Specifically, the compound that alters the binding property between the peptide of the present invention and the protein of the present invention is screened by measuring changes in extracellular pH, when the peptide of the present invention is brought in contact with the cells wherein the protein of the present invention is expressed and when the peptide of the present invention and a test compound are brought in contact with the cells wherein the protein of the present invention is expressed; and comparing the changes therebetween.

The extracellular pH change is determined using, e.g., Cytosensor Device (Molecular Device, Inc.).

In this method, the test compound that suppresses the extracellular pH change by the peptide of the present invention can be selected as a candidate substance capable of competitive inhibition.

On the other hand, an agonist may also be screened by contacting a test compound alone with the cells wherein the protein of the present invention is expressed and measuring the extracellular pH change as in the peptide of the present invention.

A specific example of the screening method is described below.

The cells wherein the protein of the present invention is expressed are cultured overnight in a capsule for Cytosensor Device, which is set in a chamber of the device to reflux 0.1% BSA-containing RPMI 1640 medium (manufactured by Molecular Device, Inc.) until the extracellular pH becomes stable. After the pH becomes stable, a medium containing the peptide of the present invention or the peptide of the present invention and a test compound is refluxed onto the cells. The pH changes in the medium caused by reflux are measured and compared.

The compound that suppresses the extracellular pH change by the peptide of the present invention can be selected as a candidate substance capable of competitive inhibition.

[11] In Saccharomyces Cerevisiae, sex pheromone receptor STe2 of haploid α-mating type (MATα) is coupled to G protein Gpa1 to activate MAP kinase in response to sex pheromone α-mating factor, whereby Far1 (cell-cycle arrest) and transcription activator Ste12 are activated. Ste12 induces expression of a wide variety of genes (e.g., FUS 1 which participates in conjugation). On the other hand, regulator Sst2 functions to inhibit the foregoing process. In this system, an attempt has been made to construct the assay system for the reaction of a receptor agonist with a receptor, which involves preparing a receptor gene-transfected yeast, activating the intracellular signal transduction system in yeast by stimulation with the receptor agonist and using the resulting growth, etc. as an indicator (Trends in Biotechnology, vol. 15, pp. 487-494, 1997). Utilizing this receptor gene-transfected yeast system, the compound that alters the binding property between the peptide of the present invention and the protein of the present invention can be screened.

A specific example is described below.

In MATα yeast, the genes coding for Ste2 and Gpa1 are removed and instead, the gene for the protein of the present invention and the genes coding for Ste2 and Gpa1 are introduced. The Far-coding gene is previously removed to cause no cell-cycle arrest and the gene encoding Sst is removed to increase the sensitivity in response to the peptide of the present invention. Furthermore, FUS1-HIS3 gene, which is FUS1 ligated with histidine biosynthesis gene HIS3, is introduced. This genetic recombinant engineering may be carried out, e.g., by replacing the protein of the present invention for somatostatin receptor type 2 (SSTR2) gene, in the method described in Molecular and Cellular Biology, vol. 15, pp. 6188-6195, 1995.

The thus constructed transformant yeast is responsive to the peptide of the present invention with a high sensitivity so that MAP kinase is activated to cause synthesis of histidine biosynthesis enzyme. Thus, the transformant becomes capable of growing in a histidine-deficient medium.

Accordingly, the compound that alters the binding property between the peptide of the present invention and the protein of the present invention can be screened by culturing the aforesaid the protein of the present invention-expressed yeast (MATα yeast wherein Ste2 gene and Gpa1 gene are removed, the protein gene of the present invention and the Gpa-Gai2 fused protein-coding gene, Far gene and Sst gene are removed, and S1-HIS3 gene is transfected) in a histidine-deficient medium, contacting the peptide of the present invention or the peptide of the present invention and a test compound with the yeast, assaying the growth of the yeast, and comparing the growth therebetween.

In this method, the test compound that suppresses growth of the yeast can be selected as a candidate substance capable of competitive inhibition.

On the other hand, an agonist may also be screened by contacting a test compound alone with the aforesaid yeast wherein the protein of the present invention is expressed and assaying growth of the yeast as in the peptide of the present invention.

A specific example of the screening method is described below.

The aforesaid yeast wherein the protein of the present invention is expressed thus produced is cultured overnight in a complete synthesis liquid medium and then added to a histidine-free, dissolved agar medium in a concentration of 2 x 104 cells/ml, followed by inoculation on a square Petri dish of 9 x 9 cm. After the agar is solidified, a sterilized filter paper impregnated with the peptide of the present invention or the peptide of the present invention and a test compound is put on the agar surface, which is incubated at 30°C for 3 days. To determine the effect of the test compound, growth of yeast around the filter paper is compared to the case wherein the sterilized filter paper impregnated only with the peptide of the present invention peptide. Alternatively, the peptide of the present invention is previously added to a histidine-free agar medium, a sterilized filter paper is impregnated with a test compound alone, and the yeast is incubated, under which observation may be made that growth of the yeast over the entire surface of Petri dish is affected at the periphery of the filter paper.

The compound that suppresses growth of the yeast can be selected as a candidate substance capable of competitive inhibition.

[12] When the protein gene RNA of the present invention is injected into Xenopus laevis oocytes and stimulated by the peptide of the present invention, the intracellular Ca ion level increases to cause a calcium-activated chloride current, which can be taken as fluctuation in membrane potential (the same applies also to the case where fluctuation occurs in K ion level gradient). Utilizing the above reaction caused by the peptide of the present invention in Xenopus laevis oocytes wherein the protein of the present invention is transfected, the compound that alters the binding property between the peptide of the present invention and the protein of the present invention can be screened by assaying the stimulating activity of the peptide of the present invention on the cells wherein the protein of the present invention is expressed.

Specifically, the compound that alters the binding property between the peptide of the present invention and the protein of the present invention can be screened by assaying changes in cell membrane potential, when the peptide of the present invention is brought in contact with Xenopus laevis oocytes wherein the protein gene RNA of the present invention is transfected and when the peptide of the present invention and a test compound are brought in contact with Xenopus laevis oocytes wherein the protein gene RNA of the present invention is transfected; and comparing the changes therebetween.

In this method, the test compound that suppresses the changes in cell membrane potential can be selected as a candidate substance capable of competitive inhibition.

On the other hand, an agonist may also be screened by contacting a test compound alone with Xenopus laevis oocytes wherein the protein gene RNA of the present invention is transfected and assaying the changes in cell membrane potential as in the peptide of the present invention.

A specific example of the screening method is described below.

Female Xenopus laevis is anesthetized by immersing in ice water and anatomized to withdraw oocytes. The oocyte clusters are treated with collagenase (0.5 mg/ml) dissolved in an MBS solution (88 mM NaCl, 1 mM KCl, 0.41 mM $CaCl_2$, 0.33 mM $Ca(NO3)_2$, 0.82 mM $MgSO_4$, 2.4 mM $NaHCO_3$, 10 mM HEPES; pH 7.4) at 19°C for 1 to 6 hours at 150 rpm, until the oocytes are loosen. Washing is performed 3 times by replacing the outer liquid by the MBS solution followed by microinjection of the protein gene of the present invention or poly A-added cRNA (50 ng/50 nl) with a micromanipulator.

The protein gene mRNA of the present invention may be prepared from tissues or cells, or may be transcribed from plasmids in vitro. The oocytes are incubated in the MBS solution at 20°C for 3 days. The oocytes are placed in a hole of a voltage clamp device, which is continuously perfused with Ringer's solution, and impaled into the cells with glass microelectrodes for voltage clamp and glass microelectrodes for potential recording, in which (-) electrode is placed outside the oocytes. When the holding potential stabilizes, Ringer's solution containing the peptide of the present invention or the peptide of the present invention and a test compound is perfused to record a change in potential. An effect of the compound can be determined by comparing a change in cell membrane potential of the Xenopus laevis oocytes wherein the protein gene RNA of the present invention is transfected, with the case wherein Ringer's solution containing the peptide of the present invention alone is perfused.

The compound that suppresses the change in cell membrane potential can be selected as a candidate substance capable of competitive inhibition.

In the system described above, since the assay becomes easy when the variation in potential increases, poly A-added RNA of various G protein genes may be introduced. Also, the amount of luminescence, not a change in membrane potential, may be assayed by co-injecting poly A-added RNA of a gene for such a protein (e.g., aequorin, etc.) that emits light in the presence of Ca.

The kit for screening the compound or its salt that alters the binding property between the peptide of the present invention and the protein of the present invention comprises the protein of the present invention, cells containing the protein of the present invention or cell membrane fractions containing the protein of the present invention, as well as the peptide of the present invention.

Examples of the screening kits of the present invention are as follow.

1. Reagents for screening

(i) Assay buffer and wash buffer

Hanks' balanced salt solution (manufactured by Gibco Co.) supplemented with 0.05% bovine serum albumin (manufactured by Sigma Co.).

The solution is sterilized by filtration through a 0.45 µm filter, and stored at 4°C or may be prepared at use.

(ii) Protein preparation of the present invention

CHO cells wherein the protein of the present invention is expressed are subcultured on a 12-well plate at a density of 5 × 105 cells/well and cultured at 37°C under 5% $CO_2$ and 95% air for 2 days.

(iii) Labeled ligand

The peptide of the present invention labeled with [$^3$H], [$^{125}$I], [$^{14}$C], [$^{35}$S], or the like. A solution of the peptide dissolved in an appropriate solvent or buffer is stored at 4°C or -20°C and upon use, diluted to 1 µM with the assay buffer.

(iv) Ligand reference solution

The peptide of the present invention is dissolved in PBS containing 0.1% bovine serum albumin (manufactured by Sigma Co.) in a volume of 1 mM, and the solution is stored at -20°C.

2. Assay method

(i) The cells wherein the protein of the present invention is expressed are cultured on a 12-well culture plate. After washing twice with 1 ml of the assay buffer, 490 µl of the assay buffer is added to each well.
(ii) After 5 µl of a solution of test compound in 10-3 to 10-10 M is added, 5 µl of a labeled form of the peptide of the present invention is added thereto. The reaction is carried out at room temperature for an hour. To examine the non-specific binding, 5 µl of the peptide of the present invention of 10-3 M is previously added in place of the test compound.
(iii) The reaction solution is removed and the wells are washed 3 times with 1 ml of the wash buffer. The labeled peptide of the present invention bound to the cells is dissolved in 0.2N NaOH-1% SDS, and mixed with 4 ml of liquid scintillator A (manufactured by Wako Pure Chemical Industries, Ltd.).
(iv) The radioactivity is measured using a liquid scintillation counter (manufactured by Beckman Co.), and the percent maximum binding (PMB) is calculated in accordance with the following equation.

$$PMB = [(B - NSB)/(B_0 - NSB)] \times 100$$

| | |
|---|---|
| PMB | Percent maximum binding |
| B | Value obtained in the presence of a test compound |
| NSB | Non-specific binding |
| B0 | Maximum binding |

The compound or its salt, which is obtained using the screening methods or the screening kits of the present invention, is a compound that alters the binding (inhibits or promotes the binding) of the peptide of the present invention to the protein of the present invention, and specifically, is a compound or its salt that has the cell stimulating activity mediated by the protein of the present invention (a so-called an agonist to the protein of the present invention); or a compound that does not have the stimulating activity such as the receptor-mediated cell stimulating activity (a so-called an antagonist to the protein of the present invention). The compounds may be peptides, proteins, non-peptide compounds, synthetic compounds, fermentation products, etc. These compounds may be novel or publicly known compounds.

To specifically determine if the compounds are agonists or antagonists to the protein of the present invention described above, the following (A) or (B) is available.

(A) The binding assay recited in the screening methods supra is performed to obtain the compound that alters the binding property between the peptide of the present invention and the protein of the present invention (especially inhibits the binding) followed by assay for the compound to determine if the compound has the cell stimulating activity mediated by the protein of the present invention as described above. The compound or its salts having the cell stimulating activity are agonists to the protein of the present invention, whereas the compound or its salts having no such activity are antagonists to the protein of the present invention.
(B) (a) A test compound is brought in contact with cells containing the protein of the present invention to assay the cell stimulating activity mediated by the protein of the present invention. The compound or its salts having the cell stimulating activity are agonists to the protein of the present invention.
(b) The cell stimulating activity mediated by the protein of the present invention is assayed in the case that a compound that activates the protein of the present invention is brought in contact with the cells containing the protein of the present invention and in the case that a compound that activates the protein of the present invention and a test compound are brought in contact with the cells containing the protein of the present invention, and comparison is made on the cell stimulating activity between the cases. The compound or its salts capable of reducing the cell stimulating activity by the compound that activates the protein of the present invention are antagonists to the protein of the present invention.

The agonists to the protein of the present invention exhibit activities similar to the physiological activities that the peptide of the present invention has, and are thus useful as safe and low toxic drugs as in the peptide of the present invention.

To the contrary, the antagonists to the protein of the present invention can suppress the physiological activities that the peptide of the present invention has, and are useful as safe and low toxic drugs for suppressing the receptor activity.

As an antagonist of the present invention, for example, a compound which alters the binding property between the peptide or MIT1 of the present invention and the protein of the present invention, preferably,

(i) a compound or the salt thereof which inhibits the activity of the peptide of the present invention,
(ii) a compound or the salt thereof which inhibits the activity of the protein of the present invention,
(iii) a compound or the salt thereof which inhibits binding of the peptide or MIT1 of the present invention to the protein of the present invention,
(iv) a compound or the salt thereof which inhibits an activated action of the peptide or MIT1 of the present invention the protein of the present invention.

Since the peptide of the present invention has activities of the growth of the vessel endothelial cell, and the like, foe example, the antagonist of the present invention is useful as pharmaceuticals of the vessel development agent.

Also, the antagonists to the protein of the present invention can be used as therapeutic/preventive agents, or the like, for diseases (e.g.,cancers (e.g., pancreas cancer, lung cancer, kidney cancer, liver cancer, non-small-cell lung cancer, ovarian cancer, prostate cancer, stomach cancer, urinary bladder cancer, breast cancer, endocervix cancer, colon cancer, rectum cancer, Kaposi's sarcoma, or the like), ovarian diseases (e.g., polycystic ovarian syndrome, ovarial hyperstimulation syndrome, or the like), inflammatory diseases (e.g., articular rheumatism, or the like), diabatic retinopathy, or the like).

As the salts of compound obtained by using the screening methods or screening kits described above, there may be employed, for example, pharmaceutically acceptable salts. Examples of the salts include salts with inorganic bases, salts with organic bases, salts with inorganic acids, salts with organic acids, salts with basic or acidic amino acids, and the like.

Preferred examples of the salts with inorganic bases include alkali metal salts such as sodium salts, potassium salts, etc.; alkaline earth metal salts such as calcium salts, magnesium salts, etc.; aluminum salts, ammonium salts, and the like.

Preferred examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc.

Preferred examples of the salts with inorganic acids include salts with hydrochloric acid, hydrobromic acid, sulfuric acid, phosphoric acid, etc.

Preferred examples of the salts with organic acids include salts with formic acid, acetic acid, propionic acid, fumaric acid, oxalic acid, tartaric acid, maleic acid, citric acid, succinic acid, malic acid, methanesulfonic acid, benzenesulfonic acid, benzoic acid, etc.

Preferred examples of the salts with basic amino acids include salts with arginine, lysine, ornithine, etc. Preferred examples of the salts with acidic amino acids include salts with aspartic acid, glutamic acid, etc.

Where the compound or its salts obtained by the screening methods or kits of the present invention are used as the pharmaceuticals described above, such can be performed as in the case where the aforesaid peptide of the present invention is provided.

When the compound or its salts obtained by the screening methods or kits of the present invention are used as the above-mentioned pharmaceuticals, they can be formulated in a conventional manner. For example, they can be administered orally as tablets coated with sugar or with enteric coating if necessary, capsules, elixirs, microcapsules, etc., or parenterally in the form of injections such as sterile solutions or suspensions in water or in pharmaceutically acceptable solutions other than water. For example, the compound or its salts can be mixed with carriers, flavoring agents, excipients, vehicles, preservatives, stabilizers, binders, etc. in a unit dosage form generally accepted. The amount of active ingredients in these preparations is adjusted so as to obtain appropriate doses within specified ranges.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, a flavoring agent such as peppermint, akamono oil and cherry, etc. When the unit dosage is in the form of a capsule, liquid carriers such as oils and fats may further be used together with the additives described above. A sterile composition for injection may be formulated in a conventional manner used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc.), etc. and may be used in combination with an appropriate dissolution aid such as an alcohol (e.g., ethanol), a polyalcohol (e.g., propylene glycol and polyethylene glycol), a nonionic surfactant (e.g., polysorbate 80™ and HCO-50), etc. Examples of the oily medium include sesame oil, soybean oil, etc., which may also be used in combination with a dissolution aid such as benzyl benzoate, benzyl alcohol, etc.

These compounds may further be formulated together with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

Since the pharmaceutical preparation thus obtained is safe and low toxic, it can be administered to a mammal (e.g., human, mouse, rat, guinea pig, rabbit, sheep, swine, bovine, cat, dog, monkey, chimpanzee, etc.).

The dose of the compound or its salts obtained using the screening methods or screening kits of the present invention varies depending on conditions, etc.; for example, in oral administration, the dose is normally about 0.1 to about 1000 mg, preferably about 1.0 to about 300 mg, more preferably about 3.0 to about 50 mg per day for adult (as 60 kg body weight). In parenteral administration, the single dose varies depending on subject to be administered, target organ, conditions, method for administration, etc.; e.g., in the form of an injection, it is advantageous to administer intravenously at a daily dose of about 0.01 to about 30 mg, preferably about 0.1 to about 20 mg, more preferably about 0.1 to about 10 mg, to adult patient (as 60 kg body weight) with a digestive disease. For other animal species, the corresponding dose as converted per 60 kg weight can be administered.

The compound or the salt thereof which is obtained by using screening method of the present invention or the screening kit of the present invention may be used combination with another agent, for example, chemical therapy agents (e.g, Ifosfamide, Adriamycin, Peplomycin, Cisplatin, Cyclophosphamide, 5-FU, UFT, Methorexate, Mitomycin C, Mitoxantrone), and the like.

(6) The screening method of angiogenesis inhibitor using polynucleotide encoding the peptide of the present invention, encoding the protein of the present invention

Specifically, for example, (a) a case in which cells having an ability of produce the peptide of the present invention or the protein of the present invention are cultured, (b) a case in which the mixture of cells having an ability of produce the peptide of the present invention or the protein of the present invention and test compound are cultured, and comparison is made on the cell stimulating activity between the cases. The screening of the angiogenesis inhibitor is carried out.

In the above screening method, for example, in the case between (a) and (b), the expression amount of the gene of the peptide of the present invention or the protein of the present invention is measured and compared.

Examples of the test compound include peptides, proteins, non-peptide compounds, synthetic compounds, fermentation products, cell extracts, plant extracts, animal tissue extracts, and the like.

For carrying out the screening method of the present invention, cells which have an ability of producing the peptide of the present invention or the protein of the present invention is suspended into the adequate buffer and prepared. As a buffer, phosphorylated buffer or borate buffer at pH about 4 to 10 (preferably, pH about 6 to 8) is used.

As cells which have an ability of producing the peptide of the present invention or the protein of the present invention, for example, host which is transformed by a vector containing DNA encoding the peptide of resent invention or the protein of the present invention (transformant) is used. As a host, for example, animal cell such as CHO cell is preferably used. In the screening, for example, transformant which is expressed the peptide of the present invention or the protein of the present invention by culturing with the above method.

According to the measurement of the peptide of the present invention or the protein of the present invention, the above protein which is present in extract of cells can be measured with accordance with Western blotting, ELISA method, or the like by known method, for example, using antibody which recognizes the peptide of the present invention or the protein of the present invention.

The amount of the gene expression of the peptide of the present invention or the protein of the present invention can be measured by known method, for example, Northern blotting, Reverse transcription-polymerase chain reaction (RT-PCR), real time PCT (manufactured by ABI, TaqMan polymerase chain reaction) or the like.

For example, test compound can be selected as an angiogenesis inhibitor which can inhibit the amount of the gene expression of the peptide of the present invention or the protein of the present invention more than at least about 20%, preferably at least 30%, more preferably at least about 50% in comparison with the case of above (a).

(7) The screening method of angiogenesis inhibitor using an antibody against the peptide of the present invention, MIT or the protein of the present invention

Specifically, for example, (a) a case in which cells having an ability of produce the peptide or MIT1 of the present invention or the protein of the present invention are cultured, (b) a case in which the mixture of cells having an ability of produce the peptide or MIT1 of the present invention or the protein of the present invention and test compound are cultured, and comparison is made on the cell stimulating activity between the cases by using an antibody against the peptide or MIT1 of the present invention or the protein of the present invention. The screening of the angiogenesis inhibitor is carried out.
In the above screening method, for example, in the case between (a) and (b), the expression amount of the gene of the peptide or MIT1 of the present invention or the protein of the present invention (specifically, protein mass) is measured and compared by using above antibody.
Examples of the test compound include peptides, proteins, non-peptide compounds, synthetic compounds, fermentation products, cell extracts, plant extracts, animal tissue extracts, and the like.
For carrying out the screening method of the present invention, cells which have an ability of producing the peptide or MIT1 of the present invention or the protein of the present invention is suspended into the adequate buffer and prepared. As a buffer, phosphorylated buffer or borate buffer at pH about 4 to 10 (preferably, pH about 6 to 8) is used.
As cells which have an ability of producing the peptide or MIT1 of the present invention or the protein of the present invention, for example, host which is transformed by a vector containing DNA encoding the peptide or MIT1 of resent invention or the protein of the present invention (transformant) is used. As a host, for example, animal cell such as CHO cell is preferably used. In the screening, for example, transformant which is expressed the peptide or MIT1 of the present invention or the protein of the present invention by culturing with the above method.
For example, test compound can be selected as an angiogenesis inhibitor which can inhibit the amount of the gene expression of the peptide or MIT1 of the present invention or the protein of the present invention more than at least about 20%, preferably at least 30%, more preferably at least about 50% in comparison with the case of above (a).

(8) Angiogenesis inhibitor containing the compound having inhibiting activity of endothelial cell proliferation or the salt thereof

A compound having inhibiting activity of endothelial cell proliferation or the salt thereof may be any compounds or the salt thereof which have inhibiting activity of the endothelial cell proliferation or the salt thereof.
The compound or the salt thereof is used as an angiogenesis inhibitor, for example, is safely used as a pharmaceuticals for the treatment/prevention of the diseases associated with angiogenesis (e.g., cancers (e.g., pancreas cancer, lung cancer, kidney cancer, liver cancer, non-small-cell lung cancer, ovarian cancer, prostate cancer, stomach cancer, urinary bladder cancer, breast cancer, endocervix cancer, colon cancer, rectum cancer, Kaposi's sarcoma, or the like), ovarian diseases (e.g., polycystic ovarian syndrome, ovarial hyperstimulation syndrome, or the like), inflammatory diseases (e.g., articular rheumatism, or the like), diabatic retinopathy, or the like).
When using such as a pharmaceutical, it is carried out as well as the compound which obtained by using screening method of the present invention or the screening kit of the present invention described above as a pharmaceutical.

(9) Quantification of the protein or the peptide of the present invention

The antibody of the present invention can specifically recognize the protein of the present invention. Therefore, the antibody can be used to quantify the protein or the peptide of the present invention (hereinafter, abbreviated as the protein of the present invention) in a test fluid, especially for quantification by the sandwich immunoassay, etc.
That is, the present invention provides, for example, the following methods of quantification:

(i) A method of quantifying the protein of the present invention in a test fluid, which comprises competitively reacting the antibody of the present invention with the test fluid and a labeled form of the protein of the present invention, and measuring the ratio of the labeled protein of the present invention bound to the antibody; and,
(ii) A method of quantifying the protein of the present invention in a test fluid, which comprises reacting the test fluid with the antibody of the present invention immobilized on a carrier and a labeled form of the antibody of the present invention simultaneously or sequentially, and measuring the activity of the label on the immobilized carrier.

Using a monoclonal antibody to the protein of the present invention (hereinafter sometimes referred to as the monoclonal antibody of the present invention), the protein of the present invention can be assayed and can further be detected by tissue staining or the like. For these purposes, the antibody molecule itself may be used, or F(ab')2, Fab' or Fab fractions of the antibody molecule may be used as well.

The methods of quantifying assay the protein of the present invention using the antibody of the present invention are not to be limited particularly. Any method can be used, so long as the amount of antibody, antigen, or antibody-antigen complex in response to the amount of antigen (e.g., the amount of the protein of the present invention) in a test fluid can be detected by chemical or physical means and can be calculated from a standard curve prepared from standard solutions containing known amounts of the antigen. For example, nephrometry, competitive method, immunometric method, and sandwich method are advantageously used, among which the sandwich method described below is particularly preferable in terms of sensitivity and specificity.

As labeling agents for the methods using labeled substances, there are employed, for example, radioisotopes, enzymes, fluorescent substances, luminescent substances, etc. As the radioisotopes, there are employed, for example, [$^{125}$I], [$^{131}$I], [$^{3}$H], [$^{14}$C], etc. As the enzymes described above, stable enzymes with a high specific activity are preferred; for example, β-galactosidase, β-glucosidase, alkaline phosphatase, peroxidase, malate dehydrogenase, and the like are used. Examples of the fluorescent substance used are fluorescamine, fluorescein isothiocyanate and the like. As the luminescent substances, there are employed, for example, luminol, luminol derivatives, luciferin, lucigenin and the like. Furthermore, the biotin-avidin system may also be used for binding an antibody or antigen to the label.

For immobilization of the antigen or antibody, physical adsorption may be used. Chemical binding methods conventionally used for insolubilization or immobilization of peptides, enzymes, etc. may be used as well. For the carriers, examples include insoluble polysaccharides such as agarose, dextran, cellulose, etc.; synthetic resin such as polystyrene, polyacrylamide, silicone, etc., or glass, etc.

In the sandwich method, the immobilized monoclonal antibody of the present invention is reacted with a test fluid (primary reaction), then with a labeled form of another monoclonal antibody of the present invention (secondary reaction), and the activity of the labeling agent on the immobilizing carrier is assayed, whereby the amount of the protein of the present invention in the test fluid can be quantified. The order of the primary and secondary reactions may be reversed, and the reactions may be performed simultaneously or with some time intervals. The methods of labeling and immobilization can be performed by modifications of those methods described above. In the immunoassay by the sandwich method, the antibody used for immobilized or labeled antibody is not necessarily from one species, but a mixture of two or more species of antibodies may be used to increase the measurement sensitivity.

In the methods of assaying the protein of the present invention by the sandwich method, antibodies that bind to different sites of the protein of the present invention are preferably used as the monoclonal antibodies of the present invention for the primary and secondary reactions. That is, in the antibodies used for the primary and secondary reactions, for example, when the antibody used in the secondary reaction recognizes the C-terminal region of the protein of the present invention, it is preferable to use the antibody capable of recognizing the region other than the C-terminal region for the primary reaction, e.g., the antibody capable of recognizing the N-terminal region.

The monoclonal antibody of the present invention can be used for the assay systems other than the sandwich method, for example, the competitive method, immunometric method, nephrometry, etc.

In the competitive method, an antigen in a test fluid and a labeled antigen are competitively reacted with an antibody, and the unreacted labeled antigen (F) and the labeled antigen bound to the antibody (B) are separated (B/F separation). The amount of the labeling agent in B or F is measured, and the amount of the antigen in the test fluid is quantified. This reaction method includes a liquid phase method using a soluble antibody as an antibody, polyethylene glycol for B/F separation and a secondary antibody, etc. to the soluble antibody, and an immobilized method either using an immobilized antibody as the primary antibody, or using a soluble antibody as the primary antibody and an immobilized antibody as the secondary antibody.

In the immunometric method, an antigen in a test fluid and an immobilized antigen are competitively reacted with a definite amount of labeled antibody, the immobilized phase is separated from the liquid phase, or an antigen in a test fluid is reacted with an excess amount of labeled antibody, the immobilized antigen is then added to bind the unreacted labeled antibody to the immobilized phase, and the immobilized phase is separated from the liquid phase. Then, the amount of the labeling agent in either phase is measured to quantify the antigen in the test fluid.

In the nephrometry, insoluble precipitates produced after the antigen-antibody reaction in gel or solution, are quantified. Even when the amount of an antigen in a test fluid is small and only a small amount of precipitates is obtained, laser nephrometry using scattering of laser can be advantageously employed.

For applying these immunological assay methods to the quantification methods of the present invention, any particular conditions or procedures are not required. The assay systems for the protein of the present invention may be constructed by adding ordinary technical consideration in the art to conventional conditions and procedures in the respective methods. For the details of these general technical means, reference can be made to the following reviews and texts.

For example, reference can be made on Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immonoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing), etc.

As described above, the protein of the present invention can be quantified with high sensitivity, by using the antibody of the present invention.

Furthermore, since the protein of the present invention highly expresses in capillary endothelial cell such as ovary or spermary, by quantifying the level of the protein of the present invention using the antibody of the present invention, when an increased level of the protein of the present invention is detected, it can be diagnosed that one suffers from the diseases which associated with the proliferation of the capillary endothelial cell in these organisms, for example, cancers (e.g., pancreas cancer, lung cancer, kidney cancer, liver cancer, non-small-cell lung cancer, ovarian cancer, prostate cancer, stomach cancer, urinary bladder cancer, breast cancer, endocervix cancer, colon cancer, rectum cancer, Kaposi's sarcoma, or the like), ovarian diseases (e.g., polycystic ovarian syndrome, ovarial hyperstimulation syndrome, or the like), inflammatory diseases (e.g., articular rheumatism, or the like), diabatic retinopathy, or the like or it is highly likely that one would suffer from these disease in the future.

Besides, the antibody of the present invention may be used for detecting the protein of the present invention present in test samples such as body fluids, tissues, etc. The antibody may also be used for preparation of antibody columns used to purify the protein of the present invention, for detection of the protein of the present invention in each fraction upon purification, for analysis of the behavior of the protein of the present invention in test cells; etc.

(10) Gene diagnostic agent

By using the DNA of the present invention, e.g., as a probe, an abnormality (gene abnormality) of the DNA or mRNA encoding the peptide of the present invention in human or other warm-blooded animal (e.g., rat, mouse, guinea pig, rabbit, chicken, sheep, swine, bovine, horse, cat, dog, monkey, etc.) can be detected. Therefore, the DNA of the present invention is useful as a gene diagnostic agent for damages to the DNA or mRNA, its mutation or decreased expression, or increased expression or overexpression of the DNA or mRNA.

The gene diagnosis described above using the DNA of the present invention can be performed by, for example, publicly known Northern hybridization or PCR-SSCP assay (Genomics, 5, 874-879 (1989); Proceedings of the National Academy of Sciences of the United States of America, 86, 2766-2770 (1989)), DNA microarray, etc.

For example, when increased expression is detected by northern hybridization or DNA microarray, or when DNA mutation is detected by PCR-SS or DNA microarray, it can be diagnosed that, for example, diseases associated with the proliferation of the capillary vessel endothelial cell, for example, cancers (e.g., pancreas cancer, lung cancer, kidney cancer, liver cancer, non-small-cell lung cancer, ovarian cancer, prostate cancer, stomach cancer, urinary bladder cancer, breast cancer, endocervix cancer, colon cancer, rectum cancer, Kaposi's sarcoma, or the like), ovarian diseases (e.g., polycystic ovarian syndrome, ovarial hyperstimulation syndrome, or the like), inflammatory diseases (e.g., articular rheumatism, or the like), diabatic retinopathy, or the like are involved or it is highly likely to suffer in the future from these diseases.

(11) Pharmaceuticals comprising anti-sense DNA

Anti-sense DNA that complementally binds to the DNA of the present invention and can suppress the DNA expression can suppress the functions of the peptide or the protein of the present invention (hereinafter, abbreviated as the protein of the present invention) or the DNA of the present invention in vivo, and can be used as the agent for the treatment/prevention of diseases associated with, e.g., overexpression of the protein of the present invention (for example, digestive diseases (e.g., enteritis, diarrhea, constipation, malabsorption syndrome, etc.)).

The anti-sense DNA described above may be employed as the above therapeutic/prophylactic agents similarly to the therapeutic/prophylactic agent for various diseases comprising the DNA of the present invention described above.

For example, the anti-sense DNA is administered solely, or the anti-sense DNA is inserted into an appropriate vector such as retrovirus vector, adenovirus vector, adenovirus-associated virus vector, etc., which is then administered in a conventional manner. The anti-sense DNA may be administered as it stands, or may be prepared into a dosage form together with a physiologically acceptable carrier to increase its uptake and administered by gene gun or through a catheter such as a catheter with a hydrogel.

In addition, the anti-sense DNA may also be employed as an oligonucleotide probe for diagnosis to examine the presence of the DNA of the present invention in tissues or cells and the conditions of its expression.

The present invention further provides:

(i) A double-stranded RNA containing a part of the RNA encoding the protein of the present invention;
(ii) A pharmaceutical comprising the double-stranded RNA;
(iii) A ribozyme containing a part of the RNA encoding the protein of the present invention; and,
(iv) A pharmaceutical comprising the ribozyme.

As in the anti-sense nucleotide described above, the double-stranded RNA (RNAi; RNA interference method), ribozyme, etc. can suppress in vivo the expression of polynucleotide (e.g., DNA) encoding the protein of the present invention and the functions of the protein or DNA of the present invention and thus, can be used as preventive/therapeutic agents for diseases, for example, cancers (e.g., pancreas cancer, lung cancer, kidney cancer, liver cancer, non-small-cell lung cancer, ovarian cancer, prostate cancer, stomach cancer, urinary bladder cancer, breast cancer, endocervix cancer, colon cancer, rectum cancer, Kaposi's sarcoma, or the like), ovarian diseases (e.g., polycystic ovarian syndrome, ovarial hyperstimulation syndrome, or the like), inflammatory diseases (e.g., articular rheumatism, or the like), diabatic retinopathy, or the like.
The double-stranded RNA can be manufactured by designing the same based on the sequence of the polynucleotide of the present invention, by a modification of publicly known methods (e.g., Nature, 411, 494, 2001).
The ribozyme can be manufactured by designing the same based on the sequence of the polynucleotide of the present invention, by a modification of publicly known methods (e.g., TRENDS in Molecular Medicine, 7, 221, 2001). For example, the ribozyme designing the same based on the sequence of the polynucleotide of the present invention, can be manufactured by ligating a publicly known ribozyme to a part of the RNA encoding the peptide of the present invention. The part of the RNA encoding the peptide of the present invention includes a contiguous part (RNA fragment) to the cleavage site on the RNA of the present invention, which can be cleaved by a publicly known ribozyme.
When the double-stranded RNA or ribozyme described above is used as the prophylactic/therapeutic agent described above, the RNA or ribozyme may be prepared into pharmaceutical preparations, as in the anti-sense polynucleotide, which is provided for administration.

(12) Pharmaceuticals and diagnostic agents comprising the antibody of the present invention

The antibody of the present invention which possesses the effect of neutralizing the activities of the peptide or the protein of the present invention (hereinafter, abbreviated as the protein of the present invention) can be used as pharmaceuticals for the treatment/prevention of diseases associated with, e.g., overexpression of the protein of the present invention, for example, cancers (e.g., pancreas cancer, lung cancer, kidney cancer, liver cancer, non-small-cell lung cancer, ovarian cancer, prostate cancer, stomach cancer, urinary bladder cancer, breast cancer, endocervix cancer, colon cancer, rectum cancer, Kaposi's sarcoma, or the like), ovarian diseases (e.g., polycystic ovarian syndrome, ovarial hyperstimulation syndrome, or the like), inflammatory diseases (e.g., articular rheumatism, or the like), diabatic retinopathy, or the like, or as diagnostic agents of diseases associated with proliferating endothelial cell overexpressing the protein of the present invention, for example, polycystic ovarian syndrome, ovarial hyperstimulation syndrome, or the like.
The therapeutic/prophylactic agent for the treatment/prevention of the diseases described above, which contains the antibody of the present invention, may be administered orally or parenterally to human or mammals (e.g., rat, rabbit, sheep, swine, bovine, cat, dog, monkey, etc.) as a liquid preparation in its original form, or as a pharmaceutical composition in an appropriate drug form. The dose varies depending on subject to be administered, target disease, conditions, route for administration, etc.; for example, when it is used for the treatment/prevention of digestive diseases, the antibody of the present invention is intravenously administered to adult normally in a single dose of about 0.01 mg to about 20 mg/kg body weight, preferably about 0.1. to about 10 mg/kg body weight, and more preferably about 0.1 to about 5 mg per day, once to about 5 times a day, preferably once to about 3 times. In parenteral administration of other routes as well as in oral administration, a dose similar to those given above can be administered. Where conditions are particularly severe, the dose may be increased depending on the conditions.
The antibody of the present invention can be administered in itself or as an appropriate pharmaceutical composition. The pharmaceutical composition used for the administration described above contains a carrier, a diluent or excipient, which is pharmaceutically acceptable with the above-mentioned antibody or salts thereof. Such a composition is provided in the preparation suitable for oral or parenteral administration.
That is, examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.
As the composition for parenteral administration, there are employed, for example, injections, suppositories, etc. and the injections include the form of intravenous, subcutaneous, transcutaneous, intramuscular, drip injections, etc. Such injections are prepared by publicly known methods, e.g., by dissolving, suspending or emulsifying the aforesaid antibody or its salts in a sterile aqueous or oily liquid medium. Examples of the aqueous medium for injection used include physiological saline, isotonic solutions containing glucose and other adjuvant, etc. Appropriate dissolution aids, for example, alcohol (e.g., ethanol), polyalcohol (e.g., propylene glycol, polyethylene glycol), nonionic surfactant [e.g., polysorbate 80TM, HCO-50 (polyoxyethylene (50 mol) adduct of hydrogenated castor oil)] may be used in combination. For the oily solution, for example, sesame oil, soybean oil and the like are used, and dissolution aids such as benzyl benzoate and benzyl alcohol may be used in combination. The thus-prepared liquid for injection is normally filled in an appropriate ampoule. The suppository used for rectal administration is prepared by mixing the aforesaid antibody or its salts with conventional suppository base.

The oral or parenteral pharmaceutical composition described above is advantageously prepared in a unit dosage form suitable for the dose of the active ingredient. Examples of such unit dosage form include tablets, pills, capsules, injections (ampoules), suppositories, etc. It is preferred that the antibody described above is contained generally in a dose of about 5 to about 500 mg per unit dosage form, about 5 to about 100 mg especially for injections and about 10 to about 250 mg for other preparations.

Each composition described above may further contain other active components, unless their formulation with the antibody causes any adverse interaction.

For example, each angiogenesis inhibitor described above, may be used combination with, for example, chemical therapy agents (e.g, Ifosfamide, Adriamycin, Peplomycin, Cisplatin, Cyclophosphamide, 5-FU, UFT, Methorexate, Mitomycin C, Mitoxantrone), and the like.

(13) Preparation of non-human animal having the DNA of the present invention

Transgenic non-human animal capable of expressing the peptide or protein of the present invention can be prepared using the DNA of the present invention. Examples of the non-human animal include mammals (e.g., rats, mice, rabbits, sheep, swine, bovine, cats, dogs, monkeys, etc.) and the like (hereinafter merely referred to as animal). In particular, mice, rabbits and the like are preferable.

To transfect the DNA of the present invention into a target animal, it is generally advantageous to employ the DNA as a gene construct ligated downstream the promoter capable of expressing the DNA in an animal cell. For example, when the rabbit-derived DNA of the present invention is transfected, the gene construct, in which the DNA is ligated downstream various promoters capable of expressing the DNA of the present invention derived from an animal that is highly homologous to the DNA of the present invention, is microinjected to, e.g., rabbit fertilized ova. Thus, the DNA-transfected animal capable of producing a high level of the peptide, or the protein of the present invention can be prepared. As the promoters, there may be used, e.g., a virus-derived promoter and a ubiquitous expression promoter such as metallothionein, etc. Preferably, NGF gene promoters, enolase gene promoter, etc., which are specifically expressed in the brain, are used.

The transfection of the DNA of the present invention to the fertilized egg cell stage secures the presence of the DNA in all germ and somatic cells in the target animal. The presence of the peptide or the protein of the present invention in the germ cells in the DNA-transfected animal means that all germ and somatic cells contain the receptor protein of the present invention in all progenies of the animal. The progenies of the animal that took over the gene contain the peptide or the protein of the present invention in all germ and somatic cells.

The animal, to which the DNA of the present invention has been transfected, can be subjected to mating and breeding for generations under common breeding circumstance, as the DNA-carrying animal, after confirming that the gene can be stably retained. Moreover, male and female animals having the desired DNA are mated to give a homozygote having the transfected gene in both homologous chromosomes and then the male and female animals are mated so that such breeding for generations that progenies contain the DNA can be performed.

The animal, to which the DNA of the present invention has been transfected, may be used to analyze the functions of the peptide of the present invention, since the peptide or the protein of the present invention is highly expressed. That is, the animal, to which he DNA of the present invention has been transfected, may be used as the cell sources for tissue culture. The peptide or the protein of the present invention can be analyzed by, for example, direct analysis of the DNA or RNA in the tissues from the DNA-transfected mouse of the present invention, or by analysis of the tissues containing the peptide of the present invention expressed from the gene. Cells from tissues containing the peptide or the protein of the present invention are cultured by the standard tissue culture technique and using these cells, the function of the cells from the tissues that are generally difficult to culture, for example, cells derived from the brain and peripheral tissues can be studied. Using these cells, it is also possible to select pharmaceuticals, for example, that potentiate the function of various tissues. Where a highly expressing cell line is available, the peptide or the protein of the present invention may also be isolated and purified from the cell line.

In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA | deoxyribonucleic acid |
| cDNA | complementary deoxyribonucleic acid |
| A | adenine |
| T | thymine |
| G | guanine |
| C | cytosine |
| Y | thymine or cytosine |
| N | thymine, cytosine, adenine or guanine |
| R | adenine or guanine |
| M | cytosine or adenine |
| W | thymine or adenine |
| S | cytosine or guanine |
| RNA | ribonucleic acid |
| mRNA | messenger ribonucleic acid |
| dATP | deoxyadenosine triphosphate |
| dTTP | deoxythymidine triphosphate |
| dGTP | deoxyguanosine triphosphate |
| dCTP | deoxycytidine triphosphate |
| ATP | adenosine triphosphate |
| Gly or G | glycine |
| Ala or A | alanine |
| Val or V | valine |
| Leu or L | leucine |
| Ile or I | isoleucine |
| Ser or S | serine |
| Thr or T | threonine |
| Cys or C | cysteine |
| Met or M | methionine |
| Glu or E | glutamic acid |
| Asp or D | aspartic acid |
| Lys or K | lysine |
| Arg or R | arginine |
| His or H | histidine |
| Phe or F | phenylalanine |
| Tyr or Y | tyrosine |
| Trp or W | tryptophan |
| Pro or P | proline |
| Asn or N | asparagine |
| Gln or Q | glutamine |
| pGlu | pyroglutamic acid |
| Xaa | unidentified amino acid residue |

Also, substituents, protecting groups and reagents, etc. frequently used in this specification are presented by the codes below.

| | |
|---|---|
| Me | methyl group |
| Et | ethyl group |
| Bu | butyl group |
| Ph | phenyl group |
| Ac | acetyl group |

(continued)

| | |
|---|---|
| TC | thiazolidine-4(R)-carboxamido group |
| Bom | benzyloxymethyl |
| Bzl | benzyl |
| Z | benzyloxycarbonyl |
| Br-Z | 2-bromobenzyloxycarbonyl |
| Cl-Z | 2-chlorobenzyloxycarbonyl |
| $Cl_2Bzl$ | 2,6-dichlorobenzyl |
| Boc | t-butoxycarbonyl |
| HOBt | 1-hydroxybenztriazole |
| HOOBt | 3,4-dihydro-3-hydroxy-4-oxo-1,2,3-benzotriazine |
| PAM | phenylacetamidomethyl |
| Tos | p-toluenesulfonyl |
| Fmoc | N-9-fluorenyl methoxycarbonyl |
| DNP | dinitrophenol |
| Bum | tertiary-butoxymethyl |
| Trt | trityl |
| Born | benzyloxymethyl |
| Z | benzyloxycarbonyl |
| MeBzl | 4-methylbenzyl |
| DCC | N,N'-dicyclohexylcarbodiimido |
| HONB | 1-hydroxy-5-norbornene-2,3-dicarboxyimido |
| NMP | N-methylpyrrolidone |
| HONB | N-hydroxy-5-norbornene-2,3-dicarboxyimido |
| NMP | N-methylpyrrolidone |
| TFA | trifluoroacetic acid |
| CHAPS | 3-[(3-cholamidopropyl)dimethylammonio]-1-propanesulfonate |
| PMSF | phenylmethylsulfonyl fluoride |
| GDP | guanosine-5'-diphosphate |
| Fura-2AM | pentacetoxymethyl 1-[6-amino-2-(5-carboxy-2-oxazolyl)-5-benzofuranyloxy]-2-(2-amino-5-methylphenoxy)ethane-N,N,N',N'-tetraacetate |
| Fluo-3AM | pentacetoxymethyl 1-[2-amino-5-(2,7-dichloro-6-hydroxy-3-oxy-9-xanthenyl)phenoxy]-2-(2-amino-5-methylphenoxy)ethane-N,N,N',N'-tetraacetate |
| EDTA | ethylenediaminetetraacetic acid |
| SDS | sodium dodecyl sulfate |
| BSA | bovine serum albumin |
| HBSS | Hanks' balanced salt solution |
| EIA | enzymeimmunoassay |
| CHAPS | 3-[(3-cholamidopropyl)dimethylammonio]-1-propanesulfonate |

The sequence identification numbers in the sequence listing of the specification indicates the following sequence, respectively.

[SEQ ID NO:1]

This shows the amino acid sequence of the protein ZAQ derived from human brain.

[SEQ ID NO:2]

This shows the base sequence of DNA encoding human brain-derived protein ZAQ (ZAQC).

[SEQ ID NO:3]

This shows the base sequence of DNA encoding human brain- derived protein ZAQ (ZAQT).

[SEQ ID NO:4]

This shows the base sequence of the primer 1 used in Reference Example 3 later described.

[SEQ ID NO:5]

This shows the base sequence of the primer 2 used in Reference Example 3 later described.

[SEQ ID NO:6]

This shows the base sequence of the human I5E receptor protein.

[SEQ ID NO:7]

This shows the base sequence of the primer hBv8-F1 used in Reference Example 3 later described.

[SEQ ID NO:8]

This shows the base sequence of the primer hBv8-R1 used in Reference Example 3 later described.

[SEQ ID NO:9]

This shows the base sequence of DNA fragment obtained from Reference Example 3 later described.

[SEQ ID NO:10]

This shows the base sequence of the primer hBv8-R2 used in Reference Example 3 later described.

[SEQ ID NO:11]

This shows the 5' terminal base sequence of DNA encoding human Bv8 peptide obtained from Reference Example 3 later described.

[SEQ ID NO:12]

This shows the base sequence of the primer hBv8-WF in Reference Example 3 later described.

[SEQ ID NO:13]

This shows the base sequence of the primer hBv8-WR in Reference Example 3 later described.

[SEQ ID NO:14]

This shows the base sequence of the primer hBv8-CF in Reference Example 3 later described.

[SEQ ID NO:15]

This shows the 3'-terminus of base sequence of the primer hBv8-SR in Reference Example 3 later described.

[SEQ ID NO:16]

This shows the base sequence of DNA fragment obtained from in Reference Example 3 later described.

[SEQ ID NO:17]

This shows the amino acid sequence base of human Bv8 precursor peptide.

[SEQ ID NO:18]

This shows the base sequence of DNA encoding human Bv8 precursor peptide.

[SEQ ID NO:19]

This shows the amino acid sequence of human Bv8 maturation peptide.

[SEQ ID NO:20]

This shows the base sequence of human Bv8 maturation peptide.

[SEQ ID NO:21]

This shows the amino acid sequence of snake poison MIT1.

[SEQ ID NO:22]

This shows the amino acid sequence of C'-terminal Lys - deleted human Bv8 maturation peptide.

[SEQ ID NO:23]

This shows the base sequence of the primer BF2 used in Reference Example 5 later described.

[SEQ ID NO:24]

This shows the base sequence of the primer BR1 used in Reference Example 5 later described.

[SEQ ID NO:25]

This shows the base sequence of DNA fragment obtained from Reference Example 5 later described.

[SEQ ID NO:26]

This shows the base sequence of the primer RB5-1 used in Reference Example 5 later described.

[SEQ ID NO:27]

This shows the base sequence of the primer RB5-3 used in Reference Example 5 later described.

[SEQ ID NO:28]

This shows the 5' terminal base sequence of DNA encoding rat Bv8 obtained from Reference Example 5 later described.

[SEQ ID NO:29]

This shows the base sequence of the primer RB3-1 used in Reference Example 5 later described.

[SEQ ID NO:30]

This shows the base sequence of the primer RB3-2 used in Reference Example 5 later described.

[SEQ ID NO:31]

This shows the 3' terminal base sequence of DNA encoding rat type Bv8 obtained from Reference Example 5 later described.

[SEQ ID NO:32]

This shows the base sequence of the primer RBv8-WF1 used in Reference Example 5 later described.

[SEQ ID NO:33]

This shows the base sequence of the primer RBv8-WF2 used in Reference Example 5 later described.

[SEQ ID NO:34]

This shows the base sequence of the primer RBvB-WR1 used in Reference Example 5 later described.

[SEQ ID NO:35]

This shows the base sequence of the primer RBv8-WR2 used in Reference Example 5 later described.

[SEQ ID NO:36]

This shows the base sequence of DNA fragment obtained from in Reference Example 5 later described.

[SEQ ID NO:37]

This shows the amino acid sequence base of rat type Bv8 precursor peptide.

[SEQ ID NO:38]

This shows the base sequence of DNA encoding rat type Bv8 precursor peptide.

[SEQ ID NO:39]

This shows the amino acid sequence of rat type Bv8 maturation peptide and mouse type Bv8 maturation peptide.

[SEQ ID NO:40]

This shows the base sequence of rat type Bv8 maturation peptide.

[SEQ ID NO:41]

This shows the base sequence of the primer used in Reference Example 6 later described.

[SEQ ID NO:42]

This shows the base sequence of the primer used in Reference Example 6.

[SEQ ID NO:43]

This shows the base sequence of CDNA encoding a novel G protein-coupled receptor protein (rZAQ1).

[SEQ ID NO:44]

This shows the amino acid sequence of cDNA encoding novel G protein-coupled receptor protein (rZAQ1).

[SEQ ID NO:45]

This shows the base sequence of the probe used in Reference Example 7 later described.

[SEQ ID NO:46]

This shows the base sequence of the probe used in Reference Example 7 later described.

[SEQ ID NO:47]

This shows the base sequence of the primer used in Reference Example 7 later described.

[SEQ ID NO:48]

This shows the base sequence of the primer used in Reference Example 7 later described.

[SEQ ID NO:49]

This shows the base sequence of the primer used in Reference Example 7 later described.

[SEQ ID NO:50]

This shows the base sequence of cDNA encoding a novel G protein-coupled receptor protein (rZAQ2).

[SEQ ID NO:51]

This shows the amino acid sequence of cDNA encoding novel G protein-coupled receptor protein (rZAQ2).

[SEQ ID NO:52]

This shows the amino acid sequence of human I5E receptor protein.

[SEQ ID NO:53]

This shows the amino acid sequence of G protein-coupled receptor protein derived from mouse (GPR73).

[SEQ ID NO:54]

This shows the amino acid sequence of G protein-coupled receptor protein derived from mouse (mI5E).

[SEQ ID NO:55]

This shows the amino acid sequence base of mouse type Bv8 precursor peptide.

[SEQ ID NO: 56]

This shows the base sequence of DNA encoding mouse type Bv8 precursor peptide.

[SEQ ID NO: 57]

This shows the base sequence of mouse type Bv8 maturation peptide.

[SEQ ID NO: 58]

This shows the base sequence of the primer 3 used in Reference Example 1 later described.

[SEQ ID NO: 59]

This shows the base sequence of the primer 4 used in Reference Example 1 later described.

[SEQ ID NO: 60]

This shows the base sequence of the ZAQ probe used in Reference Example 1 later described.

[SEQ ID NO: 61]

This shows the base sequence of the primer ZAQC Sal used in Reference Example 1 later described.

[SEQ ID NO: 62]

This shows the base sequence of the primer ZAQC Spe used in Reference Example 4 later described.

[SEQ ID NO: 63]

This shows the base sequence of G protein-coupled receptor protein derived from mouse (GPR73).

[SEQ ID NO: 64]

This shows the base sequence of G protein-coupled receptor protein derived from mouse (mI5E).

[SEQ ID NO: 65]

This shows the base sequence of DNA fragment #1 used in Reference Example 8 later described.

[SEQ ID NO: 66]

This shows the base sequence of DNA fragment #2 used in Reference Example 8 later described.

[SEQ ID NO: 67]

This shows the base sequence of DNA fragment #3 used in Reference Example 8 later described.

[SEQ ID NO: 68]

This shows the base sequence of DNA fragment #4 used in Reference Example 8 later described.

[SEQ ID NO: 69]

This shows the base sequence of DNA fragment #5 used in Reference Example 8 later described.

[SEQ ID NO: 70]

This shows the base sequence of DNA fragment #6 used in Reference Example 8 later described.

[SEQ ID NO: 71]

This shows the base sequence of synthesized DNA encoding human Bv8 shown in SEQ ID NO: 30.

[SEQ ID NO: 72]

This shows the amino acid sequence of ZAQ activated peptide purified in Reference Example 9 later described.

[SEQ ID NO: 73]

This shows the base sequence of the primer ZF1 used in Reference Example 9 (II) later described.

[SEQ ID NO: 74]

This shows the base sequence of the primer ZF2 used in Reference Example 9 (II) later described.

[SEQ ID NO:75]

This shows the base sequence of the primer ZF3 used in Reference Example 9 (II) later described.

[SEQ ID NO: 76]

This shows the 3' terminal base sequence of DNA encoding human Bv8 obtained from Reference Example 9 (II) later described.

[SEQ ID NO: 77]

This shows the base sequence of the primer ZAQL-CF used in Reference Example 9 (II) later described.

[SEQ ID NO: 78]

This shows the base sequence of the primer ZAQL-XR1 used in Reference Example 9 (II) later described.

[SEQ ID NO:79]

This shows the base sequence of DNA fragment obtained from in Reference Example 9 (II) later described.

[SEQ ID NO:80]

This shows the base sequence of DNA fragment obtained from in Reference Example 9 (II) later described.

[SEQ ID NO:81]

This shows the amino acid sequence of human ZAQ ligand maturation peptide.

[SEQ ID NO:82]

This shows the amino acid sequence of human ZAQ ligand maturation peptide.

[SEQ ID NO:83]

This shows the amino acid sequence of human ZAQ ligand precursor peptide.

[SEQ ID NO:84]

This shows the amino acid sequence of human ZAQ ligand precursor peptide.

[SEQ ID NO:85]

This shows the base sequence of DNA containing DNA encoding human ZAQ ligand precursor peptide shown in SEQ ID NO: 89.

[SEQ ID NO:86]

This shows the base sequence of DNA containing DNA encoding human ZAQ ligand precursor peptide shown in SEQ ID NO: 90.

[SEQ ID NO:87]

This shows the base sequence of DNA containing DNA encoding human ZAQ ligand maturation peptide shown in SEQ ID NO: 81.

[SEQ ID NO:88]

This shows the base sequence of DNA containing DNA encoding human ZAQ ligand maturation peptide shown in SEQ ID NO: 82.

[SEQ ID NO:89]

This shows the base sequence of DNA containing DNA encoding human ZAQ ligand precursor peptide shown in SEQ ID NO: 83.

[SEQ ID NO:90]

This shows the base sequence of DNA containing DNA encoding human ZAQ ligand precursor peptide shown in SEQ ID NO: 84.

[SEQ ID NO:91]

This shows the base sequence of DNA fragment used in Reference Example 10 (1) later described

[SEQ ID NO:92]

This shows the N-terminal amino acid sequence of human ZAQ ligand peptide analyzed in Reference Example 11(2).

[SEQ ID NO:93]

This shows the base sequence of the primer used in Reference Example 12 later described.

[SEQ ID NO:94]

This shows the base sequence of the primer used in Reference Example 12 later described.

[SEQ ID NO:95]

This shows the base sequence of DNA fragment #1 used in Reference Example 1 later described.

[SEQ ID NO:96]

This shows the base sequence of DNA fragment #2 used in Reference Example 1 later described.

[SEQ ID NO:97]

This shows the base sequence of DNA fragment #3 used in Reference Example 1 later described.

[SEQ ID NO:98]

This shows the base sequence of DNA fragment #4 used in Reference Example 1 later described.

[SEQ ID NO:99]

This shows the base sequence of DNA fragment #5 used in Reference Example 1 later described.

[SEQ ID NO:100]

This shows the base sequence of DNA fragment #6 used in Reference Example 1 later described.

[SEQ ID NO:101]

This shows the base sequence of synthesized DNA encoding human ZAQ ligand shown in SEQ ID NO:82.

[SEQ ID NO:102]

This shows the base sequence of the primer used in Reference Example 15 later described.

[SEQ ID NO:103]

This shows the base sequence of the primer used in Reference Example 15 later described.

[SEQ ID NO:104]

This shows the base sequence of the primer used in Reference Example 15 later described.

[SEQ ID NO:105]

This shows the base sequence of the primer used in Reference Example 15 later described.

[SEQ ID NO:106]

This shows the base sequence of DNA fragment obtained from Reference Example 15 later described.

[SEQ ID NO:107]

This shows the amino acid sequence base of mouse type ZAQ precursor peptide.

[SEQ ID NO:108]

This shows the base sequence of DNA encoding mouse type ZAQ precursor peptide.

[SEQ ID NO:109]

This shows the amino acid sequence of mouse type ZAQ maturation peptide.

[SEQ ID NO:110]

This shows the base sequence of mouse type ZAQ maturation peptide.

[SEQ ID NO:111]

This shows the base sequence of DNA containing DNA encoding mouse type ZAQ ligand precursor peptide.

[SEQ ID NO:112]

This shows the base sequence of the primer used in Reference Example 16 later described.

[SEQ ID NO:113]

This shows the base sequence of the primer used in Reference Example 16 later described.

[SEQ ID NO:114]

This shows the base sequence of the primer used in Reference Example 16 later described.

[SEQ ID NO:115]

This shows the base sequence of the primer used in Reference Example 16 later described.

[SEQ ID NO:116]

This shows the base sequence of DNA fragment obtained from Reference Example 16 later described.

[SEQ ID NO:117]

This shows the base sequence of DNA fragment obtained from Reference Example 16 later described.

[SEQ ID NO:118]

This shows the base sequence of the primer used in Reference Example 16 later described.

[SEQ ID NO:119]

This shows the base sequence of the primer used in Reference Example 16 later described.

[SEQ ID NO:120]

This shows the base sequence of the primer used in Reference Example 16 later described.

[SEQ ID NO:121]

This shows the 5' terminal base sequence of DNA encoding rat type ZAQ ligand obtained from Reference Example 16 later described.

[SEQ ID NO:122]

This shows the base sequence of the primer used in Reference Example 16 later described.

[SEQ ID NO:123]

This shows the base sequence of the primer used in Reference Example 16 later described.

[SEQ ID NO:124]

This shows the 5' terminal base sequence of DNA encoding rat type ZAQ ligand obtained from Reference Example 16 later described.

[SEQ ID NO:125]

This shows the base sequence of the primer used in Reference Example 16 later described.

[SEQ ID NO:126]

This shows the base sequence of the primer used in Reference Example 16 later described.

[SEQ ID NO:127]

This shows the base sequence of the primer used in Reference Example 16 later described.

[SEQ ID NO:128]

This shows the base sequence of the primer used in Reference Example 16 later described.

[SEQ ID NO:129]

This shows the base sequence of the DNA fragment obtained from Reference Example 16 later described (normal type).

[SEQ ID NO:130]

This shows the base sequence of the DNA fragment obtained from Reference Example 16 later described (Y type).

[SEQ ID NO:131]

This shows the base sequence of the DNA fragment obtained from Reference Example 16 later described (Q type).

[SEQ ID NO:132]

This shows the amino acid sequence of the rat type ZAQ ligand precursor peptide (normal type).

[SEQ ID NO:133]

This shows the base sequence of the DNA encoding rat type ZAQ ligand precursor peptide (normal type).

[SEQ ID NO:134]

This shows the amino acid sequence of the rat type ZAQ ligand precursor peptide (Y type).

[SEQ ID NO:135]

This shows the base sequence of the DNA encoding rat type ZAQ ligand precursor peptide (Y type).

[SEQ ID NO:136]

This shows the amino acid sequence of the rat type ZAQ ligand precursor peptide (Q type).

[SEQ ID NO:137]

This shows the base sequence of the DNA encoding rat type ZAQ ligand precursor peptide (Q type).

[SEQ ID NO:138]

This shows the amino acid sequence of the rat type ZAQ ligand mutation peptide (normal type).

[SEQ ID NO:139]

This shows the base sequence of the DNA encoding rat type ZAQ ligand mutation peptide (normal type).

[SEQ ID NO:140]

This shows the amino acid sequence of the rat type ZAQ ligand mutation peptide (Y type).

[SEQ ID NO:141]

This shows the base sequence of the DNA encoding rat type ZAQ ligand mutation peptide (Y type).

[SEQ ID NO:142]

This shows the amino acid sequence of the rat type ZAQ ligand mutation peptide (Q type).

[SEQ ID NO:143]

This shows the base sequence of the DNA encoding rat type ZAQ ligand mutation peptide (Q type).

[SEQ ID NO:144]

This shows the base sequence of the primer used in Example 1 later described.

[SEQ ID NO:145]

This shows the base sequence of the primer used in Example 1 later described.

[SEQ ID NO:146]

This shows the base sequence of the bovine ZAQ partial sequence obtained from Example 1 later described.

[SEQ ID NO:147]

This shows the base sequence of the bovine I5E partial sequence obtained from Example 1 later described.

[SEQ ID NO:148]

This shows the base sequence of the primer used in Example 1 later described.

[SEQ ID NO:149]

This shows the base sequence of the primer used in Example 1 later described.

[SEQ ID NO:150]

This shows the base sequence of the bovine ZAQ 5' partial sequence (type 1) obtained from Example 1 later described.

[SEQ ID NO:151]

This shows the base sequence of the bovine type ZAQ 5' partial sequence (type 2) obtained from Example 1 later described.

[SEQ ID NO: 152]

This shows the base sequence of the primer used in Example 1 later described.

[SEQ ID NO:153]

This shows the base sequence of the primer used in Example 1 later described.

[SEQ ID NO:154]

This shows the base sequence of the bovine type ZAQ 3' partial sequence (type 2) obtained from Example 1 later described.

[SEQ ID NO:155]

This shows the base sequence of the primer used in Example 1 later described.

[SEQ ID NO:156]

This shows the base sequence of the primer used in Example 1 later described.

[SEQ ID NO:157]

This shows the base sequence of the primer used in Example 1 later described.

[SEQ ID NO:158]

This shows the base sequence of the primer used in Example 1 later described.

[SEQ ID NO:159]

This shows the base sequence of the 2038bp of DNA fragment encoding full length bovine type ZAQ obtained from Example 1 later described.

[SEQ ID NO:160]

This shows the base sequence of the primer used in Example 1 later described.

[SEQ ID NO:161]

This shows the base sequence of the primer used in Example 1 later described.

[SEQ ID NO:162]

This shows the base sequence of the bovine type I5E 5' partial sequence obtained from Example 1 later described.

[SEQ ID NO:163]

This shows the base sequence of the primer used in Example 1 later described.

[SEQ ID NO:164]

This shows the base sequence of the primer used in Example 1 later described.

[SEQ ID NO:165]

This shows the base sequence of the bovine type I5E 3' partial sequence obtained from Example 1 later described.

[SEQ ID NO:166]

This shows the base sequence of the primer used in Example 1 later described.

[SEQ ID NO:167]

This shows the base sequence of the primer used in Example 1 later described.

[SEQ ID NO:168]

This shows the base sequence of the primer used in Example 1 later described.

[SEQ ID NO:169]

This shows the base sequence of the primer used in Example 1 later described.

[SEQ ID NO:170]

This shows the base sequence of the 1623bp of DNA fragment encoding full length bovine type I5E obtained from Example 1 later described.

[SEQ ID NO:171]

This shows the amino acid sequence of bovine type ZAQ.

[SEQ ID NO:172]

This shows the base sequence of bovine type ZAQ.

[SEQ ID NO:173]

This shows the amino acid sequence of bovine type I5E.

[SEQ ID NO:174]

This shows the base sequence of bovine type I5E.

[SEQ ID NO:175]

This shows the base sequence of the primer used in Example 2 later described.

[SEQ ID NO:176]

This shows the base sequence of the primer used in Example 2 later described.

[SEQ ID NO:177]

This shows the base sequence of the primer used in Example 2 later described.

[SEQ ID NO:178]

This shows the base sequence of the primer used in Example 2 later described.

[SEQ ID NO:179]

This shows the base sequence of the probe used in Example 2 later described. In the sequence, FAM shows 6-carboxyfluorescein, TAMRA shows 6-carboxy-tetramethylrhodamine, respectively.

[SEQ ID NO:180]

This shows the base sequence of the primer used in Example 2 later described.

[SEQ ID NO:181]

This shows the base sequence of the primer used in Example 2 later described.

[SEQ ID NO:182]

This shows the base sequence of the primer used in Example 2 later described.

[SEQ ID NO:183]

This shows the base sequence of the primer used in Example 2 later described.

[SEQ ID NO:184]

This shows the base sequence of the probe used in Example 2 later described. In the sequence, FAM shows 6-carboxyfluorescein, TAMRA shows 6-carboxy-tetramethylrhodamine, respectively.

Transformant Escherichia coli DH5$\alpha$/pBZAQ obtained in Example 1 later described has been deposited with the National Institute of Advanced Industrial Science and Technology, International Patent Organism Depositary (former NIBH) located at Central 6, 1-1-1 Higashi, Tsukuba, Ibaraki, Japan (postal code 305-8566) under the Accession Number FERM BP-7845 since January 9, 2002 and with the Institute for Fermentation, Osaka (IFO) located at 2-17-85, Juso-Honmachi, Yodogawa-ku, Osaka-shi, Osaka, Japan (postal code 532-8686) under the Accession Number IFO 16740 since December 18, 2001.

Transformant Escherichia coli TOP10/pB15E obtained in Example 1 later described has been deposited with the National Institute of Advanced Industrial Science and Technology, International Patent Organism Depositary (former NIBH) located at Central 6, 1-1-1 Higashi, Tsukuba, Ibaraki, Japan (postal code 305-8566) under the Accession Number FERM BP-7844 since December 18, 2001, and with the Institute for Fermentation, Osaka (IFO) located at 2-17-85, Juso-Honmachi, Yodogawa-ku, Osaka-shi, Osaka, Japan (postal code 532-8686) under the Accession Number IFO 16739 since December 18, 2001.

Transformant Escherichia coli DH5$\alpha$/pCR2.1-ZAQC obtained in Reference Example 1 later described has been deposited with the National Institute of Advanced Industrial Science and Technology, International Patent Organism Depositary (former NIBH) located at Central 6, 1-1-1 Higashi, Tsukuba, Ibaraki, Japan (postal code 305-8566) under the Accession Number FERM BP-6855 since August 23, 1999, and with the Institute for Fermentation, Osaka (IFO) under the Accession Number IFO 16301 since August 4, 1999.

Transformant Escherichia coli DH5$\alpha$/pCR2.1-ZAQT obtained in Reference Example 1 later described has been deposited with the National Institute of Advanced Industrial Science and Technology, International Patent Organism Depositary (former NIBH) located at Central 6, 1-1-1 Higashi, Tsukuba, Ibaraki, Japan (postal code 305-8566) under the Accession Number FERM BP-6856 since August 23, 1999, and with the Institute for Fermentation, Osaka (IFO) under the Accession Number IFO 16302 since August 4, 1999.

Transformant Escherichia coli TOP10/pRBv obtained in Reference Example 5 later described has been deposited with the National Institute of Advanced Industrial Science and Technology, International Patent Organism Depositary (former NIBH) located at Central 6, 1-1-1 Higashi, Tsukuba, Ibaraki, Japan (postal code 305-8566) under the Accession Number FERM BP-7427 since December 22, 2000, and with the Institute for Fermentation, Osaka (IFO) located at 2-17-85, Juso-Honmachi, Yodogawa-ku, Osaka-shi, Osaka, Japan (postal code 532-8686) under the Accession Number IFO 16522 since December 22, 2000.

Transformant Escherichia coli DH5$\alpha$/pCR2.1-rZAQ1 obtained in Reference Example 6 later described has been deposited with the National Institute of Advanced Industrial Science and Technology, International Patent Organism Depositary (former NIBH) located at Central 6, 1-1-1 Higashi, Tsukuba, Ibaraki, Japan (postal code 305-8566) under the Accession Number FERM BP-7275 since August 21, 2000, and with the Institute for Fermentation, Osaka (IFO) under the Accession Number IFO 16459 since August 1, 2000.

Transformant Escherichia coli DH10B/pCMV-rZAQ2 obtained in Reference Example 7 later described has been deposited with the National Institute of Advanced Industrial Science and Technology, International Patent Organism Depositary (former NIBH) located at Central 6, 1-1-1 Higashi, Tsukuba, Ibaraki, Japan (postal code 305-8566) under the Accession Number FERM BP-7276 since August 21, 2000, and with the Institute for Fermentation, Osaka (IFO) under the Accession Number IFO 16460 since August 1, 2000.

Transformant Escherichia coli MM294(DE3)/pTCh2ZAQ obtained in Reference Example 8 later described has been deposited with the National Institute of Advanced Industrial Science and Technology, International Patent Organism Depositary (former NIBH) located at Central 6, 1-1-1 Higashi, Tsukuba, Ibaraki, Japan (postal code 305-8566) under the Accession Number FERM BP-7572 sinceApril 27, 2001, and with the Institute for Fermentation, Osaka (IFO) located at 2-17-85, Juso-Honmachi, Yodogawa-ku, Osaka-shi, Osaka, Japan (postal code 532-8686) under the Accession Number IFO 16587 since March 15, 2001.

Transformant Escherichia coli TOP10/pHMITA obtained in Reference Example 9 later described has been deposited with the National Institute of Advanced Industrial Science and Technology, International Patent Organism Depositary (former NIBH) located at Central 6, 1-1-1 Higashi, Tsukuba, Ibaraki, Japan (postal code 305-8566) under the Accession Number FERM BP-7219 since July 13, 2000, and with the Institute for Fermentation, Osaka (IFO) located at 2-17-85, Juso-Honmachi, Yodogawa-ku, Osaka-shi, Osaka, Japan (postal code 532-8686) under the Accession Number IFO 16440 since May 26, 2000.

Transformant Escherichia coli TOP10/pHMITG obtained in Reference Example 9 later described has been deposited with the National Institute of Advanced Industrial Science and Technology, International Patent Organism Depositary (former NIBH) located at Central 6, 1-1-1 Higashi, Tsukuba, Ibaraki, Japan (postal code 305-8566) under the Accession Number FERM BP-7220 since July 13, 2000, and with the Institute for Fermentation, Osaka (IFO) located at 2-17-85, Juso-Honmachi, Yodogawa-ku, Osaka-shi, Osaka, Japan (postal code 532-8686) under the Accession Number IFO 16441 since May 26, 2000.

Transformant Escherichia coli MM294(DE3)/pTCh1ZAQ obtained in Reference Example 14 later described has been deposited with the National Institute of Advanced Industrial Science and Technology, International Patent Organism Depositary (former NIBH) located at Central 6, 1-1-1 Higashi, Tsukuba, Ibaraki, Japan (postal code 305-8566) under the Accession Number FERM BP-7571 since April 27, 2001, and with the Institute for Fermentation, Osaka (IFO) located at 2-17-85, Juso-Honmachi, Yodogawa-ku, Osaka-shi, Osaka, Japan (postal code 532-8686) under the Accession Number IFO 16527 since January 16, 2001.

Transformant Escherichia coli TOP10/pMMIT obtained in Reference Example 15 later described has been deposited with the National Institute of Advanced Industrial Science and Technology, International Patent Organism Depositary (former NIBH) located at Central 6, 1-1-1 Higashi, Tsukuba, Ibaraki, Japan (postal code 305-8566) under the Accession Number FERM BP-7425 since January 11, 2001, and with the Institute for Fermentation, Osaka (IFO) located at 2-17-85, Juso-Honmachi, Yodogawa-ku, Osaka-shi, Osaka, Japan (postal code 532-8686) under the Accession Number IFO 16520 since December 22, 2000.

Transformant Escherichia coli TOP10/pRMIT obtained in reference Example 16 later described has been deposited with the National Institute of Advanced Industrial Science and Technology, International Patent Organism Depositary (former NIBH) located at Central 6, 1-1-1 Higashi, Tsukuba, Ibaraki, Japan (postal code 305-8566) under the Accession Number FERM BP-7426 since January 11, 2001, and with the Institute for Fermentation, Osaka (IFO) under the Accession Number IFO 16521 since December 22, 2000.

## EXAMPLES

The present invention will be further specifically described in the following examples and reference examples. However, these examples are not intended to limit the scope of the present invention. It is to be noted that genetic manipulation using *Escherichia coli* was carried out according to the method described in Molecular Cloning.

### Example 1 Cloning of bovine type ZAQ and bovine type I5E receptor cDNAs

BACE-derived total RNA (BACE tRNA) and BAE-derived total RNA (BAE tRNA) were obtained from bovine adrenal capillary endothelial cells (BACE) and bovine aorta endothelial cells (BAE), using RNeasy Mini Kit (QIAGEN) according to the method described in a manual supplied with the kit. Using these RNAs as templates, BACE-derived cDNA (BACE cDNA) and BAE-derived cDNA (BAE cDNA) were obtained using SUPERSCRIPT First-Strand Synthesis System for RT-PCR Kit (GIBCO BRL) according to the method described in a manual supplied with the kit. Subsequently, using BACE cDNA as a template, degenerate primers ZAQ-B1F (SEQ ID NO: 144) and ZAQ-B1R (SEQ ID NO: 145) were prepared based on information regarding human ZAQ, mouse ZAQ, rat ZAQ, human I5E, and mouse I5E and GPR73. Using the obtained primers, a PCR reaction described below was carried out.

ZAQ-B1F: 5'-ATYGTSTGCTGCCCCTTYGAGATG-3' (SEQ ID NO: 144)

ZAQ-B1R: 5'-TAGTAGAGCTGCTGRTCCACBGRCC-3' (SEQ ID NO: 145)

A PCR reaction solution was prepared by mixing 0.5 µl of AmpliTaq Gold (Applied Biosystems), 5 µl of 10 x PCR buffer II (100 mM Tris-Hce, 500 mM KCl) supplied with the kit, 4 µl of 25 mM $MgCl_2$, 5 µl of dNTP mixture (2.0 mM each, Applied Biosystems), 2.5 µl each of 12.5 µM primers ZAQ-B1F and ZAQ-B1R, 1 µl of template cDNA, and 29.5 µl of distilled water. After the early denaturation at 95°C for 10 minutes, the reaction conditions were set to repeat 35 cycles of reactions at 95°C for 20 seconds and at 66°C for 30 seconds, and a final extension reaction at 66°C for 10 minutes.

The obtained DNA fragment was cloned using TOPO TA Cloning Kit (Invitrogen) according to the method described in a manual supplied with the kit. The sequences of the cloned DNA were determined using ABI3100 DNA Sequencer, so as to obtain the nucleotide sequence of a partial sequence of bovine type ZAQ (SEQ ID NO: 146) and the nucleotide sequence of a partial sequence of bovine type I5E (SEQ ID NO: 147).

BACE-derived total RNA (BACE tRNA) was obtained from bovine adrenal capillary endothelial cells (BACE) using TRIzol Reagent (GIBCO BRL) according to the method described in a manual supplied therewith. Using the BACE-derived total RNA as a template, BACE-derived mRNA (BACE mRNA) was obtained using mRNA Purification Kit (Amersham Pharmacia Biotech) according to the method described in a manual supplied therewith. Using the BACE mRNA as a template, BACE-derived Library of adaptor-ligated ds cDNA (BACE AL ds cDNA) was prepared using Marathon cDNA Amplification Kit (CLONTECH) according to the method described in a manual supplied therewith.

Primers bZAQ5-2 (SEQ ID NO: 148) and bZAQ5-3 (SEQ ID NO: 149) were prepared based on the information regarding SEQ ID NO: 146, and using BACE AL ds cDNA as a template, a 5' RACE experiment described below was carried out.

bZAQ5-2: 5′-GGCCAGCAGGGCGTTGGTGGAGA-3′ (SEQ ID NO: 148)

bZAQ5-3: 5′-GTGCGCAGGTAGTTGACAGAGGCGCACA-3′ (SEQ ID 149)

A 5' RACE PCR reaction solution was prepared by mixing 0.5 µl of 50X Advantage-GC 2 Polymerase Mix (CLONTECH), 5 µl of 5x Advantage-GC 2 PCR buffer supplied with the kit (200 mM Tricine-KOH, 75 mM KOAc, 17.5 mM $Mg(OAc)_2$, 25% dimethyl sulfoxide, 18.75 µg/ml BSA, 0.025% Tween-20, and 0.025% Nonidet-P40), 2 µl of dNTP mixture (2.5 mM each, Takara Shuzo Co., Ltd.), 0.5 µl of 10 µM primer bZAQ5-2, 0.5 µl of 10 µM primer AP1 (which was supplied with Marathon-Ready cDNA Kit available from CLONTECH), 2.5 µl of template cDNA (which was a 50 times diluted solution of BACE AL ds cDNA), and 14 µl of distilled water. After the early denaturation at 94°C for 30 seconds, the reaction conditions were set to repeat 5 cycles of reactions at 94°C for 5 seconds and at 72°C for 4 minutes, 5 cycles of reactions at 94°C for 5 seconds and at 70°C for 4 minutes, and 25 cycles of reactions at 94°C for 5 seconds and at 68°C for 4 minutes.

Subsequently, using the reaction solution obtained from the above PCR reaction as a template, a nested PCR was carried out. A reaction solution was prepared by mixing 0.5 µl of 50X Advantage-GC 2 Polymerase Mix (CLONTECH), 5 µl of 5x Advantage-GC 2 PCR buffer supplied with the kit (200 mM Tricine-KOH, 75 mM KOAc, 17.5 mM $Mg(OAc)_2$, 25% dimethyl sulfoxide, 18.75 µg/ml BSA, 0.025% Tween-20, and 0.025% Nonidet-P40), 2.0 µl of dNTP mixture (2.5 mM each, Takara Shuzo Co., Ltd.), 0.5 µl of 10 µM primer bZAQ5-3, 0.5 µl of 10 µM primer AP2 (which was supplied with Marathon-Ready cDNA Kit available from CLONTECH), 2.5 µl of template DNA (which was a 50 times diluted solution of the above PCR reaction solution), and 14 µl of distilled water. After the early denaturation at 94°C for 30 seconds, the reaction conditions were set to repeat 5 cycles of reactions at 94°C for 5 seconds and at 72°C for 4 minutes, 5 cycles of reactions at 94°C for 5 seconds and at 70°C for 4 minutes, and 25 cycles of reactions at 94°C for 5 seconds and 68°C for 4 minutes.

The obtained DNA fragment was cloned using TOPO TA Cloning Kit (Invitrogen) according to the method described in a manual supplied with the kit. The sequences of the cloned DNA were determined using ABI3100 DNA Sequencer, so as to obtain 2 types of 5' partial sequences of bovine ZAQ (type 1: SEQ ID NO: 150; type 2: SEQ ID NO: 151).

Primers bZAQa-F1 (SEQ ID NO: 152) and bZAQa-F2 (SEQ ID NO: 153) were prepared based on the information regarding SEQ ID NO: 150. Using the obtained primers, a 3' RACE experiment described below was carried out.

bZAQa-F1: 5′-TCATCAGCCCGACCGCCCACTCTGGC-3′ (SEQ ID NO: 152)

bZAQa-F2: 5′-TAGGATGAAGCTTGGATTCTGGACGCCA-3′ (SEQ ID 153)

A 3' RACE PCR reaction solution was prepared by mixing 1 µl of PfuTurbo DNA polymerase (Stratagene), 5 µl of 10x PCR buffer supplied with the kit, 4 µl of dNTP mixture (2.5 mM each, Takara Shuzo Co., Ltd.), 2.5 µl each of a 10 µM primer bZAQa-F1 and a 10 µM primer AP1 (which was supplied with Marathon-Ready cDNA Kit available from CLONTECH), 1 µl of template cDNA (which was a 50 times diluted solution of BACE AL ds cDNA), and 34 µl of distilled water. After the early denaturation at 94°C for 5 seconds, the reaction conditions were set to repeat 5 cycles of reactions and at 72°C for 4 minutes, 5 cycles of reactions at 94°C for 5 seconds and at 70°C for 4 minutes, and 25 cycles of reactions at 94°C for 5 seconds and at 68°C for 4 minutes.

Subsequently, using, as a template, a solution obtained by 50 times diluting the reaction solution obtained from the above PCR reaction with distilled water, a nested PCR was carried out. A PCR reaction solution was prepared by mixing 1 μl of PfuTurbo DNA polymerase (Stratagene), 5 μl of 10x PCR buffer supplied with the kit, 4 μl of dNTP mixture (2.5 mM each, Takara Shuzo Co., Ltd.), 2.5 μl each of a 10 μM primer bZAQa-F2 and a 10 μM primer AP1 (primer was supplied with Marathon-Ready cDNA Kit available from CLONTECH), 1 μl of template DNA, and 34 μl of distilled water. After the early denaturation at 94°C for 30 seconds, the reaction conditions consisted of an initial denaturation reaction 5 cycles of reactions at 94°C for 5 seconds and at 72°C for 4 minutes, 5 cycles of reactions at 94°C for 5 seconds and at 70°C for 4 minutes, and 25 cycles of reactions at 94°C for 5 seconds and 68°C for 4 minutes.

The obtained DNA fragment was cloned using Zaro Blunt TOPO PCR Cloning Kit (Invitrogen) according to the method described in a manual supplied with the kit. The sequence of the cloned DNA was determined using ABI3100 DNA Sequencer, so as to obtain the nucleotide sequence located at the 3'-terminus of bovine ZAQ (SEQ ID NO: 154).

Primers bZAQa-EF1 (SEQ ID NO: 155), bZAQa-IF1 (SEQ ID NO: 156), bZAQa-IR1 (SEQ ID NO: 157), and bZAQa-ER1 (SEQ ID NO: 158) were prepared based on the information regarding the 5' RACE and the 3' RACE. Using a 50 times diluted solution of BACE AL ds cDNA as a template, a PCR reaction described below was carried out.

bZAQa-EF1: 5'-TTTGAGTCGCTCCCAGTGTGTCCT-3' (SEQ ID NO: 155)

bZAQa-IF1: 5'-GCTCCTCCCGGTTCTTTGAAATC-3' (SEQ ID 156)

bZAQa-IR1: 5'-TGTAGTCTCTGAATCTCACTGGTGCC-3' (SEQ ID 157)

bZAQa-ER1: 5'-GGTGGATTAAAAAGGCCCTCAATAAGCC-3' (SEQ ID 158)

A PCR reaction solution was prepared by mixing 1 μl of PfuTurbo DNA polymerase (Stratagene), 5 μl of 10x PCR buffer supplied with the kit, 4 μl of 2.5 mM dNTP mixture, 2.5 μl each of 10 μM primers bZAQa-EF1 and bZAQa-ER1, 1 μl of template DNA, and 34 μl of distilled water. After the early denaturation at 95°C for 1 minute, the reaction conditions were set to repeat 35 cycles of reactions at 95°C for 30 seconds, at 60°C for 30 seconds and at 72°C for 3 minutes, and a final extension reaction at 72°C for 10 minutes.

Subsequently, using, as a template, a solution obtained by 50 times diluting the reaction solution obtained from the above PCR reaction with distilled water, a nested PCR was carried out. A PCR reaction solution was prepared by mixing 1 μl of PfuTurbo DNA polymerase (Stratagene), 5 μl of 10x PCR buffer supplied with the kit, 4 μl of 2.5 mM dNTP mixture, 2.5 μl each of 10 μM primers bZAQa-IF1 and bZAQa-IR1, 1 μl of template DNA, and 34 μl of distilled water. After the early denaturation at 95°C for 1 minute, the reaction conditions were set to repeat 35 cycles of reactions at 95°C for 30 seconds, at 60°C for 30 seconds and at 72°C for 3 minutes, and a final extension reaction at 72°C for 10 minutes.

The obtained DNA fragment was cloned using Zaro Blunt TOPO PCR Cloning Kit (Invitrogen) according to the method described in a manual supplied with the kit. The sequence of the cloned DNA was determined using ABI3100 DNA Sequencer, so as to obtain pBZAQ having a 2,038 bp DNA fragment (SEQ ID NO: 159) encoding full-length bovine type ZAQ. *Escherichia coli* DH5α was transformed with the obtained plasmid, and it was named as *Escherichia coli* DH5α/pBZAQ.

The cDNA fragment having the nucleotide sequence shown in SEQ ID NO: 159 encodes an amino acid sequence (SEQ ID NO: 171) consisting of 393 amino acids (SEQ ID NO: 172). A protein having the amino acid sequence was named as bovine type ZAQ.

Primers bI5E5-1 (SEQ ID NO: 1-60) and bI5E5-3 (SEQ ID NO: 161) were prepared based on the information regarding SEQ ID NO: 147, and using BACE AL ds cDNA as a template, a 5' RACE experiment described below was carried out.

bI5E5-1: 5'-GTAGGCTGATGGGATGGAGATGAGAATGGA-3' (SEQ ID NO: 160)

bI5E5-3: 5'-GCAGGTAGTTGATACAGGCACAGAGCACAT-3' (SEQ ID 161)

A 5' RACE PCR reaction solution was prepared by mixing 0.5 μl of 50x Advantage-GC 2 Polymerase Mix (CLONTECH), 5 μl of 5x Advantage-GC 2 PCR buffer supplied with the kit (200 mM Tricine-KOH, 75 mM KOAc, 17.5 mM $Mg(OAc)_2$, 25% dimethyl sulfoxide, 18.75 μg/ml BSA, 0.025% Tween-20, and 0.025% Nonidet-P40), 2 μl of dNTP mixture (2.5 mM each, Takara Shuzo Co., Ltd.), 0.5 μl of a 10 μM primer bI5E5-1, 0.5 μl of a 10 μM primer AP1 (which was supplied with Marathon-Ready cDNA Kit available from CLONTECH), 2.5 μl of template cDNA (which was a 50 times diluted solution of BACE AL ds cDNA), and 14 μl of distilled water. After the early denaturation at 94°C for 30 seconds, the reaction condition were set to repeat 5 cycles of reactions at 94°C for 5 seconds and at 72°C for 4 minutes, 5 cycles of reactions at 94°C for 5 seconds and at 70°C for 4 minutes, and 25 cycles of reactions at 94°C for 5 seconds and at 68°C for 4 minutes.

Subsequently, using the reaction solution obtained from the above PCR reaction as a template, a nested PCR was carried out. A reaction solution was prepared by mixing 0.5 μl of 50X Advantage-GC 2 Polymerase Mix (CLONTECH), 5 μl of 5x Advantage-GC 2 PCR buffer supplied with the kit (200 mM Tricine-KOH, 75 mM KOAc, 17.5 mM Mg $(OAc)_2$, 25% dimethyl sulfoxide, 18.75 μg/ml BSA, 0.025% Tween-20, and 0.025% Nonidet-P40), 2.0 μl of dNTP mixture (2.5 mM each, Takara Shuzo Co., Ltd.), 0.5 μl of a 10 μM primer bI5E5-3, 0.5 μl of a 10 μM primer AP2 (which was supplied with Marathon-Ready cDNA Kit available from CLONTECH), 2.5 μl of template DNA (which was a 50 times diluted solution of the above PCR reaction solution), and 14 μl of distilled water. After the early denaturation at 94°C for 30 seconds, the reacxtion conditions were set to repeat 5 cycles of reactions at 94°C for 5 seconds and at 72°C for 4 minutes, 5 cycles of reactions at 94°C for 5 seconds and at 70°C for 4 minutes, and 25 cycles of reactions at 94°C for 5 seconds and 68°C for 4 minutes.

The obtained DNA fragment was cloned using TOPO TA Cloning Kit (Invitrogen) according to the method described in a manual supplied with the kit. The sequence of the cloned DNA was determined using ABI3100 DNA Sequencer, so as to obtain a 5' partial sequence of bovine I5E (SEQ ID NO: 162).

Primers bI5E3-1 (SEQ ID NO: 163) and bI5E3-3 (SEQ ID NO: 164) were prepared based on the information regarding SEQ ID NO: 147. Using the obtained primers, a 3' RACE experiment described below was carried out.

bI5E3-1: 5'-AGCCTCCTTCCTGATCGCTCTGGTCTG-3' (SEQ ID NO: 163)

bI5E3-3: 5'-CCCATCAGCCTACTTCACAAAGGAAACCG-3' (SEQ ID 164)

A 3' RACE PCR reaction solution was prepared by mixing 0.5 μl of 50X Advantage-GC 2 Polymerase Mix (CLONTECH), 5 μl of 5x Advantage-GC 2 PCR buffer supplied with the kit (200 mM Tricine-KOH, 75 mM KOAc, 17.5 mM $Mg(OAc)_2$, 25% dimethyl sulfoxide, 18.75 μg/ml BSA, 0.025% Tween-20, and 0.025% Nonidet-P40), 2 μl of dNTP mixture (2.5 mM each, Takara Shuzo Co., Ltd.), 0.5 μl of a 10 μM primer bI5E3-1, 0.5 μl of a 10 μM primer AP1 (which was supplied with Marathon-Ready cDNA Kit available from CLONTECH), 2.5 μl of template cDNA (which was a 50 times diluted solution of BACE AL ds cDNA), and 14 μl of distilled water. After the early denaturation at 94°C for 30 seconds, the reaction conditions were set to repeat 5 cycles of reactions at 94°C for 5 seconds and at 72°C for 4 minutes, 5 cycles of reactions at 94°C for 5 seconds and at 70°C for 4 minutes, and 25 cycles of reactions at 94°C for 5 seconds and at 68°C for 4 minutes.

Subsequently, using the reaction solution obtained from the above PCR reaction as a template, a nested PCR was carried out. A reaction solution was prepared by mixing 0.5 μl of 50X Advantage-GC 2 Polymerase Mix (CLONTECH), 5 μl of 5x Advantage-GC 2 PCR buffer supplied with the kit (200 mM Tricine-KOH, 75 mM KOAc, 17.5 mM Mg $(OAc)_2$, 25% dimethyl sulfoxide, 18.75 μg/ml BSA, 0.025% Tween-20, and 0.025% Nonidet-P40), 2.0 μl of dNTP mixture

(2.5 mM each, Takara Shuzo Co., Ltd.), 0.5 µl of a 10 µM primer bI5E3-3, 0.5 µl of 10 µM primer AP2 (which was supplied with Marathon-Ready cDNA Kit available from CLONTECH), 2.5 µl of template DNA (which was a 50 times diluted solution of the PCR reaction solution), and 14 µl of distilled water. After the early denaturation at 94°C for 30 seconds, the reaction conditions were set to repeat 5 cycles of reactions at 94°C for 5 seconds and at 72°C for 4 minutes, 5 cycles of reactions at 94°C for 5 seconds and at 70°C for 4 minutes, and 25 cycles of reactions at 94°C for 5 seconds and 68°C for 4 minutes.

The obtained DNA fragment was cloned using TOPO TA Cloning Kit (Invitrogen) according to the method described in a manual supplied with the kit. The sequence of the cloned DNA was determined using ABI3100 DNA Sequencer, so as to obtain a 3' partial sequence of bovine I5E (SEQ ID NO: 1-65).

Primers bZAQb-F (SEQ ID NO: 166), bZAQb-F2 (SEQ ID NO: 167), bZAQb-R (SEQ ID NO: 168), and bZAQb-R2 (SEQ ID NO: 169) were prepared based on the information regarding the 5' RACE and the 3' RACE. Using a 50 times diluted solution of BACE AL ds cDNA as a template, a PCR reaction described below was carried out.

bZAQb-F: 5′-CCATCCTAATACGACTCACTATAGGGC-3′ (SEQ ID NO: 166)

bZAQb-F2: 5′-GCTGGGTGAGAAGGAATAGGGA-3′ (SEQ ID 167)

bZAQb-R: 5′-GCAGGTGCCAATTCATTTTGC-3′ (SEQ ID 168)

bZAQb-R2: 5′-TGCTCTTTAATCTCGCTGGTGGT-3′ (SEQ ID 169)

A PCR reaction solution was prepared by mixing 1 µl of PfuTurbo DNA polymerase (Stratagene), 5 µl of 10x PCR buffer supplied with the kit, 4 µl of 2.5 mM dNTP mixture, 2.5 µl each of 10 µM primers bZAQb-F and bZAQb-R, 1 µl of template DNA, and 34 µl of distilled water. After the early denaturation at 95°C for 1 minute, the reaction conditions were set to repeat consisted of an initial denaturation reaction at, 40 cycles of reactions at 95°C for 30 seconds, at 58°C for 30 seconds and at 72°C for 3 minutes, and a final extension reaction at 72°C for 10 minutes.

Subsequently, using, as a template, a solution obtained by 50 times diluting the reaction solution obtained from the above PCR reaction with distilled water, a nested PCR was carried out. A PCR reaction solution was prepared by mixing 1 µl of PfuTurbo DNA polymerase (Stratagene), 5 µl of 10x PCR buffer supplied with the kit, 4 µl of 2.5 mM dNTP mixture, 2.5 µl each of 10 µM primers bZAQb-F2 and bZAQb-R2, 1 µl of template DNA, and 34 µl of distilled water. After the early denaturation at 95°C for 1 minute, the reaction conditions were set to repeat 40 cycles of reactions at 95°C for 30 seconds, at 58°C for 30 seconds and at 72°C for 3 minutes, and a final extension reaction at 72°C for 10 minutes.

The obtained DNA fragment was cloned using Zaro Blunt TOPO PCR Cloning Kit (Invitrogen) according to the method described in a manual supplied with the kit. The sequence of the cloned DNA was determined using ABI3100 DNA Sequencer, so as to obtain pBI5E having a 1,623 bp DNA fragment (SEQ ID NO: 170) encoding full-length bovine type I5E. *Escherichia coli* TOP10 was transformed with the obtained plasmid, and it was named as *Escherichia coli* TOP10/pBI5E.

The cDNA fragment having the nucleotide sequence shown in SEQ ID NO: 170 encodes an amino acid sequence (SEQ ID NO: 173) consisting of 384 amino acids (SEQ ID NO: 174). A protein having the amino acid sequence was named as bovine type I5E.

Example 2 Identification of bovine type ZAQ and bovine type I5E receptors expressed in bovine adrenal capillary endothelial cells (BACE) and bovine aorta endothelial cells (BAE)

Using pBZAQ as a template, primers bZAQa-IF1 (SEQ ID NO: 175) and bZAQa-tempIR (SEQ ID NO: 176) were prepared based on the information regarding the bovine type ZAQ. Using the obtained primers, a standard DNA fragment for ZAQ was prepared by a method described below.

bZAQa-IF1: 5′-GCTCCTCCCGGTTCTTTGAAATC-3′ (SEQ ID NO: 175)

bZAQa-tempIR: 5'-TGGTGAGGTTGCGTAGCTTCTTG-3' (SEQ ID NO: 176)

A PCR reaction solution was prepared by mixing 4 μl of PfuTurbo hotstart DNA polymerase (Stratagene), 20 μl of 10 x PCR buffer supplied with the kit, 16 μl of dNTP mixture (2.5 mM each, Takara Shuzo Co., Ltd.), 10 μl each of 10 μM primers bZAQa-IF1 and bZAQa-tempIR, 4 μl of template cDNA (pBZAQ), and 136 μl of distilled water. After the early denaturation at 95°C for 1 minute, the reaction conditions were set to repeat 40 cycles of reactions at 95°C for 30 seconds, at 60°C for 30 seconds and at 72°C for 1 minute. The obtained DNA fragment was defined as a standard DNA fragment for bovine type ZAQ.

Employing Primer Express software, forward and reverse TaqMan primers used for bovine type ZAQ, bZAQa-taqF (SEQ ID NO: 177) and bZAQa-taqR (SEQ ID NO: 178), and a TaqMan probe used for bovine type ZAQ, bZAQa-probe (SEQ ID NO: 179), were prepared based on the information regarding bovine type ZAQ.

bZAQa-taqF: 5'-AACCAACTATTTCCCTCTGCTTGAC-3' (SEQ ID NO: 177)

bZAQa-taqR: 5'-TCACCGTAGCTGAAGTTGAAGG-3' (SEQ ID NO: 178)

bZAQa-probe: 5'-FAM-CCTCGGAGCCCAAGCTGCTTCTTT-3' (SEQ ID NO: 179)

In combined use of the TaqMan probes and TaqMan primers for a bovine type ZAQ gene, a bovine type ZAQ gene transcript was assayed by TaqMan PCR analysis, using BACE-derived cDNA (BACE cDNA) and BAE-derived cDNA (BAE cDNA) as templates. A reaction solution was prepared by mixing 12.5 μl of 2x TaqMan PCR Master Mix, 1.5 μl each of 5 μM primers bZAQa-taqF and bZAQa-taqR, 1 μl each of 5 μM probes bZAQa-probes, 4 μl of standard DNA fragments for bovine type ZAQ each having a different dilution concentration or template cDNA (200 ng, derived from tRNA), and 4.5 μl of distilled water.

A TaqMan PCR reaction was carried out using SDS7700 (Sequence Detection System, ABI) under reaction conditions consisting of an initial reaction at 50°C for 2 minutes and 95°C for 10 minutes, and 50 cycles of reactions at 95°C for 15 seconds and 60°C for 1 minute. After completion of the reaction, a standard curve was plotted on the software, using fluorescence intensity to standard DNA fragments each having a different dilution concentration, so that a bovine type ZAQ gene transcript existing in the template cDNA sample was assayed.

Using pBI5E as a template, primers bZAQb-F2 (SEQ ID NO: 180) and bZAQb-tempIR (SEQ ID NO: 181) were prepared based on the information regarding the bovine type I5E. Using the obtained primers, a standard DNA fragment for ZAQ was prepared by a method described below.

bZAQb-F2: 5'-GCTGGGTGAGAAGGAATAGGGA-3' (SEQ ID NO: 180)

bZAQb-tempIR: 5'-GGAGAGCTGATGCACCACATAGTAG-3' (SEQ ID NO: 181)

A PCR reaction solution was prepared by mixing 4 μl of PfuTurbo hotstart DNA polymerase (Stratagene), 20 μl of 10 x PCR buffer supplied with the kit, 16 μl of dNTP mixture (2.5 mM each, Takara Shuzo Co., Ltd.), 10 μl each of 10 μM primers bZAQb-F2 and bZAQb-tempIR, 4 μl of template cDNA (pBI5E), and 136 μl of distilled water. After the early denaturation at 95°C for 1 minute, the reaction conditions were set to repeat 40 cycles of reactions at 95°C for 30 seconds, at 60°C for 30 seconds and at 72°C for 1 minute. The obtained DNA fragment was defined as a standard DNA fragment for bovine type I5E.

Employing Primer Express software, forward and reverse TaqMan primers used for bovine type I5E, bZAQb-taqF (SEQ ID NO: 182) and bZAQb-taqR (SEQ ID NO: 183), and a TaqMan probe used for bovine type I5E, bZAQb-probe (SEQ ID NO: 184), were prepared based on the information regarding bovine type I5E.

bZAQb-taqF: 5′-TCATGAGAGCAGAAGGTCTGGA-3′ (SEQ ID NO: 182)

bZAQb-taqR: 5′-TGGCTGGAAAACTAGCATTTCC-3′ (SEQ ID NO: 183)

bZAQb-probe:
5′-FAM-CACACACCGCTCACTGGAAAGCTTCA-TAMRA-3′ (SEQ ID NO: 184)

In combined use of the TaqMan probes and TaqMan primers for a bovine type I5E gene, a bovine type I5E gene transcript was assayed by TaqMan PCR analysis, using BACE-derived cDNA (BACE cDNA) and BAE-derived cDNA (BAE cDNA) as templates. A reaction solution was prepared by mixing 12.5 μl of 2x TaqMan PCR Master Mix, 1.5 μl each of 5 μM primers bZAQb-taqF and bZAQb-taqR, 1 μl each of 5 μM probes bZAQb-probes, 4 μl of standard DNA fragments for bovine type I5E each having a different dilution concentration or template cDNA (200 ng, derived from tRNA), and 4.5 μl of distilled water.

A TaqMan PCR reaction was carried out using SDS7700 (Sequence Detection System, ABI) under reaction conditions were set to repeat 50 cycles of reactions at 95°C for 15 seconds and 60°C for 1 minute, after the early denaturation at 50°C for 2 minutes and 95°C for 10 minutes. After completion of the reaction, a standard curve was plotted on the software, using fluorescence intensity to standard DNA fragments each having a different dilution concentration, so that a bovine type I5E gene transcript existing in the template cDNA sample was assayed.

The results are shown in [Figure 6]. From the results, it is found that bovine type ZAQ and bovine type I5E were expressed in bovine adrenal capillary endothelial cells. It is also found that bovine type I5E was expressed in bovine aorta endothelial cells.

Example 3

(1) Determination of activity to increase intracellular calcium ion concentration by FLIPR using bovine adrenal capillary endothelial cells (BACE)

BACE was insolated by a method reported by Gospodarowicz et al. (Journal of Cellular Physiology 127, 121-136, 1986). BACE dispersed in a growth medium (DMEM containing 10% fetal bovine serum (FBS), 100 units/ml penicillin, and 100 μg/ml streptomycin) was inoculated in a 96-well plate for FLIPR (Black plate clear bottom, Coster) such that the concentration became $3 \times 10^4$ cells/200 μl/well. It was cultured in a 5% $CO_2$ incubator at 37°C overnight. Thereafter, it was used for the subsequent experiment (hereinafter referred to as a cell plate). 10 ml of FLIPR assay buffer (9.8 g of Nissui Hanks' 2 (Nissui Pharmaceutical Co., Ltd.), 0.35 g of sodium bicarbonate, 4.77 g of HEPES, and 6M sodium hydroxide solution were mixed, the mixture was adjusted to pH 7.4, and it was filled up to 1 liter, followed by sterilization with a filter), 100 μl of 250 mM Probenecid, and 100 μl of FBS were mixed. Moreover, 20 μl of dimethyl sulfoxide and 20 μl of 20% pluronic acid (Molecular Probes) were added to and dissolved in 1 vial (25 μg) of Fluo 3-AM (Dojindo Laboratories). Thereafter, the obtained solution was added to the above Hanks'/HBSS-Probenecid-FBS. 100 μl each of the obtained mixture was poured into each well of the cell plate, from which a culture supernatant had been removed. The cell plate was then incubated in 5% $CO_2$ incubator at 37°C for 1 hour.

On the other hand, each of a human type ZAQ ligand peptide, a human type Bv8 peptide, and snake venom MIT1, which had been prepared by the methods described in Reference examples 14, 8 and 13, was diluted with Hanks'/HBSS containing 2.5 mM Probenecid, 0.2% bovine serum albumin (BSA) and 0.1% CHAPS, and the thus diluted solution was poured into a 96-well plate (sample plate). After pigment was loaded on the cell plate, the cell plate was washed 4 times with a FLIPR assay buffer (washing buffer) containing 2.5 mM probenecid, employing a plate washer (Molecular Devices). Thereafter, 100 μl of the washing buffer was kept. The cell plate and the sample plate were placed in FLIPR, followed by performing assay (50 μl of the sample was transferred from the sample plate to the cell plate by FLIPR).

The results are shown in [Figure 7].

From the results, it is clearly found that in response to the human ZAQ ligand peptide, the human Bv8 peptide and the snake venom MIT1, bovine adrenal capillary endothelial cells (BACE) cause increase of intracellular calcium ions.

Accordingly, bovine adrenal capillary endothelial cells are stimulated with the peptide of the present invention such as the human ZAQ ligand peptide, the human Bv8 peptide or the snake venom MIT1 in the presence or absence of a test compound, so as to know whether or not the test compound has an activity to inhibit the signal transduction of ZAQ or I5E, thereby obtaining an angiogenesis inhibitor.

(2) Detection of phosphorylated p42 MAP kinase or phosphorylated p44 MAP kinase by western blotting

BACE dispersed in a growth medium was inoculated in a 12-well plate such that the concentration became $2 \times 10^5$ cells/ml/well. It was then cultured in a 5% $CO_2$ incubator at 37°C overnight, and thereafter, it was used in an experiment. 10 μl each of 10 nM, 100 nM and 1,000 nM ligand solutions was added thereto, and the mixture was incubated at 37°C for 5 minutes.

As such ligand solutions, the human type ZAQ ligand peptide, the human type Bv8 peptide, and the snake venom MIT1, which had been prepared by the methods described in Reference examples 14, 8 and 13, were dissolved in H/HBSS containing 0.2% BSA and 0.1% CHAPS, and used.

Thereafter, a culture supernatant was removed, and 100 μl of a lysis buffer (50 mM Tris, 150 mM NaCl, 1 mM EDTA, 2 mM $Na_3VO_4$, 50 mM NaF, 4 mM $Na_4P_2O_7$, 1% NP40, 1 mM phenylmethylsulfonyl fluoride (PMSF), 20 μg/ml leupeptin, 10 μg/ml pepstatin A, pH 7.4) was added to each well. The mixture was left on ice for 30 minutes, and the supernatant was then subjected to centrifugal separation at 15,000 rpm for 10 minutes. To the obtained supernatant, 20 μl of a sample buffer for SDS-PAGE (300 mM Tris, 10% sodium dodesylsulfate, 0.025% bromophenolblue, 50% glycerol, pH 6.8) was added, followed by boiling for 3 minutes. 18 μl of the resultant product was separated by SDS-PAGE using 12.5% gel (BIO-RAD). After performing electrophoresis, the gel was shaken in a transfer buffer (25 mM Tris, 192 mM glycine, 10% MeOH) for 15 minutes. Thereafter, a protein contained in the gel was transferred to a PVDF membrane (MiniProBlot, Applied Biosystems). The transferred membrane (blot) was blocked using a blocking buffer (50 mM Tris, 150 mM NaCl, 0.1% Tween 20, 1% BSA, pH 7.4) at 4°C overnight. Thereafter, 30 ml of a primary antibody solution (obtained by 5,000 times diluting Phospho p42/44 MAP Kinase Antibody available from Cell Signaling TECHNOLOGY with the above blocking buffer) was added to the blot, and the mixture was incubated at room temperature for 2 hours. Thereafter, the blot was washed with a washing buffer (50 mM Tris, 150 mM NaCl, 0.1% Tween 20, pH 7.4) (20 minutes × 3). Thereafter, 30 ml of a secondary antibody solution (obtained by 1,000 times diluting Anti Rabbit IgG HRP-linked (Goat) available from KPL with the above blocking buffer) was added to the blot, and the mixture was reacted at room temperature for 1 hour. Thereafter, the blot was washed with the washing buffer (20 minutes × 3), and it was further washed with a buffer (50 mM Tris, 150 mM NaCl, pH 7.4) (2 minutes × 2), followed by detection of phosphorylated p42/44 MAP kinase with ECL Plus (Amersham Pharmacia Biotech).

The results are shown in [Figure 8].

It can be read from the figure that, when compared with a control to which no ligands were added, in the cases of adding the human type ZAQ ligand peptide, the human type Bv8 peptide, or the snake venom MIT1, the staining intensity of a phosphorylated MAP kinase (MAPK-P) band was increased. In addition, it was also found that the snake venom MIT1 exhibited such an effect of increasing the staining intensity in a low concentration such as 0.1 nM, and that the human type Bv8 peptide exhibited the same effect in a concentration of 1 nM and the human type ZAQ ligand peptide exhibited the same effect in a concentration of 10 nM.

It is clear that MAP kinase is activated in bovine adrenal capillary endothelial cells (BACE) by addition of a peptide such as the human type ZAQ ligand peptide, the human type Bv8 peptide or the snake venom MIT1.

Accordingly, bovine adrenal capillary endothelial cells are stimulated with the peptide of the present invention such as the human ZAQ ligand peptide, the human Bv8 peptide or the snake venom MIT1 in the presence or absence of a test compound, so as to know whether or not the test compound has an activity to inhibit the signal transduction of ZAQ or I5E, thereby obtaining an angiogenesis inhibitor.

(3) Detection of phosphorylated p38 MAP kinase by western blotting

BACE dispersed in a growth medium was inoculated in a 12-well plate such that the concentration became $2 \times 10^5$ cells/ml/well. It was then cultured in a 5% $CO_2$ incubator at 37°C overnight, and thereafter, it was used in an experiment. 10 μl each of 10 nM, 100 nM and 1,000 nM ligand solutions was added thereto, and the mixture was incubated at 37°C for 5 minutes.

As such ligand solutions, the human type ZAQ ligand peptide, the human type Bv8 peptide, and the snake venom MIT1, which had been prepared by the methods described in Reference examples 14, 8 and 13, were dissolved in H/HBSS containing 0.2% BSA and 0.1% CHAPS, and used.

Thereafter, a culture supernatant was removed, and 100 μl of a lysis buffer (50 mM Tris, 150 mM NaCl, 1 mM EDTA, 2 mM $Na_3VO_4$, 50 mM NaF, 4 mM $Na_4P_2O_7$, 1% NP40, 1 mM PMSF, 20 μg/ml leupeptin, 10 μg/ml pepstatin A, pH 7.4) was added to each well. The mixture was left on ice for 30 minutes, and the supernatant was then subjected to centrifugal separation at 15,000 rpm for 10 minutes. To the obtained supernatant, 20 μl of the sample buffer for SDS-PAGE was added, followed by boiling for 3 minutes. 18 μl of the resultant product was separated by SDS-PAGE using 12.5% gel (BIO-RAD). After performing electrophoresis, the gel was shaken in a transfer buffer (25 mM Tris, 192 mM glycine, 10% MeOH) for 15 minutes. Thereafter, a protein contained in the gel was transferred to a PVDF membrane (MiniProBlot, Applied Biosystems). The transferred membrane (blot) was blocked using a blocking buffer (50 mM Tris, 150 mM NaCl, 0.1% Tween 20, 1% BSA, pH 7.4) at room temperature for 1 hour. Thereafter, 30 ml of a primary antibody solution (obtained by 1,000 times diluting Phospho p38 MAP Kinase (Thr180/Tyr182) Antibody available from Cell Signaling TECHNOLOGY with the above blocking buffer) was added to the blot, and the mixture was incubated at 4°C overnight. Thereafter, the blot was washed with a washing buffer (20 minutes × 3). Thereafter, 30 ml of a secondary antibody solution (obtained by 1,000 times diluting Anti Rabbit IgG, HRP-linked Antibody available from Cell Signaling TECHNOLOGY with the above blocking buffer) was added to the blot, and the mixture was reacted at room temperature for 1 hour. Thereafter, the blot was washed with a washing buffer (50 mM Tris, 150 mM NaCl, 0.1% Tween 20, pH 7.4) (20 minutes × 3), and it was further washed with a buffer (50 mM Tris, 150 mM NaCl, pH 7.4) (2 minutes × 2), followed by detection of phosphorylated p38 MAP kinase with ECL Plus (Amersham Pharmacia Biotech).

The results are shown in [Figure 9].

It can be read from the figure that, when compared with a control to which no ligands were added, in the cases of adding the human type ZAQ ligand peptide, the human type Bv8 peptide, or the snake venom MIT1, the staining intensity of a phosphorylated p38 MAP kinase (p38 MAPK-P) band was increased. In addition, it was also found that the snake venom MIT1 exhibited such an effect of increasing the staining intensity in a low concentration such as 0.1 nM, and that the human type Bv8 peptide exhibited the same effect in a concentration of 1 nM and the human type ZAQ ligand peptide exhibited the same effect in a concentration of 10 nM.

It is clear that p38 MAP kinase is activated in bovine adrenal capillary endothelial cells (BACE) by addition of a peptide such as the human type ZAQ ligand peptide, the human type Bv8 peptide or the snake venom MIT1.

Accordingly, bovine adrenal capillary endothelial cells are stimulated with the peptide of the present invention such as the human type ZAQ ligand peptide, the human Bv8 peptide or the snake venom MIT1 in the presence or absence of a test compound, so as to know whether or not the test compound has an activity to inhibit the signal transduction of ZAQ or I5E, thereby obtaining an angiogenesis inhibitor.

(4) [$^3$H]thymidine uptake assay

BACE dispersed in a growth medium was inoculated in a 48-well plate such that the concentration became $1.5 \times 10^4$ cells/200 μl/well, followed by culturing overnight. Thereafter, a culture supernatant was removed, and 495 μl of an assay buffer (DMEM containing 0.5% BSA and 0.1% FBS) and 5 μl of a ligand solution were added to the residual culture, followed by further culturing for 15 to 18 hours.

As such a ligand solution, the human type ZAQ ligand peptide, the human type Bv8 peptide, or the snake venom MIT1, which had been prepared by the methods described in Reference examples 14, 8 and 13, was dissolved in H/HBSS containing 0.2% BSA and 0.1% CHAPS, and used.

Thereafter, 50 μl of [$^3$H]thymidine solution (obtained by 100 times diluting NET-027 available from NEN with serum free DMEM) was added to each well, followed by further culturing for 6 hours. Thereafter, a culture supernatant was removed, and 500 μl of a washing buffer was added to each well so as to wash it, and 500 μl of methanol was then added thereto, followed by leaving the mixture on ice for 15 minutes. Thereafter, a supernatant was removed from each well, and 500 μl of 5% trichloroacetic acid (TCA) was added thereto, followed by leaving the mixture on ice for 15 minutes. Thereafter, TCA was removed, and 500 μl of water was added thereto for washing. Finally, 200 μl of 0.3 M NaOH was added thereto and dissolved therein. 3 ml of liquid scintillator was added to the mixture, so that radioactivity was measured with a liquid scintillator counter.

The results are shown in [Figure 10].

From the figure, it is found that when compared with a control to which no ligands were added, in the cases of adding the human type ZAQ ligand peptide, the human type Bv8 peptide, or the snake venom MIT1, the uptake of [$^3$H]thymidine by bovine adrenal capillary endothelial cells was increased.

Accordingly, bovine adrenal capillary endothelial cells are stimulated with the peptide of the present invention such as the human ZAQ ligand peptide, the human Bv8 peptide or the snake venom MIT1 in the presence or absence of a test compound, so as to know whether or not the test compound has an activity to inhibit the signal transduction of ZAQ or I5E, thereby obtaining an angiogenesis inhibitor.

(5) Cell growth assay

BACE dispersed in a growth medium was inoculated in a 24-well plate such that the concentration became $5 \times 10^3$ cells/ml/well, followed by culturing overnight. Thereafter, 10 μl of a solution, which was obtained by dissolving the human type ZAQ ligand peptide or human type Bv8 peptide prepared by the methods described in Reference examples 14 and 8 in H/HBSS containing 0.2% BSA and 0.1% CHAPS, was added to the culture, followed by further culturing for 4 or 5 days. Thereafter, a culture supernatant was removed, and 1 ml of PBS was added to each well so as to wash the cells. Thereafter, 200 μl of a trypsin solution was added thereto, and the cells were recovered with a pipette. The number of the cells was counted with a hemacytometer.
The results are shown in [Figure 11].
From the figure, it is found that when compared with a control to which the above peptide was not added, in the cases of adding the human type ZAQ ligand peptide or the human type Bv8 peptide, the number of bovine adrenal capillary endothelial cells was increased.
Accordingly, bovine adrenal capillary endothelial cells are stimulated with the peptide of the present invention such as the human ZAQ ligand peptide or the human Bv8 peptide in the presence or absence of a test compound, so as to know whether or not the test compound has an activity to inhibit the signal transduction of ZAQ or I5E, thereby obtaining an angiogenesis inhibitor.

Reference example 1 Cloning of cDNA encoding G protein-conjugated receptor protein ZAQ, determination of nucleotide sequence thereof, and analysis of expression distribution of ZAQ

(1) Cloning of cDNA encoding G protein-conjugated receptor protein ZAQ, and determination of nucleotide sequence thereof

Using human pituitary gland cDNA (CLONTECH) as a template, a PCR reaction was carried out with two types of primers, primer 1 (5'-GTC GAC ATG GAG ACC ACC ATG GGG TTC ATG G-3'; SEQ ID NO: 4) and primer 2 (5'-ACT AGT TTA TTT TAG TCT GAT GCA GTC CAC CTC TTC-3'; SEQ ID NO: 5).
As the composition of a reaction solution in the above reaction, using the one tenth of the amount of the above cDNA as a template, a 1/50 amount of Advantage 2 polymerase Mix (CLONTECH), the primers 1 and 2 each having 0.2 μM, 200 μM dNTPs, an enzyme and a buffer supplied with the kit were mixed, so as to set the total liquid amount at 25 μl. The PCR reaction was carried out under reaction conditions consisting of an initial denaturation reaction at 94°C for 2 minutes, 3 cycles of reactions at 94°C for 20 seconds and at 72°C for 100 seconds, 3 cycles of reactions at 94°C for 20 seconds and at 68°C for 100 seconds, 38 cycles of reactions at 94°C for 20 seconds, at 64°C for 20 seconds and at 68°C for 100 seconds, and a final extension reaction at 68°C for 7 minutes. A reaction product obtained as a result of the PCR reaction was subcloned into a plasmid vector pCR2.1 (Invitrogen) using a TA cloning kit (Invitrogen). The plasmid vector was then introduced into *Escherichia coli* DH5α. Thereafter, clones having cDNA were selected in an LB agar medium containing ampicillin, and the sequence of each clone was analyzed, so as to obtain two types of cDNA sequences, ZAQC (SEQ ID NO: 2) and ZAQT (SEQ ID NO: 3), which encode novel G protein-conjugated receptor proteins. Since all of proteins having amino acid sequences derived from the cDNAs have a sequence (SEQ ID NO: 1), these proteins were named as ZAQ. A transformant having DNA shown in SEQ ID NO: 2 was named as *Escherichia coli* DH5α/pCR2.1-ZAQC, and a transformant having DNA shown in SEQ ID NO: 3 was named as *Escherichia coli* DH5α/pCR2.1-ZAQT.

(2) Analysis of expression distribution of ZAQ by Taqman PCR

Primers and probes used in Taqman PCR were searched using Primer Express ver. 1.0 (PE Biosystems Japan), so as to select primer 3 (5'-TCATGTTGCTCCACTGGAAGG-3' (SEQ ID NO: 58)), primer 4 (5'-CCAATTGTCTTGAGGTCCAGG-3' (SEQ ID NO: 29)), and ZAQ probe (5'-TTCTTACAATGGCGGTAAGTCCAGTGCAG-3' (SEQ ID NO: 60)). FAM (6-carboxyfluorescein) was added as a reporter pigment to the probe.
A PCR fragment was amplified with pAK-ZAQC as a template, using a primer ZAQC Sal (5'-GTCGACATGGAGACCACCATGGGGTTCATGG-3' (SEQ ID NO: 61)) and a primer ZAQC Spe

(5'-ACTAGTTTATTTTAGTCTGATGCAGTCCACCTCTTC-3' (SEQ ID NO: 62)).

The amplified PCR fragment was purified using CHROMA SPIN200 (CLONTECH Laboratories, Inc. (CA, USA)), and it was then adjusted at $10^0$-$10^6$ copies/μl, so as to use it as standard DNA. As a cDNA source of each tissue, Human Multiple Tissue cDNA Panel I and Panel II (CLONTECH Laboratories, Inc.) were used. Taqman Universal PCR Master Mix (PE Biosystems Japan) was added to the primers, the probes and the template, in an amount prescribed in the documents attached therewith. Thereafter, a PCR reaction and an analysis were carried out using ABI PRISM 7700 Sequence Detection System (PE Biosystems Japan).

The results are shown in Table 1. Expression of ZAQ was observed, mainly in the testis, and then, in sites such as the lung or brain.

[Table 1]

| Tissue | ZAQ (copies/μl) |
|---|---|
| Brain | 6.1 |
| Heart | 2.9 |
| Kidney | 2.8 |
| Liver | 2.6 |
| Lung | 7.0 |
| Pancreas | 2.1 |
| Placenta | 3.2 |
| Skeletal Muscle | 2.6 |
| Colon | 1.8 |
| Ovary | 3.4 |
| Leukocyte | 0.0 |
| Prostate | 0.7 |
| Small Intestine | 2.2 |
| Spleen | 2.1 |
| Testis | 28.0 |
| Thymus | 1.1 |

Reference example 2 Production of receptor stably expressed CHO cells

(2-1) Production of ZAQ stably expressed cell line

A ZAQ stably expressed cell line was prepared as follows. This is to say, a clone of the DH5α/pCR2.1-ZAQC obtained in Reference example 1 was cultured with shaking in an LB medium containing ampicillin, so as to obtain a plasmid pCR2.1-ZAQC. The plasmid was then treated with restriction enzymes SalI and SpeI, so as to cut out an insert portion encoding ZAQC. Likewise, a portion pAKKO-1.11H (Biochemica et Biophysica Acta 1219 (1994) 251-259) was obtained by treatment with restriction enzymes SalI and SpeI. Thereafter, the pAKKO-1.11H portion was ligated to the above insertion portion using Ligation Express Kit (CLONTECH Laboratories, Inc. (CA, USA)), and the thus ligated portion was introduced into *Escherichia coli* DH10B by electroporation. The structure of a plasmid contained in the obtained clone was confirmed by a restriction enzyme treatment and analysis of the sequence, and a plasmid having a correct structure was used as a plasmid for expression of CHO cells, pAK-ZAQC.

The plasmid pAK-ZAQC was introduced into CHO/dhFr⁻ cells (American Type Culture Collection) for genetic transduction, using CellPhect Transfection kit (Amersham Pharmacia Biotech), so as to obtain cells of interest. First, 120 μl of Buffer A (which was supplied with the CellPhect Transfection Kit) was added to 4 μg of plasmid DNA dissolved in 120 μl of distilled water. The mixture was stirred and then left at rest for 10 minutes. Thereafter, 240 μl of Buffer B (which was supplied with the CellPhect Transfection Kit) was added thereto, and the mixture was intensively stirred, so as to form a DNA-calcium phosphate complex containing the DNA. CHO/dhFr⁻ cells were dispersed in an amount of $5 \times 10^5$ cells on a 60 mm Petri dish, and the cells were cultured in a Ham's F-12 medium (Nissui Pharmaceutical Co., Ltd.) containing 10% fetal bovine serum (BIO WHITTAKER) at 37°C in 5% carbon dioxide for 1 day. Thereafter, 480 μl of a suspension containing the DNA-calcium phosphate complex was added dropwise to the cells on the Petri dish. The mixture was cultured 37°C in 5% carbon dioxide for 6 hours. Thereafter, the cells were washed twice with a Ham's F-12 medium containing no serum. Thereafter, 1.2 ml of buffer solution (140 mM NaCl, 25 mM HEPES, 1.4 mM $Na_2PO_4$, pH 7.1) containing 15% glycerol was added onto the cells on the Petri dish, followed by a treatment for 2 minutes. The thus treated cells were again washed twice with a Ham's F-12 medium containing no serum, and they were then cultured in a Ham's F-12 medium containing 10% fetal bovine serum at 37°C in 5% carbon dioxide overnight. Thereafter, the cells were treated with trypsin and dispersed, and then recovered from the Petri dish. They were then inoculated upon a 6-well plate in a concentration of $2 \times 10^4$ cells/well. Culture of the cells began in Dulbecco's modified Eagle medium (DMEM) (Nissui Pharmaceutical Co., Ltd.) containing dialyzed 10% fetal bovine serum (JRH BIOSCIENCES), 1 mM MEM nonessential amino acid solution (Dainippon Pharmaceutical Co., Ltd.), 100 units/ml penicillin and 100 μg/ml streptomycin at 37°C in 5% carbon dioxide. While the transformed CHO cells in which the plasmid had been introduced were grown in the medium, not-introduced cells were gradually died. Thus, on the 1st and 2nd days after beginning of the culture, the medium was exchanged with a fresh one, so as to eliminate dead cells. At 8, 9 or 10 days after beginning of the culture, approximately 21 colonies of the grown transformed CHO cells were selected. RNA was recovered from each of the selected cells, using a commercially available RNA isolation kit. Thereafter, ZAQ expression CHO cell B-1 clones (hereinafter abbreviated as ZAQC-B1 cells), which highly express ZAQ, were selected by the known RT-PCR method.

(2-2) Production of I5E stably expressed cell line

Human I5E receptor cDNA (SEQ ID NO: 6) was cloned by a known PCR method, and the obtained clone was incorporated into a pAKKO1.11H expression vector. The expression vector was introduced into CHO/dhFr⁻ cells (American Type Culture Collection) for genetic transduction by the method described in the above section (2-1). At 10, 11, 12, 13 or 14 days after beginning of the culture, approximately 20 colonies of the grown transformed CHO cells were selected. The selected cells were inoculated upon a 96-well plate in a concentration of $3 \times 10^4$ cells/well. Reactivity of the cells to the snake venom MTT1 (SEQ ID NO: 21) was studied by a test method described later in the section (2-3). I5E expression CHO cell No. 4 clones (hereinafter abbreviated as I5E-4 cells) with good reactivity were selected.

(2-3) Determination of action to activate receptor

Each of the ZAQC-B1 cells obtained in the above section (2-1) and the I5E-4 cells obtained in the above section (2-2) was suspended in a medium (10% d FBS-DMEM) such that the number of cells became $15 \times 10^4$ cells/ml. 200 μl each of the suspension was inoculated into each well of a FLIPR 96-well plate (Black plate clear bottom, Coster), using a dispersion burette ($3.0 \times 10^4$ cells/200 μl/well). Thereafter, the cells were cultured in a 5% $CO_2$ incubator at 37°C overnight, and then used (hereinafter referred to as a cell plate). 20 ml of H/HBSS (9.8 g of Nissui Hanks' 2 (Nissui Pharmaceutical Co., Ltd.), 0.35 g of sodium bicarbonate, 4.77 g of HEPES, and 6M sodium hydroxide solution were mixed, and the mixture was adjusted to pH 7.4, followed by sterilization with a filter), 200 μl of 250 mM Probenecid, and 200 μl of bovine fetal serum (FBS) were mixed. Moreover, 2 vials (50 μg) of Fluo 3-AM (Dojindo Labolatories) were dissolved in 40 μl of dimethyl sulfoxide and 40 μl of 20% pluronic acid (Molecular Probes), and the obtained solution was added to the above H/HBSS-Probenecid-FBS followed by mixing. Thereafter, using a 8-channel pipette, 100 μl each of the obtained mixture was poured into each well of the cell plate, from which a culture supernatant had been removed. The cell plate was then incubated in 5% $CO_2$ incubator at 37°C for 1 hour (pigment loading). Thereafter, the snake venom MIT1 was diluted with 150 μl of H/HBSS containing 2.5 mM Probenecid and 0.2% BSA, and the thus diluted solution was poured into a FLIPR 96-well plate (V-Bottom plate, Coster) (hereinafter referred to as a sample plate). After pigment was loaded on the cell plate, the cell plate was washed 4 times with a washing buffer prepared by adding 2.5 mM Probenecid to H/HBSS, employing a plate washer (Molecular Devices). After completion of the washing, 100 μl of the washing buffer was kept. The cell plate and the sample plate were placed in FLIPR (50 μl of the sample was transferred from the sample plate to the cell plate by FLIPR). A change in fluorescence intensity was measured over time, so as to determine an activity to increase an intracellular Ca ion concentration.

Reference example 3 Production of human type Bv8 peptide

(3-1) Cloning of human type Bv8 peptide cDNA

Using human testis Marathon-Ready cDNA (CLONTECH) as a template, a primer hBv8-F1 (SEQ ID NO: 7) and a primer hBv8-R1 (SEQ ID NO: 8) were prepared. Using the obtained primers, a PCR reaction described below was carried out.

hBv8-F1: 5'-CTACTTCTGCTGCTGCCGCTGCTGTT-3' (SEQ ID NO: 7)

hBv8-R1: 5'-TTGGAAAGTTGAGGAAGCAAGAGCATTT-3' (SEQ ID NO: 8)

A PCR reaction solution was prepared by mixing 1 μl of 50X Advantage 2 Polymerase Mix (CLONTECH), 5

μl of 10x Advantage 2 PCR buffer supplied with the kit (400 mM Tricine-KOH, 150 mM KOAc, 35 mM $Mg(OAc)_2$, 37.5 μg/ml BSA, 0.05% Tween-20, and 0.05% Nonidet-P40), 4 μl of dNTP mixture (2.5 mM each, Takara Shuzo Co., Ltd.), 1 μl each of 10 μM primers hBv8-F1 and hBv8-R1, 5 μl of template cDNA, and 33 μl of distilled water. After the early denaturation at 95°C for 1 minute, the reaction conditions were set to repeat 35 cycles of reactions at 95°C for 30 seconds and at 68°C for 1 minute, and a final extension reaction at 68°C for 1 minute.

The obtained DNA fragment was cloned using TOPO TA Cloning Kit (Invitrogen) according to the method described in a manual supplied with the kit. The sequence of the cloned DNA was determined using ABI377 DNA Sequencer, so as to obtain a sequence shown in SEQ ID NO: 9.

Using the human tentis Marathon-Ready cDNA (CLONTECH) as a template, a primer hBv8-R2 (SEQ ID NO: 10) was prepared. Using the primer, a 5' RACE experiment described below was carried out.

hBv8-R2: 5′-TGTCTCCCAGTTTGCCCATAGGTGTGC-3′ (SEQ ID NO: 10)

A 5' RACE PCR reaction solution was prepared by mixing 1 μl of 50X Advantage-GC 2 Polymerase Mix (CLONTECH), 10 μl of 5x Advantage-GC 2 PCR buffer supplied with the kit (200 mM Tricine-KOH, 75 mM KOAc, 17.5 mM $Mg(OAc)_2$, 25% dimethyl sulfoxide, 18.75 μg/ml BSA, 0.025% Tween-20, and 0.025% Nonidet-P40), 4 μl of dNTP mixture (2.5 mM each, Takara Shuzo Co., Ltd.), 1 μl of 10 μM primer hBV8-R1, 1 μl of 10 μM primer AP1 (which was supplied with Marathon-Ready cDNA Kit available from CLONTECH), 5 μl of template cDNA (CLONTECH, human tentis Marathon-Ready cDNA), and 28 μl of distilled water. After the early denaturation at 94°C for 1 minute, the reaction conditions were set to repeat 10 cycles of reactions at 94°C for 30 seconds and at 72°C for 3 minutes, 5 cycles of reactions at 94°C for 30 seconds and at 70°C for 3 minutes, and 25 cycles of reactions at 94°C for 30 seconds and at 68°C for 3 minutes.

Subsequently, using a reaction solution obtained from the above PCR reaction as a template, a nested PCR was carried out. A reaction solution was prepared by mixing 1 μl of 50X Advantage-GC 2 Polymerase Mix (CLONTECH), 10 μl of 5x Advantage-GC 2 PCR buffer supplied with the kit (200 mM Tricine-KOH, 75 mM KOAc, 17.5 mM $Mg(OAc)_2$, 25% dimethyl sulfoxide, 18.75 μg/ml BSA, 0.025% Tween-20, and 0.025% Nonidet-P40), 4 μl of dNTP mixture (2.5 mM each, Takara Shuzo Co., Ltd.), 1 μl of 10 μM primer hBv8-R2, 1 μl of 10 μM primer AP2 (which was supplied with Marathon-Ready cDNA Kit available from CLONTECH), 5 μl of template DNA (which was a 50 times diluted solution of the above PCR reaction solution), and 28 μl of distilled water. After the early denaturation at 94°C for 1 minute, the reaction conditions were set to repeat 35 cycles of reactions at 94°C for 30 seconds and at 68°C for 3 minutes.

The obtained DNA fragment was cloned using TOPO TA Cloning Kit (Invitrogen) according to the method described in a manual supplied with the kit. The sequence of the cloned DNA was determined using ABI377 DNA Sequencer, so as to obtain a sequence located at the 5'-terminus (SEQ ID NO: 11).

Primers hBv8-WF (SEQ ID NO: 12), hBv8-WR (SEQ ID NO: 13), hBv8-CF (SEQ ID NO: 14), and hBv8-SR (SEQ ID NO: 15) were prepared based on the information regarding SEQ ID NOS: 9 and 11. Using the obtained primers, a PCR reaction described below was carried out.

hBv8-WF: 5′-CCATGAGGAGCCTGTGCTGCGCC-3′ (SEQ ID NO: 12)

hBv8-WR: 5′-CTATTCACATTTGGTTTCTACTC-3′ (SEQ ID 13)

hBv8-CF: 5′-GTCGACCACCATGAGGAGCCTGTGCTGCG-3′ (SEQ ID NO: 14)

hBv8-SR: 5′-ACTAGTCGATTACTTTTGGGCTAAAC-3′ (SEQ ID NO: 15)

A PCR reaction solution was prepared by mixing 1 μl of PfuTurbo DNA polymerase (Stratagene), 5 μl of 10x PCR buffer supplied with the kit, 4 μl of 2.5 mM dNTP mixture, 2.5 μl each of 10 μM primers hBv8-WF and hBv8-WR, 5 μl of template DNA (human testis Marathon-Ready cDNA, CLONTECH) used as a template, and 30 μl of distilled

water. After the early denaturation at 95°C for 1 minute, the reaction conditions were set to repeat 35 cycles of reactions at 95°C for 1 minute, at 65°C for 1 minute and at 72°C for 1 minute, and a final extension reaction at 72°C for 5 minutes.

Subsequently, using a reaction solution obtained from the above PCR reaction as a template, a nested PCR was carried out. A reaction solution was prepared by mixing 1 µl of PfuTurbo DNA polymerase (Stratagene), 5 µl of 10x PCR buffer supplied with the kit, 4 µl of 2.5 mM dNTP mixture, 2.5 µl each of 10 µM primers hBv8-CF and hBv8-SR, 1 µl of template DNA, and 34 µl of distilled water. After the early denaturation at 95°C for 1 minute, the reaction conditions were set to repeat 35 cycles of reactions at 95°C for 1 minute, at 65°C for 1 minute and at 72°C for 1 minute, and a final extension reaction at 72°C for 5 minutes.

The obtained DNA fragment was cloned using TOPO TA Cloning Kit (Invitrogen) according to the method described in a manual supplied with the kit. The nucleotide sequence of the cloned DNA was determined using ABI377 DNA Sequencer. As a result, it was found that the cloned DNA has a 346 bp nucleotide sequence shown in SEQ ID NO: 16. A plasmid containing a DNA fragment having the nucleotide sequence shown in SEQ ID NO: 16 was named as pHBv. *Escherichia coli* was transformed with the plasmid pHBv, and the thus transformed *Escherichia coli* was named as *Escherichia coli* TOP10/pHBv.

The nucleotide sequence of this DNA fragment was analyzed. As a result, it was found that a DNA fragment shown in SEQ ID NO: 1-6 contains DNA (SEQ ID NO: 18) encoding a human type Bv8 precursor peptide (108 amino acid residues) shown in SEQ ID NO: 17.

Moreover, it was also found that the nucleotide sequence shown in SEQ ID NO: 18 has a typical signal sequence, and that the DNA having the nucleotide sequence shown in SEQ ID NO: 18 contains DNA (SEQ ID NO: 20) consisting of 243 base pairs encoding a human type Bv8 mature peptide (81 amino acid residues) shown in SEQ ID NO: 19.

(3-2) Establishment of human type Bv8 peptide expression CHO cells

An insertion cDNA was cut out of the plasmid pHBv described in the above section (3-1), using restriction enzymes SalI and SpeI, and it was incorporated into a pAKKO1.11H expression vector. The plasmid was introduced into CHO/dhFr⁻ cells (American Type Culture Collection) for genetic transduction, using CellPhect Transfection kit (Amersham Pharmacia Biotech), so as to obtain cells of interest. First, 120 µl of Buffer A (which was supplied with the CellPhect Transfection Kit) was added to 10 µg of plasmid DNA dissolved in 300 µl of distilled water. The mixture was stirred and then left at rest for 10 minutes. Thereafter, 240 µl of Buffer B (which was supplied with the CellPhect Transfection Kit) was added thereto, and the mixture was intensively stirred, so as to form a DNA-calcium phosphate complex containing the DNA. CHO/dhFr⁻ cells were dispersed in an amount of $4 \times 10^5$ cells on a 60 mm Petri dish, and the cells were cultured in a Ham's F-12 medium (Nissui Pharmaceutical Co., Ltd.) containing 10% fetal bovine serum (BIO WHITTAKER) and nonessential amino acid for MEM at 37°C in 5% carbon dioxide for 1 day. Thereafter, the above complex was added dropwise onto the cells on the Petri dish. The mixture was cultured 37°C in 5% carbon dioxide for 7 hours. Thereafter, the cells were washed twice with a Ham's F-12 medium containing no serum. Thereafter, 3 ml of 15% glycerol was added onto the cells on the Petri dish, followed by a treatment for 2 minutes. The thus treated cells were again washed twice with a Ham's F-12 medium containing no serum, and they were then cultured in a Ham's F-12 medium containing 10% fetal bovine serum at 37°C in 5% carbon dioxide for 15 hours. Thereafter, the cells were treated with trypsin and dispersed, and then recovered from the Petri dish. They were then inoculated upon a 6-well plate in a concentration of $1.25 \times 10^4$ cells/well. Culture of the cells began in Dulbecco's modified Eagle medium (DMEM) (Nissui Pharmaceutical Co., Ltd.) containing dialyzed 10% fetal bovine serum (JRH BIOSCIENCES), 1 mM MEM nonessential amino acid solution, 100 units/ml penicillin and 100 µg/ml streptomycin at 37°C in 5% carbon dioxide. While the transformed CHO cells in which the plasmid had been introduced were grown in the medium, not-introduced cells were gradually died. Thus, the medium was exchanged with a fresh one every two days, so as to eliminate dead cells. At 10, 11, 12, 13 or 14 days after beginning of the culture, approximately 29 colonies of the grown transformed CHO cells were selected. Each selected cell was inoculated on a 24-well plate. After the cell had become confluent, the medium was exchanged with a Dulbecco's modified Eagle medium (not containing Phenol Red, GIBCO) containing 10% fetal bovine serum (JRH BIOSCIENCES), 1 mM MEM nonessential amino acid solution, 100 units/ml penicillin and 100 µg/ml streptomycin, followed by culturing for 3 days. Thereafter, a culture supernatant was recovered, and the ability of ZAQ expression CHO cells to activate ZAQ was determined using a FLIPR calcium assay kit according to the method described in the above section (2-3). Thus, a ZAQL-2 expression CHO cell clone No. 2 (hereinafter abbreviated as ZAQL-2/CHO No. 4), which highly expresses human type Bv8, was selected.

(3-3) Preparation of serum free culture supernatant of human type Bv8 peptide expression CHO cells

4 pieces of Single Tray (Nunc) were cultured in a Dulbecco's Modified Eagle Medium (DMEM) (Nissui Pharmaceutical Co., Ltd.) containing dialyzed 10% fetal bovine serum (JRH BIOSCIENCES), 1 mM MEM nonessential amino acid solution, 100 units/ml penicillin and 100 μg/ml streptomycin, until they became confluent. The obtained ZAQL-2/CHO No. 4 was treated with trypsin and dispersed, and then recovered by centrifugal separation. Cells contained in a piece of the above Single Tray were suspended in 1.5 L of the above medium, and the suspension was inoculated on Cell Factories 10 (Nunc). Thereafter, 4 sets of Cell Factories 10 were cultured at 37°C in 5% carbon dioxide for 3 days. A culture supernatant was removed, and then, a set of Cell Factories 10 was washed with 1 L of the above H/HBSS. After the H/HBSS was removed, 2 L of serum free medium (which was a Dulbecco's Modified Eagle Medium containing 1 mM MEM nonessential amino acid solution, 100 units/ml penicillin and 100 μg/ml streptomycin) was added per a set of Cell Factories, followed by further culturing for 2 days. The recovered culture supernatant was centrifuged at 1,000 rpm for 10 minutes using a Hitachi high speed centrifuge, and the resultant product was filtrated with gauze, so as to obtain a pure supernatant. Acetic acid was added to the supernatant to a final concentration of 1 M, and the following operation was carried out.

(3-4) Rough fractionation of serum free culture supernatant of human type Bv8 peptide expression CHO cells by octadodecyl reverse phase chromatography

Silica gel, to which an octadodecyl group was fixed, was filled in PrepC18 (Waters), and it was swollen with methanol. The obtained product was filled in a glass column (manufactured by BioRad; internal diameter of 5 cm, height of 10 cm). Thereafter, the extract obtained in the above section (3-3) was applied to the column that had been equilibrated with 1 M acetic acid. Thereafter, the column was washed with 800 ml of 1 M acetic acid. Thereafter, 1,000 ml of 60% acetonitrile/0.1% trifluoroacetic acid was run through the column, so as to elute rough peptide components of interest. The obtained eluant was concentrated with an evaporator, and the obtained concentrate was freeze-dried with a freeze-drier (12EL; VirTis).

(3-5) Separation by Wakosil-II 5C18HG Prep reverse phase high performance liquid chromatography

A solution consisting of 91.7 % by volume of liquid A (0.1% trifluoroacetic acid/distilled water) and 8.3% by volume of liquid B (0.1% trifluoroacetic acid/60% acetonitrile) was run at 40°C at a flow rate of 5 ml/minute through a Wakosil-II 5C18HG Prep reverse phase high performance liquid chromatography column (Wako Pure Chemical Industries, Ltd., 20 mm × 250 mm), so that the column was equilibrated. The freeze-dried product obtained in the above section (3-4) was subjected to a chromatographic operation. This is to say, 36 ml of 1 M acetic acid was added to and dissolved in the freeze-dried product, and the mixture was centrifuged. The one third of the resultant product was applied to the above column. Thereafter, the concentration of the liquid B was increased in a linear gradient manner at a flow rate of 5 ml/minute over 1 minute to 66.7 % by volume of liquid A/33.3 % by volume of liquid B, and then further increased over 120 minutes to 16.7 % by volume of liquid A/83.3 % by volume of liquid B. The eluant was divided into fractions each consisting of 5 ml, and a fraction number was assigned to each fraction. This operation was carried out 3 times, so that the freeze-dried product of the above section (3-4) was treated. 3 μl of an aliquot from the above each fraction was mixed with 150 μl of 0.2% BSA, and the mixture was freeze-dried with a freeze-drier (12EL; VirTis). 150 μl of assay buffer [prepared by adding 2.5 mM Probenecid and 0.1% CHAPS to H/HBSS (prepared by mixing 9.8 g of Nissui Hanks' 2 (Nissui Pharmaceutical Co., Ltd.), 0.35 g of sodium bicarbonate, 4.77 g of HEPES and sodium hydroxide solution, and adjusting the mixture to pH 7.4, followed by sterilization with a filter)] was added to and dissolved in the above freeze-dried product. Using 50 μl of the obtained solution, an action to activate a ZAQ receptor was determined according to the method described in the above section (2-3). As a result, it was found that a component of interest having an action to activate a ZAQ receptor was eluted mainly in fractions with fraction Nos. 73 and 74.

(3-6) Separation by TSKgel CM-2SW ion exchange high performance liquid chromatography

A solution consisting of 100% by volume of liquid A (4 M ammonium formate : distilled water : acetonitrile = 1 : 299 : 100) and 0% by volume of liquid B (4 M ammonium formate : distilled water : acetonitrile = 1 : 2 : 1) was run at 25°C at a flow rate of 1 ml/minute through a TSKgel CM-2SW ion exchange high performance liquid chromatography column (Tosoh Corp., 4.6 mm × 250 mm), so that the column was equilibrated. The product obtained by freeze-drying fraction Nos. 73 and 74 obtained in the above section (3-5) was dissolved in 4 ml of liquid A, and the obtained solution was applied to the TSKgel CM-2SW ion exchange column. Thereafter, the concentration of the liquid B was increased in a linear gradient manner at a flow rate of 2 ml/minute over 120 minutes to 25% by volume of liquid A/75% by volume of liquid B, and the eluant was then recovered. The eluant was divided into fractions each consisting of 2 ml, and a fraction number was assigned to each fraction. 2 μl of an aliquot from the above each fraction was mixed with 100 μl of 0.2% BSA, and the mixture was freeze-dried with a freeze-drier (12EL; VirTis). 100 μl of the above described assay buffer was added to and dissolved in the freeze-dried product. The obtained solution was 100 times diluted with the same above buffer. Thereafter, using the obtained solution, an action to activate a ZAQ receptor was determined according to the method described in the above section

(2-3). As a result, it was found that a component of interest having an action to activate a ZAQ receptor was eluted mainly in fractions with fraction Nos. 83 to 85.

(3-7) Purification by TSKgel ODS-80Ts reverse phase high performance liquid chromatography

A solution consisting of 91.7% by volume of liquid A (0.1% trifluoroacetic acid/distilled water) and 8.3% by volume of liquid B (0.1% trifluoroacetic acid/60% acetonitrile) was run at 40°C at a flow rate of 1 ml/minute through a TSKgel ODS-80Ts reverse phase high performance liquid chromatography column (Tosoh Corp., 4.6 mm × 100 mm), so that the column was equilibrated. The product obtained by freeze-drying fraction Nos. 83 to 85 obtained in the above section (3-6) was subjected to a chromatographic operation. This is to say, 2 ml of 1 M acetic acid was added to the freeze-dried product to dissolve it, and the obtained solution was applied to the above column. Thereafter, the concentration of the liquid B was increased in a linear gradient manner at a flow rate of 1 ml/minute over 1 minute to 75% by volume of liquid A/25% by volume of liquid B, and then further increased over 60 minutes to 25% by volume of liquid A/75% by volume of liquid B. Then, the eluant was recovered. The eluant was divided into fractions each consisting of 0.5 ml, and a fraction number was assigned to each fraction. 1 μl of an aliquot was collected from each of fraction Nos. 121 to 130, and it was then 100 times diluted with the same above buffer. Thereafter, using the obtained solution, an action to activate a ZAQ receptor was determined according to the method described in the above section (2-3). As a result, it was found that a component of interest having an action to activate a ZAQ receptor was eluted mainly in fractions with fraction Nos. 123 to 125.

(3-8) Purification by TSKgel Super-Phenyl reverse phase high performance liquid chromatography

A solution consisting of 91.7% by volume of liquid A (0.1% trifluoroacetic acid/distilled water) and 8.3% by volume of liquid B (0.1% trifluoroacetic acid/60% acetonitrile) was run at 40°C at a flow rate of 1 ml/minute through a TSKgel Super-Phenyl reverse phase high performance liquid chromatography column (Tosoh Corp., 4.6 mm × 100 mm), so that the column was equilibrated. The fractions with fraction Nos. 123 to 125 obtained in the section (3-7) were subjected to a chromatographic operation. This is to say, a fraction solution was applied to the above column by direct loading. Thereafter, the concentration of the liquid B was increased in a linear gradient manner at a flow rate of 1 ml/minute over 1 minute to 75% by volume of liquid A/25% by volume of liquid B, and then further increased over 60 minutes to 25% by volume of liquid A/75% by volume of liquid B. Then, the eluant was recovered. The eluant was divided into fractions each consisting of 0.5 ml, and a fraction number was assigned to each fraction. 1 μl of an aliquot was collected from each of fraction Nos. 91 to 100, and it was then 100 times diluted with the same above buffer. Thereafter, using the obtained solution, an action to activate a ZAQ receptor was determined according to the method described in the above section (2-3). As a result, it was found that a component of interest having an action to activate a ZAQ receptor was eluted mainly in fractions with fraction Nos. 97 and 98.

(3-9) Repurification by TSKgel Super-Phenyl reverse phase high performance liquid chromatography

A solution consisting of 91.7% by volume of liquid A (0.1% trifluoroacetic acid/distilled water) and 8.3% by volume of liquid B (0.1% trifluoroacetic acid/60% acetonitrile) was run at 40°C at a flow rate of 1 ml/minute through a TSKgel Super-Phenyl reverse phase high performance liquid chromatography column (Tosoh Corp., 4.6 mm × 100 mm), so that the column was equilibrated. The fractions with fraction Nos. 97 and 98 obtained in the section (3-8) were subjected to a chromatographic operation. This is to say, a fraction solution was applied to the above column by direct loading. Thereafter, the concentration of the liquid B was increased in a linear gradient manner at a flow rate of 1 ml/minute over 1 minute to 75% by volume of liquid A/25% by volume of liquid B, and then further increased over 60 minutes to 25% by volume of liquid A/75% by volume of liquid B. Then, the eluant was recovered. As a result, it was found that a component of interest having an action to activate a ZAQ receptor was eluted as a single peak.

(3-10) Analysis of structure of purified human type Bv8 peptide

The structure of the main component for activating ZAQ obtained in the above section (3-9) was determined by a method described below. The purified human type Bv8 peptide was freeze-dried with a freeze-drier (12EL; VirTis). The obtained freeze-dried peptide was dissolved in DMSO (dimethyl sulfoxide). An aliquot from the obtained solution was subjected to analysis of an N-terminal amino acid sequence, using a protein sequencer (Perkin-Elmer, PE Biosystems Procise 491cLC). As a result, it was found that the obtained N-terminal amino acid sequence was identical to the amino acid sequence shown in SEQ ID NO: 19, which is predicted from the human type Bv8 mature peptide. In addition, using a Finnigan LCQ LC/MS device, mass spectrometry was carried out on the main component for activating ZAQ according to the electron spray ionization method. The molecular weight was calculated to be 8664.34. This measurement value is smaller by 128.2 than 8792.54, a theoretical value for the human type Bv8 mature peptide (SEQ ID NO: 19). Thus, it was confirmed that a peptide (SEQ ID NO: 22) formed by omission of a Lys residue at the C terminus from the mature peptide was obtained.

Reference example 4 Determination of reactivity of human type Bv8 peptide

The action of the purified human type Bv8 peptide obtained in Reference example 3 to activate ZAQ and I5E receptors was determined according to the method described in the above section (2-3).
As a result, it was found that the human type Bv8 peptide concentration-dependently induces increase of an intracellular calcium concentration in ZAQ receptor expression CHO cells and I5E receptor expression CHO cells. Moreover, it was also found that the snake venom MIT1 had an action to activate an I5E receptor, which was 10 times greater than that of the human type Bv8 peptide. The results are shown in [Figure 1] and [Figure 2].

Reference example 5 Cloning of rat type Bv8 peptide cDNA

Using rat testis Marathon-Ready cDNA (CLONTECH) as a template, degenerate primers BF2 (SEQ ID NO: 23) and BR1 (SEQ ID NO: 24) were prepared. Using the obtained primers, a PCR reaction described below was carried out.

BF2: 5'-GCTTGYGACAAGGACTCYCA-3' (SEQ ID NO: 23)

BR1: 5'-GTTYCTACTYCAGAGYGAT-3' (SEQ ID NO: 24)

A PCR reaction solution was prepared by mixing 0.4 µl of 50X Advantage 2 Polymerase Mix (CLONTECH), 2 µl of 10x Advantage 2 PCR buffer supplied with the kit (400 mM Tricine-KOH, 150 mM KOAc, 35 mM $Mg(OAc)_2$, 37.5 µg/ml BSA, 0.05% Tween-20, and 0.05% Nonidet-P40), 1 to 6 µl of dNTP mixture (2.5 mM each, Takara Shuzo Co., Ltd.), 0.4 µl each of 10 µM primers BF2 and BR1, 2 µl of template cDNA, and 13.2 µl of distilled water. After the early denaturation at 95°C for 1 minute, the reaction conditions were set to repeat 40 cycles of reactions at 95°C for 30 seconds, at 55°C for 1 minute and at 68°C for 1 minute, and a final extension reaction at 68°C for 5 minutes.
The obtained DNA fragment was cloned using TOPO TA Cloning Kit (Invitrogen) according to the method described in a manual supplied with the kit. The sequence of the cloned DNA was determined using ABI377 DNA Sequencer, so as to obtain a partial sequence shown in SEQ ID NO: 25.
A primer RB5-1 (SEQ ID NO: 26) and a primer RB5-3 (SEQ ID NO: 27) were prepared based on the information of the above sequence. Using the obtained primers, a 5' RACE experiment described below was carried out.

RB5-1: 5'-GTGCATCCTCCGCCCCCAAAATGGAA-3' (SEQ ID NO: 26)

RB5-3: 5'-GACAGCGCAGCACATTCCTCCTCCACAC-3' (SEQ ID NO: 27)

A 5' RACE PCR reaction solution was prepared by mixing 1 µl of 50X Advantage 2 Polymerase Mix (CLONTECH), 5 µl of 10x Advantage 2 PCR buffer supplied with the kit (400 mM Tricine-KOH, 150 mM KOAc, 35 mM $Mg(OAc)_2$, 37.5 µg/ml BSA, 0.05% Tween-20, and 0.05% Nonidet-P40), 4 µl of dNTP mixture (2.5 mM each, Takara Shuzo Co., Ltd.), 1 µl of 10 µM primer RB5-1, 1 µl of 10 µM primer AP1 (which was supplied with Marathon-Ready cDNA Kit available from CLONTECH), 5 µl of template cDNA (CLONTECH, rat tentis Marathon-Ready cDNA), and 33 µl of distilled water. After the early denaturation at 94°C for 1 minute, the reaction conditions were set to repeat 5 cycles of reactions at 94°C for 30 seconds and at 72°C for 3 minutes, 5 cycles of reactions at 94°C for 30 seconds and at 70°C for 3 minutes, and 25 cycles of reactions at 94°C for 30 seconds and at 68°C for 3 minutes.
Subsequently, using a reaction solution obtained from the above PCR reaction as a template, a nested PCR was carried out. A reaction solution was prepared by mixing 1 µl of 50X Advantage 2 Polymerase Mix (CLONTECH), 5 µl of 10x Advantage 2 PCR buffer supplied with the kit (400 mM Tricine-KOH, 150 mM KOAc, 35 mM $Mg(OAc)_2$,

37.5 μg/ml BSA, 0.05% Tween-20, and 0.05% Nonidet-P40), 4 μl of dNTP mixture (2.5 mM each, Takara Shuzo Co., Ltd.), 1 μl of 10 μM primer RB5-3, 1 μl of 10 μM primer AP2 (which was supplied with Marathon-Ready cDNA Kit available from CLONTECH), 5 μl of template cDNA (which was a 50 times diluted solution of the above PCR reaction solution), and 33 μl of distilled water. After the early denaturation at 94°C for 1 minute, the reaction conditions were set to repeat 35 cycles of reactions at 94°C for 30 seconds and at 68°C for 3 minutes.

The obtained DNA fragment was cloned using TOPO TA Cloning Kit (Invitrogen) according to the method described in a manual supplied with the kit. The nucleotide sequence of the cloned DNA was determined using ABI377 DNA Sequencer, so as to obtain a sequence located at the 5'-terminus (SEQ ID NO: 28).

Primers RB3-1 (SEQ ID NO: 29) and RB3-2 (SEQ ID NO: 30) were prepared based on the information regarding SEQ ID NO: 25. Using the obtained primers, a 3' RACE experiment described below was carried out.

RB3-1: 5'-GAGACAGCTGCCACCCCCTGACTCGGAA-3' (SEQ ID NO: 29)

RB3-2: 5'-GGCGGAGGATGCACCACACTTGTCCCTG-3' (SEQ ID NO: 30)

A 3' RACE PCR reaction solution was prepared by mixing 1 μl of 50X Advantage 2 Polymerase Mix (CLONTECH), 5 μl of 10x Advantage 2 PCR buffer supplied with the kit (400 mM Tricine-KOH, 150 mM KOAc, 35 mM Mg$(OAc)_2$, 37.5 μg/ml BSA, 0.05% Tween-20, and 0.05% Nonidet-P40), 4 μl of dNTP mixture (2.5 mM each, Takara Shuzo Co., Ltd.), 1 μl of 10 μM primer RB3-1, 1 μl of 10 μM primer AP1 (which was supplied with Marathon-Ready cDNA Kit available from CLONTECH), 5 μl of template cDNA (CLONTECH, rat tentis Marathon-Ready cDNA), and 33 μl of distilled water. After the early denaturation at 94°C for 1 minute, the reaction conditions were set to repeat 5 cycles of reactions at 94°C for 30 seconds and at 72°C for 3 minutes, 5 cycles of reactions at 94°C for 30 seconds and at 70°C for 3 minutes, and 25 cycles of reactions at 94°C for 30 seconds and at 68°C for 3 minutes.

Subsequently, using a reaction solution obtained from the above PCR reaction as a template, a nested PCR was carried out. A reaction solution was prepared by mixing 1 μl of 50X Advantage 2 Polymerase Mix (CLONTECH), 5 μl of 10x Advantage 2 PCR buffer supplied with the kit (400 mM Tricine-KOH, 150 mM KOAc, 35 mM Mg$(OAc)_2$, 37.5 μg/ml BSA, 0.05% Tween-20, and 0.05% Nonidet-P40), 4 μl of dNTP mixture (2.5 mM each, Takara Shuzo Co., Ltd.), 1 μl of 10 μM primer RB3-2, 1 μl of 10 μM primer AP2 (which was supplied with Marathon-Ready cDNA Kit available from CLONTECH), 5 μl of template cDNA (which was a 50 times diluted solution of the above PCR reaction solution), and 33 μl of distilled water. After the early denaturation at 94°C for 1 minute, the reaction conditions were set to repeat 35 cycles of reactions at 94°C for 30 seconds and at 68°C for 3 minutes.

The obtained DNA fragment was cloned using TOPO TA Cloning Kit (Invitrogen) according to the method described in a manual supplied with the kit. The nucleotide sequence of the cloned DNA was determined using ABI377 DNA Sequencer, so as to obtain a sequence located at the 3'-terminus (SEQ ID NO: 31).

Using rat brain Marathon Ready cDNA (CLONTECH) as a template, primers RBv8-WF1 (SEQ ID NO: 32), RBv8-WF2 (SEQ ID NO: 33), RBv8-WR1 (SEQ ID NO: 34) and RBv8-WR2 (SEQ ID NO: 35) were prepared based on the information obtained from the above 5' RACE and 3' RACE. Using the obtained primers, a PCR reaction described below was carried out.

RBv8-WF1: 5'-TAACCGCCACCGCCTCCT-3' (SEQ ID NO: 32)

RBv8-WF2: 5'-GGGACGCCATGGAGGAC-3' (SEQ ID NO: 33)

RBv8-WR1: 5'-CGAGACTTGACAGACATTGTTCAGTG-3' (SEQ ID NO: 34)

RBv8-WR2: 5'-TTTCCAGCTCCTGCTTCAGA-3' (SEQ ID NO: 35)

A PCR reaction solution was prepared by mixing 0.6 μl of PfuTurbo DNA polymerase (Stratagene), 3 μl of 10x PCR buffer supplied with the kit, 2.4 μl of 2.5 mM dNTP mixture, 1.5 μl each of 10 μM primers RBv8-WF1 and RBv8-WR1, 3 μl of template DNA, and 18 μl of distilled water. After the early denaturation at 95°C for 1 minute, the reaction conditions were set to repeat 35 cycles of reactions at 95°C for 30 seconds, at 55°C for 30 seconds and at 72°C for 1 minute, and a final extension reaction at 72°C for 5 minutes.

Subsequently, using, as a template, a solution prepared by 50 times diluting the reaction solution from the above PCR reaction with distilled water, a nested PCR was carried out. A reaction solution was prepared by mixing 0.6 μl of PfuTurbo DNA polymerase (Stratagene), 3 μl of 10x PCR buffer supplied with the kit, 2.4 μl of 2.5 mM dNTP mixture, 1.5 μl each of 10 μM primers RBv8-WF2 and RBv8-WR2, 3 μl of template DNA, and 18 μl of distilled water. After the early denaturation at 95°C for 1 minute, the reaction conditions were set to repeat 35 cycles of reactions at 95°C for 30 seconds, at 55°C for 30 seconds and at 72°C for 1 minute, and a final extension reaction at 72°C for 5 minutes.

The obtained DNA fragment was cloned using Zero Blunt TOPO PCR Cloning Kit (Invitrogen) according to the method described in a manual supplied with the kit. The nucleotide sequence of the cloned DNA was determined using ABI377 DNA Sequencer. As a result, it was found that the cloned DNA has a 356 bp nucleotide sequence shown in SEQ ID NO: 36. A plasmid containing a DNA fragment having the nucleotide sequence shown in SEQ ID NO: 36 was named as pRBv. *Escherichia coli* was transformed with the plasmid pRBv, and the transformed *Escherichia coli* was named as *Escherichia coli* TOP10/pRBv.

The nucleotide sequence of this DNA fragment was analyzed. As a result, it was found that a DNA fragment shown in SEQ ID NO: 36 contains DNA (SEQ ID NO: 38) encoding a rat type Bv8 precursor peptide (107 amino acid residues) shown in SEQ ID NO: 37.

Moreover, it was also found that the nucleotide sequence shown in SEQ ID NO: 38 has a typical signal sequence, and that the DNA having the nucleotide sequence shown in SEQ ID NO: 38 contains DNA (SEQ ID NO: 40) consisting of 243 base pairs encoding a rat type Bv8 mature peptide (81 amino acid residues) shown in SEQ ID NO: 39.

Reference example 6 Cloning of cDNA encoding rat brain cDNA-derived novel G protein-conjugated receptor protein (rZAQ1), and determination of nucleotide sequence thereof

Using rat total brain cDNA library (CLONTECH) as a template, a PCR reaction was carried out with two types of primers (SEQ ID NOS: 41 and 42). As the composition of a reaction solution in the above reaction, using the one tenth of the amount of the above cDNA as a template, a 1/50 amount of Advantage 2 cDNA polymerase Mix (CLONTECH), the above primers each having 0.2 μM, 200 μM dNTPs, an enzyme and a buffer supplied with the kit were mixed, so as to set the total liquid amount at 25 μl. The PCR reaction was carried out under reaction conditions consisting of (i) an initial denaturation reaction at 94°C for 2 minutes, (ii) 3 cycles of reactions at 94°C for 20 seconds and at 72°C for 90 seconds, (iii) 3 cycles of reactions at 94°C for 20 seconds and at 68°C for 90 seconds, (iv) 36 cycles of reactions at 94°C for 20 seconds, at 62°C for 20 seconds and at 68°C for 1 minute, and a final extension reaction at 68°C for 7 minutes. A reaction product obtained as a result of the PCR reaction was subcloned into a plasmid vector pCR2.1-TOPO (Invitrogen) using a TOPO-TA cloning kit (Invitrogen). The plasmid vector was then introduced into *Escherichia coli* DH5α. Thereafter, clones having cDNA were selected in an LB agar medium containing ampicillin. The sequence of each clone was analyzed, so as to obtain cDNA (SEQ ID NO: 43) encoding a novel G protein-conjugated receptor protein. The amino acid sequence (SEQ ID NO: 44) derived from the nucleotide sequence of the above cDNAs had homology of 83.7% with the amino acid sequence shown in SEQ ID NO. 1. A novel G protein-conjugated receptor protein having this amino acid sequence was named as rZAQ1. Moreover, a transformant (*Escherichia coli*) containing DNA having the nucleotide sequence shown in SEQ ID NO: 43 was named as *Escherichia coli* DH5α/pCR2.1-rZAQ1.

Reference example 7 Cloning of cDNA encoding rat brain cDNA-derived novel G protein-conjugated receptor protein (rZAQ2), and determination of nucleotide sequence thereof

A clone encoding rZAQ2 was obtained by the gene trapper method. This is to say, probes (SEQ ID NOS: 45 and 46) were biotinylated. Thereafter, the probe was subjected to hybridization with a single-stranded rat total brain cDNA library (GIBCO-BRL), so as to obtain a single-stranded gene. The obtained single-stranded gene was repaired with primers (SEQ ID NOS: 47 and 48) and thereby converted into a double-stranded gene. The obtained gene was introduced into *Escherichia coli* DH10B by electroporation, so as to obtain a transformant, using resistance to ampicillin as an indicator. Moreover, a colony PCR was carried out using a probe (SEQ ID NO: 45) and a primer (SEQ ID NO: 49), so as to select a clone encoding a nucleotide sequence of interest. The amino acid sequence (SEQ ID NO: 51) derived from the nucleotide sequence (SEQ ID NO: 50) of an ORF (open reading frame) estimated from the nucleotide sequence of the obtained clone had homology of 80.6% with rZAQ1. A novel G protein-conjugated receptor protein having the above amino acid sequence was named as rZAQ2. Moreover, a transformant (*Escherichia coli*) obtained by the gene trapper method was named as *Escherichia coli* DH10B/pCMV-rZAQ2.

Reference example 8 Production of human type Bv8 peptide (SEQ ID NO: 19) in *Escherichia coli*

(1) Construction of human type Bv8 peptide expression plasmid in *Escherichia coli*

(a) Preparation of structural gene of human type Bv8

Using 6 types of DNA fragments #1 to #6 (SEQ ID NOS: 65 to 70), a structural gene of human type Bv8 was prepared.

(b) Phosphorylation of DNA oligomer

Each of 4 types of DNA oligomers (#2 to #5), from which the above fragments #1 and #6 to be on the 5'-terminal side had been eliminated, was reacted at 37°C for 1 hour in 25 µl of phosphoryalation reaction solution [10 µg of DNA oligomer, 50 mM Tris-HCl, pH 7.6, 10 mM $MgCl_2$, 1 mM spermidine, 10 mM dithiothreitol (hereinafter abbreviated as DTT), 0.1 mg/ml bovine serum albumin (hereinafter abbreviated as BSA), 1 mM ATP, 10 units of T4 polynucleotide kinase (Takara Shuzo Co., Ltd.)], so that the 5'-terminus of each oligomer was phosphorylated. The resultant product was then treated with phenol, and ethanol was added thereto in an amount of 2 times its volume. The mixture was cooled to -70°C and then subjected to centrifugal separation, so as to deposit DNA.

(c) Ligation of DNA fragment

The DNA fragments obtained in (b) above were mixed with the above fragments #1 and #6, and the total amount was set at 120 µl. The mixed solution was retained at 90°C for 10 minutes, and then it was gradually cooled to room temperature. Thereafter, the solution was subjected to annealing, and then subjected to a ligation reaction using TaKaRa DNA Ligation Kit ver. 2 (Takara Shuzo Co., Ltd.). 30 µl of solution II attached with the kit was added to 30 µl of annealing solution, and the mixture was well blended. Thereafter, 60 µl of solution I attached with the kit was added to the mixture, followed by a reaction at 37°C for 1 hour for ligation. Thereafter, the reaction product was treated with phenol, and water layers were recovered. Ethanol was added to the layers in an amount of 2 times their volume. The mixture was cooled to -70°C and then subjected to centrifugal separation, so as to deposit DNA. The thus obtained DNA fragments were phosphorylated using T4 polynucleotide kinase (Takara Shuzo Co., Ltd.), and the obtained fragments were used in the following step (d).

(d) Construction of human type Bv8 expression vector

To construct an expression vector, pTCII (Japanese Patent Laid-Open No. 2000-178297) was digested with NdeI and BamHI (Takara Shuzo Co., Ltd.) at 37°C for 2 hours. Thereafter, the digest was subjected to 1% agarose gel electrophoresis, so that a 4.3 kb DNA fragment was extracted using QIAquick Gel Extraction Kit (QIAGEN). The obtained fragment was then dissolved in 25 µl of TE buffer solution. The NdeI-BalHI fragment of the pTCII and the structural gene of human type Bv8 (SEQ ID NO: 71) as prepared above were subjected to a ligation reaction, using TaKaRa DNA ligation kit ver. 2 (Takara Shuzo Co., Ltd.). *Escherichia coli* JM109 competent cells (Toyobo Co., Ltd.) were transformed with 10 µl of the obtained reaction solution. The transformed cells were dispersed on an LB agar medium containing 10 µg/ml tetracycline, and they were cultured at 37°C overnight, followed by selection of the generated tetracycline-resistant colonies. The obtained transformant was cultured in an LB medium overnight, and then, a plasmid pTCh2ZAQ was prepared using QIAprep8 Miniprep Kit (QIAGEN). The nucleotide sequence of the human type Bv8 DNA was determined using Applied Biosystems Model 377 DNA Sequencer. *Escherichia coli* MM294 (DE3) was transformed with the above plasmid pTCh2ZAQ, so as to obtain human type Bv8 expression line *Escherichia coli* MM294 (DE3)/pTCh2ZAQ.

(2) Production of human type Bv8 peptide

The above *Escherichia coli* MM294 (DE3)/pTCh2ZAQ was added to 1 L of LB medium (1% peptone, 0.5% yeast extract, 0.5% sodium chloride) containing 5.0 mg/L tetracycline placed in a 2 L volume flask, and it was subjected to a shake culture at 37°C for 8 hours. The obtained culture solution was transferred into a 50 L volume fermenter in which 19 L of main fermentation medium (1 %-68% sodium hydrogenphosphate monobasic, 0.3% potassium hydrogenphosphate dibasic, 0.1% ammonium chloride, 0.05% sodium chloride, 0.05% magnesium sulfate, 0.02% antifoaming agent, 0.00025% ferrous sulfate, 0.0005% thiamine hydrochloride, 1.5% glucose, 1.5% high-case amino) was stored, and then, aeration agitation began at 30°C. When the turbidity of the culture solution became 500 Klett units, isopropyl-β-D-thiogalactopyranoside was added thereto to a final concentration of 12 mg/L, followed by further culturing for 4 hours. After completion of the culture, the culture solution was centrifuged to obtain approximately 500 g of wet cell bodies, which were then conserved at -80°C.

(3) Activation of human type Bv8 peptide

800 ml of solution (pH 8.0) containing 200 mM Tris/HCl and 7 M guanidine hydrochloride was added to 400 g of the cell bodies obtained in (2) above, so as to dissolve the cell bodies. Thereafter, the obtained solution was subjected to centrifugal separation (10,000 rpm, 1 hour). 20 L of solution (pH 8.0) containing 0.4 M arginine, 50 mM Tris/HCl, 0.2 mM GSSG (oxidized glutathione) and 1 mM GSH (reduced glutathione) was added to the obtained supernatant, and activation was carried out at 4°C overnight.

(4) Purification of human type Bv8 peptide

A regenerant obtained as a result of activation in (3) above was adjusted to pH 6.0, and it was adsorbed to an SP-sepharose column (11.3 cm × 15 cm) equilibrated with a 50 mM phosphate buffer solution (pH 6.0). Thereafter, elution was carried out with a 600 mM NaCl/50 mM phosphate buffer solution (pH 6.0), so as to pool a fraction containing human type Bv8. This fraction was passed through SP-5PW (21.5 mm × 150 mmL) equilibrated with a 50 mM phosphate buffer solution (pH 6.0), and it was adsorbed thereto and washed. Thereafter, elution was carried out with a stepwise gradient from 0% to 100% B (B = 50 mM phosphate buffer solution + 1 M NaCl, pH 6.05) (60 minutes), so as to obtain a human type Bv8 fraction (elution time: approximately 40 minutes). This fraction was passed through C4P-50 (21.5 mm ID × 300 mm L, Showa Denko K.K.) equilibrated with 0.1% trifluoroacetic acid, and it was adsorbed thereto and washed. Thereafter, elution was carried out with a stepwise gradient from 25% to 50% B (B = 80% acetonitrile/0.1% trifluoroacetic acid) (60 minutes), so as to pool a human type Bv8 fraction (elution time: approximately 30 minutes). The fraction was then freeze-dried, so as to obtain approximately 25 mg of human type Bv8 freeze-dried powders.

(5) Determination of characteristics of human type Bv8 peptide

(a) Analysis by SDS polyacrylamide gel electrophoresis

Human type Bv8 obtained in (4) above was suspended in a sample buffer [Laemmli, Nature, 227, 680 (1979)] to which 100 mM DTT was added, and the suspension was heated at 95°C for 1 minute, followed by electrophoresis with Multigel 15/25 (Daiichi Pure Chemicals Co., Ltd.). The electrophoresed gel was stained with Coomassie brilliant blue. As a result, a single protein band was observed at a position of 9 kDa. From this result, it was found that the recombinant human type Bv8 sample derived from *Escherichia coli* obtained in (4) above has high purity.

(b) Analysis of amino acid composition

Amino acid composition was determined by an amino acid analyzer (Hitachi L-8500A Amino Acid Analyzer). As a result, it was found that the analyzed amino acid composition was identical to the amino acid composition estimated from the nucleotide sequence of DNA of human type Bv8 (a peptide consisting of an amino acid sequence shown in SEQ ID NO: 19) (Table 2).

[Table 2]

| Amino acid | Number of residues per mole | Value estimated from nucleotide sequence of human type Bv8 |
|---|---|---|
| Asx | 4.0 | 4 |
| Thr[1)] | 4.6 | 5 |
| Ser[1)] | 4.3 | 5 |
| Glx | 2.3 | 2 |
| Pro | 5.2 | 5 |
| Gly | 7.8 | 8 |
| Ala | 5.0 | 5 |

1) Value extrapolated at 0 time

[Table 2] (continued)

| Amino acid | Number of residues per mole | Value estimated from nucleotide sequence of human type Bv8 |
|---|---|---|
| Cys[2)] | N.D. | 10 |
| Val | 3.8 | 4 |
| Met | 2.8 | 3 |
| Ile | 4.2 | 5 |
| Leu | 6 | 6 |
| Tyr | 0 | 0 |
| Phe | 3.7 | 4 |
| His | 2.9 | 3 |
| Lys | 4.9 | 5 |
| Arg | 5.8 | 6 |
| Trp | 0.9 | 1 |
| Acid hydrolysis (6 N HCl-4% thioglycolic acid, 110°C, mean value obtained from hydrolyses for 24 and 48 hours) | | |

2) Undetected

(c) Analysis of N-terminal amino acid sequence

An N-terminal amino acid sequence was determined using a gas phase protein sequencer (PE Applied Biosystems Model 492). As a result, it was found that the determined amino acid sequence was identical to the N-terminal amino acid sequence of human type Bv8, which was estimated from the nucleotide sequence of DNA of the human type Bv8 (Table 3).

[Table 3]

| Residue No. | Detected PTH-amino acid[1)] (pmol) | Amino acid estimated from nucleotide sequence of human type Bv8 |
|---|---|---|
| 1 | Ala(103) | Ala |
| 2 | Val(99) | Val |
| 3 | Ile(88) | Ile |
| 4 | Thr(54) | Thr |
| 5 | Gly(66) | Gly |
| 6 | Ala(79) | Ala |
| 7 | N.D. | Cys |
| 8 | Asp(47) | Asp |
| 9 | Lys(62) | Lys |
| 10 | Asp(50) | Asp |
| 11 | Ser(36) | Ser |
| 12 | Gln(52) | Gln |
| 13 | N.D. | Cys |
| 14 | Gly(44) | Gly |
| 15 | Gly(55) | Gly |
| 16 | Gly(56) | Gly |
| 17 | Met(50) | Met |
| 18 | N.D. | Cys |
| 19 | N.D. | Cys |
| 20 | Ala(33) | Ala |

1) Amino acid was detected as a PTH (phenylthiohydantoin) derivative.
Human type Bv8 was analyzed in an amount of 150 pmol.
N. D. indicates "undetected."

(d) Analysis of C-terminal amino acid

A C-terminal amino acid was determined using an amino acid analyzer (Hitachi L-8500A Amino Acid Analyzer). The determined C-terminal amino acid was identical to the C-terminal amino acid estimated from the nucleotide sequence of the DNA encoding the human type Bv8 (Table 4).

[Table 4]

| C-terminal amino acid | Recovery rate (%) |
|---|---|
| Lys | 18.9 |
| Gas phase hydrazinolysis (100°C, 3.5 hours) | |

(6) Determination of activity of human type Bv8 peptide (determination of activity thereof to increase intracellular Ca ion concentration by FLIPR)

The activity of the purified recombinant human type Bv8 sample derived from *Escherichia coli* obtained in (4) above was determined by the method described in the section (2-3) in Reference example 2 (determination of the activity thereof to increase an intracellular Ca ion concentration by FLIPR). As a result, it was found that the above sample had an activity equivalent to that of a recombinant sample derived from CHO cells (a purified product of human type Bv8; Reference example 3).

Reference example 9 Cloning of cDNA of human type ZAQ ligand peptide

(I) Isolation of peptide for activating ZAQ

(1) Preparation of cow milk extract

The following operations were performed on commercially available low-temperature sterilized cow milk to prepare an extract. 2 liter of cow milk was centrifuged at 10,000 rpm for 15 minutes at 4°C using a high speed centrifuge (CR26H, R10A rotor; Hitachi, Ltd.). The obtained supernatant was filtrated with gauze, so as to eliminate lipid sections. Acetic acid was added to the supernatant to a final concentration of 1 M, and the mixture was stirred at 4°C for 30 minutes. Subsequently, the resultant product was centrifuged at 10,000 rpm for 15 minutes using a high speed centrifuge (CR26H, R10A rotor; Hitachi, Ltd.). The obtained supernatant was filtrated with gauze, so as to eliminate insoluble portions. While stirring, acetone was added to the supernatant in an amount of two times its volume, followed by stirring at 4°C for 3 hours. Subsequently, the resultant product was centrifuged at 10,000 rpm for 15 minutes using a high speed centrifuge (CR26H, R10A rotor; Hitachi, Ltd.). The obtained supernatant was filtrated with gauze, so as to eliminate insoluble portions. The obtained supernatant was concentrated with a rotary evaporator to eliminate acetone, and eventually it was concentrated to 1,350 ml. 675 ml each from the obtained concentrate was mixed with 338 ml of diethyl ether, and the mixture was intensively blended in a separatory funnel. The mixture was then separated into two phases, and thus, a water phase was obtained. The same above operations were performed once again on the obtained water phase, so as to obtain a pure water phase. The obtained water phase was concentrated to 800 ml with a rotary evaporator, so as to obtain a final extract.

(2) Rough fractionation of cow milk extract by C18 reverse phase chromatography

10 g of silica gel, to which an octadodecyl group was fixed, was filled in a column Sep-Pak C18 (Waters), and it was then swollen with methanol. The obtained product was equilibrated with 1 M acetic acid. The extract (corresponding to 2 liter of cow milk) prepared in (1) above was applied to the column. Subsequently, 100 ml of 1 M acetic acid was run through the column, so that the gel was washed. Thereafter, 200 ml of solution consisting of 60% acetonitrile/0.1% trifluoroacetic acid was run through the column, so as to elute rough peptide components of interest. The obtained eluant was concentrated with an evaporator, and the obtained concentrate was freeze-dried with a freeze-drier (12EL; VirTis).

(3) Rough fractionation of cow milk extract by sulfopropyl ion exchange chromatography

SP Sephadex C-25 (Amersham Pharmacia Biotech) was swollen in 100 mM hydrochloric acid, and it was then filled in a polypropylene column such that the volume became 2 ml. Thereafter, it was washed with distilled water and 2 M ammonium formate (pH 4.0) and then equilibrated with liquid I (2 M ammonium formate : acetonitrile : water = 1 : 25 : 74). The freeze-dried product obtained in (2) above was dissolved in 20 ml of liquid I, and the obtained solution was then loaded onto 2 ml of SP Sephadex C-25. After washing with 10 ml of liquid I, elution was carried out successively with 10 ml each of liquid II (2 M ammonium formate : acetonitrile : water = 1 : 2.5 : 6.5), liquid III (2 M ammonium formate : acetonitrile : water = 1 : 1 : 2), and liquid IV (2 M ammonium formate : acetonitrile : water = 1 : 0.5 : 0.5). Each of the obtained liquids I to IV was freeze-dried with a freeze-drier (12EL; VirTis).

(4) Fractionation of cow milk extract by TSKgel ODS80Ts reverse phase high performance liquid chromatography

A solution consisting of 91.7 % by volume of liquid A (0.1% trifluoroacetic acid/distilled water) and 8.3% by volume of liquid B (0.1% trifluoroacetic acid/60% acetonitrile) was run at 40°C at a flow rate of 1 ml/minute through a TSKgel ODS-80Ts reverse phase high performance liquid chromatography column (Tosoh Corp., 4.6 mm × 25 cm), so that the column was equilibrated. Each of the freeze-dried products of liquids I to IV obtained in (3) above was dissolved in 4 ml of 1M acetic acid, and the obtained solution was subjected to a chromatographic operation. This is to say, 4 ml of the solution containing the above freeze-dried product was applied to the above column. Thereafter, the concentration of the liquid B was increased in a linear gradient manner at a flow rate of 1 ml/minute over 1 minute to 67% by volume of liquid A/33% by volume of liquid B, and then further increased over 40 minutes from 67% by volume of liquid A/33% by volume of liquid B to 0% by volume of liquid A/100% by volume of liquid B.

The eluant was divided into fractions each consisting of 1 ml, and a fraction number was assigned to each fraction. 2 µl of an aliquot from the above each fraction was mixed with 150 µl of 0.2% bovine serum albumin (BSA)/ distilled water, and the mixture was then freeze-dried. The obtained dried product was used as an assay sample used in determination of an activity to increase an intracellular $Ca^{2+}$ ion concentration, which will be described later in (5).

(5) Determination of activity to increase intracellular $Ca^{2+}$ ion concentration by FLIPR

A ZAQ stably expressed cell line was prepared as follows. This is to say, a clone of the DH5α/pCR2.1-ZAQC obtained in Example 1 was subjected to a shake culture in an LB medium containing ampicillin, so as to obtain a plasmid pCR2.1-ZAQC. The plasmid was then treated with restriction enzymes SalI and SpeI, so as to cut out an insert portion encoding ZAQC. Likewise, a portion pAKKO-1.11H (Biochemica et Biophysica Acta 1219 (1994) 251-259) was obtained by treatment with restriction enzymes SalI and SpeI. Thereafter, the pAKKO-1.11H portion (Biochemica et Biophysica Acta 1219 (1994) 251-259) was ligated to the above insertion portion using Ligation Express Kit (CLONTECH Laboratories, Inc. (CA, USA)), and the thus ligated portion was introduced into *Escherichia coli* DH10B by electroporation. The structure of a plasmid contained in the obtained clone was confirmed by a restriction enzyme treatment and analysis of the sequence, and a plasmid having a correct structure was used as a plasmid for expression of CHO cells, pAK-ZAQC.

The plasmid pAK-ZAQC was introduced into CHO/dhFr⁻ cells (American Type Culture Collection) for genetic transduction, using CellPhect Transfection kit (Amersham Pharmacia Biotech), so as to obtain cells of interest. First, 120 µl of Buffer A (which was supplied with the CellPhect Transfection Kit) was added to 4 µg of plasmid DNA dissolved in 120 µl of distilled water. The mixture was stirred and then left at rest for 10 minutes. Thereafter, 240 µl of Buffer B (which was supplied with the CellPhect Transfection Kit) was added thereto, and the mixture was intensively stirred, so as to form a DNA-calcium phosphate complex containing the DNA. CHO/dhFr⁻ cells were dispersed in an amount of $5 \times 10^5$ cells on a 60 mm Petri dish, and the cells were cultured in a Ham's F-12 medium (Nissui Pharmaceutical Co., Ltd.) containing 10% fetal bovine serum (BIO WHITTAKER) at 37°C in 5% carbon dioxide for 1 day. Thereafter, 480 µl of a suspension containing the DNA-calcium phosphate complex was added dropwise to the cells on the Petri dish. The mixture was cultured 37°C in 5% carbon dioxide for 6 hours. Thereafter, the cells were washed twice with a Ham's F-12 medium containing no serum. Thereafter, 1.2 ml of buffer solution (140 mM NaCl, 25 mM HEPES, 1.4 mM $Na_2HPO_4$, pH 7.1) containing 15% glycerol was added onto the cells on the Petri dish, followed by a treatment for 2 minutes. The thus treated cells were washed twice with a Ham's F-12 medium containing no serum again, and they were then cultured in a Ham's F-12 medium containing 10% fetal bovine serum at 37°C in 5% carbon dioxide overnight. Thereafter, the cells were dispersed by a trypsin treatment and recovered from the Petri dish, and they were inoculated upon a 6-well plate in a concentration of $2 \times 10^4$ cells/well. Culture of the cells began in Dulbecco's modified Eagle medium (DMEM) (Nissui Pharmaceutical Co., Ltd.) containing dialyzed 10% fetal bovine serum (JRH BIOSCIENCES), 1 mM MEM nonessential amino acid solution (Dainippon Pharmaceutical Co., Ltd.), 100 units/ml penicillin and 100 µg/ ml streptomycin at 37°C in 5% carbon dioxide. While the transformed CHO cells in which the plasmid had been introduced were grown in the medium, not-introduced cells were gradually died. Thus, after beginning of the culture, the medium was exchanged with a fresh one every two days, so as to eliminate dead cells. At 8, 9 or 10 days after beginning of the culture, 21 colonies of the grown transformed CHO cells were selected. RNA was recovered from each of the selected cells, using a commercially available RNA isolation kit. Thereafter, ZAQ expression CHO cell B-1 clones (hereinafter abbreviated as ZAQC-B1 cells), which highly express ZAQ, were selected by the known RT-PCR method.

Further, as a control, an ETA (endothelin A receptor) expression CHO cell No. 24 clone (hereinafter abbreviated as an ETA 24 cell; refer to Journal of Pharmacology and Experimental Therapeutics, vol. 279, pp. 675 to 685, 1996) was used.

The activity of the assay sample obtained in (4) above to increase an intracellular $Ca^{2+}$ ion concentration both in ZAQC-B1 cells and in ETA 24 cells was determined using FLIPR (Molecular Devices). In both cases of the ZAQC-B1 cells and the ETA 24 cells, the cells used herein had been subcultured in DMEM to which 10% dialyzed fetal bovine serum (hereinafter referred to as d FBS) had been added. Each of the ZAQC-B1 cells and the ETA 24 cells was suspended in a medium (10% d FBS-DMEM) such that the number of cells became $15 \times 10^4$ cells/ml. 200 μl each of the suspension was inoculated into each well of a FLIPR 96-well plate (Black plate clear bottom, Coster), using a dispersion burette ($3.0 \times 10^4$ cells/200 μl/well). Thereafter, the cells were cultured in a 5% $CO_2$ incubator at 37°C overnight, and then used (hereinafter referred to as a cell plate). 20 ml of H/HBSS (9.8 g of Nissui Hanks' 2 (Nissui Pharmaceutical Co., Ltd.), 0.35 g of sodium bicarbonate, 4.77 g of HEPES and sodium hydroxide solution were mixed, and the mixture was adjusted to pH 7.4, followed by sterilization with a filter), 200 μl of 250 mM Probenecid, and 200 μl of bovine fetal serum (FBS) were mixed. Moreover, 2 vials (50 μg) of Fluo 3-AM (Dojindo Labolatories) were dissolved in 40 μl of dimethyl sulfoxide and 40 μl of 20% pluronic acid (Molecular Probes), and the obtained solution was added to the above H/HBSS-Probenecid-FBS followed by mixing. Thereafter, using a 8-channel pipette, 100 μl each of the obtained mixture was poured into each well of the cell plate, from which a culture supernatant had been removed. The cell plate was then incubated in 5% $CO_2$ incubator at 37°C for 1 hour (pigment loading). Thereafter, each fraction of the assay sample obtained in (4) above was diluted with 150 μl of H/HBSS containing 2.5 mM Probenecid and 0.1 % CHAPS, and the thus diluted solution was transferred to a FLIPR 96-well plate (V-Bottom plate, Coster) (hereinafter referred to as a sample plate). After pigment was loaded on the cell plate, the cell plate was washed 4 times with a washing buffer prepared by adding 2.5 mM Probenecid to H/HBSS, employing a plate washer (Molecular Devices). After completion of the washing, 100 μl of the washing buffer was kept. The cell plate and the sample plate were placed in FLIPR, followed by performing assay (50 μl of the sample was transferred from the sample plate to the cell plate by FLIPR).

As a result, an activity to increase an intracellular $Ca^{2+}$ ion concentration that is specific for ZAQC-B1 cells was observed in fraction No. 53 obtained by performing the reverse phase high performance liquid chromatography described in (4) above on the IV liquid described in (3) above.

(6) Purification by TSKgel Super-Phenyl reverse phase high performance liquid chromatography

A solution consisting of 91.7% by volume of liquid A (0.1% trifluoroacetic acid/distilled water) and 8.3% by volume of liquid B (0.1% trifluoroacetic acid/60% acetonitrile) was run at 40°C at a flow rate of 1 ml/minute through a TSKgel Super-Phenyl reverse phase high performance liquid chromatography column (Tosoh Corp., 0.46 cm × 10 cm), so that the column was equilibrated. The fraction with fraction No. 53 obtained in (4) above was subjected to a chromatographic operation. This is to say, 1 ml of the solution with fraction No. 53 was applied to the above column, and then, the concentration of the liquid B was increased in a linear gradient manner at a flow rate of 1 ml/minute over 1 minute to 75% by volume of liquid A/25% by volume of liquid B, and then further increased over 75 minutes to 67% by volume of liquid A/33% by volume of liquid B.

The eluant was recovered and divided into fractions each consisting of 500 μl, and a fraction number was assigned to each fraction. 25 μl of an aliquot was collected from each fraction, and it was mixed with 150 μl of 0.2% BSA. The mixture was then freeze-dried with a freeze-drier (21EL; VirTis). 150 μl of H/HBSS containing 2.5 mM Probenecid and 0.1% CHAPS was added to and dissolved in the freeze-dried product. Thereafter, using 50 μl of the obtained solution, an activity to increase an intracellular $Ca^{2+}$ ion concentration was determined according to the test method described in (5) above, so as to determine an action to activate receptors to the ZAQC-B1 cells of interest. As a result, it was found that a component of interest having an action to activate receptors to the ZAQC-B1 cells, that is, a ZAQ-activating component was eluted mainly in fractions with fraction Nos. 103 to 105.

(7) Purification by μRPC C2/C18 ST 4.6/100 reverse phase high performance liquid chromatography

A solution consisting of 95% by volume of liquid A (heptafluorobutyric acid/distilled water) and 5% by volume of liquid B (0.1% heptafluorobutyric acid/100% acetonitrile) was run at 40°C at a flow rate of 1 ml/minute through a μRPC C2/C18 ST 4.6/100 reverse phase high performance liquid chromatography column (Amersham Pharmacia Biotech, 0.46 cm × 10 cm), so that the column was equilibrated.

The fractions with fraction Nos. 103 to 105 obtained by the TSKgel Super-Phenyl reverse phase high performance liquid chromatography described in (6) above were directly added to the μRPC C2/C18 ST 4.6/100 reverse phase column. Thereafter, the concentration of the liquid B was rapidly increased in a linear gradient manner at a flow rate of 1 ml/minute over 1 minute from 95% by volume of liquid A (0.1% heptafluorobutyric acid/distilled water) and 5% by volume of liquid B (0.1% heptafluorobutyric acid/100% acetonitrile) to 65% by volume of liquid A/35% by volume of liquid B, and then further increased at a flow rate of 1 ml/minute over 60 minutes to 50% by volume of liquid A/50% by volume of liquid B. Then, the eluant was recovered. The eluant was detected as a single peak by ultraviolet absorption at 210 nm.

The eluant was recovered and divided into fractions each consisting of 500 µl, and a fraction number was assigned to each fraction. 10 µl of an aliquot was collected from each fraction, and it was mixed with 150 µl of 0.2% BSA. The mixture was then freeze-dried with a freeze-drier (21EL; VirTis). 75 µl of H/HBSS containing 2.5 mM Probenecid and 0.1% CHAPS was added to and dissolved in the freeze-dried product. Thereafter, using 50 µl of the obtained solution, an action to activate receptors to ZAQC-B1 cells was determined according to the test method described in (5) above. As a result, it was found that a component of interest having an action to activate receptors to the ZAQC-B 1 cells, that is, a ZAQ-activating component was eluted mainly in fractions with fraction Nos. 82 to 84. This activity peak was completely identical to the ultraviolet absorption peak at 210 nm, and it is therefore considered that it was purified to be a single peptide.

(8) Analysis of structure of purified ZAQ-activating peptide

The structure of the ZAQ-activating component obtained in (7) above was determined by a method described below. The purified sample of a ZAQ-activating component was freeze-dried. The obtained freeze-dried product was dissolved in DMSO (dimethyl sulfoxide). An aliquot from the obtained solution was subjected to analysis of an N-terminal amino acid sequence, using a protein sequencer (Perkin-Elmer, PE Biosystems Procise 491cLC). As a result, 14 amino acid residues out of the 16 amino acid residues from the N-terminus could be identified (Ala Val Ile Thr Gly Ala Xaa Glu Arg Asp Val Gln Xaa Arg Ala Gly (SEQ ID NO: 72; Xaa was an unidentified residue herein)).

(II) Cloning of cDNA of human type ZAQ ligand peptide

The N-terminal amino acid sequence (SEQ ID NO: 72) of a peptide for activating ZAQ purified from cow milk obtained in (I) above, was used as a query, and a Blast search of the given query was performed against a database of sequences. As a result, a human EST (X40467) was found, which contains a sequence equivalent to the nucleotide sequence of DNA encoding a peptide having the amino acid sequence shown in SEQ ID NO: 72. Since this sequence did not have a full-length open reading frame, the sequence of an identified portion was clarified by an RACE method described below, and a cDNA clone having a full-length open reading frame was then obtained.

Primers ZF1 (SEQ ID NO: 73), ZF2 (SEQ ID NO: 74) and ZF3 (SEQ ID NO: 75) were prepared based on the information regarding the EST (E40467). Using human testis Marathon-Ready cDNA (CLONTECH) as a template, a 3' RACE experiment described below was carried out.

ZF1: 5'-GGTGCCACGCGAGTCTCAATCATGCTCC-3' (SEQ ID NO: 73)

ZF2: 5'-GGGGCCTGTGAGCGGGATGTCCAGTGTG-3' (SEQ ID NO: 74)

ZF3: 5'-CTTCTTCAGGAAACGCAAGCACCACACC-3' (SEQ ID NO: 75)

A 3' RACE PCR reaction solution was prepared by mixing 1 µl of 50x Advantage 2 Polymerase Mix (CLONTECH), 5 µl of 10x Advantage 2 PCR buffer supplied with the kit (400 mM Tricine-KOH, 150 mM KOAc, 35 mM Mg $(OAc)_2$, 37.5 µg/ml BSA, 0.05% Tween-20, and 0.05% Nonidet-P40), 4 µl of dNTP mixture (2.5 mM each, Takara Shuzo Co., Ltd.), 1 µl of 10 µM primer ZF1, 1 µl of 10 µM primer AP1 (which was supplied with Marathon-Ready cDNA Kit available from CLONTECH), 5 µl of template cDNA (CLONTECH, human tentis Marathon-Ready cDNA), and 33 µl of distilled water. After the early denaturation at 94°C for 60 seconds, the reaction conditions were set to repeat 5 cycles of reactions at 94°C for 30 seconds and at 72°C for 4 minutes, 5 cycles of reactions at 94°C for 30 seconds and at 70°C for 4 minutes, and 25 cycles of reactions at 94°C for 30 seconds and at 68°C for 44 minutes.

Subsequently, using a reaction solution obtained from the above PCR reaction as a template, a nested PCR was carried out. A reaction solution was prepared by mixing 1 µl of 50x Advantage 2 Polymerase Mix (CLONTECH), 5 µl of 10x Advantage 2 PCR buffer supplied with the kit (400 mM Tricine-KOH, 150 mM KOAc, 35 mM Mg(OAc)$_2$, 37.5 µg/ml BSA, 0.05% Tween-20, and 0.05% Nonidet-P40), 4 µl of dNTP mixture (2.5 mM each, Takara Shuzo Co., Ltd.), 1 µl of 10 µM primer ZF2, 1 µl of 10 µM primer AP2 (which was supplied with Marathon-Ready cDNA Kit available from CLONTECH), 5 µl of template DNA (which was a 50 times diluted solution of the above PCR reaction solution), and 33 µl of distilled water. After the early denaturation at 94°C for 60 seconds, the reaction conditions were set to repeat 5 cycles of reactions at 94°C for 30 seconds and at 72°C for 4 minutes, 5 cycles of reactions at 94°C for 30 seconds and at 70°C for 4 minutes, and 25 cycles of reactions at 94°C for 30 seconds and at 68°C for 44 minutes.

Subsequently, using a reaction solution obtained from the above PCR reaction as a template, a second nested PCR was carried out. A reaction solution was prepared by mixing 1 µl of 50x Advantage 2 Polymerase Mix (CLONTECH), 5 µl of 10x Advantage 2 PCR buffer supplied with the kit (400 mM Tricine-KOH, 150 mM KOAc, 35 mM Mg (OAc)$_2$, 37.5 µg/ml BSA, 0.05% Tween-20, and 0.05% Nonidet-P40), 4 µl of dNTP mixture (2.5 mM each, Takara Shuzo Co., Ltd.), 1 µl of 10 µM primer ZF3, 1 µl of 10 µM primer AP2 (which was supplied with Marathon-Ready cDNA Kit available from CLONTECH), 5 µl of template DNA (which was a 50 times diluted solution of the above PCR reaction solution), and 33 µl of distilled water. After the early denaturation at 94°C for 60 seconds, the reaction conditions were set to repeat 5 cycles of reactions at 94°C for 30 seconds and at 72°C for 4 minutes, 5 cycles of reactions at 94°C for 30 seconds and at 70°C for 4 minutes, and 25 cycles of reactions at 94°C for 30 seconds and at 68°C for 44 minutes. The obtained DNA fragment was cloned using TOPO TA Cloning Kit (Invitrogen) according to the method described in a manual supplied with the kit. The nucleotide sequence of the cloned DNA was determined using ABI377 DNA Sequencer, so as to obtain a sequence located at the 3'-terminus (SEQ ID NO: 76).

Primers ZAQL-CF (SEQ ID NO: 77) and ZAQL-XR1 (SEQ ID NO: 78) were prepared based on the information obtained regarding the nucleotide sequence shown in SEQ ID NO: 76 and the EST (X40467). Using human testis Marathon-Ready cDNA (CLONTECH), a PCR reaction was carried out with the primers ZAQL-CF and ZAQL-XR1.

ZAQL-CF: 5'-CCACCATGAGAGGTGCCACG-3' (SEQ ID NO: 77)

ZAQL-XR1: 5'-CTCGAGCTCAGGAAAAGGATGGTG-3' (SEQ ID NO: 78)

A PCR reaction solution was prepared by mixing 1 µl of PfuTurbo DNA polymerase (Stratagene), 5 µl of 10x PCR buffer supplied with the kit, 4 µl of 2.5 mM dNTP mixture, 2.5 µl each of 10 µM primers ZAQL-CF and ZAQL-XR1, 5 µl of template DNA, and 30 µl of distilled water. After the early denaturation at 95°C for 1 minute, the reaction conditions were set to repeat 40 cycles of reactions at 95°C for 1 minute, at 60°C for 1 minute and at 72°C for 1 minute, and a final extension reaction at 72°C for 10 minutes. The obtained DNA fragment was cloned using TOPO TA Cloning Kit (Invitrogen) according to the method described in a manual supplied with the kit. The nucleotide sequences of the cloned DNA fragments were determined using ABI377 DNA Sequencer. As a result, it was found that the cloned DNA fragments have a 371 bp nucleotide sequence shown in SEQ ID NO: 79 and a 371 bp nucleotide sequence shown in SEQ ID NO: 80. A plasmid containing a DNA fragment having the nucleotide sequence shown in SEQ ID NO: 79 was named as pHMITA, and a plasmid containing a DNA fragment having the nucleotide sequence shown in SEQ ID NO: 80 was named as pHMITG

*Escherichia coli* was transformed with each of the plasmids pHMITA and pHMITG, and the 2 types of the transformed *Escherichia coli* were named as *Escherichia coli* TOP10/pHMITA and *Escherichia coli* TOP10/pHMITG, respectively.

The nucleotide sequences of these DNA fragments were analyzed. As a result, it was found that the DNA fragment shown in SEQ ID NO: 79 contains DNA (SEQ ID NO: 89) encoding a human type ZAQ ligand precursor peptide (type A, 105 amino acid residues) shown in SEQ ID NO: 83, and that the DNA fragment shown in SEQ ID NO: 80 contains DNA (SEQ ID NO: 90) encoding a human type ZAQ ligand precursor peptide (type G, 105 amino acid residues) shown in SEQ ID NO: 84.

Moreover, it was also found that the nucleotide sequences shown in SEQ ID NOS: 89 and 90 have typical signal sequences, that the DNA having the nucleotide sequence shown in SEQ ID NO: 89 contains DNA (SEQ ID NO: 87) consisting of 258 base pairs encoding a human type ZAQ ligand mature peptide (type A, 86 amino acid residues) shown in SEQ ID NO: 81, and that the DNA having the nucleotide sequence shown in SEQ ID NO: 90 contains DNA (SEQ ID NO: 88) consisting of 258 base pairs encoding a human type ZAQ ligand mature peptide (type G, 86 amino acid residues) shown in SEQ ID NO: 82.

Reference example 10 Generation of human type ZAQ ligand peptide in mammalian cells (1)

(1) Construction of human type ZAQ ligand precursor peptide mammalian cell expression vector

A 382 bp DNA fragment (SEQ ID NO: 91) containing cDNA encoding a human type ZAQ ligand precursor peptide was cut out of the plasmid pHMITG obtained in (II) of Reference example 9 by digestion with restriction enzymes EcoRI and XhoI.

This is to say, the plasmid pHMITG was digested with restriction enzymes EcoRI and XhoI, and the obtained DNA was electrophoresed with 1.5% agarose gel. A gel portion containing a band with a size of approximately 382 bp to be stained with cyber green was cut out with a razor. Thereafter, a DNA fragment was recovered from the gel portion using Gene Clean spin DNA extraction kit (BIO 101). The obtained DNA fragment was cloned into the EcoRI-XhoI restriction site of a mammalian cell expression vector pCAN618 containing a CMV-IE enhancer and a chicken beta-actin promoter as an expression promoter. The nucleotide sequence of the cloned DNA fragment was determined by the above described method. As a result, it was confirmed that the DNA fragment has a nucleotide sequence shown in SEQ ID NO: 91. This mammalian cell expression vector containing DNA encoding a human type ZAQ ligand precursor peptide was named as pCANZAQLg2.

(2) Introduction of expression vector into COS7 cells

COS7 cells were purchased from ATCC, and they were subcultured in a DMEM medium (containing 10% FBS) and used. A DMEM medium containing COS7 cells was dispersed on a 10 cm Petri dish, such that the concentration became $1.5 \times 10^6$ cells/dish. The cells were cultured at 37°C in a 5% $CO_2$ incubator overnight. 298 μl of buffer EC (Effectene transfection reagent, QIAGEN) was added to 2 μg of the human type ZAQ ligand precursor peptide expression plasmid (pCANZAQLg2) (which was dissolved in 2 μl of TE buffer), and 16 μl of the enhancer was further added thereto, followed by mixing for 1 second. Thereafter, the mixture was left at room temperature for 3 minutes. Thereafter, 60 μl of Effectene Transfection Reagent was further added thereto, followed by mixing for 10 seconds. Thereafter, the mixture was left at room temperature for 10 minutes. A supernatant obtained from the cells that had been dispersed on the preceding day was eliminated, and the residue was washed once with 10 ml of DMEM medium. Thereafter, 9 ml of DMEM medium was added thereto. 1 ml of DMEM medium was added to a plasmid solution, and they were blended. The obtained mixture was added dropwise to the cells, and the mixture was blended. Thereafter, the mixture was cultured at 37°C in a 5% $CO_2$ incubator overnight. The obtained culture was washed twice with 10 ml of DMEM medium, and 10 ml of DMEM medium was added to the culture, followed by culturing at 37°C in a 5% $CO_2$ incubator overnight. 2 days later, the culture supernatant was recovered.

(3) Partial purification of peptide for activating ZAQ from culture supernatant of human type ZAQ ligand precursor peptide expression COS7 cells

(3-1) Preparation of culture supernatant extract of human type ZAQ ligand precursor peptide expression COS7 cells

The culture supernatant of human type ZAQ ligand precursor peptide expression COS7 cells was recovered, and an extract was prepared in the following operations. First, 1.1 ml of acetic acid was added dropwise to a cell culture supernatant (approximately 18.5 ml) to a final concentration of 1 M, followed by stirring for 1 hour. Thereafter acetone was added thereto in an amount of 2 times the volume of the mixture, followed by stirring at 4°C for 30 minutes. Subsequently, the mixture was centrifuged at 15,000 rpm for 30 minutes using a high speed centrifuge (CR26H, 23 type rotor, Hitachi Ltd.), so as to obtain a supernatant. Acetone was removed from the obtained supernatant using an evaporator, and the resultant product was then freeze-dried with a freeze-drier (12EL; VirTis).

(3-2) Sephadex G50 gel filtration chromatography and SepPak column chromatography on culture supernatant of human type ZAQ ligand precursor peptide expression COS7 cells

The freeze-dried powders obtained in the above section (3-1) were dissolved in 2 ml of 1 M acetic acid, and the obtained solution was adsorbed onto a Sephadex G15 column (diameter: 3 cm, 35 ml, Pharmacia Biotech) equilibrated with 1 M acetic acid. Thereafter, 1 M acetic acid was run through the column, and the eluant was divided into fractions each consisting of 5 ml, and a fraction number was assigned to each fraction. The fractions were then freeze-dried with a freeze-drier (12E1; VirTis).

A SepPak C18-5g column (10 ml) was swollen with methanol, and 0.1% trifluoroacetic acid/distilled water was run through the column, so that the column was equilibrated. Of the fractions obtained as a result of the Sephadex G50 gel filtration chromatography, freeze-dried products with fraction Nos. 1 to 16 were all dissolved in 3 ml of 0.1% trifluoroacetic acid/distilled water. The obtained solution was applied to the SepPak C18-5g column, and the column was then washed with 24 ml of 0.1% trifluoroacetic acid/distilled water, followed by elution with 20 ml of 0.1% trifluroacetic acid/60% acetonitrile. The obtained eluant was evaporated on a Savant centrifugal concentrator.

(3-3) Purification by Super ODS reverse phase high performance liquid chromatography

Liquid A (0.1% trifluoroacetic acid/distilled water) was run at 40°C at a flow rate of 1 ml/minute through a TSKgel Super ODS reverse phase high performance liquid chromatography column (Tosoh Corp., 0.46 cm × 10 cm), so that the column was equilibrated. A SepPak C18-5g column fraction obtained in the section (5-3-2) was evaporated on a Savant centrifugal concentrator, and the resultant product was then subjected to Super ODS reverse phase high performance liquid chromatography. Thereafter, the concentration of the liquid B was increased in a linear gradient manner at a flow rate of 1 ml/minute over 60 minutes from 100% by volume of liquid A (0.1% trifluoroacetic acid/distilled water)/0% by volume of liquid B (0.1% trifluoroacetic acid/60% acetonitrile) to 0% by volume of liquid A/100% by volume of liquid B, and the eluant was then recovered.
The eluant was divided into fractions each consisting of 1 ml, and a fraction number was assigned to each fraction. The total amount of the divided fraction was freeze-dried with a freeze-drier (12EL; VirTis). 150 µl of a mixture, which was obtained by adding 2.5 mM Probenecid and 0.2% BSA to H/HBSS, was added to and dissolved in the freeze-dried product. Using the obtained solution, an action to activate receptors to ZAQC-B1 cells was determined according to a test method described in the following section (3-4).

(3-4) Determination of activity to increase intracellular $Ca^{2+}$ ion concentration by FLIPR

Using the sample obtained in the above section (3-3), an activity thereof to increase an intracellular $Ca^{2+}$ ion concentration in ZAQ expression cells (ZAQC-B1) obtained in Reference example 9 (I)(5) was determined by FLIPR. In addition, hOT7T175 expression cells (hOT7T175-16; described in WO00/24890) were used as control cells.
Before use, both the ZAQC-B1 cells and the hOT7T1751-6 cells were subcultured in DMEM to which 10% dialyzed fetal bovine serum (hereinafter referred to as d FBS) had been added. Each of the ZAQC-B1 cells and the hOT7T1751-6 cells was suspended in a medium (10% d FBS-DMEM) such that the number of cells became $15 \times 10^4$ cells/ml. 200 µl each of the suspension was inoculated into each well of a FLIPR 96-well plate (Black plate clear bottom, Coster), using a dispersion burette ($3.0 \times 10^4$ cells/200 µl/well). Thereafter, the cells were cultured in a 5% $CO_2$ incubator at 37°C overnight, and then used (hereinafter referred to as a cell plate). 21 ml of H/HBSS (9.8 g of Hanks', 0.35 g of sodium bicarbonate, 4.77 g of HEPES and sodium hydroxide solution were mixed, and the mixture was adjusted to pH 7.4, followed by sterilization with a filter), 210 µl of 250 mM Probenecid, and 210 µl of bovine fetal serum (FBS) were mixed. Moreover, 2 vials (50 µg) of Fluo 3-AM were dissolved in 42 µl of dimethyl sulfoxide and 42 µl of 20% pluronic acid, and the obtained solution was added to the above H/HBSS-Probenecid-FBS followed by mixing. Thereafter, using a 8-channel pipette, 100 µl each of the obtained mixture was poured into each well of the cell plate, from which a culture supernatant had been removed. The cell plate was then incubated in 5% $CO_2$ incubator at 37°C for 1 hour (pigment loading). Thereafter, 150 µl of a solution, which was prepared by adding 2.5 mM Probenecid and 0.2% BSA to H/HBSS, was added to and dissolved in each fraction of the assay sample obtained in the above (5-3-3), and the obtained solution was transferred to a FLIPR 96-well plate (V-Bottom plate, Coster) (hereinafter referred to as a sample plate). After pigment was loaded on the cell plate, the cell plate was washed 4 times with a washing buffer prepared by adding 2.5 mM Probenecid to H/HBSS, employing a plate washer (Molecular Devices). After completion of the washing, 100 µl of the washing buffer was kept. The cell plate and the sample plate were placed in FLIPR, followed by performing assay (0.05 ml of the sample was transferred from the sample plate to the cell plate by FLIPR). An activity to increase an intracellular $Ca^{2+}$ ion concentration, which was specific for the ZAQC-B1 cells, was observed in fractions with fraction Nos. 48 to 68. From these results, it was found that a component of interest having an action to activate receptors to the ZAQC-B1 cells, that is, a ZAQ-activating component was eluted in fractions with fraction Nos. 48 to 68. Reference example 11 Generation of human type ZAQ ligand peptide in mammalian cells (2)

(1) Preparation of culture supernatant

A human type ZAQ ligand precursor peptide expression plasmid (pCANZAQLg2) was introduced in COS7 cells by the method described in Reference example 10. This is to say, a DMEM medium containing COS7 cells was dispersed on a 15 cm Petri dish, such that the concentration became $3.0 \times 10^6$ cells/dish. The cells were cultured at 37°C in a 5% $CO_2$ incubator overnight. 600 µl of buffer EC (Effectene transfection reagent, QIAGEN) was added to 4 µg of the human type ZAQ ligand precursor peptide expression plasmid (pCANZAQLg2) (which was dissolved in 4 µl of TE buffer), and 32 µl of the enhancer was further added thereto, followed by mixing for 1 second. Thereafter, the mixture was left at room temperature for 3 minutes. Thereafter, 120 µl of Effectene Transfection Reagent was further added thereto, followed by mixing for 10 seconds. Thereafter, the mixture was left at room temperature for 10 minutes. A supernatant obtained from the cells that had been dispersed on the preceding day was eliminated, and the residue was washed once with 10 ml of DMEM medium. Thereafter, 30 ml of DMEM medium was added thereto. 1 ml of DMEM medium was added to a plasmid solution, and they were blended. The obtained mixture was added dropwise to the cells, and the mixture was blended. Thereafter, the mixture was cultured at 37°C in a 5% $CO_2$ incubator overnight. The obtained culture was washed once with 10 ml of DMEM medium, and 20 ml of DMEM medium was added to the culture, followed by culturing at 37°C in a 5% $CO_2$ incubator overnight. 1 day later, the culture supernatant was recovered. 20 ml of DMEM medium was further added thereto, followed by culturing at 37°C in a 5% $CO_2$ incubator overnight. Thereafter, the culture supernatant was recovered.

(2) Purification of human type ZAQ ligand peptide from culture supernatant

A culture supernatant in an amount equivalent to that obtained from eighty 15-cm Petri dishes was recovered in the method described in (1) above, and thereafter, acetic acid was added thereto to a final concentration of 1 M. After the mixture was stirred for 1 hour, acetone was added thereto in an amount of 2 times the volume of the mixture to deposit proteins. The deposited proteins were stirred at 4°C for 30 minutes. Subsequently, the mixture was centrifuged at 10,000 rpm for 30 minutes using a high speed centrifuge (CR26H, RR10A type rotor, Hitachi Ltd.), so as to obtain a supernatant. Acetone was removed from the obtained supernatant using an evaporator, and the resultant product was run through a reverse phase column (Waters C18, 100 g) that had previously been equilibrated with 0.1% trifluoroacetic acid/distilled water. The column was washed with 1,000 ml of 0.1% trifluoroacetic acid/distilled water, and then with 1,000 ml of 0.1% trifluoroacetic acid/20% acetonitrile. Thereafter, peptides were eluted with 1,000 ml of 0.1% trifluoroacetic acid/60% acetonitrile. The obtained eluant was applied to an evaporator, and it was then freeze-dried with a freeze-drier (12EL; VirTis).

Liquid A (0.1% triluoroacetic acid/distilled water) was run at 40°C at a flow rate of 4 ml/minute through a TSKgel ODS80TM reverse phase high performance liquid chromatography column (Tosoh Corp., 21.5 mm × 30 cm), so that the column was equilibrated. The obtained freeze-dried powders were dissolved in the liquid A, and it was applied to the ODS80TM column. Thereafter, the concentration of the liquid B was increased in a linear gradient manner at a flow rate of 4 ml/minute over 120 minutes from 60% by volume of liquid A (0.1% trifluoroacetic acid/distilled water) /40% by volume of liquid B (0.1% trifluoroacetic acid/60% acetonitrile) to 0% by volume of liquid A/100% by volume of liquid B, so as to elute peptides.

The eluant was divided into fractions each consisting of 8 ml, and a fraction number was assigned to each fraction. 50 µl of an aliquot from the above each fraction was freeze-dried with a freeze-drier (12EL; VirTis). 200 µl of a solution prepared by adding 2.5 mM Probenecid and 0.2% BSA to H/HBSS was added to and dissolved in the above freeze-dried product. Using the obtained solution, an action to activate receptors to ZAQC-B1 cells was determined according to the test method described in the above section (5-3-4). As a result, it was found that a component of interest having an action to activate receptors to the ZAQC-B 1 cells, that is, a ZAQ-activating component was eluted in a fraction with fraction No. 32.

Liquid A (10 mM ammonium formate/10% acetonitrile) was run at 25°C at a flow rate of 1 ml/minute through a TSKgel CM-2SW ion exchange high performance liquid chromatography column (Tosoh Corp., 4.6 mm × 25 cm), so that the column was equilibrated. The above fraction with fraction No. 32 was applied to the CM-2SW column. Thereafter, the concentration of the liquid B was increased in a linear gradient manner at a flow rate of 1 ml/minute over 60 minutes from 100% by volume of liquid A (10 mM ammonium formate/10% acetonitrile)/0% by volume of liquid B (1,000 mM ammonium formate/10% acetonitrile) to 0% by volume of liquid A/100% by volume of liquid B, so as to elute peptides.

The eluant was divided into fractions each consisting of 1 ml, and a fraction number was assigned to each fraction. 1.5 µl of an aliquot was obtained from the above each fraction, and it was then diluted with 200 µl of a solution prepared by adding 2.5 mM Probenecid and 0.2% BSA to H/HBSS. Using the obtained solution, an action to activate receptors to ZAQC-B1 cells was determined according to the test method described in the above section (5-3-4). As a result, it was found that a component of interest having an action to activate receptors to the ZAQC-B 1 cells, that is, a ZAQ-activating component was eluted in fractions with fraction Nos. 56 and 57.

Liquid A (0.1 % trifluoroacetic acid/distilled water) was run at 40°C at a flow rate of 1 ml/minute through a TSKgel Super phenyl reverse phase high performance liquid chromatography column (Tosoh Corp., 4.6 mm × 10 cm), so that the column was equilibrated. The above fractions with fraction Nos. 56 and 57 were applied to the Super phenyl column. Thereafter, the concentration of the liquid B was increased in a linear gradient manner at a flow rate of 1 ml/minute over 60 minutes from 70% by volume of liquid A (0.1% trifluoroacetic acid/distilled water)/30% by volume of liquid B (0.1% trifluoroacetic acid/60% acetonitrile) to 50% by volume of liquid A/50% by volume of liquid B, so as to elute peptides.

The eluant was divided into fractions each consisting of 1 ml, and a fraction number was assigned to each fraction. 1.5 µl of an aliquot was obtained from the above each fraction, and it was then diluted with 200 µl of a solution prepared by adding 2.5 mM Probenecid and 0.2% BSA to H/HBSS. Using the obtained solution, an action to activate receptors to ZAQC-B 1 cells was determined according to the test method described in Reference example 10. As a result, it was found that a component of interest having an action to activate receptors to the ZAQC-B1 cells, that is, a ZAQ-activating component was eluted in fractions with fraction Nos. 54, 55 and 56. This activity peak was identical to a single ultraviolet absorption peak, and it is therefore considered that the above activating component was purified to be a single component.

A solvent contained in the purified sample of the ZAQ-activating component was freeze-dried and eliminated. The obtained freeze-dried product was dissolved in DMSO (dimethyl sulfoxide). An aliquot (approximately 7.5 pmol) from the obtained solution was subjected to analysis of an N-terminal amino acid sequence, using a protein sequencer (Perkin-Elmer, PE Biosystems Procise 491cLC). As a result, 9 amino acid residues out of the 10 amino acid residues from the N-terminus could be identified (Ala Val Ile Thr Gly Ala Xaa Glu Arg Asp (SEQ ID NO: 92; Xaa was an unidentified residue herein)). The obtained amino acid sequence was identical to the N-terminal amino acid sequence predicted from the human type ZAQ ligand mature peptide. In addition, using a Finnigan LCQ LC/MS device (Thermoquest, San Jose, CA), mass spectrometry was carried out on the purified sample of the ZAQ-activating component according to the electron spray ionization method. The molecular weight was confirmed to be 9657.6. This value is close to 9657.3, a theoretical value for the human type ZAQ ligand mature peptide (SEQ ID NO: 82) consisting of 86 residues wherein 10 cysteine residues all form a disulfide bond. Thus, it was confirmed that the purified sample of the ZAQ-activating component has a human type ZAQ ligand mature peptide having an amino acid sequence shown in SEQ ID NO: 82.

(3) Determination of action of purified human type ZAQ ligand peptide to activate ZAQ

The action of the human type ZAQ ligand mature peptide purified in (2) above to activate receptors to ZAQC-B1 cells was determined by the test method described in Reference example 10. As a result, it was found that the human type ZAQ ligand mature peptide concentration-dependently induces increase of an intracellular calcium concentration in ZAQ expression CHO cells (ZAQC-B1 cells). $EC_{50}$ value was 96 pM, and thus, it became clear that the human type ZAQ ligand mature peptide exhibits an extremely strong agonist activity. The results are shown in [Figure 3].

Reference example 12 Generation of human type ZAQ ligand peptide in mammalian cells (3)

(1) Establishment of human type ZAQ ligand peptide stable expression CHO cell line

Using the plasmid pHMITG described in Reference example 9 (II) as a template, human type ZAQ ligand cDNA was amplified by PCR with primers indicated below, and the amplified product was then cloned into a pCR Blunt II vector (Invitrogen).

5′ GTCGACCACCATGAGAGGTGCCACGC 3′ (SEQ ID NO: 93)

5′ ACTAGTCGCAGAACTGGTAGGTATGG 3′ (SEQ ID NO: 94)

An insert cDNA was cut out of the obtained clone having a correct sequence using restriction enzymes SalI and SpeI, and it was then incorporated into a pAKKO1.11H expression vector. This plasmid was introduced into CHO/dhFr⁻ cells (American Type Culture Collection) for genetic transduction, using the CellPhect Transfection kit (Amersham Pharmacia Biotech) described in Reference example 9. A culture supernatant was recovered from several transduced clones, and using the culture supernatant, the ZAQ ligand activity of ZAQC-B1 cells to increase an intracellular $Ca^{2+}$ ion concentration was determined by the test method described in Reference example 9. By this determination, a ZAQL-1 expression CHO cell clone No. 4, which highly expresses ZAQ ligand, was selected.

(2) Preparation of serum free culture supernatant of human type ZAQ ligand (ZAQL-1) expression CHO cells

4 pieces of Single Tray (Nunc) were cultured in a Dulbecco's Modified Eagle Medium (DMEM) (Nissui Pharmaceutical Co., Ltd.) containing dialyzed 10% fetal bovine serum (JRH BIOSCIENCES), 1 mM MEM nonessential amino acid solution, 100 units/ml penicillin and 100 µg/ml streptomycin, until they became confluent, so as to obtain a ZAQL-1 expression CHO cell clone No. 4. The obtained ZAQL-1 expression CHO cell clone No. 4 was treated with trypsin and dispersed, and then recovered by centrifugal separation. Cells contained in a piece of the above Single Tray were suspended in 1.5 L of the above medium, and the suspension was inoculated on Cell Factories 10 (Nunc).

Thereafter, 4 sets of Cell Factories 10 were cultured at 37°C in 5% carbon dioxide for 3 days. A culture supernatant was removed, and then, a set of Cell Factories 10 was washed with 1 L of the above H/HBSS. After the H/HBSS was removed, 2 L of serum free medium (which was a Dulbecco's Modified Eagle Medium containing 1 mM MEM nonessential amino acid solution, 100 units/ml penicillin and 100 μg/ml streptomycin) was added per a set of Cell Factories 10, followed by further culturing for 2 days. The recovered culture supernatant was centrifuged at 1,000 rpm for 10 minutes using a Hitachi high speed centrifuge, and the resultant product was filtrated with gauze, so as to obtain a pure supernatant. Acetic acid was added to the supernatant to a final concentration of 1 M, and the following operation was carried out.

(3) Rough fractionation of serum free culture supernatant of ZAQL-1 expression CHO cells by octadodecyl reverse phase chromatography

Silica gel, to which an octadodecyl group was fixed, was filled in PrepC18 (Waters), and it was swollen with methanol. The obtained product was filled in a glass column (50 mm × 100 mm). Thereafter, the extract prepared in Reference example 11 (2) was applied to the column that had been equilibrated with 1 M acetic acid. Thereafter, the column was washed with 800 ml of 1 M acetic acid. Thereafter, 1,000 ml of 60% acetonitrile/0.1% trifluoroacetic acid was run through the column, so as to elute rough peptide components of interest. The obtained eluant was concentrated with an evaporator, and the obtained concentrate was freeze-dried with a freeze-drier (12EL; VirTis).

(4) Separation by Wakosil-II 5C18HG Prep reverse phase high performance liquid chromatography

A solution consisting of 91.7 % by volume of liquid A (0.1% trifluoroacetic acid/distilled water) and 8.3% by volume of liquid B (0.1% trifluoroacetic acid/60% acetonitrile) was run at 40°C at a flow rate of 5 ml/minute through a Wakosil-II 5C18HG Prep reverse phase high performance liquid chromatography column (Wako Pure Chemical Industries, Ltd., 20 mm × 250 mm), so that the column was equilibrated. The freeze-dried product obtained in (3) above was subjected to a chromatographic operation. This is to say, 36 ml of 1 M acetic acid was added to and dissolved in the freeze-dried product, and the mixture was centrifuged. The one third of the resultant product was applied to the above column. Thereafter, the concentration of the liquid B was increased in a linear gradient manner at a flow rate of 5 ml/minute over 1 minute to 66.7 % by volume of liquid A/33.3 % by volume of liquid B, and then further increased over 120 minutes to 16.7 % by volume of liquid A/83.3 % by volume of liquid B. The eluant was divided into fractions each consisting of 5 ml, and a fraction number was assigned to each fraction. 3 μl of an aliquot from the above each fraction was mixed with 150 μl of 0.2% BSA, and the mixture was freeze-dried with a freeze-drier (12EL; VirTis). 150 μl of assay buffer [prepared by adding 2.5 mM Probenecid and 0.1% CHAPS to H/HBSS (prepared by mixing 9.8 g of Nissui Hanks' 2 (Nissui Pharmaceutical Co., Ltd.), 0.35 g of sodium bicarbonate, 4.77 g of HEPES and sodium hydroxide solution, and adjusting the mixture to pH 7.4, followed by sterilization with a filter)] was added to and dissolved in the freeze-dried product. Using 50 μl of the obtained solution, an action to activate a ZAQ-B1 receptor was determined according to the test method described in Reference example 9. As a result, it was found that a component of interest having an action to activate a ZAQ-B1 receptor was eluted mainly in fractions with fraction Nos. 73 to 75.

(5) Separation by TSKgel CM-2SW ion exchange high performance liquid chromatography

A solution consisting of 100% by volume of liquid A (4 M ammonium formate : distilled water : acetonitrile = 1 : 299 : 100) and 0% by volume of liquid B (4 M ammonium formate : distilled water : acetonitrile = 1 : 2 : 1) was run at 25°C at a flow rate of 2 ml/minute through a TSKgel CM-2SW ion exchange high performance liquid chromatography column (Tosoh Corp., 7.8 × 300 mm), so that the column was equilibrated. The fractions with fraction Nos. 73 to 75 out of the fractions separated by the Wakosil-II 5C18HG Prep reverse phase high performance liquid chromatography in (4) above were freeze-dried. The obtained freeze-dried product was then dissolved in 4 ml of liquid A, and the obtained solution was applied to the TSKgel CM-2SW ion exchange column. Thereafter, the concentration of the liquid B was increased in a linear gradient manner at a flow rate of 1 ml/minute over 120 minutes to 25% by volume of liquid A/75% by volume of liquid B, and the eluant was then recovered. The eluant was divided into fractions each consisting of 2 ml, and a fraction number was assigned to each fraction. 10 μl of an aliquot from the above each fraction was mixed with 100 μl of 0.2% BSA, and the mixture was freeze-dried with a freeze-drier (12EL; VirTis). 100 μl of the above described assay buffer was added to and dissolved in the freeze-dried product. The obtained solution was 100 times diluted with the same above buffer. Thereafter, using the obtained solution, an action to activate a ZAQ receptor was determined according to the test method described in the above section (3-5). As a result, it was found that a component of interest having an action to activate a ZAQ receptor was eluted mainly in fractions with fraction Nos. 95 to 100.

(6) Purification by TSKgel ODS-80Ts reverse phase high performance liquid chromatography

A solution consisting of 91.7% by volume of liquid A (0.1% trifluoroacetic acid/distilled water) and 8.3% by volume of liquid B (0.1% trifluoroacetic acid/60% acetonitrile) was run at 40°C at a flow rate of 1 ml/minute through a TSKgel ODS-80Ts reverse phase high performance liquid chromatography column (Tosoh Corp., 4.6 mm × 100 mm), so that the column was equilibrated. The product obtained by freeze-drying the fractions with fraction Nos. 95 to 100 obtained in (5) above was subjected to a chromatographic operation. This is to say, 4 ml of 1 M acetic acid was added to the freeze-dried product to dissolve it, and the obtained solution was applied to the above column. Thereafter, the concentration of the liquid B was increased in a linear gradient manner at a flow rate of 1 ml/minute over 1 minute to 75% by volume of liquid A/25% by volume of liquid B, and then further increased over 60 minutes to 25% by volume of liquid A/75% by volume of liquid B. Then, the eluant was recovered. The eluant was divided into fractions each consisting of 1 ml, and a fraction number was assigned to each fraction. Thereafter, a ZAQ ligand activity was determined according to the method described in Reference example 9, and it was confirmed that the activity was eluted in a fraction with a single ultraviolet absorption peak. Thus, it was considered that the ZAQ ligand was purified to be a single ligand.

(7) Analysis of structure of purified ZAQ ligand peptide

The structure of the ZAQ ligand peptide obtained in (6) above was determined by a method described below. The purified sample of a ZAQ-activating main component was freeze-dried with a freeze-drier (12EL; VirTis). The freeze-dried product was dissolved in a solvent DMSO (dimethyl sulfoxide). An aliquot from the obtained solution was subjected to analysis of an N-terminal amino acid sequence, using a protein sequencer (Perkin-Elmer, PE Biosystems Procise 491cLC). As a result, it was found that the determined N-terminal amino acid sequence was identical to the amino acid sequence predicted from the human type ZAQ ligand mature peptide (SEQ ID NO: 82). In addition, using a Finnigan LCQ LC/MS device, mass spectrometry was carried out on the ZAQ-activating main component according to the electron spray ionization method. The molecular weight was calculated to be 9658.0. This measurement value is close to 9657.3, a theoretical value for the human type ZAQ ligand mature peptide (SEQ ID NO: 82). Thus, it was confirmed that a peptide of interest having an amino acid (SEQ ID NO: 82) was obtained.

Reference example 13 Reactivity of snake venom MIT1 and human type ZAQ ligand with ZAQ and I5E

(1) Isolation of snake venom MIT1

(1-1) Separation by Wakosil-II 5C18HG Prep reverse phase high performance liquid chromatography

A solution consisting of 91.7 % by volume of liquid A (0.1% trifluoroacetic acid/distilled water) and 8.3% by volume of liquid B (0.1% trifluoroacetic acid/60% acetonitrile) was run at 40°C at a flow rate of 5 ml/minute through a Wakosil-II SC18HG Prep reverse phase high performance liquid chromatography column (Wako Pure Chemical Industries, Ltd., 20 mm × 250 mm), so that the column was equilibrated. 4 ml of 1 M AcOH was added to and dissolved in 50 mg of the freeze-dried product of a Black Mamba venom (Sigma), and the obtained solution was subjected to centrifugal separation at 15,000 rpm for 10 minutes. The obtained supernatant was then subjected to a chromatographic operation. This is to say, the sample was applied to the above column. Thereafter, the concentration of the liquid B was increased in a linear gradient manner at a flow rate of 5 ml/minute over 1 minute to 91.7 % by volume of liquid A/8.3 % by volume of liquid B, and then further increased over 120 minutes to 33.4% by volume of liquid A/66.6% by volume of liquid B. 20 minutes after beginning of the operation, the eluant was divided into fractions each consisting of 10 ml, and a fraction number was assigned to each fraction. 1 μl of an aliquot was collected from the above each fraction, and it was then 10,000 times diluted with the above described assay buffer. Thereafter, an action to activate a ZAQ-B1 receptor was determined according to the test method described in Reference example 9. As a result, it was found that a component of interest having an action to activate a ZAQ-B1 receptor was eluted mainly in fractions with fraction Nos. 21 to 23.

(1-2) Separation by TSKgel CM-2SW ion exchange high performance liquid chromatography

A solution consisting of 100% by volume of liquid A (4 M ammonium formate : distilled water : acetonitrile = 1 : 299 : 100) and 0% by volume of liquid B (4 M ammonium formate : distilled water: acetonitrile = 1 : 2 : 1) was run at 25°C at a flow rate of 1 ml/minute through a TSKgel CM-2SW ion exchange high performance liquid chromatography column (Tosoh Corp., 4.6 × 250 mm), so that the column was equilibrated. The fractions with fraction Nos. 21 to 23 out of those obtained by the Wakosil-II 5C18HG Prep reverse phase high performance liquid chromatography in Reference example 12 were freeze-dried. The obtained freeze-dried product was dissolved in 4 ml of the liquid A, and the obtained solution was then applied to the TSKgel CM-2SW ion exchange column. Thereafter, the concentration of the liquid B was increased in a linear gradient manner at a flow rate of 1 ml/minute over 90 minutes to 0% by volume of liquid A/100% by volume of liquid B, and the eluant was then recovered. The eluant was divided into fractions each consisting of 1 ml, and a fraction number was assigned to each fraction. 1 μl of an aliquot collected from the above each fraction was 10,000 times diluted with the above described assay buffer. Thereafter, an action to activate a ZAQ-B1 receptor was determined according to the test method described in Reference example 9. As a result, it was found that a component of interest having an action to activate a ZAQ-B1 receptor was eluted mainly in fractions with fraction Nos. 50 and 51.

(1-3) Separation by Vydac238TP3410 reverse phase high performance liquid chromatography

A solution consisting of 91.7 % by volume of liquid A (0.1% trifluoroacetic acid/distilled water) and 8.3% by volume of liquid B (0.1% trifluoroacetic acid/60% acetonitrile) was run at 40°C at a flow rate of 1 ml/minute through a Vydac238TP3410 reverse phase high performance liquid chromatography column (Vydac, 4.6 mm × 100 mm), so that the column was equilibrated. The fractions with fraction Nos. 50 and 51 out of the fractions obtained by the TSKgel CM-2SW ion exchange high performance liquid chromatography in (1-2) above were directly applied to the above column. Thereafter, the concentration of the liquid B was increased in a linear gradient manner at a flow rate of 1 ml/minute over 1 minute to 75% by volume of liquid A/25% by volume of liquid B, and then further increased over 75 minutes to 41.7% by volume of liquid A/58.3% by volume of liquid B. The eluant was divided into fractions each consisting of 0.5 ml, and a fraction number was assigned to each fraction. 1 μl of an aliquot was collected from the above each fraction, and it was then 10,000 times diluted with the above described assay buffer. Thereafter, an action to activate a ZAQ-B 1 receptor was determined according to the test method described in Reference example 9. As a result, it was found that a component of interest having an action to activate a ZAQ-B1 receptor was eluted mainly in fractions with fraction Nos. 108 to 115.

(1-4) Analysis of structure of purified snake venom MIT1

The structure of the snake venom MIT1 obtained in (1-3) above was determined by a method described below. The purified MIT1 was freeze-dried with a freeze-drier (12EL; VirTis). The obtained freeze-dried peptide was dissolved in a solvent DMSO (dimethyl sulfoxide). An aliquot from the obtained solution was subjected to analysis of an N-terminal amino acid sequence, using a protein sequencer (Perkin-Elmer, PE Biosystems Procise 491cLC). As a result, it was found that the obtained N-terminal amino acid sequence was identical to the amino acid sequence predicted from the snake venom MIT1 (SEQ ID NO: 21). In addition, using a Finnigan LCQ LC/MS device, mass spectrometry was carried out on the snake venom MIT1 according to the electron spray ionization method. The molecular weight was calculated to be 8506.8. This measurement value is close to 8506.4, a theoretical value for the snake venom MIT1. Thus, it was confirmed that a peptide of interest having an amino acid sequence shown in SEQ ID NO: 21 was obtained.

(2) Establishment of I5E stably expressed cell line

Human type I5E receptor cDNA (SEQ ID NO: 6) was cloned by a known PCR method, and the obtained clone was incorporated into a pAKKO1.11H expression vector. The expression vector was introduced into CHO/dhFr⁻ cells (American Type Culture Collection) for genetic transduction according to the method described in Reference example 9. At 10, 11, 12, 13 or 14 days after beginning of the culture, approximately 20 colonies of the grown transformed CHO cells were selected. The selected cells were inoculated upon a 96-well plate in a concentration of $3 \times 10^4$ cells/well. Reactivity of the cells to the MIT1 was studied according to the test method described in Reference example 9. I5E expression CHO cell No. 4 clones (hereinafter abbreviated as I5E-4 cells) with good reactivity were selected. An amino acid sequence encoded by the nucleotide sequence shown in SEQ ID NO: 6 is shown in SEQ ID NO: 52.

(3) Determination of actions of human type ZAQ ligand peptide and MIT1 to activate ZAQ receptor and I5E receptor

With regard to the human type ZAQ ligand peptide purified by the method described in Reference example 12 and the snake venom MIT1 purified by the method described in (1) above, their action to activate a ZAQ receptor and an I5E receptor was determined by the method described in Reference example 9. The results are shown in [Figure 4] and [Figure 5].

As a result, it was found that the human type ZAQ ligand peptide and the snake venom MIT1 concentration-dependently induce increase of an intracellular calcium concentration. Moreover, the snake venom MIT1 had an action to activate a ZAQ receptor, which was 10 times greater than that of the human type ZAQ ligand peptide. Furthermore, the snake venom MIT1 had an action to activate a human type I5E receptor, which was about 100 times greater than that of the human type ZAQ ligand peptide.

Reference example 14 Production of human ZAQ ligand (SEQ ID NO: 82) in *Escherichia coli*

(1) Construction of human ZAQ ligand expression plasmid in *Escherichia coli*

(a) Using 6 types of DNA fragments #1 to #6 (SEQ ID NOS: 95 to 100), structural DNA of a ZAQ ligand was prepared.
(b) Phosphorylation of DNA oligomer

Each of 4 types of DNA oligomers (#2 to #5), from which the above fragments #1 and #6 to be on the 5'-terminal side had been eliminated, was reacted at 37°C for 1 hour in 25 μl of phosphoryalation reaction solution [10 μg of DNA oligomer, 50 mM Tris-HCl, pH 7.6, 10 mM $MgCl_2$, 1 mM spermidine, 10 mM dithiothreitol (hereinafter abbreviated as DTT), 0.1 mg/ml bovine serum albumin (hereinafter abbreviated as BSA), 1 mM ATP, 10 units of T4 polynucleotide kinase (Takara Shuzo Co., Ltd.)], so that the 5'-terminus of each oligomer was phosphorylated. The resultant product was then treated with phenol, and ethanol was added thereto in an amount of 2 times its volume. The mixture was cooled to - 70°C and then subjected to centrifugal separation, so as to deposit DNA.

(c) Ligation of DNA fragment

The DNA fragments obtained in (b) above were mixed with the above fragments #1 and #6, and the total amount was set at 120 μl. The mixed solution was retained at 90°C for 10 minutes, and then it was gradually cooled to room temperature. Thereafter, the solution was subjected to annealing, and then subjected to a ligation reaction using TaKaRa DNA Ligation Kit ver. 2 (Takara Shuzo Co., Ltd.). 30 μl of solution II attached with the kit was added to 30 μl of annealing solution, and the mixture was well blended. Thereafter, 60 μl of solution I attached with the kit was added to the mixture, followed by a reaction at 37°C for 1 hour for ligation. Thereafter, the reaction product was treated with phenol, and water layers were recovered. Ethanol was added to the layers in an amount of 2 times their volume. The mixture was cooled to -70°C and then subjected to centrifugal separation, so as to deposit DNA. The thus obtained DNA fragments were phosphorylated using T4 polynucleotide kinase (Takara Shuzo Co., Ltd.), and the obtained fragments were used in the following step (d).

(d) Construction of ZAQ ligand expression vector

To construct an expression vector, pTCII (Japanese Patent Laid-Open No. 2000-178297) was digested with NdeI and BamHI (Takara Shuzo Co., Ltd.) at 37°C for 2 hours. Thereafter, the digest was subjected to 1% agarose gel electrophoresis, so that a 4.3 kb DNA fragment was extracted using QIAquick Gel Extraction Kit (QIAGEN). The obtained fragment was then dissolved in 25 μl of TE buffer solution. The NdeI, BamHI fragment of the pTCII and the structural gene (SEQ ID NO: 101) of a ZAQ ligand as prepared above were subjected to a ligation reaction, using TaKaRa DNA ligation kit ver. 2 (Takara Shuzo Co., Ltd.). *Escherichia coli* JM109 competent cells (Toyobo Co., Ltd.) were transformed with 10 μl of the above obtained reaction solution. The transformed cells were dispersed on an LB agar medium containing 10 μg/ml tetracycline, and they were cultured at 37°C overnight, followed by selection of the generated tetracycline-resistant colonies. The obtained transformant was cultured in an LB medium overnight, and then, a plasmid pTCh1ZAQ was prepared using QIAprep8 Miniprep Kit (QIAGEN). The nucleotide sequence of the ZAQ ligand DNA was determined using Applied Biosystems Model 377 DNA Sequencer. *Escherichia coli* MM294 (DE3) was transformed with the above plasmid pTCh1ZAQ, so as to obtain ZAQ ligand expression line *Escherichia coli* MM294 (DE3)/pTCh1ZAQ.

(2) Production of ZAQ ligand

The above *Escherichia coli* MM294 (DE3)/pTCh1ZAQ was added to 1 L of LB medium (1% peptone, 0.5% yeast extract, 0.5% sodium chloride) containing 5.0 mg/L tetracycline placed in a 2 L volume flask, and it was subjected to a shake culture at 37°C for 8 hours. The obtained culture solution was transferred into a 50 L volume fermenter in which 19 L of main fermentation medium (1-68% sodium hydrogenphosphate monobasic, 0.3% potassium hydrogenphosphate dibasic, 0.1% ammonium chloride, 0.05% sodium chloride, 0.05% magnesium sulfate, 0.02% antifoaming agent, 0.00025% ferrous sulfate, 0.0005% thiamine hydrochloride, 1.5% glucose, 1.5% high-case amino) was stored, and then, aeration agitation began at 30°C. When the turbidity of the culture solution became 500 Klett units, isopropyl-β-D-thiogalactopyranoside was added thereto to a final concentration of 12 mg/L, followed by further culturing for 4 hours. After completion of the culture, the culture solution was centrifuged to obtain approximately 200 g of wet cell bodies, which were then conserved at -80°C.
This transformed *Escherichia coli* MM294 (DE3)/pTCh1ZAQ was deposited with the Institution for Fermentation, Osaka (IFO) under accession No. IFO1-6527.

(3) Activation of ZAQ ligand

400 ml of solution (pH 8.0) containing of 200 mM Tris/HCl and 7 M guanidine hydrochloride was added to 200 g of the cell bodies obtained in (2) above, so as to dissolve the cell bodies. Thereafter, the obtained solution was subjected to centrifugal separation (10,000 rpm, 1 hour). 10 L of solution (pH 8.0) containing 0.4 M arginine, 50 mM Tris/HCl, 0.2 mM GSSG and 1 mM GSH was added to the obtained supernatant, and activation was carried out at 4°C overnight.

(4) Purification of ZAQ ligand

A regenerant obtained as a result of activation in (3) above was adjusted to pH 6.0, and it was adsorbed to an SP-sepharose column (11.3 cm × 15 cm) equilibrated with a 50 mM phosphate buffer solution (pH 6.0). Thereafter, elution was carried out with a 600 mM NaCl/50 mM phosphate buffer solution (pH 6.0), so as to pool a fraction containing a ZAQ ligand. This fraction was passed through CM-5PW (21.5 mm × 150 mmL) equilibrated with a 50 mM phosphate buffer solution (pH 6.0), and it was adsorbed thereto and washed. Thereafter, elution was carried out with a stepwise gradient from 0% to 100% B (B = 50 mM phosphate buffer solution + 1 M NaCl, pH 6.05) (60 minutes), so as to obtain a ZAQ ligand fraction (elution time: approximately 40 minutes). This fraction was passed through C4P-50 (21.5 mm ID × 300 mm L, Showa Denko K.K.) equilibrated with 0.1% trifluoroacetic acid, and it was adsorbed thereto and washed. Thereafter, elution was carried out with a stepwise gradient from 25% to 50% B (B = 80% acetonitrile/0.1% trifluoroacetic acid) (60 minutes), so as to pool a ZAQ ligand fraction (elution time: approximately 40 minutes). The fraction was then freeze-dried, so as to obtain approximately 80 mg of ZAQ ligand freeze-dried powders.

(5) Determination of characteristics of ZAQ ligand

(a) Analysis by SDS polyacrylamide gel electrophoresis

The ZAQ ligand obtained in (4) above was suspended in a sample buffer [Laemmli, Nature, 227, 680 (1979)] to which 100 mM DTT was added, and the suspension was heated at 95°C for 1 minute, followed by electrophoresis with Multigel 15/25 (Daiichi Pure Chemicals Co., Ltd.). The electrophoresed gel was stained with Coomassie brilliant blue. As a result, a single protein band was observed at the same position as of the recombinant ZAQ ligand sample derived from COS7 cells obtained in Reference example 10. From this result, it was found that the recombinant ZAQ ligand sample derived from *Escherichia coli* obtained in (4) above has high purity, and that it is identical to the recombinant ZAQ ligand prepared from COS7 cells in terms of molecular weight.

(b) Analysis of amino acid composition

Amino acid composition was determined by an amino acid analyzer (Hitachi L-8500A Amino Acid Analyzer). As a result, it was found that the analyzed amino acid composition was identical to the amino acid composition estimated from the nucleotide sequence of DNA of a ZAQ ligand (a peptide consisting of an amino acid sequence shown in SEQ ID NO: 82) (Table 5).

[Table 5]

| Amino acid | Number of residues per mole | Value estimated from the nucleotide sequence encoding ZAQ ligand |
|---|---|---|
| Asx | 5.7 | 6 |
| Thr[1)] | 3.3 | 4 |
| Ser[1)] | 3.4 | 4 |
| Glx | 5.0 | 5 |
| Pro | 5.6 | 6 |
| Gly | 7.7 | 8 |
| Ala | 3.9 | 4 |
| Cys[2)] | N.D. | 10 |
| Val | 2.9 | 3 |
| Met | 1.9 | 2 |

1) Value extrapolated to time 0

2) Undetected

[Table 5] (continued)

| Amino acid | Number of residues per mole | Value estimated from the nucleotide sequence encoding ZAQ ligand |
|---|---|---|
| Ile | 2.6 | 3 |
| Leu | 8 | 8 |
| Tyr | 1.0 | 1 |
| Phe | 3.7 | 4 |
| His | 3.8 | 4 |
| Lys | 3.8 | 4 |
| Arg | 8.5 | 9 |
| Trp | 0.9. | 1 |
| Acid hydrolysis (6 N HCl-1% phenol, 110°C, mean value obtained from hydrolyses for 24 and 48 hours) | | |

(c) Analysis of N-terminal amino acid sequence

An N-terminal amino acid sequence was determined using a gas phase protein sequencer (PE Applied Biosystems Model 492). As a result, it was found that the determined amino acid sequence was identical to the N-terminal amino acid sequence of a ZAQ ligand, which was estimated from the nucleotide sequence of DNA of the ZAQ ligand (Table 6).

[Table 6]

| Residue No. | Detected PTH-amino acid[1)] (pmol) | Amino acid estimated from the nucleotide sequence encoding ZAQ ligand |
|---|---|---|
| 1 | Ala (99) | Ala |
| 2 | Val (100) | Val |
| 3 | Ile (91) | Ile |
| 4 | Thr (57) | Thr |
| 5 | Gly (70) | Gly |
| 6 | Ala (89) | Ala |
| 7 | N.D. | Cys |
| 8 | Glu (60) | Glu |
| 9 | Arg (49) | Arg |
| 10 | Asp (54) | Asp |
| 11 | Val (19) | Val |
| 12 | Gln (67) | Gln |
| 13 | N.D. | Cys |
| 14 | Gly (54) | Gly |
| 15 | Ala (65) | Ala |
| 16 | Gly (47) | Gly |
| 17 | Thr (32) | Thr |
| 18 | N.D. | Cys |
| 19 | N.D. | Cys |
| 20 | Ala (36) | Ala |

1) Analysis was carried out with 150 pmol phenylthiohydantoin.
N. D. indicates "undetected."

(d) Analysis of C-terminal amino acid

A C-terminal amino acid was determined using an amino acid analyzer (Hitachi L-8500A Amino Acid Analyzer). The C-terminal amino acid of the obtained ZAQ ligand was identical to the C-terminal amino acid estimated from the nucleotide sequence of the DNA (Table 7).

[Table 7]

| C-terminal amino acid | Recovery rate (%) |
|---|---|
| Phe | 49.8 |
| Gas phase hydrazinolysis (100°C, 3.5 hours) | |

(e) Mass spectrometry

Mass spectrometry was carried out using an LCQ ion trap mass spectrometer (ThermoQuest) equipped with nano ESI ion source. As a result, the molecular weight was confirmed to be 9657.55 ± 0.89. This value is close to a theoretical molecular weight (9657.3) for the ZAQ ligand shown in SEQ ID NO: 82 wherein 10 Cys residues form 5 pairs of disulfide bonds.

(6) Determination of activity of ZAQ ligand (determination of activity thereof to increase intracellular Ca ion concentration by FLIPR)

The activity of the purified recombinant ZAQ ligand sample derived from *Escherichia coli* obtained in (4) above was determined by the method described in Reference example 10 (determination of the activity thereof to increase an intracellular Ca ion concentration by FLIPR). As a result, it was found that the above sample had an activity equivalent to that of a recombinant sample derived from COS7 cells (a purified ZAQ ligand) which was obtained in Reference example 10.

Reference example 15 Cloning of mouse type ZAQ ligand peptide cDNA

Using the brain of Mouse Multiple Tissue cDNA Panel (CLONTECH) as a template, primers mMIT1-F3 (SEQ ID NO: 102), mMIT1-R3 (SEQ ID NO: 103), mMIT1-F4 (SEQ ID NO: 104) and mMIT1-R4 (SEQ ID NO: 105) were prepared. Using the primers, a PCR reaction described below was carried out.

mMIT1-F3: 5′-CTTGGCCTTCTCGGCTTGTCTAG-3′ (SEQ ID NO: 102)

mMIT1-R3: 5′-GGTGTAAAGCAAGAGGTCACCCAGT -3′ (SEQ ID NO: 103)

mMIT1-F4: 5′-ACAAGGCAGCGCAGAAGGAAGT-3′ (SEQ ID NO: 104)

mMIT1-R4: 5′-GGCTCCAGTACAGAGTCCAGACAA-3′ (SEQ ID NO: 105)

A PCR reaction solution was prepared by mixing 0.6 µl of 50X Advantage-GC 2 Polymerase Mix (CLONTECH), 6 µl of 5x Advantage-GC 2 PCR buffer supplied with the kit (200 mM Tricine-KOH, 75 mM KOAc, 17.5 mM $Mg(OAc)_2$, 25% dimethyl sulfoxide, 18.75 µg/ml BSA, 0.025% Tween-20, and 0.025% Nonidet-P40), 2.4 µl of 2.5 mM dNTP mixture, 0.6 µl each of 10 µM primers mMIT1-F3 and mMIT1-R3, 3 µl of template DNA, and 16.8 µl of distilled water. After the early denaturation at 94°C for 3 minutes, the reaction conditions were set to repeat 35 cycles of reactions at 94°C for 30 seconds and at 68°C for 3 minutes, and a final extension reaction at 68°C for 3 minutes.

Subsequently, using, as a template, a solution obtained by 50 times diluting the reaction solution obtained as a result of the above PCR reaction with distilled water, a nested PCR was carried out. A reaction solution was prepared by mixing 0.6 µl of 50X Advantage-GC 2 Polymerase Mix (CLONTECH), 6 µl of 5x Advantage-GC 2 PCR buffer supplied with the kit (200 mM Tricine-KOH, 75 mM KOAc, 17.5 mM $Mg(OAc)_2$, 25% dimethyl sulfoxide, 18.75 µg/ml BSA, 0.025% Tween-20, and 0.025% Nonidet-P40), 2.4 µl of 2.5 mM dNTP mixture, 0.6 µl each of 10 µM primers mMIT1-F4 and

mMIT1-R4, 3 μl of template DNA, and 16.8 μl of distilled water. After the early denaturation at 94°C for 3 minutes, the reaction conditions were set to repeat 35 cycles of reactions at 94°C for 30 seconds and at 68°C for 3 minutes, and a final extension reaction at 68°C for 3 minutes.

The obtained DNA fragment was cloned using TOPO TA Cloning Kit (Invitrogen) according to the method described in a manual supplied with the kit. The sequence of the cloned DNA was determined using ABI377 DNA Sequencer, so as to obtain a plasmid having a 410 bp DNA fragment (SEQ ID NO: 106) encoding a mouse ZAQ ligand full-length peptide. The obtained plasmid was named as pMMIT. Moreover, a sequence obtained by eliminating a portion determined by the primers from the above nucleotide sequence was determined to be the nucleotide sequence of cDNA (SEQ ID NO: 111). *Escherichia coli* TOP10 was transformed with the above plasmid, and the transformed *Escherichia coli* was named as *Escherichia coli* TOP10/pMMIT.

The nucleotide sequence of this DNA fragment was analyzed. As a result, it was found that a DNA fragment shown in SEQ ID NO: 106 contains DNA (SEQ ID NO: 108) encoding a mouse type ZAQ ligand precursor peptide (105 amino acid residues) shown in SEQ ID NO: 107.

Moreover, it was also found that the nucleotide sequence shown in SEQ ID NO: 108 has a typical signal sequence, and that the DNA having the nucleotide sequence shown in SEQ ID NO: 108 contains DNA (SEQ ID NO: 110) consisting of 258 base pairs encoding a mouse type ZAQ ligand mature peptide (86 amino acid residues) shown in SEQ ID NO: 109.

Reference example 16 Cloning of rat type ZAQ ligand peptide cDNA

Using rat brain QUICK-clone cDNA (CLONTECH) as a template, degenerate primers MF1 (SEQ ID NO: 112), MR1 (SEQ ID NO: 113), MF2 (SEQ ID NO: 114) and MR2 (SEQ ID NO: 115) were prepared. Using the primers, a PCR reaction described below was carried out.

MF1: 5'-TCACCYCAAGTGAYCATGAGAGG-3' (SEQ ID NO: 112)

MR1: 5'-CTAAAARTTGRYRTTCTTCAAGTCC-3' (SEQ ID NO: 113)

MF2: 5'-ATCACAGGGGCCTGTGARCG-3' (SEQ ID NO: 114)

MR2: 5'-AGCAGCGGTACCTGCCGTCC-3' (SEQ ID NO: 115)

A PCR reaction solution was prepared by mixing 0.6 μl of 50X Advantage 2 Polymerase Mix (CLONTECH), 3 μl of 10x Advantage 2 PCR buffer supplied with the kit (400 mM Tricine-KOH, 150 mM KOAc, 35 mM $Mg(OAc)_2$, 37.5 μg/ml BSA, 0.05% Tween-20, and 0.05% Nonidet-P40), 2.4 μl of dNTP mixture (2.5 mM each, Takara Shuzo Co., Ltd.), 0.6 μl each of 10 μM primers MF1 and MR1, 1 μl of template cDNA, and 20.6 μl of distilled water. After the early denaturation at 95°C for 1 minute, the reaction conditions were set to repeat 35 cycles of reactions at 95°C for 30 seconds, at 55°C for 1 minute and at 68°C for 1 minute, and a final extension reaction at 68°C for 5 minutes.

Subsequently, using, as a template, a solution obtained by 15 times diluting the reaction solution obtained as a result of the above PCR reaction with distilled water, a nested PCR was carried out. A reaction solution was prepared by mixing 0.6 μl of 50X Advantage 2 Polymerase Mix (CLONTECH), 3 μl of 10x Advantage 2 PCR buffer supplied with the kit (400 mM Tricine-KOH, 150 mM KOAc, 35 mM $Mg(OAc)_2$, 37.5 μg/ml BSA, 0.05% Tween-20, and 0.05% Nonidet-P40), 2.4 μl of dNTP mixture (2.5 mM each, Takara Shuzo Co., Ltd.), 0.6 μl each of 10 μM primers MF1 and MR1, 1 μl of template cDNA, and 20.6 μl of distilled water. After the early denaturation at 95°C for 1 minute, the reaction conditions were set to repeat 35 cycles of reactions at 95°C for 30 seconds, at 55°C for 1 minute and at 68°C for 1 minute, and a final extension reaction at 68°C for 5 minutes.

The obtained DNA fragment was cloned using Zaro Blunt TOPO PCR Cloning Kit (Invitrogen) according to the method described in a manual supplied with the kit. The sequence of the cloned DNA was determined using ABI377 DNA sequencer, so as to obtain partial sequences shown in SEQ ID NO: 116 (type T) and SEQ ID NO: 117 (type C).

Primers RM3-1 (SEQ ID NO: 118), RM3-2 (SEQ ID NO: 119) and RM3-3 (SEQ ID NO: 120) were prepared based on the information regarding the above sequences. Using the primers, a 5' RACE experiment described below was carried out.

RM3-1: 5′-GTGGCACTCCTCTCCTTCCCGCCCCAGA-3′ (SEQ ID NO: 118)

RM3-2: 5′-CAGGCCCCGCAGCCACAGGCTGATAGCA-3′ (SEQ ID NO: 119)

RM3-3: 5′-AGCAGGTGCCAGCCCCACACTGGACATC-3′ (SEQ ID NO: 120)

A 5' RACE PCR reaction solution was prepared by mixing 0.6 μl of 50X Advantage-GC 2 Polymerase Mix (CLONTECH), 6 μl of 5x Advantage-GC 2 PCR buffer supplied with the kit (200 mM Tricine-KOH, 75 mM KOAc, 17.5 mM $Mg(OAc)_2$, 25% dimethyl sulfoxide, 18.75 μg/ml BSA, 0.025% Tween-20, and 0.025% Nonidet-P40), 2.4 μl of dNTP mixture (2.5 mM each, Takara Shuzo Co., Ltd.), 0.6 μl of 10 μM primer RM3-1, 0.6 μl of 10 μM primer AP1 (which was supplied with Marathon-Ready cDNA Kit available from CLONTECH), 3 μl of template cDNA (CLONTECH, rat brain Marathon-Ready cDNA), and 16.8 μl of distilled water. After the early denaturation at 94°C for 30 seconds, the reaction conditions were set to repeat 5 cycles of reactions at 94°C for 5 seconds and at 72°C for 3 minutes, 5 cycles of reactions at 94°C for 5 seconds and at 70°C for 3 minutes, and 25 cycles of reactions at 94°C for 5 seconds and at 68°C for 3 minutes.

Subsequently, using a reaction solution obtained from the above PCR reaction as a template, a nested PCR was carried out. A reaction solution was prepared by mixing 0.6 μl of 50X Advantage-GC 2 Polymerase Mix (CLONTECH), 6 μl of 5x Advantage-GC 2 PCR buffer supplied with the kit (200 mM Tricine-KOH, 75 mM KOAc, 17.5 mM $Mg(OAc)_2$, 25% dimethyl sulfoxide, 18.75 μg/ml BSA, 0.025% Tween-20, and 0.025% Nonidet-P40), 2.4 μl of dNTP mixture (2.5 mM each, Takara Shuzo Co., Ltd.), 0.6 μl of 10 μM primer RM3-2 or RM3-3, 0.6 μl of 10 μM primer AP2 (which was supplied with Marathon-Ready cDNA Kit available from CLONTECH), 3 μl of template DNA (which was a 50 times diluted solution of the above PCR reaction solution), and 16.8 μl of distilled water. After the early denaturation at 94°C for 30 seconds, the reaction conditions were set to repeat 30 cycles of reactions at 94°C for 5 seconds and at 68°C for 3 minutes.

The obtained DNA fragment was cloned using TOPO TA Cloning Kit (Invitrogen) according to the method described in a manual supplied with the kit. The sequence of the cloned DNA was determined using ABI377 DNA sequencer, so as to obtain a sequence located at the 5'-terminus (SEQ ID NO: 121).

Primers RM5-1 (SEQ ID NO: 122) and RM5-4 (SEQ ID NO: 123) were prepared based on the information regarding SEQ ID NOS: 116 and 117. Using the obtained primers, a 3' RACE experiment described below was carried out.

RM5-1: 5′-GGAAGGAGAGGAGTGCCACCCTGGAAG-3′ (SEQ ID NO: 122)

RM5-4: 5′-ACCATACCTGTCCCTGTTCACCCAGCCT-3′ (SEQ ID NO: 123)

A 3' RACE PCR reaction solution was prepared by mixing 0.6 μl of 50X Advantage-GC 2 Polymerase Mix (CLONTECH), 6 μl of 5x Advantage-GC 2 PCR buffer supplied with the kit (200 mM Tricine-KOH, 75 mM KOAc, 17.5 mM $Mg(OAc)_2$, 25% dimethyl sulfoxide, 18.75 μg/ml BSA, 0.025% Tween-20, and 0.025% Nonidet-P40), 2.4 μl of dNTP mixture (2.5 mM each, Takara Shuzo Co., Ltd.), 0.6 μl of 10 μM primer RM5-1, 0.6 μl of 10 μM primer AP1 (which was supplied with Marathon-Ready cDNA Kit available from CLONTECH), 3 μl of template cDNA (CLONTECH, rat brain Marathon-Ready cDNA), and 16.8 μl of distilled water. After the early denaturation at 94°C for 1 minute, the reaction conditions were set to repeat 5 cycles of reactions at 94°C for 30 seconds and at 72°C for 3 minutes, 5 cycles of reactions at 94°C for 30 seconds and at 70°C for 3 minutes, 25 cycles of reactions at 94°C for 30 seconds and at 68°C for 3 minutes, and a final extension reaction at 68°C for 3 minutes.

Subsequently, using a reaction solution obtained from the above PCR reaction as a template, a nested PCR was carried out. A reaction solution was prepared by mixing 0.6 µl of 50X Advantage-GC 2 Polymerase Mix (CLONTECH), 6 µl of 5x Advantage-GC 2 PCR buffer supplied with the kit (200 mM Tricine-KOH, 75 mM KOAc, 17.5 mM Mg $(OAc)_2$, 25% dimethyl sulfoxide, 18.75 µg/ml BSA, 0.025% Tween-20, and 0.025% Nonidet-P40), 2.4 µl of dNTP mixture (2.5 mM each, Takara Shuzo Co., Ltd.), 0.6 µl of 10 µM primer RM5-4 or RM3-3, 0.6 µl of 10 µM primer AP2 (which was supplied with Marathon-Ready cDNA Kit available from CLONTECH), 3 µl of template DNA (which was a 50 times diluted solution of the above PCR reaction solution), and 16.8 µl of distilled water. After the early denaturation at 94°C for 1 minute, the reaction conditions were set to repeat 35 cycles of reactions at 94°C for 30 seconds and at 68°C for 3 minutes, and a final extension reaction at 68°C for 3 minutes.

The obtained DNA fragment was cloned using TOPO TA Cloning Kit (Invitrogen) according to the method described in a manual supplied with the kit. The sequence of the cloned DNA was determined using ABI377 DNA Sequencer, so as to obtain a sequence located at the 3'-terminus (SEQ ID NO: 124).

Using rat brain QUICK-clone cDNA (CLONTECH) or rat brain cDNA (Wistar rat) as a template, primers RBv8-WF1 (SEQ ID NO: 125), RBv8-WF2 (SEQ ID NO: 126), RBv8-WR1 (SEQ ID NO: 127) and RBv8-WR2 (SEQ ID NO: 128) were prepared based on the information obtained by the 5' RACE and the 3' RACE. Using the obtained primers, a PCR reaction described below was carried out.

RMIT-WF1: 5′-ATTCCAGAGTGGACAGTGTTTGCCTTCACC-3′ (SEQ ID NO: 125)

RMIT-WF2: 5′-GATCATGAGAGGTGCTGTGCAAGTCTTC-3′ (SEQ ID NO: 126)

RMIT-WR1: 5′-CTCTCTGCACGCTGCTGGACTGTTCC-3′ (SEQ ID NO: 127)

RMIT-WR2: 5′-CAGATGTAACACAAGAGGTCACCCAGTAGG-3′ (SEQ ID NO: 128)

A PCR reaction solution was prepared by mixing 0.6 µl of PfuTurbo DNA polymerase (Stratagene), 3 µl of 10x PCR buffer supplied with the kit, 2.4 µl of 2.5 mM dNTP mixture, 1.5 µl each of 10 µM primers RMIT-WF1 and RMIT-WR1, 1 µl of template DNA, and 20 µl of distilled water. After the early denaturation at 95°C for 1 minute, the reaction conditions were set to repeat 35 cycles of reactions at 95°C for 30 seconds, at 55°C for 30 seconds and at 72°C for 1 minute, and a final extension reaction at 72°C for 5 minutes.

Subsequently, using, as a template, a solution obtained by 50 times diluting the reaction solution obtained as a result of the above PCR reaction, a nested PCR was carried out. A PCR reaction solution was prepared by mixing 0.6 µl of PfuTurbo DNA polymerase (Stratagene), 3 µl of 10x PCR buffer supplied with the kit, 2.4 µl of 2.5 mM dNTP mixture, 1.5 µl each of 10 µM primers RMIT-WF2 and RMIT-WR2, 3 µl of template DNA, and 18 µl of distilled water. After the early denaturation at 95°C for 1 minute, the reaction conditions were set to repeat 35 cycles of reactions at 95°C for 30 seconds, at 55°C for 30 seconds and at 72°C for 1 minute, and a final extension reaction at 72°C for 5 minutes.

The obtained DNA fragment was cloned using Zaro Blunt TOPO PCR Cloning Kit (Invitrogen) according to the method described in a manual supplied with the kit. The sequence of the cloned DNA was determined using ABI377DNA sequencer, so as to obtain plasmids having 3 types of 375 bp DNA fragments (SEQ ID NOS: 129, 130 and 131; the fragments with base substitution are referred to as normal type, type Y and type Q, respectively.) encoding a rat ZAQ ligand full-length peptide. The obtained plasmids were named as pRMIT, pRMITY and pHMITQ, respectively. *Escherichia coli* TOP10 was transformed with each of the above plasmids, and the transformed *Escherichia coli* were named as *Escherichia coli* TOP10/pRMIT, *Escherichia coli* TOP10/pRMITY and *Escherichia coli* TOP10/pRMITQ, respectively.

The nucleotide sequences of these DNA fragments were analyzed. As a result, it was found that a DNA fragment shown in SEQ ID NO: 129 contains DNA (SEQ ID NO: 133) encoding a rat type ZAQ ligand precursor peptide (105 amino acid residues) shown in SEQ ID NO: 132, that a DNA fragment shown in SEQ ID NO: 130 contains DNA (SEQ ID NO: 135) encoding a rat type ZAQ ligand precursor peptide (105 amino acid residues) shown in SEQ ID NO: 134, and that a DNA fragment shown in SEQ ID NO: 131 contains DNA (SEQ ID NO: 137) encoding a rat type ZAQ ligand precursor peptide (105 amino acid residues) shown in SEQ ID NO: 136.

Moreover, it was also found that the nucleotide sequences shown in SEQ ID NOS: 132, 134 and 136 have typical signal sequences. It was clarified that the DNA having the nucleotide sequence shown in SEQ ID NO: 132 contains DNA (SEQ ID NO: 139) consisting of 258 base pairs encoding a rat type ZAQ ligand mature peptide (86 amino acid residues) shown in SEQ ID NO: 138, that the DNA having the nucleotide sequence shown in SEQ ID NO: 134 contains DNA (SEQ ID NO: 141) consisting of 258 base pairs encoding a rat type ZAQ ligand mature peptide (86 amino acid residues) shown in SEQ ID NO: 140, and that the DNA having the nucleotide sequence shown in SEQ ID NO: 136 contains DNA (SEQ ID NO: 143) consisting of 258 base pairs encoding a rat type ZAQ ligand mature peptide (86 amino acid residues) shown in SEQ ID NO: 142.

When compared with the amino acid sequence shown in SEQ ID NO: 138, His at position 46 is substituted by Tyr in SEQ ID NO: 140, and Arg at position 36 is substituted by Gln in SEQ ID NO: 142.

INDUSTRIAL APPLICABILITY

The peptide, MIT1 or protein of the present invention, an antibody against them, and a polynucleotide encoding the peptide or protein of the present invention are useful in a simple searching (screening) method for obtaining an angiogenesis inhibitor. In particular, an antagonist to the protein of the present invention is useful as an excellent angiogenesis inhibitor. In addition, the screening method of the present invention is carried out using endothelial cells derived from mammals, whereby an angiogenesis inhibitor can be screened under more physiological conditions than usual.

Moreover, the protein, DNA, antisense DNA, and antibody of the present invention is useful to practice (i) an agent for preventing or treating various types of diseases with which the protein of the present invention is associated, (ii) screening of a compound or salt thereof changing the binding of the peptide of the present invention with the protein of the present invention, (iii) assay of the protein of the present invention, (iv) a gene diagnosis agent, (v) a pharmaceutical containing antisense DNA, (vi) a pharmaceutical and a diagnostic agent containing the antibody of the present invention, (vii) production of a non-human animal containing the DNA of the present invention, and (viii) drug design based on a comparison with a structurally similar ligand receptor. In particularly, using a receptor binding assay system in which an expression system of the recombinant protein of the present invention or the like is used, a compound (e.g., an agonist, an antagonist, etc.) changing the binding of a ligand with the protein of the present invention specific for humans or mammals can be screened. Thus, such an agonist or antagonist can be used safely as an agent for preventing or treating various diseases such as diseases accompanying angiogenesis [for example, cancers (e.g., pancreatic cancer, lung cancer, kidney cancer, liver cancer, non-small cell lung cancer, ovarian cancer, prostatic cancer, stomach cancer, bladder cancer, breast cancer, cervical cancer, colonic cancer, rectal cancer, Kaposi's sarcoma, etc.), diseases of ovary (e.g., polycystic ovary syndrome (polycystic ovarian disease), ovarian hyperstimulation syndrome, etc.), inflammatory diseases (e.g., arthrorheumatism, etc.), diabetic retinopathy].

## Claims

1. An angiogenesis inhibitor, which comprises a compound or salt thereof inhibiting the activity of a peptide or a salt thereof comprising the same or substantially the same amino acid sequence shown in SEQ ID NO: 81, SEQ ID NO:82, SEQ ID NO:109, SEQ ID NO:138, SEQ ID NO:19 or SEQ ID NO: 39.

2. An angiogenesis inhibitor, which comprises a compound or salt thereof inhibiting the activity of a protein or a salt thereof comprising the same or substantially the same the amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO:173.

3. An angiogenesis inhibitor, which comprises a compound or salt thereof inhibiting the binding of (a) a peptide or salt thereof comprising the same or substantially the same amino acid sequence shown in SEQ ID NO: 21, SEQ ID NO:81, SEQ ID NO:82, SEQ ID NO: 109, SEQ ID NO:138, SEQ ID NO:19 or SEQ ID NO:39, with (b) a protein or salt thereof comprising the same or substantially the same the amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO:173.

4. An angiogenesis inhibitor, which comprises a compound or salt thereof inhibiting the action of a peptide or salt thereof comprising the same or substantially the same amino acid sequence shown in SEQ ID NO: 21, SEQ ID NO:81, SEQ ID NO:82, SEQ ID NO:109, SEQ ID NO:138, SEQ ID NO:19 or SEQ ID NO:39, to activate a protein or salt thereof comprising the same or substantially the same the amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO:173.

5. An angiogenesis inhibitor, which comprises an antisense nucleotide having a nucleotide sequence or portion thereof complementary to or substantially complementary to the nucleotide sequence of a DNA encoding a peptide or partial peptide thereof comprising the same or substantially the same amino acid sequence shown in SEQ ID NO: 81, SEQ ID NO:82, SEQ ID NO:109, SEQ ID NO:138, SEQ ID NO:19 or SEQ ID NO:39.

6. An angiogenesis inhibitor, which comprises an antibody against a peptide, partial peptide thereof or salt thereof comprising the same or substantially the same amino acid sequence shown in SEQ ID NO: 81, SEQ ID NO:82, SEQ ID NO:109, SEQ ID NO:138, SEQ ID NO:19 or SEQ ID NO:39.

7. An angiogenesis inhibitor, which comprises an antisense nucleotide having a nucleotide sequence or portion thereof complementary to or substantially complementary to the nucleotide sequence of a DNA encoding a protein or partial peptide thereof comprising the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO:173.

8. An angiogenesis inhibitor, which comprises an antibody against a protein, partial peptide thereof or salt thereof comprising the same or substantially the same amino acid sequence shown in SEQ ID NO: 1, SEQ ID NO:44, SEQ ID NO:51, SEQ ID NO: 52, SEQ ID NO:53, SEQ ID NO:54, SEQ ID NO:171 or SEQ ID NO:173.

9. The angiogenesis inhibitor according to any one of claims 1 to 8, which is an agent for preventing or treating cancer, polycystic ovary syndrome or ovarian hyperstimulation syndrome.

**10.** An agent for diagnosing cancer, polycystic ovary syndrome or ovarian hyperstimulation syndrome, which comprises (a) a polynucleotide encoding a peptide or salt thereof comprising the same or substantially the same amino acid sequence shown in SEQ ID NO: 81, SEQ ID NO:82, SEQ ID NO:109, SEQ ID NO:138, SEQ ID NO:19 or SEQ ID NO:39, or (b) a polynucleotide encoding a protein or salt thereof having an amino acid sequence identical to or substantially identical to the amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO:173.

**11.** A method for screening a compound or salt thereof inhibiting the activity of a protein or salt thereof comprising the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO:173, wherein endothelial cells are used in the screening test.

**12.** An angiogenesis inhibitor, which comprises a compound or salt thereof selected by the screening method according to claim 11.

**13.** A method for screening an angiogenesis inhibitor, **characterized in that** said method uses (a) a peptide or salt thereof comprising the same or substantially the same amino acid sequence shown in SEQ ID NO: 21, SEQ ID NO:81, SEQ ID NO:82, SEQ ID NO:109, SEQ ID NO:138, SEQ ID NO:19 or SEQ ID NO:39, and/or (b) a protein or salt thereof comprising the same or substantially the same amino acid sequence shown in SEQ ID NO:1, SEQ ID NO:44, SEQ ID NO:51, SEQ ID NO:52, SEQ ID NO:53, SEQ ID NO:54, SEQ ID NO:171 or SEQ ID NO:173.

**14.** A kit for screening an angiogenesis inhibitor, **characterized in that** said method comprises (a) a peptide or salt thereof comprising the same or substantially the same amino acid sequence shown in SEQ ID NO: 21, SEQ ID NO:81, SEQ ID NO:82, SEQ ID NO:109, SEQ ID NO:138, SEQ ID NO:19 or SEQ ID NO:39, and/or (b) a protein or salt thereof comprising the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO:173.

**15.** A method for screening an angiogenesis inhibitor, **characterized in that** said method uses (a) a polynucleotide encoding a peptide or salt thereof comprising the same or substantially the same amino acid sequence shown in SEQ ID NO: 81, SEQ ID NO:82, SEQ ID NO:109, SEQ ID NO:138, SEQ ID NO:19 or SEQ ID NO:39, and/or (b) a polynucleotide encoding a protein or salt thereof comprising the same or substantially the same amino acid sequence shown in SEQ ID NO: 1, SEQ ID NO:44, SEQ ID NO:51, SEQ ID NO:52, SEQ ID NO:53, SEQ ID NO: 54, SEQ ID NO:171 or SEQ ID NO:173.

**16.** A method for screening an angiogenesis inhibitor, **characterized in that** said method uses (a) an antibody against a peptide, partial peptide thereof or salt thereof comprising the same or substantially the same amino acid sequence shown in SEQ ID NO: 21, SEQ ID NO:81, SEQ ID NO:82, SEQ ID NO:109, SEQ ID NO:138, SEQ ID NO:19 or SEQ ID NO:39, and/or (b) an antibody against a protein, partial peptide thereof or salt thereof comprising the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO:173.

**17.** An angiogenesis inhibitor, which is obtained using the screening method according to claim 13, 15 or 16, or the screening kit according to claim 14.

**18.** An angiogenesis inhibitor, which comprises a compound or salt thereof having an action to inhibit the growth of endothelial cells.

**19.** A protein or salt thereof, **characterized in that** said protein contains the same or substantially the same amino acid sequence shown in SEQ ID NO: 171 or SEQ ID NO:137.

**20.** A protein or salt thereof consisting of the amino acid sequence shown in SEQ ID NO: 171.

**21.** A protein or salt thereof consisting of the amino acid sequence shown in SEQ ID NO: 137.

**22.** A partial peptide or salt thereof of the protein according to claim 19.

**23.** A polynucleotide, which comprises a polynucleotide encoding the protein according to claim 19.

**24.** The polynucleotide according to claim 23, wherein said polynucleotide is DNA.

**25.** A polynucleotide consisting of a nucleotide sequence shown in SEQ ID NO: 172 or SEQ ID NO:174.

**26.** A recombinant vector comprising the polynucleotide according to claim 24.

**27.** A transformant transformed by the recombinant vector according to claim 26.

**28.** A method for producing the protein or salt thereof according to claim 19, **characterized in that** said method comprises culturing the transformant according to claim 27, and producing and accumulating the protein according to claim 19.

**29.** An antibody against the protein, partial peptide thereof or salt thereof according to claim 19.

**30.** A drug comprising the antibody according to claim 29.

**31.** A diagnostic agent comprising the antibody according to claim 29.

**32.** A method for screening a compound or salt thereof promoting or inhibiting the activity of the protein or salt thereof according to claim 19, **characterized in that** said method uses the protein, partial peptide thereof or salt thereof according to claim 19.

**33.** A kit for screening a compound or salt thereof promoting or inhibiting the activity of the protein or salt thereof according to claim 19, **characterized in that** said method uses the protein, partial peptide thereof or salt thereof according to claim 19.

**34.** A compound or salt thereof promoting or inhibiting the activity of the protein or salt thereof according to claim 19, which is selected using the screening method according to claim 32 or the screening kit according to claim 33.

**35.** A drug comprising the compound or salt thereof according to claim 34.

**36.** A method for inhibiting angiogenesis in a mammal, **characterized in that** said method comprises administration of an effective dose of a compound or salt thereof inhibiting the activity of a protein or salt thereof comprising the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO:173, to said mammal.

**37.** Use of a compound or salt thereof inhibiting the activity of a protein or salt thereof comprising the same or substantially the same amino acid sequence shown in SEQ ID NO: 1, SEQ ID NO:44, SEQ ID NO:51, SEQ ID NO: 52, SEQ ID NO:53, SEQ ID NO:54, SEQ ID NO:171 or SEQ ID NO:173 to produce an angiogenesis inhibitor.

**38.** A method for preventing or treating cancer in a mammal, polycystic ovary syndrome or ovarian hyperstimulation syndrome, **characterized in that** said method comprises administration of an effective dose of a compound or salt thereof inhibiting the activity of a protein or salt thereof comprising the same or substantially the same amino acid sequence shown in SEQ ID NO: 1, SEQ ID NO:44, SEQ ID NO:51, SEQ ID NO:52, SEQ ID NO:53, SEQ ID NO:54, SEQ ID NO:171 or SEQ ID NO:173, to said mammal.

**39.** Use of a compound or salt thereof inhibiting the activity of a protein or salt thereof comprising the same or substantially the same amino acid sequence shown in SEQ ID NO: 1, SEQ ID NO:44, SEQ ID NO:51, SEQ ID NO: 52, SEQ ID NO:53, SEQ ID NO:54, SEQ ID NO:171 or SEQ ID NO:173 to produce an agent for preventing or treating cancer, polycystic ovary syndrome or ovarian hyperstimulation syndrome.

FIG. 1

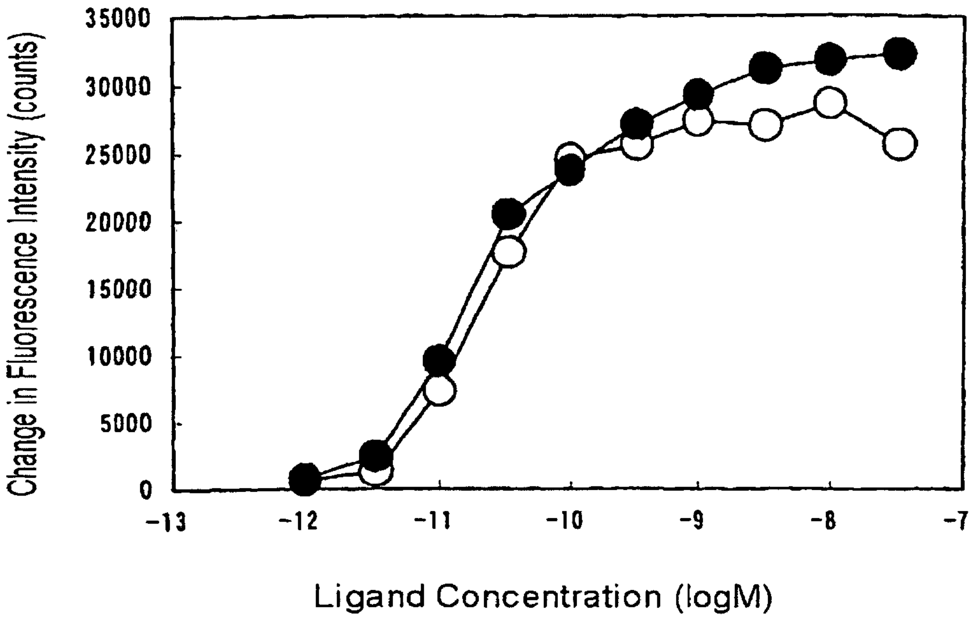
Change in Fluorescence Intensity (counts)
35000
30000
25000
20000
15000
10000
5000
0
-13
-12
-11
-10
-9
-8
-7
Ligand Concentration (logM)

FIG. 2

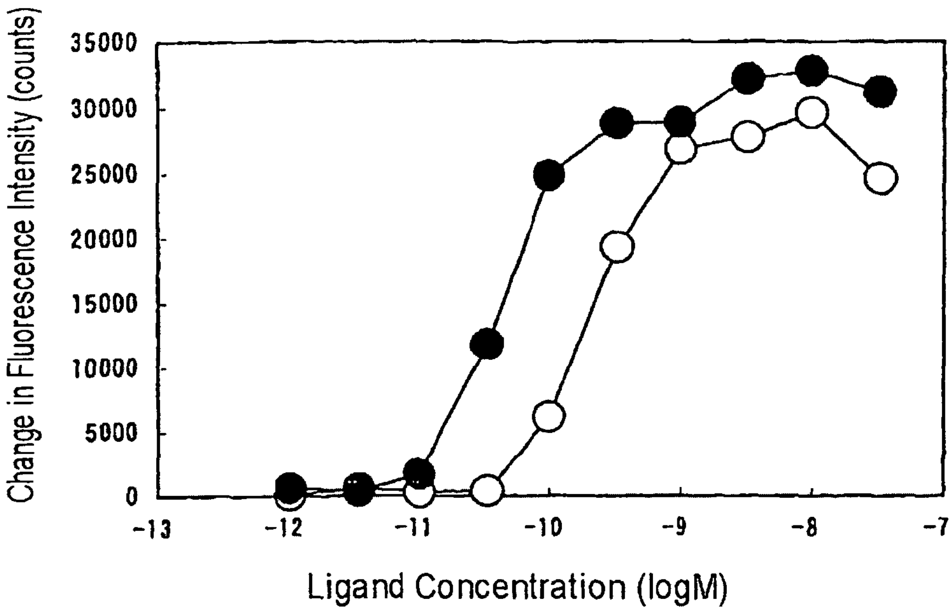
Change in Fluorescence Intensity (counts)
35000
30000
25000
20000
15000
10000
5000
0
-13
-12
-11
-10
-9
-8
-7
Ligand Concentration (logM)

FIG. 3

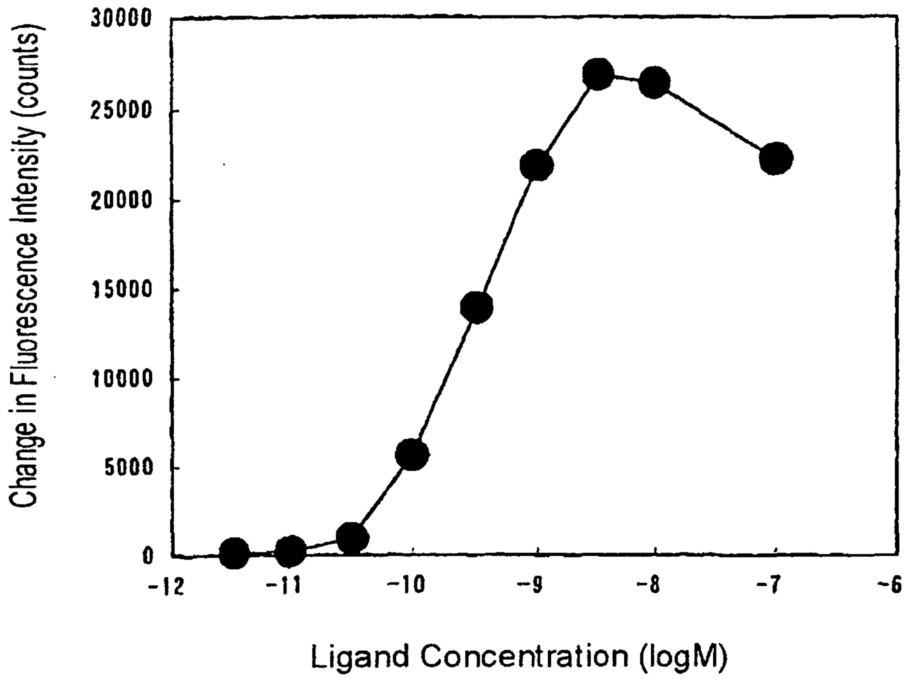
Change in Fluorescence Intensity (counts)
30000
25000
20000
15000
10000
5000
0
-12
-11
-10
-9
-8
-7
-6
Ligand Concentration (logM)

FIG. 4

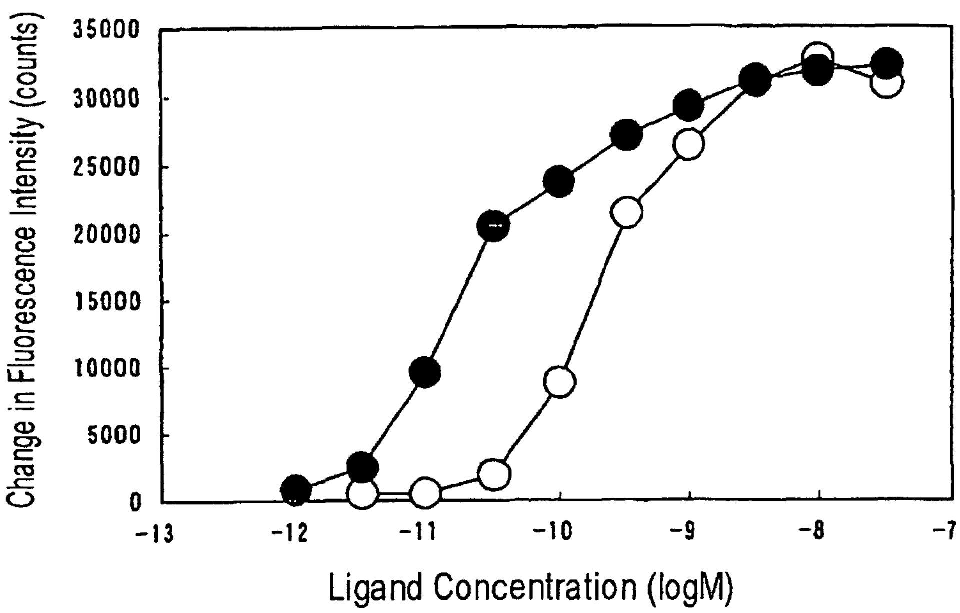
Change in Fluorescence Intensity (counts)
35000
30000
25000
20000
15000
10000
5000
0
-13
-12
-11
-10
-9
-8
-7
Ligand Concentration (logM)

FIG. 5

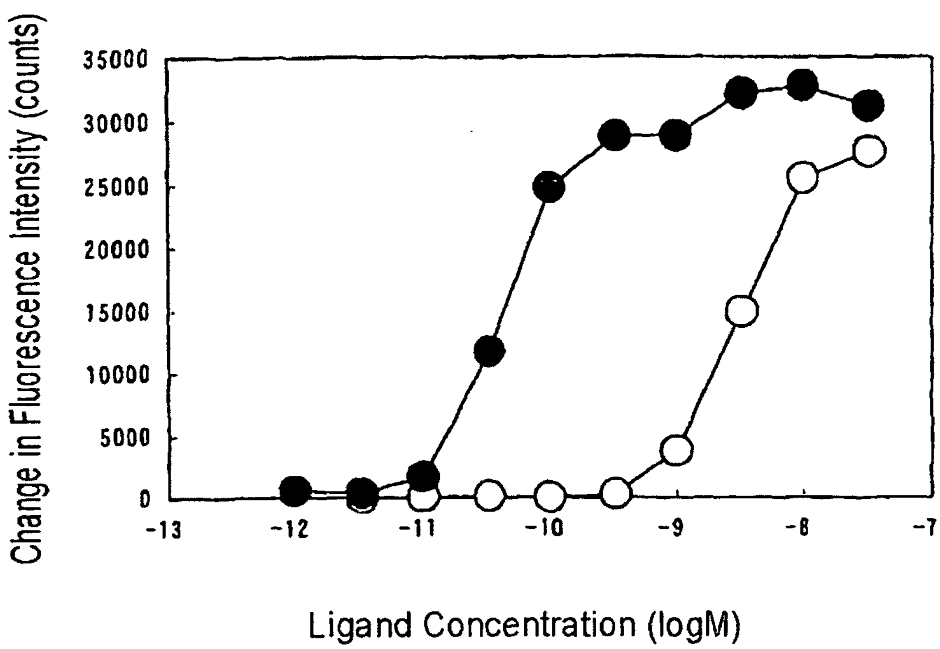
Change in Fluorescence Intensity (counts)
35000
30000
25000
20000
15000
10000
5000
0
-13
-12
-11
-10
-9
-8
-7
Ligand Concentration (logM)

FIG. 6

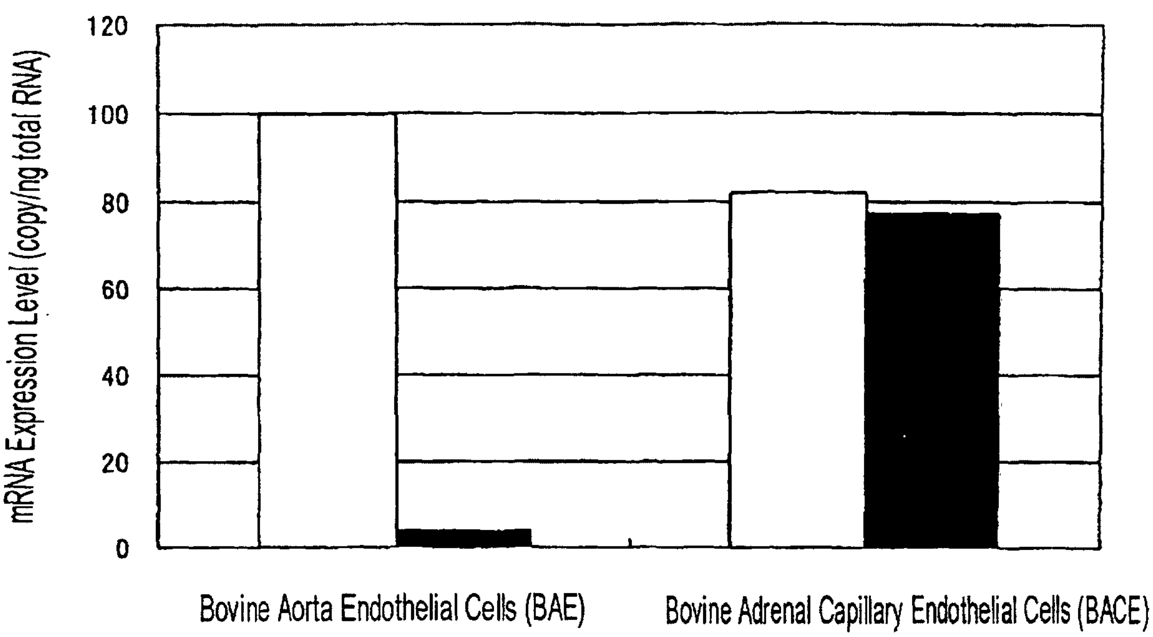

120
100
80
60
40
20
0
mRNA Expression Level (copy/ng total RNA)
Bovine Aorta Endothelial Cells (BAE)
Bovine Adrenal Capillary Endothelial Cells (BACE)

FIG. 7

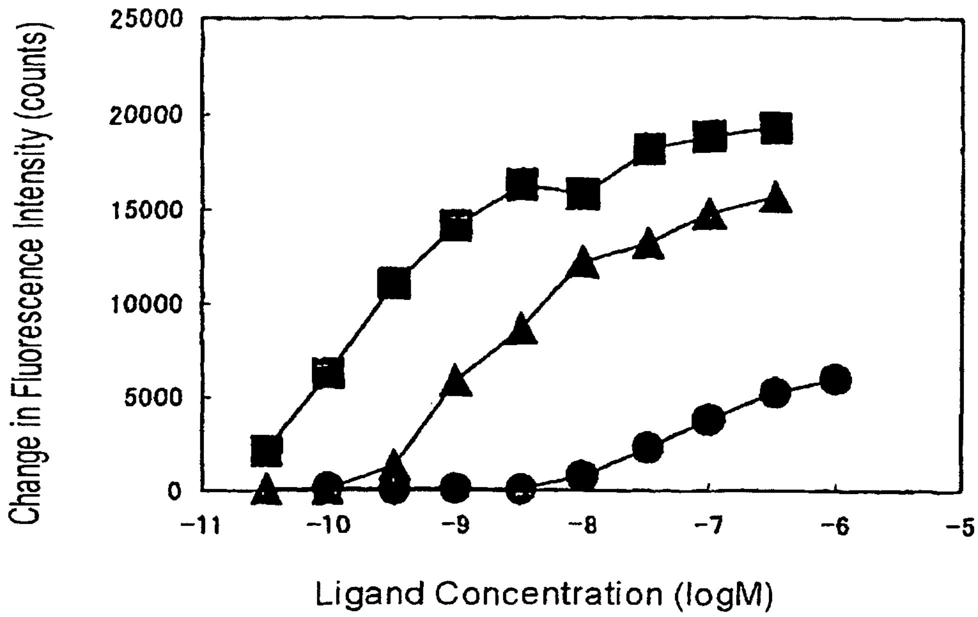
Change in Fluorescence Intensity (counts)
25000
20000
15000
10000
5000
0
−11
−10
−9
−8
−7
−6
−5
Ligand Concentration (logM)

FIG. 8

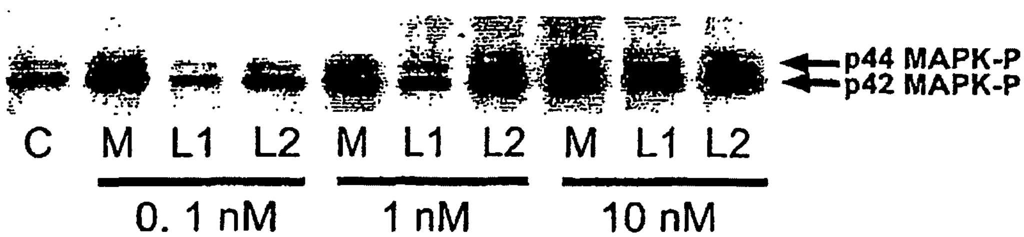

FIG. 9

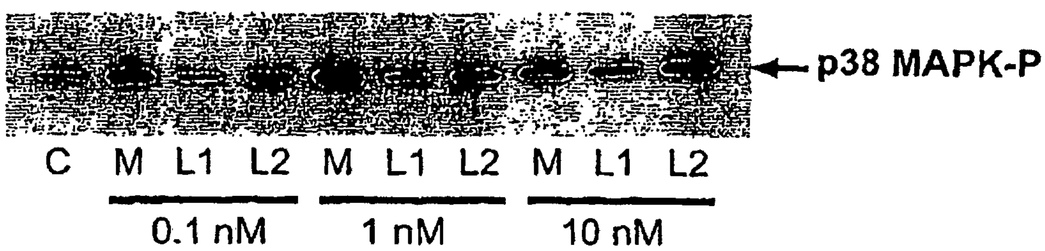

FIG. 10

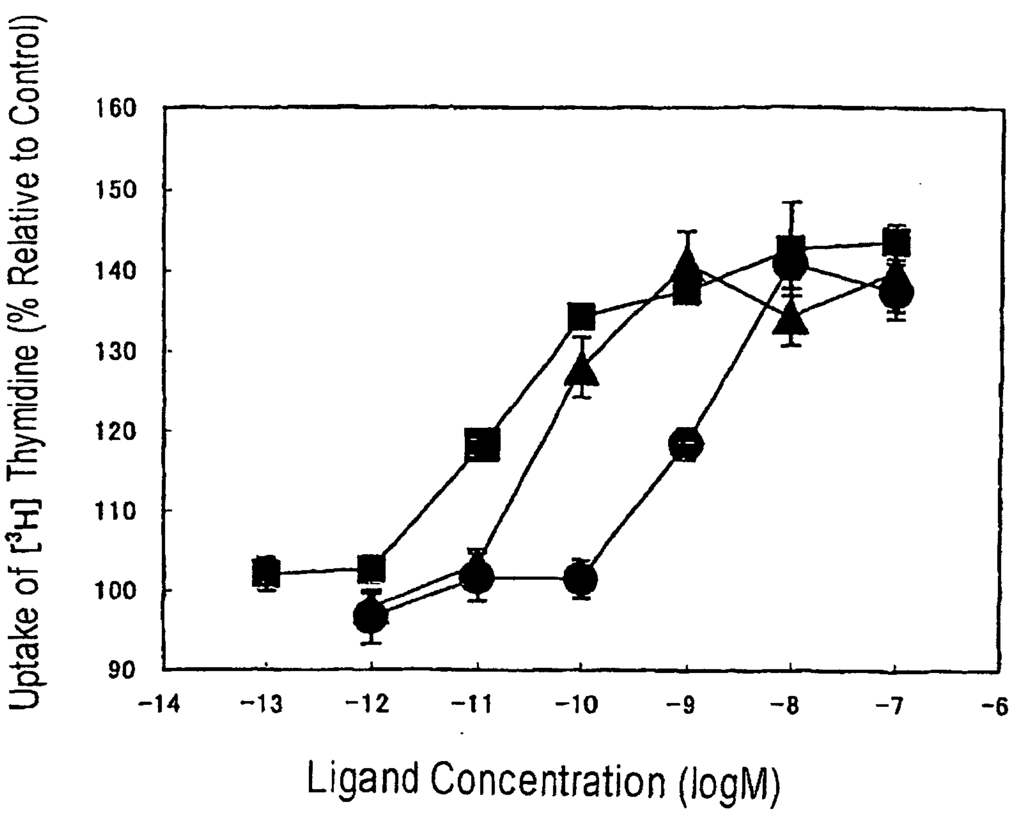
Uptake of [$^3$H] Thymidine (% Relative to Control)
160
150
140
130
120
110
100
90
−14
−13
−12
−11
−10
−9
−8
−7
−6
Ligand Concentration (logM)

FIG. 11

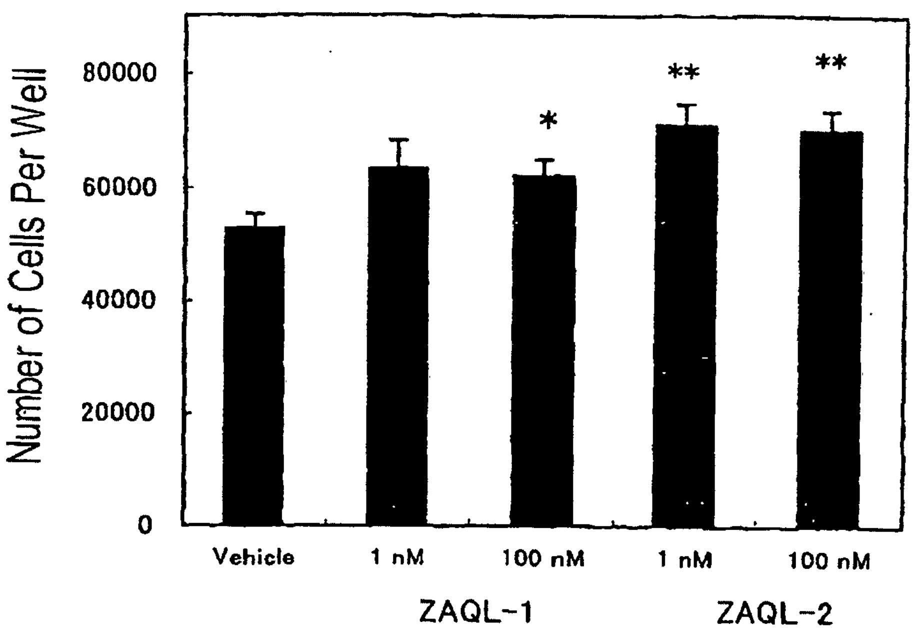
Number of Cells Per Well
80000
60000
40000
20000
0
**
**
*
Vehicle
1 nM
100 nM
1 nM
100 nM
ZAQL-1
ZAQL-2

## INTERNATIONAL SEARCH REPORT

International application No.
PCT/JP03/01057

**A. CLASSIFICATION OF SUBJECT MATTER**

Int.Cl$^7$ C12N15/12, C07K14/47, C07K16/18, C12N5/10, C12P21/02, C12Q1/68, A61K31/7088, A61K39/395, A61K45/00, A61K48/00, A61P15/08, A61P35/00, A61P43/00

According to International Patent Classification (IPC) or to both national classification and IPC

**B. FIELDS SEARCHED**

Minimum documentation searched (classification system followed by classification symbols)

Int.Cl$^7$ C12N15/12, C07K14/47, C07K16/18, C12N5/10, C12P21/02, C12Q1/68, A61K31/7088, A61K39/395, A61K45/00, A61K48/00, A61P15/08, A61P35/00, A61P43/00

Documentation searched other than minimum documentation to the extent that such documents are included in the fields searched

Electronic data base consulted during the international search (name of data base and, where practicable, search terms used)

WPI(DIALOG), BIOSIS(DIALOG), GenBank/EMBL/DDBJ/GeneSeq, SwissProt/PIR/GeneSeq

**C. DOCUMENTS CONSIDED TO BE RELEVANT**

| Category* | Citation of document, with indication, where appropriate, of the relevant passages | Relevant to claim No. |
|---|---|---|
| A | WO 02/06483 A1 (Takeda Chemical Industries, Ltd.), 24 January, 2002 (24.01.02), & JP 2002-335976 A & AU 200172735 A | 5,6,10,13-16 |
| A | WO 01/57190 A2 (HYSEQ, INC.), 09 August, 2001 (09.08.01), & US 2002/0128187 A1 & AU 200134847 A | 5,6,10,13-16 |
| A | WO 01/16309 A1 (Takeda Chemical Industries, Ltd.), 08 March, 2001 (08.03.01), & EP 1207198 A1 & JP 2002-95474 A & AU 200067280 A | 5,6,10,13-16 |

☐ Further documents are listed in the continuation of Box C. ☐ See patent family annex.

* Special categories of cited documents:
- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 24 April, 2003 (24.04.03) | 13 May, 2003 (13.05.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/01057

**Box I Observations where certain claims were found unsearchable (Continuation of item 2 of first sheet)**

This international search report has not been established in respect of certain claims under Article 17(2)(a) for the following reasons:

1. [X] Claims Nos.: 36, 38
because they relate to subject matter not required to be searched by this Authority, namely:
The method of inhibiting angiogenesis and the prevention/treatment method as set forth in claims 36 and 38 pertain to methods for treatment of the human body by therapy and thus relate to a subject matter which this International Searching Authority is not required, under the (continued to extra sheet)

2. [X] Claims Nos.: 1-4, 12, 17, 18, 34, 35, 37, 39
because they relate to parts of the international application that do not comply with the prescribed requirements to such an extent that no meaningful international search can be carried out, specifically:
The angiogenesis inhibitors and compounds as set forth in claims 1 to 4, 12, 17, 18, 34, 35, 37 and 39 are specified exclusively by a function or a screening method. However, the description presents neither any compounds having this function nor any compounds obtained by this screening method.

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:
The amino acid sequences relating to SEQ ID NOS:81, 82, 109, 138, 19 and 39 as set forth in claim 5 are common to each other in being I5E receptor ligands.
However, the acquisition of a ZAQ ligand peptide had been publicly known as reported in the document WO 02/06483 A1. Thus, the above matter is not considered as a special technical feature in the meaning as described in the second sentence in PCT Rule 13.2.
Such being the case, these inventions are not considered as relating to a group of inventions so linked a to form a single general inventive concept but consisting of six groups of inventions relating (continued to extra sheet)

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [X] No required additional search fees were timely paid by the applicant. Consequently, this international search report is restricted to the invention first mentioned in the claims; it is covered by claims Nos.:
In claims 5, 6, 10 and 13 to 16, inventions relating to the amino acid sequence represented by SEQ ID NO:81.

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No.
PCT/JP03/01057

Continuation of Box No.I-1 of continuation of first sheet(1)

provisions of Article 17(2)(a)(i) of the PCT and Rule 39.1(iV) of the Regulations under the PCT, to search.

Continuation of Box No.II of continuation of first sheet(1)

to the peptides having six amino acid sequences respectively.

For the same reason, the whole claims are considered as consisting of 16 groups of inventions relating to the peptides having the amino acid sequences of SEQ ID NOS:81, 82, 109, 138, 19, 39, 21, 1, 44, 51, 52, 53, 54, 171, 173 and 137.

Accordingly, claims 1 to 4, 12, 17, 18, 34, 35, 37 and 39 are neither disclosed in the description in the meaning as described in PCT Article 5 nor supported thereby in the meaning as described in PCT Article 6. Even though the common technical knowledge at the point of the application is considered, it is completely unknown what specific compounds are involved and what are not. Therefore, the above claims are described in an extremely unclear manner and thus fail to fulfill the requirement of clearness as described in PCT Article 6.

Such being the case, no meaningful search can be made on the inventions as set forth in the above claims.

Form PCT/ISA/210 (extra sheet) (July 1998)

SEQUENCE LISTING
<110> Takeda Chemical Industries, Ltd.

<120> Angiogenesis inhibitor

<130> 3021WOOP

<150> JP2002-27299
<151> 2002-2-4

<160> 184

<210> 1
<211> 393
<212> PRT
<213> Homo sapiens

<400> 1
```
Met Glu Thr Thr Met Gly Phe Met Asp Asp Asn Ala Thr Asn Thr Ser
                5                   10                  15
Thr Ser Phe Leu Ser Val Leu Asn Pro His Gly Ala His Ala Thr Ser
            20                  25                  30
Phe Pro Phe Asn Phe Ser Tyr Ser Asp Tyr Asp Met Pro Leu Asp Glu
        35                  40                  45
Asp Glu Asp Val Thr Asn Ser Arg Thr Phe Phe Ala Ala Lys Ile Val
    50                  55                  60
Ile Gly Met Ala Leu Val Gly Ile Met Leu Val Cys Gly Ile Gly Asn
65                  70                  75                  80
Phe Ile Phe Ile Ala Ala Leu Val Arg Tyr Lys Lys Leu Arg Asn Leu
                85                  90                  95
Thr Asn Leu Leu Ile Ala Asn Leu Ala Ile Ser Asp Phe Leu Val Ala
            100                 105                 110
Ile Val Cys Cys Pro Phe Glu Met Asp Tyr Tyr Val Val Arg Gln Leu
        115                 120                 125
Ser Trp Glu His Gly His Val Leu Cys Thr Ser Val Asn Tyr Leu Arg
    130                 135                 140
Thr Val Ser Leu Tyr Val Ser Thr Asn Ala Leu Leu Ala Ile Ala Ile
145                 150                 155                 160
Asp Arg Tyr Leu Ala Ile Val His Pro Leu Arg Pro Arg Met Lys Cys
                165                 170                 175
Gln Thr Ala Thr Gly Leu Ile Ala Leu Val Trp Thr Val Ser Ile Leu
            180                 185                 190
Ile Ala Ile Pro Ser Ala Tyr Phe Thr Thr Glu Thr Val Leu Val Ile
        195                 200                 205
Val Lys Ser Gln Glu Lys Ile Phe Cys Gly Gln Ile Trp Pro Val Asp
    210                 215                 220
Gln Gln Leu Tyr Tyr Lys Ser Tyr Phe Leu Phe Ile Phe Gly Ile Glu
225                 230                 235                 240
Phe Val Gly Pro Val Val Thr Met Thr Leu Cys Tyr Ala Arg Ile Ser
                245                 250                 255
Arg Glu Leu Trp Phe Lys Ala Val Pro Gly Phe Gln Thr Glu Gln Ile
            260                 265                 270
Arg Lys Arg Leu Arg Cys Arg Arg Lys Thr Val Leu Val Leu Met Cys
        275                 280                 285
Ile Leu Thr Ala Tyr Val Leu Cys Trp Ala Pro Phe Tyr Gly Phe Thr
    290                 295                 300
Ile Val Arg Asp Phe Phe Pro Thr Val Phe Val Lys Glu Lys His Tyr
305                 310                 315                 320
Leu Thr Ala Phe Tyr Ile Val Glu Cys Ile Ala Met Ser Asn Ser Met
                325                 330                 335
Ile Asn Thr Leu Cys Phe Val Thr Val Lys Asn Asp Thr Val Lys Tyr
            340                 345                 350
Phe Lys Lys Ile Met Leu Leu His Trp Lys Ala Ser Tyr Asn Gly Gly
        355                 360                 365
Lys Ser Ser Ala Asp Leu Asp Leu Lys Thr Ile Gly Met Pro Ala Thr
    370                 375                 380
Glu Glu Val Asp Cys Ile Arg Leu Lys
385                 390
```

```
<210> 2
<211> 1179
<212> DNA
<213> Homo sapiens

<400> 2
atggagacca ccatggggtt catggatgac aatgccacca acacttccac cagcttcctt   60
tctgtgctca accctcatgg agcccatgcc acttccttcc cattcaactt cagctacagc  120
gactatgata tgcctttgga tgaagatgag gatgtgacca attccaggac gttctttgct  180
gccaagattg tcattgggat ggccctggtg ggcatcatgc tggtctgcgg cattggaaac  240
ttcatcttta tcgctgccct ggtccgctac aagaaactgc gcaacctcac caacctgctc  300
atcgccaacc tggccatctc tgacttcctg gtggccattg tctgctgccc ctttgagatg  360
gactactatg tggtgcgcca gctctcctgg gagcacggcc acgtcctgtg cacctctgtc  420
aactacctgc gcactgtctc tctctatgtc tccaccaatg ccctgctggc catcgccatt  480
gacaggtatc tggctattgt ccatccgctg agaccacgga tgaagtgcca aacagccact  540
ggcctgattg ccttggtgtg gacggtgtcc atcctgatcg ccatcccttc cgcctacttc  600
accaccgaga cggtcctcgt cattgtcaag agccaggaaa agatcttctg cggccagatc  660
tggcctgtgg accagcagct ctactacaag tcctacttcc tctttatctt tggcatagaa  720
ttcgtgggcc ccgtggtcac catgaccctg tgctatgcca ggatctcccg ggagctctgg  780
ttcaaggcgg tccctggatt ccagacagag cagatccgca agaggctgcg ctgccgcagg  840
aagacggtcc tggtgctcat gtgcatcctc accgcctacg tgctatgctg ggcgcccttc  900
tacggcttca ccatcgtgcg cgacttcttc cccaccgtgt tcgtgaagga gaagcactac  960
ctcactgcct tctacatcgt cgagtgcatc gccatgagca acagcatgat caacactctg 1020
tgcttcgtga ccgtcaagaa cgacaccgtc aagtacttca aaaagatcat gttgctccac 1080
tggaaggctt cttacaatgg cggtaagtcc agtgcagacc tggacctcaa gacaattggg 1140
atgcctgcca ccgaagaggt ggactgcatc agactaaaa                        1179

<210> 3
<211> 1179
<212> DNA
<213> Homo sapiens

<400> 3

atggagacca ccatggggtt catggatgac aatgccacca acacttccac cagcttcctt   60
tctgtgctca accctcatgg agcccatgcc acttccttcc cattcaactt cagctacagc  120
gactatgata tgcctttgga tgaagatgag gatgtgacca attccaggac gttctttgct  180
gccaagattg tcattgggat ggccctggtg ggcatcatgc tggtctgcgg cattggaaac  240
ttcatcttta tcgctgccct ggtccgctac aagaaactgc gcaacctcac caacctgctc  300
atcgccaacc tggccatctc tgacttcctg gtggccattg tctgctgccc ctttgagatg  360
gactactatg tggtgcgcca gctctcctgg gagcacggcc acgtcctgtg cacctctgtc  420
aactacctgc gcactgtctc tctctatgtc tccaccaatg ccctgctggc catcgccatt  480
gacaggtatc tggctattgt ccatccgctg agaccacgga tgaagtgcca aacagccact  540
ggcctgattg ccttggtgtg gacggtgtcc atcctgatcg ccatcccttc cgcctacttc  600
accaccgaga cggtcctcgt cattgtcaag agccaggaaa agatcttctg cggccagatc  660
tggcctgtgg accagcagct ctactacaag tcctacttcc tctttatctt tggcatagaa  720
ttcgtgggcc ccgtggtcac catgaccctg tgctatgcca ggatctcccg ggagctctgg  780
ttcaaggcgg tccctggatt ccagacagag cagatccgca agaggctgcg ctgccgcagg  840
aagacggtcc tggtgctcat gtgcatcctc accgcctacg tgctatgctg ggcgcccttc  900
tacggcttca ccatcgtgcg cgacttcttc cccaccgtgt ttgtgaagga gaagcactac  960
ctcactgcct tctacatcgt cgagtgcatc gccatgagca acagcatgat caacactctg 1020
tgcttcgtga ccgtcaagaa cgacaccgtc aagtacttca aaaagatcat gttgctccac 1080
tggaaggctt cttacaatgg cggtaagtcc agtgcagacc tggacctcaa gacaattggg 1140
atgcctgcca ccgaagaggt ggactgcatc agactaaaa                        1179

<210> 4
<211> 31
<212> DNA
<213> Artificial Sequence

<220>
<223> misc feature

<400> 4

gtcgacatgg agaccaccat ggggttcatg g                31
```

<210> 5
<211> 36
<212> DNA
<213> Artificial Sequence

<220>
<223> misc feature

<400> 5

actagtttat tttagtctga tgcagtccac ctcttc 36

<210> 6
<211> 1152
<212> DNA
<213> Homo sapiens

<400> 6
atggcagccc agaatggaaa caccagtttc acacccaact ttaatccacc ccaagaccat 60
gcctcctccc tctcctttaa cttcagttat ggtgattatg acctccctat ggatgaggat 120
gaggacatga ccaagacccg gaccttcttc gcagccaaga tcgtcattgg cattgcactg 180
gcaggcatca tgctggtctg cggcatcggt aactttgtct ttatcgctgc cctcacccgc 240
tataagaagt tgcgcaacct caccaatctg ctcattgcca acctggccat ctccgacttc 300
ctggtggcca tcatctgctg cccccttcgag atggactact acgtggtacg gcagctctcc 360
tgggagcatg gccacgtgct ctgtgcctcc gtcaactacc tgcgcaccgt ctccctctac 420
gtctccacca atgccttgct ggccattgcc attgacagat atctcgccat cgttcacccc 480
ttgaaaccac ggatgaatta tcaaacggcc tccttcctga tcgccttggt ctggatggtg 540
tccattctca ttgccatccc atcggcttac tttgcaacag aaaccgtcct ctttattgtc 600
aagagccagg agaagatctt ctgtggccag atctggcctg tggatcagca gctctactac 660
aagtcctact tcctcttcat ctttggtgtc gagttcgtgg gccctgtggt caccatgacc 720
ctgtgctatg ccaggatctc ccgggagctc tggttcaagg cagtccctgg gttccagacg 780
gagcagattc gcaagcggct gcgctgccgc aggaagacgg tcctggtgct catgtgcatt 840
ctcacggcct atgtgctgtg ctgggcaccc ttctacggtt tcaccatcgt tcgtgacttc 900
ttccccactg tgttcgtgaa ggaaaagcac tacctcactg ccttctacgt ggtcgagtgc 960
atcgccatga gcaacagcat gatcaacacc gtgtgcttcg tgacggtcaa gaacaacacc 1020
atgaagtact tcaagaagat gatgctgctg cactggcgtc cctcccagcg ggggagcaag 1080
tccagtgctg accttgacct cagaaccaac ggggtgccca ccacagaaga agtggactgt 1140
atcaggctga ag 1152

<210> 7
<211> 26
<212> DNA
<213> Artificial Sequence

<220>
<223> misc feature

<400> 7

ctacttctgc tgctgccgct gctgtt 26

<210> 8
<211> 28
<212> DNA
<213> Artificial Sequence

<220>
<223> misc feature

<400> 8

ttggaaagtt gaggaagcaa gagcattt 28

<210> 9
<211> 359
<212> DNA
<213> Homo sapiens

<400> 9

cacgccccgc gctggggacg ccgccgtgat caccggggct tgtgacaagg actcccaatg 60
tggtggaggc atgtgctgtg ctgtcagtat ctgggtcaag agcataagga tttgcacacc 120
tatgggcaaa ctgggagaca gctgccatcc actgactcgt aaagttccat tttttgggcg 180
gaggatgcat cacacttgcc catgtctgcc aggcttggcc tgtttacgga cttcatttaa 240
ccgatttatt tgtttagccc aaaagtaatc gctctggagt agaaaccaaa tgtgaatagc 300
cacatcttac ctgtaaagtc ttacttgtga ttgtgccaaa caaaaaatgt gccagaaag 359

<210> 10
<211> 27
<212> DNA
<213> Artificial Sequence

<220>
<223> misc feature

<400> 10
tgtctcccag tttgcccata ggtgtgc 27

<210> 11
<211> 184
<212> DNA
<213> Homo sapiens

<400> 11

cccgagggcg ccatgaggag cctgtgctgc gccccactcc tgctcctctt gctgctgccg 60
ccgctgctgc tcacgccccg cgctggggac gccgccgtga tcaccggggc ttgtgacaag 120
gactcccaat gtggtggagg catgtgctgt gctgtcagta tctgggtcaa gagcataagg 180
attt 184

<210> 12
<211> 23
<212> DNA
<213> Artificial Sequence

<220>
<223> misc feature

<400> 12

ccatgaggag cctgtgctgc gcc 23

<210> 13
<211> 23
<212> DNA
<213> Artificial Sequence

<220>
<223> misc feature

<400> 13

ctattcacat ttggtttcta ctc 23

<210> 14
<211> 29
<212> DNA
<213> Artificial Sequence

<220>
<223> misc feature

<400> 14

gtcgaccacc atgaggagcc tgtgctgcg 29

<210> 15
<211> 26
<212> DNA
<213> Artificial Sequence

<220>
<223> misc feature

<400> 15

actagtcgat tacttttggg ctaaac 26

<210> 16
<211> 346
<212> DNA
<213> Homo sapiens

<400> 16

gtcgaccacc atgaggagcc tgtgctgcgc cccactcctg ctcctcttgc tgctgccgcc 60
gctgctgctc acgccccgcg ctggggacgc cgccgtgatc accggggctt gtgacaagga 120
ctcccaatgt ggtggaggca tgtgctgtgc tgtcagtatc tgggtcaaga gcataaggat 180
ttgcacacct atgggcaaac tgggagacag ctgccatcca ctgactcgta aagttccatt 240
ttttgggcgg aggatgcatc acacttgccc atgtctgcca ggcttggcct gtttacggac 300
ttcatttaac cgatttattt gtttagccca aaagtaatcg actagt 346

<210> 17
<211> 108
<212> PRT
<213> Homo sapiens

<400> 17

Met Arg Ser Leu Cys Cys Ala Pro Leu Leu Leu Leu Leu Leu Leu Pro
1 5 10 15
Pro Leu Leu Leu Thr Pro Arg Ala Gly Asp Ala Ala Val Ile Thr Gly
20 25 30
Ala Cys Asp Lys Asp Ser Gln Cys Gly Gly Gly Met Cys Cys Ala Val
35 40 45
Ser Ile Trp Val Lys Ser Ile Arg Ile Cys Thr Pro Met Gly Lys Leu
50 55 60
Gly Asp Ser Cys His Pro Leu Thr Arg Lys Val Pro Phe Phe Gly Arg
65 70 75 80
Arg Met His His Thr Cys Pro Cys Leu Pro Gly Leu Ala Cys Leu Arg
85 90 95
Thr Ser Phe Asn Arg Phe Ile Cys Leu Ala Gln Lys
100 105

<210> 18
<211> 324
<212> DNA
<213> Homo sapiens

<400> 18

atgaggagcc tgtgctgcgc cccactcctg ctcctcttgc tgctgccgcc gctgctgctc 60
acgccccgcg ctggggacgc cgccgtgatc accggggctt gtgacaagga ctcccaatgt 120
ggtggaggca tgtgctgtgc tgtcagtatc tgggtcaaga gcataaggat ttgcacacct 180
atgggcaaac tgggagacag ctgccatcca ctgactcgta aagttccatt ttttgggcgg 240
aggatgcatc acacttgccc atgtctgcca ggcttggcct gtttacggac ttcatttaac 300
cgatttattt gtttagccca aaag 324

<210> 19
<211> 81
<212> PRT
<213> Homo sapiens

<400> 19

Ala Val Ile Thr Gly Ala Cys Asp Lys Asp Ser Gln Cys Gly Gly Gly
5 10 15
Met Cys Cys Ala Val Ser Ile Trp Val Lys Ser Ile Arg Ile Cys Thr
20 25 30
Pro Met Gly Lys Leu Gly Asp Ser Cys His Pro Leu Thr Arg Lys Val
35 40 45
Pro Phe Phe Gly Arg Arg Met His His Thr Cys Pro Cys Leu Pro Gly
50 55 60
Leu Ala Cys Leu Arg Thr Ser Phe Asn Arg Phe Ile Cys Leu Ala Gln
65 70 75 80
Lys

<210> 20
<211> 243
<212> DNA
<213> Homo sapiens

<400> 20

gccgtgatca ccggggcttg tgacaaggac tcccaatgtg gtggaggcat gtgctgtgct 60
gtcagtatct gggtcaagag cataaggatt tgcacaccta tgggcaaact gggagacagc 120
tgccatccac tgactcgtaa agttccattt tttgggcgga ggatgcatca cacttgccca 180
tgtctgccag gcttggcctg tttacggact tcatttaacc gatttatttg tttagcccaa 240
aag 243

<210> 21
<211> 80
<212> PRT
<213> Dendroaspip polylepis

<400> 21

Ala Val Ile Thr Gly Ala Cys Glu Arg Asp Leu Gln Cys Gly Lys Gly
5 10 15
Thr Cys Cys Ala Val Ser Leu Trp Ile Lys Ser Val Arg Val Cys Thr
20 25 30
Pro Val Gly Thr Ser Gly Glu Asp Cys His Pro Ala Ser His Lys Ile
35 40 45
Pro Phe Ser Gly Gln Arg Met His His Thr Cys Pro Cys Ala Pro Asn
50 55 60
Leu Ala Cys Val Gln Thr Ser Pro Lys Lys Phe Lys Cys Leu Ser Lys
65 70 75 80

<210> 22
<211> 80
<212> PRT
<213> Homo sapiens

<400> 22

Ala Val Ile Thr Gly Ala Cys Asp Lys Asp Ser Gln Cys Gly Gly Gly
5 10 15
Met Cys Cys Ala Val Ser Ile Trp Val Lys Ser Ile Arg Ile Cys Thr
20 25 30
Pro Met Gly Lys Leu Gly Asp Ser Cys His Pro Leu Thr Arg Lys Val
35 40 45
Pro Phe Phe Gly Arg Arg Met His His Thr Cys Pro Cys Leu Pro Gly
50 55 60
Leu Ala Cys Leu Arg Thr Ser Phe Asn Arg Phe Ile Cys Leu Ala Gln
65 70 75 80

<210> 23
<211> 20
<212> DNA
<213> Artificial Sequence

<220>
<223> misc feature

<400> 23

gcttgygaca aggactcyca 20

<210> 24
<211> 19
<212> DNA
<213> Artificial Sequence

<220>
<223> misc feature

<400> 24

gttyctacty cagagygat 19

<210> 25
<211> 210
<212> DNA
<213> Homo sapiens

<400> 25

gtgtggagga ggaatgtgct gcgctgtcag tatctgggtt aagagcataa ggatctgcac 60
acctatgggc caagtgggag acagctgcca ccccctgact cggaaagttc cattttgggg 120
gcggaggatg caccacactt gtccctgcct gccaggtttg gcatgtttaa ggacttcttt 180
caaccgtttt atttgtttgg cccggaagtg 210

<210> 26
<211> 26
<212> DNA
<213> Artificial Sequence

<220>
<223> misc feature

<400> 26

gtgcatcctc cgcccccaaa atggaa 26

<210> 27
<211> 28
<212> DNA
<213> Artificial Sequence

<220>
<223> misc feature

<400> 27

gacagcgcag cacattcctc ctccacac 28

<210> 28
<211> 148
<212> DNA
<213> Homo sapiens

<400> 28

cgcgtcccta accgccaccg cctcctcggg acgccatgga ggacccgcgc tgtgccccgc 60
tactgctact tttgctgcta ccgctgctgc tcacaccgcc cgccggggat gccgcggtca 120
tcaccggggc ttgcgacaag gactctca 148

<210> 29
<211> 28
<212> DNA
<213> Artificial Sequence

<220>
<223> misc feature

<400> 29

gagacagctg ccaccccctg actcggaa 28

<210> 30
<211> 28
<212> DNA
<213> Artificial Sequence

<220>
<223> misc feature

<400> 30

ggcggaggat gcaccacact tgtccctg 28

<210> 31
<211> 150
<212> DNA
<213> Rattus spp

<400> 31

cctgcctgcc aggtttggca tgtttaagga cttctttcaa ccgttttatt tgtttggccc 60
ggaagtgatc actctgaagc aggagctgga aatgtgaacc tctactcact gaacaatgtc 120
tgtcaagtct cgcttgtaat tgtgtcaaag 150

<210> 32
<211> 18
<212> DNA
<213> Artificial Sequence

<220>
<223> misc feature

<400> 32

taaccgccac cgcctcct 18

<210> 33
<211> 17
<212> DNA
<213> Artificial Sequence

<220>
<223> misc feature

<400> 33

gggacgccat ggaggac 17

<210> 34
<211> 26
<212> DNA
<213> Artificial Sequence

<220>
<223> misc feature

<400> 34

cgagacttga cagacattgt tcagtg 26

<210> 35

<211> 20
<212> DNA
<213> Artificial Sequence

<220>
<223> misc feature

<400> 35

tttccagctc ctgcttcaga 20

<210> 36
<211> 356
<212> DNA
<213> Rattus spp

<400> 36

```
gggacgccat ggaggacccg cgctgtgccc cgctactgct acttttgctg ctaccgctgc  60
tgctcacacc gcccgccggg gatgccgcgg tcatcaccgg ggcttgcgac aaggactctc 120
agtgtggagg aggaatgtgc tgcgctgtca gtatctgggt taagagcata aggatctgca 180
cacctatggg ccaagtggga gacagctgcc accccctgac tcggaaagtt ccattttggg 240
ggcggaggat gcaccacact tgtccctgcc tgccaggttt ggcatgttta aggacttctt 300
tcaaccgttt tatttgtttg gcccggaagt gatcactctg aagcaggagc tggaaa     356
```

<210> 37
<211> 107
<212> PRT
<213> Rattus spp

<400> 37

```
Met Glu Asp Pro Arg Cys Ala Pro Leu Leu Leu Leu Leu Leu Leu Pro
                5                   10                  15
Leu Leu Leu Thr Pro Pro Ala Gly Asp Ala Ala Val Ile Thr Gly Ala
            20                  25                  30
Cys Asp Lys Asp Ser Gln Cys Gly Gly Gly Met Cys Cys Ala Val Ser
        35                  40                  45
Ile Trp Val Lys Ser Ile Arg Ile Cys Thr Pro Met Gly Gln Val Gly
    50                  55                  60
Asp Ser Cys His Pro Leu Thr Arg Lys Val Pro Phe Trp Gly Arg Arg
65                  70                  75                  80
Met His His Thr Cys Pro Cys Leu Pro Gly Leu Ala Cys Leu Arg Thr
                85                  90                  95
Ser Phe Asn Arg Phe Ile Cys Leu Ala Arg Lys
            100                 105
```

<210> 38
<211> 321
<212> DNA
<213> Rattus spp

<400> 38

```
atggaggacc cgcgctgtgc cccgctactg ctacttttgc tgctaccgct gctgctcaca  60
ccgcccgccg gggatgccgc ggtcatcacc ggggcttgcg acaaggactc tcagtgtgga 120
ggaggaatgt gctgcgctgt cagtatctgg gttaagagca taaggatctg cacacctatg 180
ggccaagtgg gagacagctg ccaccccctg actcggaaag ttccattttg ggggcggagg 240
atgcaccaca cttgtccctg cctgccaggt ttggcatgtt taaggacttc tttcaaccgt 300
tttatttgtt tggcccggaa g                                           321
```

<210> 39
<211> 81
<212> PRT
<213> Rattus spp or Mus spp

<400> 39

```
Ala Val Ile Thr Gly Ala Cys Asp Lys Asp Ser Gln Cys Gly Gly Gly
```

```
                        5                   10                  15
Met Cys Cys Ala Val Ser Ile Trp Val Lys Ser Ile Arg Ile Cys Thr
                20                  25                  30
Pro Met Gly Gln Val Gly Asp Ser Cys His Pro Leu Thr Arg Lys Val
            35                  40                  45
Pro Phe Trp Gly Arg Arg Met His His Thr Cys Pro Cys Leu Pro Gly
    50                  55                  60
Leu Ala Cys Leu Arg Thr Ser Phe Asn Arg Phe Ile Cys Leu Ala Arg
65                  70                  75                  80
Lys
```

<210> 40
<211> 243
<212> DNA
<213> Rattus spp

<400> 40

```
gcggtcatca ccggggcttg cgacaaggac tctcagtgtg gaggaggaat gtgctgcgct  60
gtcagtatct gggttaagag cataaggatc tgcacaccta tgggccaagt gggagacagc 120
tgccaccccc tgactcggaa agttccattt tgggggcgga ggatgcacca cacttgtccc 180
tgcctgccag gtttggcatg tttaaggact tctttcaacc gttttatttg tttggcccgg 240
aag                                                               243
```

<210> 41
<211> 30
<212> DNA
<213> Artificial Sequence

<220>
<223> misc feature

<400> 41

```
gtcgacatgg agaccactgt ggggaccctg      30
```

<210> 42
<211> 30
<212> DNA
<213> Artificial Sequence

<220>
<223> misc feature

<400> 42

```
actagtttat ttcagtcgga tgcagtccac      30
```

<210> 43
<211> 1179
<212> DNA
<213> Rattus spp

<400> 43

```
atggagacca ctgtggggac cctgggcgag aataccacaa acactttcac cgacttcttt   60
tctgcacgtg atggcagtgg agccgaaacc tccccccttgc cattcacttt cagctatggt  120
gactatgaca tgccctcgga tgaagaggag gatgtgacca actctcggac tttctttgct  180
gccaagattg tcattggcat ggctttggtg ggcatcatgc tggtgtgtgg catcggcaac  240
ttcatcttca tcactgcgct ggcccgctac aaaaagcttc gcaacctcac caacctgctt  300
atcgccaacc tggccatttc ggacttcctg gtagccatcg tgtgctgccc ctttgagatg  360
gactactatg tggtacgcca gctctcctgg gagcacggcc atgtcctgtg cgcctccgtc  420
aactacttgc gcaccgtctc cctctacgtg tccactaacg ccctactggc cattgccatt  480
gacaggtatc tggccattgt gcacccgctg agaccgcgga tgaagtgtca aacggctgca  540
ggcctgatct tcctggtgtg gtctgtgtcc atcctcatcg ccatcccagc cgcctacttc  600
accactgaga cggtgttggt catcgtggaa agccaggaga agatcttctg cggccagatc  660
tggccggtgg atcagcagtt ctactacagg tcctatttcc ttttggtctt cggcctcgag  720
ttcgtgggtc ctgtaatcgc catgaccctg tgctatgcca gggtgtcccg agagctctgg  780
ttcaaggcgg tgcccggctt ccagacagag cagatccgcc ggaggctgcg ctgtcgccga  840
```

```
cggacggtac tggggctcgt gtgcgtcctt tccgcctatg tgctgtgctg ggctcccttc  900
tatggcttca ccatcgtgcg tgacttcttc ccctccgtgt ttgtgaaaga gaagcactac  960
ctcaccgcct tttatgtggt ggagtgcatc gccatgagca acagtatgat caatacgctg 1020
tgctttgtga ctgtcaggaa taacaccagt aagtacctca agaggatcct gcggctccag 1080
tggagggcct ctcctagcgg gagcaaggcc agcgctgacc tcgacctcag gaccacgggg 1140
attcctgcca cggaggaggt ggactgcatc cgactgaaa                       1179

<210> 44
<211> 393
<212> PRT
<213> Rattus spp

<400> 44

Met Glu Thr Thr Val Gly Thr Leu Gly Glu Asn Thr Thr Asn Thr Phe
1               5                   10                  15
Thr Asp Phe Phe Ser Ala Arg Asp Gly Ser Gly Ala Glu Thr Ser Pro
            20                  25                  30
Leu Pro Phe Thr Phe Ser Tyr Gly Asp Tyr Asp Met Pro Ser Asp Glu
        35                  40                  45
Glu Glu Asp Val Thr Asn Ser Arg Thr Phe Phe Ala Ala Lys Ile Val
    50                  55                  60
Ile Gly Met Ala Leu Val Gly Ile Met Leu Val Cys Gly Ile Gly Asn
65                  70                  75                  80
Phe Ile Phe Ile Thr Ala Leu Ala Arg Tyr Lys Lys Leu Arg Asn Leu
                85                  90                  95
Thr Asn Leu Leu Ile Ala Asn Leu Ala Ile Ser Asp Phe Leu Val Ala
            100                 105                 110
Ile Val Cys Cys Pro Phe Glu Met Asp Tyr Tyr Val Val Arg Gln Leu
        115                 120                 125
Ser Trp Glu His Gly His Val Leu Cys Ala Ser Val Asn Tyr Leu Arg
    130                 135                 140
Thr Val Ser Leu Tyr Val Ser Thr Asn Ala Leu Leu Ala Ile Ala Ile
145                 150                 155                 160
Asp Arg Tyr Leu Ala Ile Val His Pro Leu Arg Pro Arg Met Lys Cys
                165                 170                 175
Gln Thr Ala Ala Gly Leu Ile Phe Leu Val Trp Ser Val Ser Ile Leu
            180                 185                 190
Ile Ala Ile Pro Ala Ala Tyr Phe Thr Thr Glu Thr Val Leu Val Ile
        195                 200                 205
Val Glu Ser Gln Glu Lys Ile Phe Cys Gly Gln Ile Trp Pro Val Asp
    210                 215                 220
Gln Gln Phe Tyr Tyr Arg Ser Tyr Phe Leu Leu Val Phe Gly Leu Glu
225                 230                 235                 240
Phe Val Gly Pro Val Ile Ala Met Thr Leu Cys Tyr Ala Arg Val Ser
                245                 250                 255
Arg Glu Leu Trp Phe Lys Ala Val Pro Gly Phe Gln Thr Glu Gln Ile
            260                 265                 270
Arg Arg Arg Leu Arg Cys Arg Arg Arg Thr Val Leu Gly Leu Val Cys
        275                 280                 285
Val Leu Ser Ala Tyr Val Leu Cys Trp Ala Pro Phe Tyr Gly Phe Thr
    290                 295                 300
Ile Val Arg Asp Phe Phe Pro Ser Val Phe Val Lys Glu Lys His Tyr
305                 310                 315                 320
Leu Thr Ala Phe Tyr Val Val Glu Cys Ile Ala Met Ser Asn Ser Met
                325                 330                 335
Ile Asn Thr Leu Cys Phe Val Thr Val Arg Asn Asn Thr Ser Lys Tyr
            340                 345                 350
Leu Lys Arg Ile Leu Arg Leu Gln Trp Arg Ala Ser Pro Ser Gly Ser
        355                 360                 365
Lys Ala Ser Ala Asp Leu Asp Leu Arg Thr Thr Gly Ile Pro Ala Thr
    370                 375                 380
Glu Glu Val Asp Cys Ile Arg Leu Lys
385                 390

<210> 45
<211> 31
<212> DNA
```

<213> Artificial Sequence

<220>
<223> misc feature

<400> 45

cctcaccaay ctgctyatyg ccaacctggc c 31

<210> 46
<211> 26
<212> DNA
<213> Artificial Sequence

<220>
<223> misc feature

<400> 46

gtggtrcgsc agctctcctg ggagca 26

<210> 47
<211> 23
<212> DNA
<213> Artificial Sequence

<220>
<223> misc feature

<400> 47

tcccgggagc tctggttcaa ggc 23

<210> 48
<211> 27
<212> DNA
<213> Artificial Sequence

<220>
<223> misc feature

<400> 48

gagtgcatcg ccatgagcaa cagcatg 27

<210> 49
<211> 23
<212> DNA
<213> Artificial Sequence

<220>
<223> misc feature

<400> 49
ggcttgaacc agagctcccg gga 23

<210> 50
<211> 1263
<212> DNA
<213> Rattus spp

<400> 50

atggtatcag ttctgtccaa cagggacctc cacacactgg ccccagctga agtgctgaac 60
tccacgtggg cctatctccc tgacacatac cagcctacct gccacatcat caacatggga 120
gaccagaacg gaaacacaag ctttgcacca gacttgaacc caccccaaga ccacgtctcc 180
ttgctcccct taaactacag ttatggagat tatgacatcc ccctggatga cgatgaggat 240
gtgaccaaga cacagacctt ctttgcagcc aaaatcgtca ttggcgtagc cctggcaggc 300

atcatgctag tctgcggcgt tggcaacttt gtcttcattg ctgccctcgc ccgctacaag 360
aagctgcgca accttaccaa cctcctcatc gctaacctgg ccatctctga cttcctggtg 420
gcgatcgtct gctgcccctt tgagatggac tactacgtag tacgtcagct ttcctgggag 480
catggtcacg tgctttgtgc ctccgtcaac taccttcgta cagtctccct gtacgtctcc 540
accaatgctc tgctggccat cgctattgac agatatctcg ctattgtcca ccccttaaaa 600
cggatgaatt accagaccgc ctccttcctg atcgctttgg tctggatggt ctccatcctc 660
atcgccatcc catctgccta cttcaccaca gaaaccatcc ttgttatcgt caagaatcag 720
gaaaagctct tctgtggtca gatctggccc gtggaccagc agctctacta caaatcctac 780
ttcctcttcg tcttcgggct tgagttcgtg ggtcccgtgg tcactatgac cctgtgctat 840
gccaggatct cccaggagct ctggttcaag gctgtacctg gtttccagac ggagcagatc 900
cgcaagcgac tgcgctgccg ccgaaagaca gtgctattgc tcatgggtat cctcacagcc 960
tacgtgctgt gctgggcgcc tttctatggc tttaccatag tgcgagactt cttccccacg 1020
ctggttgtga aggagaagca ctacctcacc gccttctatg tcgtcgagtg catcgccatg 1080
agcaacagca tgatcaatac tatatgcttc gtgacggtca agaacaacac catgaaatac 1140
ttcaagaaga tgctgctgct gcactggcgg ccctctcact acgggagtaa gtccagcgcg 1200
gacctcgacc tcaaaaccag tggggttcct gccaccgaag aggtggactg tatcaggcta 1260
aag 1263

<210> 51
<211> 421
<212> PRT
<213> Rattus spp

<400> 51

Met Val Ser Val Leu Ser Asn Arg Asp Leu His Thr Leu Ala Pro Ala
5 10 15
Glu Val Leu Asn Ser Thr Trp Ala Tyr Leu Pro Asp Thr Tyr Gln Pro
20 25 30
Thr Cys His Ile Ile Asn Met Gly Asp Gln Asn Gly Asn Thr Ser Phe
35 40 45
Ala Pro Asp Leu Asn Pro Pro Gln Asp His Val Ser Leu Leu Pro Leu
50 55 60
Asn Tyr Ser Tyr Gly Asp Tyr Asp Ile Pro Leu Asp Asp Asp Glu Asp
65 70 75 80
Val Thr Lys Thr Gln Thr Phe Phe Ala Ala Lys Ile Val Ile Gly Val
85 90 95
Ala Leu Ala Gly Ile Met Leu Val Cys Gly Val Gly Asn Phe Val Phe
100 105 110
Ile Ala Ala Leu Ala Arg Tyr Lys Lys Leu Arg Asn Leu Thr Asn Leu
115 120 125
Leu Ile Ala Asn Leu Ala Ile Ser Asp Phe Leu Val Ala Ile Val Cys
130 135 140
Cys Pro Phe Glu Met Asp Tyr Tyr Val Val Arg Gln Leu Ser Trp Glu
145 150 155 160
His Gly His Val Leu Cys Ala Ser Val Asn Tyr Leu Arg Thr Val Ser
165 170 175
Leu Tyr Val Ser Thr Asn Ala Leu Leu Ala Ile Ala Ile Asp Arg Tyr
180 185 190
Leu Ala Ile Val His Pro Leu Lys Arg Met Asn Tyr Gln Thr Ala Ser
195 200 205
Phe Leu Ile Ala Leu Val Trp Met Val Ser Ile Leu Ile Ala Ile Pro
210 215 220
Ser Ala Tyr Phe Thr Thr Glu Thr Ile Leu Val Ile Val Lys Asn Gln
225 230 235 240
Glu Lys Leu Phe Cys Gly Gln Ile Trp Pro Val Asp Gln Gln Leu Tyr
245 250 255
Tyr Lys Ser Tyr Phe Leu Phe Val Phe Gly Leu Glu Phe Val Gly Pro
260 265 270
Val Val Thr Met Thr Leu Cys Tyr Ala Arg Ile Ser Gln Glu Leu Trp
275 280 285
Phe Lys Ala Val Pro Gly Phe Gln Thr Glu Gln Ile Arg Lys Arg Leu
290 295 300
Arg Cys Arg Arg Lys Thr Val Leu Leu Leu Met Gly Ile Leu Thr Ala
305 310 315 320
Tyr Val Leu Cys Trp Ala Pro Phe Tyr Gly Phe Thr Ile Val Arg Asp
325 330 335
Phe Phe Pro Thr Leu Val Val Lys Glu Lys His Tyr Leu Thr Ala Phe

```
                340                     345                     350
Tyr Val Val Glu Cys Ile Ala Met Ser Asn Ser Met Ile Asn Thr Ile
            355                 360                 365
Cys Phe Val Thr Val Lys Asn Asn Thr Met Lys Tyr Phe Lys Lys Met
    370                 375                 380
Leu Leu Leu His Trp Arg Pro Ser His Tyr Gly Ser Lys Ser Ser Ala
385                 390                 395                 400
Asp Leu Asp Leu Lys Thr Ser Gly Val Pro Ala Thr Glu Glu Val Asp
                405                 410                 415
Cys Ile Arg Leu Lys
            420
<210> 52
<211> 384
<212> PRT
<213> Homo sapiens

<400> 52

Met Ala Ala Gln Asn Gly Asn Thr Ser Phe Thr Pro Asn Phe Asn Pro
                5                   10                  15
Pro Gln Asp His Ala Ser Ser Leu Ser Phe Asn Phe Ser Tyr Gly Asp
            20                  25                  30
Tyr Asp Leu Pro Met Asp Glu Asp Glu Asp Met Thr Lys Thr Arg Thr
        35                  40                  45
Phe Phe Ala Ala Lys Ile Val Ile Gly Ile Ala Leu Ala Gly Ile Met
    50                  55                  60
Leu Val Cys Gly Ile Gly Asn Phe Val Phe Ile Ala Ala Leu Thr Arg
65                  70                  75                  80
Tyr Lys Lys Leu Arg Asn Leu Thr Asn Leu Leu Ile Ala Asn Leu Ala
                85                  90                  95
Ile Ser Asp Phe Leu Val Ala Ile Ile Cys Cys Pro Phe Glu Met Asp
            100                 105                 110
Tyr Tyr Val Val Arg Gln Leu Ser Trp Glu His Gly His Val Leu Cys
        115                 120                 125
Ala Ser Val Asn Tyr Leu Arg Thr Val Ser Leu Tyr Val Ser Thr Asn
    130                 135                 140
Ala Leu Leu Ala Ile Ala Ile Asp Arg Tyr Leu Ala Ile Val His Pro
145                 150                 155                 160
Leu Lys Pro Arg Met Asn Tyr Gln Thr Ala Ser Phe Leu Ile Ala Leu
                165                 170                 175
Val Trp Met Val Ser Ile Leu Ile Ala Ile Pro Ser Ala Tyr Phe Ala
            180                 185                 190
Thr Glu Thr Val Leu Phe Ile Val Lys Ser Gln Glu Lys Ile Phe Cys
        195                 200                 205
Gly Gln Ile Trp Pro Val Asp Gln Gln Leu Tyr Tyr Lys Ser Tyr Phe
    210                 215                 220
Leu Phe Ile Phe Gly Val Glu Phe Val Gly Pro Val Val Thr Met Thr
225                 230                 235                 240
Leu Cys Tyr Ala Arg Ile Ser Arg Glu Leu Trp Phe Lys Ala Val Pro
                245                 250                 255
Gly Phe Gln Thr Glu Gln Ile Arg Lys Arg Leu Arg Cys Arg Arg Lys
            260                 265                 270
Thr Val Leu Val Leu Met Cys Ile Leu Thr Ala Tyr Val Leu Cys Trp
        275                 280                 285
Ala Pro Phe Tyr Gly Phe Thr Ile Val Arg Asp Phe Phe Pro Thr Val
    290                 295                 300
Phe Val Lys Glu Lys His Tyr Leu Thr Ala Phe Tyr Val Val Glu Cys
305                 310                 315                 320
Ile Ala Met Ser Asn Ser Met Ile Asn Thr Val Cys Phe Val Thr Val
                325                 330                 335
Lys Asn Asn Thr Met Lys Tyr Phe Lys Lys Met Met Leu Leu His Trp
            340                 345                 350
Arg Pro Ser Gln Arg Gly Ser Lys Ser Ser Ala Asp Leu Asp Leu Arg
        355                 360                 365
Thr Asn Gly Val Pro Thr Thr Glu Glu Val Asp Cys Ile Arg Leu Lys
    370                 375                 380                 384

<210> 53
```

<211> 393
<212> PRT
<213> Mus spp

<400> 53

```
Met Glu Thr Thr Val Gly Ala Leu Gly Glu Asn Thr Thr Asp Thr Phe
                5                   10                  15
Thr Asp Phe Phe Ser Ala Leu Asp Gly His Glu Ala Gln Thr Gly Ser
            20                  25                  30
Leu Pro Phe Thr Phe Ser Tyr Gly Asp Tyr Asp Met Pro Leu Asp Glu
        35                  40                  45
Glu Glu Asp Val Thr Asn Ser Arg Thr Phe Phe Ala Ala Lys Ile Val
    50                  55                  60
Ile Gly Met Ala Leu Val Gly Ile Met Leu Val Cys Gly Ile Gly Asn
65                  70                  75                  80
Phe Ile Phe Ile Thr Ala Leu Ala Arg Tyr Lys Lys Leu Arg Asn Leu
                85                  90                  95
Thr Asn Leu Leu Ile Ala Asn Leu Ala Ile Ser Asp Phe Leu Val Ala
            100                 105                 110
Ile Val Cys Cys Pro Phe Glu Met Asp Tyr Tyr Val Val Arg Gln Leu
        115                 120                 125
Ser Trp Glu His Gly His Val Leu Cys Ala Ser Val Asn Tyr Leu Arg
    130                 135                 140
Thr Val Ser Leu Tyr Val Ser Thr Asn Ala Leu Leu Ala Ile Ala Ile
145                 150                 155                 160
Asp Arg Tyr Leu Ala Ile Val His Pro Leu Arg Pro Arg Met Lys Cys
                165                 170                 175
Gln Thr Ala Ala Gly Leu Ile Phe Leu Val Trp Ser Val Ser Ile Leu
            180                 185                 190
Ile Ala Ile Pro Ala Ala Tyr Phe Thr Thr Glu Thr Val Leu Val Ile
        195                 200                 205
Val Glu Arg Gln Glu Lys Ile Phe Cys Gly Gln Ile Trp Pro Val Asp
    210                 215                 220
Gln Gln Phe Tyr Tyr Arg Ser Tyr Phe Leu Leu Val Phe Gly Leu Glu
225                 230                 235                 240
Phe Val Gly Pro Val Val Ala Met Thr Leu Cys Tyr Ala Arg Val Ser
                245                 250                 255
Arg Glu Leu Trp Phe Lys Ala Val Pro Gly Phe Gln Thr Glu Gln Ile
            260                 265                 270
Arg Arg Thr Val Arg Cys Arg Arg Arg Thr Val Leu Gly Leu Val Cys
        275                 280                 285
Val Leu Ser Ala Tyr Val Leu Cys Trp Ala Pro Phe Tyr Gly Phe Thr
    290                 295                 300
Ile Val Arg Asp Phe Phe Pro Ser Val Phe Val Lys Glu Lys His Tyr
305                 310                 315                 320
Leu Thr Ala Phe Tyr Val Val Glu Cys Ile Ala Met Ser Asn Ser Met
                325                 330                 335
Ile Asn Thr Leu Cys Phe Val Thr Val Arg Asn Asn Thr Ser Lys Tyr
            340                 345                 350
Leu Lys Arg Ile Leu Arg Leu Gln Trp Arg Ala Ser Pro Ser Gly Ser
        355                 360                 365
Lys Ala Ser Ala Asp Leu Asp Leu Arg Thr Thr Gly Ile Pro Ala Thr
    370                 375                 380
Glu Glu Val Asp Cys Ile Arg Leu Lys
385                 390         393
```

<210> 54
<211> 381
<212> PRT
<213> Mus spp

<400> 54

```
Met Gly Pro Gln Asn Arg Asn Thr Ser Phe Ala Pro Asp Leu Asn Pro
                5                   10                  15
Pro Gln Asp His Val Ser Leu Asn Tyr Ser Tyr Gly Asp Tyr Asp Leu
            20                  25                  30
```

```
Pro Leu Gly Glu Asp Glu Asp Val Thr Lys Thr Gln Thr Phe Phe Ala
        35                  40                  45
Ala Lys Ile Val Ile Gly Val Ala Leu Ala Gly Ile Met Leu Val Cys
    50                  55                  60
Gly Ile Gly Asn Phe Val Phe Ile Ala Ala Leu Ala Arg Tyr Lys Lys
65                  70                  75                  80
Leu Arg Asn Leu Thr Asn Leu Leu Ile Ala Asn Leu Ala Ile Ser Asp
                85                  90                  95
Phe Leu Val Ala Ile Val Cys Cys Pro Phe Glu Met Asp Tyr Tyr Val
            100                 105                 110
Val Arg Gln Leu Ser Trp Ala His Gly His Val Leu Cys Ala Ser Val
        115                 120                 125
Asn Tyr Leu Arg Thr Val Ser Leu Tyr Val Ser Thr Asn Ala Leu Leu
    130                 135                 140
Ala Ile Ala Ile Asp Arg Tyr Leu Ala Ile Val His Pro Leu Lys Pro
145                 150                 155                 160
Arg Met Asn Tyr Gln Thr Ala Ser Phe Leu Ile Ala Leu Val Trp Met
                165                 170                 175
Val Ser Ile Leu Ile Ala Val Pro Ser Ala Tyr Phe Thr Thr Glu Thr
            180                 185                 190
Ile Leu Val Ile Val Lys Asn Gln Glu Lys Ile Phe Cys Gly Gln Ile
        195                 200                 205
Trp Ser Val Asp Gln Gln Leu Tyr Tyr Lys Ser Tyr Phe Leu Phe Val
    210                 215                 220
Phe Gly Leu Glu Phe Val Gly Pro Val Val Thr Met Thr Leu Cys Tyr
225                 230                 235                 240
Ala Arg Ile Ser Gln Glu Leu Trp Phe Lys Ala Val Pro Gly Phe Gln
                245                 250                 255
Thr Glu Gln Ile Arg Lys Arg Leu Arg Cys Arg Arg Lys Thr Val Leu
            260                 265                 270
Leu Leu Met Gly Ile Leu Thr Ala Tyr Val Leu Cys Trp Ala Pro Phe
        275                 280                 285
Tyr Gly Phe Thr Ile Val Arg Asp Phe Phe Pro Thr Val Val Val Lys
    290                 295                 300
Glu Lys His Tyr Leu Thr Ala Phe Tyr Val Val Glu Cys Ile Ala Met
305                 310                 315                 320
Ser Asn Ser Met Ile Asn Thr Ile Cys Phe Val Thr Val Lys Asn Asn
                325                 330                 335
Thr Met Lys Tyr Phe Lys Lys Met Leu Arg Leu His Trp Arg Pro Ser
            340                 345                 350
His Tyr Gly Ser Lys Ser Ser Ala Asp Leu Asp Leu Lys Thr Ser Gly
        355                 360                 365
Val Pro Ala Thr Glu Glu Val Asp Cys Ile Arg Leu Lys
    370                 375                 380
```

<210> 55
<211> 107
<212> PRT
<213> Mus spp

<400> 55

```
Met Gly Asp Pro Arg Cys Ala Pro Leu Leu Leu Leu Leu Leu Leu Pro
                5                   10                  15
Leu Leu Phe Thr Pro Pro Ala Gly Asp Ala Ala Val Ile Thr Gly Ala
            20                  25                  30
Cys Asp Lys Asp Ser Gln Cys Gly Gly Gly Met Cys Cys Ala Val Ser
        35                  40                  45
Ile Trp Val Lys Ser Ile Arg Ile Cys Thr Pro Met Gly Gln Val Gly
    50                  55                  60
Asp Ser Cys His Pro Leu Thr Arg Lys Val Pro Phe Trp Gly Arg Arg
65                  70                  75                  80
Met His His Thr Cys Pro Cys Leu Pro Gly Leu Ala Cys Leu Arg Thr
                85                  90                  95
Ser Phe Asn Arg Phe Ile Cys Leu Ala Arg Lys
            100                 105
```

<210> 56

<211> 321
<212> DNA
<213> Mus spp

<400> 56

atgggggacc cgcgctgtgc cccgctactg ctacttctgc tgctaccgct gctgttcaca 60
ccgcccgccg ggatgccgc ggtcatcacc ggggcttgcg acaaggactc tcagtgcgga 120
ggaggcatgt gctgtgctgt cagtatctgg gttaagagca taaggatctg cacacctatg 180
ggccaagtgg gcgacagctg ccaccccctg actcggaaag ttccattttg ggggcggagg 240
atgcaccaca cctgcccctg cctgccaggc ttggcgtgtt taaggacttc tttcaaccgg 300
tttatttgct tggcccggaa a 321

<210> 57
<211> 243
<212> DNA
<213> Mus spp

<400> 57

gcggtcatca ccggggcttg cgacaaggac tctcagtgcg gaggaggcat gtgctgtgct 60
gtcagtatct gggttaagag cataaggatc tgcacaccta tgggccaagt gggcgacagc 120
tgccaccccc tgactcggaa agttccattt tgggggcgga ggatgcacca cacctgcccc 180
tgcctgccag gcttggcgtg tttaaggact tctttcaacc ggtttatttg cttggcccgg 240
aaa 243

<210> 58
<211> 21
<212> DNA
<213> Artificial Sequence

<220>
<223> misc feature

<400> 58

tcatgttgct ccactggaag g 21

<210> 59
<211> 21
<212> DNA
<213> Artificial Sequence

<220>
<223> misc feature

<400> 59

ccaattgtct tgaggtccag g 21

<210> 60
<211> 29
<212> DNA
<213> Artificial Sequence

<220>
<223> misc feature

<400> 60

ttcttacaat ggcggtaagt ccagtgcag 29

<210> 61
<211> 31
<212> DNA
<213> Artificial Sequence

<220>

<223> misc feature

<400> 61

gtcgacatgg agaccaccat ggggttcatg g 31

<210> 62
<211> 36
<212> DNA
<213> Artificial Sequence

<220>
<223> misc feature

<400> 62

actagtttat tttagtctga tgcagtccac ctcttc 36

<210> 63
<211> 1179
<212> DNA
<213> Mus spp

<400> 63

```
atggagacca ctgtcggggc tctgggtgag aataccacag acaccttcac cgacttcttt   60
tctgcactcg atggccatga agcccaaacc ggctcgttac cattcacttt cagctacggt  120
gactatgaca tgcccctgga tgaagaggaa gatgtgacca attctcggac tttctttgct  180
gccaagattg tcattggcat ggctttggtg ggtatcatgc tagtgtgtgg catcggcaac  240
ttcatcttta tcactgccct ggcccgctac aaaaagctcc gcaacctcac caacctgctt  300
atcgccaacc tggccatttc agacttcctc gtggccatcg tgtgctgccc ctttgagatg  360
gactactatg tggtgcgcca gctctcctgg gagcatggtc atgtcctgtg cgcctctgtc  420
aactacttgc gtaccgtctc cctctacgtc tccactaacg ccctactggc cattgccatt  480
gacaggtatc tggccattgt gcacccgctg agaccgcgga tgaagtgtca aacagccgcc  540
ggcctgatct tcctggtgtg gtcagtatcc atcctcatcg ccattccagc tgcctacttc  600
accactgaga ccgtgctggt catcgtggag agacaggaga agatcttctg tggtcagatc  660
tggccggtgg atcagcagtt ctactacagg tcctatttcc ttttggtttt cggcctcgag  720
ttcgtgggcc ccgtagtcgc catgaccttg tgctatgcca gggtgtcccg ggagctctgg  780
ttcaaggcgg tgccaggctt ccagacagag cagatccgcc ggacggtgcg ctgccgccgc  840
aggacggtgc tggggctcgt gtgcgtcctc tctgcctatg tgctgtgctg ggctcccttc  900
tatggcttca ctatcgtgcg tgacttcttc ccctccgtgt ttgtgaagga gaagcactac  960
ctcaccgcct tctatgtggt ggagtgcatc gccatgagca acagcatgat caatacgctc 1020
tgctttgtga ctgtcaggaa taacaccagt aagtacctca agaggatcct gcggcttcag 1080
tggagggcct ctcccagcgg gagcaaggcc agcgctgacc tcgacctcag gaccacggga 1140
atacctgcca ccgaggaggt ggactgcatc cgactgaaa                        1179
```

<210> 64
<211> 1143
<212> DNA
<213> Mus spp

<400> 64

```
atgggacccc agaacagaaa cactagcttt gcaccagact tgaatccacc ccaagaccat   60
gtctccttaa actacagtta tggtgattat gacctccccc tgggtgagga tgaggatgtg  120
accaagacac agaccttctt tgcagccaaa attgtcattg gcgtggcact ggcaggcatc  180
atgctggtct gcggcattgg caactttgtc ttcattgctg ccctcgcccg ctacaagaag  240
ctgcgcaacc ttaccaacct cctcattgct aacctggcca tctctgactt cctggtggcg  300
atcgtctgct gcccctttga gatggactat tatgtagtac ggcagctttc ctgggcgcat  360
ggtcacgtgc tttgtgcctc cgtcaactac cttcgtacgg tctccctgta cgtctccacc  420
aacgctctgc tggccatcgc tattgacaga tacctcgcta ttgtccaccc tttgaaacca  480
cggatgaatt atcagaccgc ttccttcctg atcgctttgg tctggatggt ctccatcctc  540
atcgctgtcc catctgccta cttcaccaca gaaaccatcc tcgttatcgt caagaatcaa  600
gaaaaaatct tctgtggtca gatctggtcg gtggaccagc agctctacta caaatcctac  660
ttcctcttcg tcttcgggct tgagttcgtg ggtcccgtgg tcactatgac cctgtgctat  720
gccaggatct cccaagagct ctggttcaag gctgtacctg gcttccagac ggagcaaatc  780
cgcaagcggc tgcgttgccg ccgcaagaca gtgctactgc tcatgggcat cctcacagcc  840
tacgtgctgt gctgggcgcc gttctatggc tttaccatag tgcgagactt cttccccacg  900
```

gtagttgtga aggagaagca ctacctcacc gccttctacg tcgtggagtg cattgccatg 960
agcaacagca tgatcaatac tatatgcttc gtgacggtca agaacaacac catgaaatac 1020
ttcaagaaga tgctgcggct ccactggcgg ccctctcact acgggagtaa gtccagcgct 1080
gacctcgacc tcaaaaccag cggggtgcct gccactgaag aggtggattg tatcagacta 1140
aag 1143

<210> 65
<211> 82
<212> DNA
<213> Artificial Sequence

<220>
<223> Primer

<400> 65

tatggcggtg attaccggtg cgtgcgataa agatagccag tgcggtggcg gtatgtgctg 60
tgcggtgagc atttgggtga aa 82

<210> 66
<211> 82
<212> DNA
<213> Artificial Sequence

<220>
<223> Primer

<400> 66

agcattcgta tttgcacccc gatgggcaaa ctgggcgata gctgccatcc gctgacccgt 60
aaagtgccgt tttttggccg cc 82

<210> 67
<211> 87
<212> DNA
<213> Artificial Sequence

<220>
<223> Primer

<400> 67

gtatgcatca tacctgcccg tgcctgccgg gcctggcgtg cctgcgcacc agctttaacc 60
gctttatttg cctggcgcag aaatagg 87

<210> 68
<211> 88
<212> DNA
<213> Artificial Sequence

<220>
<223> Primer

<400> 68

cgaatgcttt tcacccaaat gctcaccgca cagcacatac cgccaccgca ctggctatct 60
ttatcgcacg caccggtaat caccgcca 88

<210> 69
<211> 85
<212> DNA
<213> Artificial Sequence

<220>
<223> Primer

<400> 69

atgatgcata cggcggccaa aaaacggcac tttacgggtc agcggatggc agctatcgcc 60
cagtttgccc atcggggtgc aaata 85

<210> 70
<211> 80
<212> DNA
<213> Artificial Sequence

<220>
<223> Primer

<400> 70

gatccctatt tctgcgccag gcaaataaag cggttaaagc tggtgcgcag gcacgccagg 60
cccggcaggc acgggcaggt 80

<210> 71
<211> 243
<212> DNA
<213> Artificial Sequence

<220>
<223> misc feature

<400> 71

gcggtgatta ccggtgcgtg cgataaagat agccagtgcg gtggcggtat gtgctgtgcg 60
gtgagcattt gggtgaaaag cattcgtatt tgcaccccga tgggcaaact gggcgatagc 120
tgccatccgc tgacccgtaa agtgccgttt tttggccgcc gtatgcatca tacctgcccg 180
tgcctgccgg gcctggcgtg cctgcgcacc agctttaacc gctttatttg cctggcgcag 240
aaa 243

<210> 72
<211> 16
<212> PRT
<213> Bos spp

<220>
<221> misc feature
<222> (7)..(7)
<223> Xaa stands for any amino acid

<220>
<221> misc feature
<222> (13)..(13)
<223> Xaa stands for any amino acid

<400> 72

Ala Val Ile Thr Gly Ala Xaa Glu Arg Asp Val Gln Xaa Arg Ala Gly
1 5 10 15

<210> 73
<211> 28
<212> DNA
<213> Artificial Sequence

<220>
<223> misc feature

<400> 73

ggtgccacgc gagtctcaat catgctcc 28

<210> 74
<211> 28
<212> DNA
<213> Artificial Sequence

<220>
<223> misc feature

<400> 74

ggggcctgtg agcgggatgt ccagtgtg 28

<210> 75
<211> 28
<212> DNA
<213> Artificial Sequence

<220>
<223> misc feature

<400> 75

cttcttcagg aaacgcaagc accacacc 28

<210> 76
<211> 409
<212> DNA
<213> Homo sapiens

<400> 76

```
cttcttcagg aaacgcaagc accacacctg tccttgcttg cccaacctgc tgtgctccag  60
gttcccggac ggcaggtacc gctgctccat ggacttgaag aacatcaatt tttaggcgct 120
tgcctggtct caggataccc accatccttt tcctgagcac agcctggatt tttatttctg 180
ccatgaaacc cagctcccat gactctccca gtccctacac tgactaccct gatctctctt 240
gtctagtacg cacatatgca cacaggcaga catacctccc atcatgacat ggtccccagg 300
ctggcctgag gatgtcacag cttgaggctg tggtgtgaaa ggtggccagc ctggttctct 360
tccctgctca ggctgccaga gaggtggtaa atggcagaaa ggacattcc             409
```

<210> 77
<211> 20
<212> DNA
<213> Artificial Sequence

<220>
<223> misc feature

<400> 77

ccaccatgag aggtgccacg 20

<210> 78
<211> 24
<212> DNA
<213> Artificial Sequence

<220>
<223> misc feature

<400> 78

ctcgagctca ggaaaaggat ggtg 24

<210> 79
<211> 371
<212> DNA
<213> Homo sapiens

<400> 79

```
ccaccatgag aggtgccacg cgagtctcaa tcatgctcct cctagtaact gtgtctgact  60
gtgctgtgat cacaggggcc tgtgagcggg atgtccagtg tggggcaggc acctgctgtg 120
```

ccatcagcct gtggcttcga gggctgcgga tgtgcacccc gctggggcgg gaaggcgagg 180
agtgccaccc cggcagccac aagatcccct tcttcaggaa acgcaagcac cacacctgtc 240
cttgcttgcc caacctgctg tgctccaggt tcccggacgg caggtaccgc tgctccatgg 300
acttgaagaa catcaatttt taggcgcttg cctggtctca ggatacccac catccttttc 360
ctgagctcga g 371

<210> 80
<211> 371
<212> DNA
<213> Homo sapiens

<400> 80

ccaccatgag aggtgccacg cgagtctcaa tcatgctcct cctagtaact gtgtctgact 60
gtgctgtgat cacaggggcc tgtgagcggg atgtccagtg tggggcaggc acctgctgtg 120
ccatcagcct gtggcttcga gggctgcgga tgtgcacccc gctggggcgg gaaggcgagg 180
agtgccaccc cggcagccac aaggtcccct tcttcaggaa acgcaagcac cacacctgtc 240
cttgcttgcc caacctgctg tgctccaggt tcccggacgg caggtaccgc tgctccatgg 300
acttgaagaa catcaatttt taggcgcttg cctggtctca ggatacccac catccttttc 360
ctgagctcga g 371

<210> 81
<211> 86
<212> PRT
<213> Homo sapiens

<400> 81

Ala Val Ile Thr Gly Ala Cys Glu Arg Asp Val Gln Cys Gly Ala Gly
1               5                   10                  15
Thr Cys Cys Ala Ile Ser Leu Trp Leu Arg Gly Leu Arg Met Cys Thr
            20                  25                  30
Pro Leu Gly Arg Glu Gly Glu Glu Cys His Pro Gly Ser His Lys Ile
        35                  40                  45
Pro Phe Phe Arg Lys Arg Lys His His Thr Cys Pro Cys Leu Pro Asn
    50                  55                  60
Leu Leu Cys Ser Arg Phe Pro Asp Gly Arg Tyr Arg Cys Ser Met Asp
65                  70                  75                  80
Leu Lys Asn Ile Asn Phe
                85

<210> 82
<211> 86
<212> PRT
<213> Homo sapiens

<400> 82

Ala Val Ile Thr Gly Ala Cys Glu Arg Asp Val Gln Cys Gly Ala Gly
                5                   10                  15
Thr Cys Cys Ala Ile Ser Leu Trp Leu Arg Gly Leu Arg Met Cys Thr
            20                  25                  30
Pro Leu Gly Arg Glu Gly Glu Glu Cys His Pro Gly Ser His Lys Val
        35                  40                  45
Pro Phe Phe Arg Lys Arg Lys His His Thr Cys Pro Cys Leu Pro Asn
    50                  55                  60
Leu Leu Cys Ser Arg Phe Pro Asp Gly Arg Tyr Arg Cys Ser Met Asp
65                  70                  75                  80
Leu Lys Asn Ile Asn Phe
                85

<210> 83
<211> 105
<212> PRT
<213> Homo sapiens

<400> 83

Met Arg Gly Ala Thr Arg Val Ser Ile Met Leu Leu Leu Val Thr Val
                5                   10                  15

```
Ser Asp Cys Ala Val Ile Thr Gly Ala Cys Glu Arg Asp Val Gln Cys
            20                  25                  30
Gly Ala Gly Thr Cys Cys Ala Ile Ser Leu Trp Leu Arg Gly Leu Arg
        35                  40                  45
Met Cys Thr Pro Leu Gly Arg Glu Gly Glu Glu Cys His Pro Gly Ser
    50                  55                  60
His Lys Ile Pro Phe Phe Arg Lys Arg Lys His His Thr Cys Pro Cys
65                  70                  75                  80
Leu Pro Asn Leu Leu Cys Ser Arg Phe Pro Asp Gly Arg Tyr Arg Cys
                85                  90                  95
Ser Met Asp Leu Lys Asn Ile Asn Phe
            100                 105
```

<210> 84
<211> 105
<212> PRT
<213> Homo sapiens

<400> 84

```
Met Arg Gly Ala Thr Arg Val Ser Ile Met Leu Leu Leu Val Thr Val
                5                   10                  15
Ser Asp Cys Ala Val Ile Thr Gly Ala Cys Glu Arg Asp Val Gln Cys
            20                  25                  30
Gly Ala Gly Thr Cys Cys Ala Ile Ser Leu Trp Leu Arg Gly Leu Arg
        35                  40                  45
Met Cys Thr Pro Leu Gly Arg Glu Gly Glu Glu Cys His Pro Gly Ser
    50                  55                  60
His Lys Val Pro Phe Phe Arg Lys Arg Lys His His Thr Cys Pro Cys
65                  70                  75                  80
Leu Pro Asn Leu Leu Cys Ser Arg Phe Pro Asp Gly Arg Tyr Arg Cys
                85                  90                  95
Ser Met Asp Leu Lys Asn Ile Asn Phe
            100                 105
```

<210> 85
<211> 678
<212> DNA
<213> Homo sapiens

<400> 85

```
aaggctgagc gggaggaagc gagaggcatc taagcaggca gtgttttgcc ttcaccccaa   60
gtgaccatga gaggtgccac gcgagtctca atcatgctcc tcctagtaac tgtgtctgac  120
tgtgctgtga tcacaggggc ctgtgagcgg gatgtccagt gtggggcagg cacctgctgt  180
gccatcagcc tgtggcttcg agggctgcgg atgtgcaccc cgctggggcg ggaaggcgag  240
gagtgccacc ccggcagcca caagatcccc ttcttcagga aacgcaagca ccacacctgt  300
ccttgcttgc ccaacctgct gtgctccagg ttcccggacg gcaggtaccg ctgctccatg  360
gacttgaaga acatcaattt ttaggcgctt gcctggtctc aggataccca ccatcctttt  420
cctgagcaca gcctggattt ttatttctgc catgaaaccc agctcccatg actctcccag  480
tccctacact gactaccctg atctctcttg tctagtacgc acatatgcac acaggcagac  540
atacctccca tcatgacatg gtccccaggc tggcctgagg atgtcacagc ttgaggctgt  600
ggtgtgaaag gtggccagcc tggttctctt ccctgctcag gctgccagag aggtggtaaa  660
tggcagaaag gacattcc                                                678
```

<210> 86
<211> 678
<212> DNA
<213> Homo sapiens

<400> 86

```
aaggctgagc gggaggaagc gagaggcatc taagcaggca gtgttttgcc ttcaccccaa   60
gtgaccatga gaggtgccac gcgagtctca atcatgctcc tcctagtaac tgtgtctgac  120
tgtgctgtga tcacaggggc ctgtgagcgg gatgtccagt gtggggcagg cacctgctgt  180
gccatcagcc tgtggcttcg agggctgcgg atgtgcaccc cgctggggcg ggaaggcgag  240
gagtgccacc ccggcagcca caaggtcccc ttcttcagga aacgcaagca ccacacctgt  300
ccttgcttgc ccaacctgct gtgctccagg ttcccggacg gcaggtaccg ctgctccatg  360
gacttgaaga acatcaattt ttaggcgctt gcctggtctc aggataccca ccatcctttt  420
cctgagcaca gcctggattt ttatttctgc catgaaaccc agctcccatg actctcccag  480
```

tccctacact gactaccctg atctctcttg tctagtacgc acatatgcac acaggcagac 540
atacctccca tcatgacatg gtccccaggc tggcctgagg atgtcacagc ttgaggctgt 600
ggtgtgaaag gtggccagcc tggttctctt ccctgctcag gctgccagag aggtggtaaa 660
tggcagaaag gacattcc 678

<210> 87
<211> 258
<212> DNA
<213> Homo sapiens

<400> 87

gctgtgatca caggggcctg tgagcgggat gtccagtgtg gggcaggcac ctgctgtgcc 60
atcagcctgt ggcttcgagg gctgcggatg tgcaccccgc tggggcggga aggcgaggag 120
tgccaccccg gcagccacaa gatccccttc ttcaggaaac gcaagcacca cacctgtcct 180
tgcttgccca acctgctgtg ctccaggttc ccggacggca ggtaccgctg ctccatggac 240
ttgaagaaca tcaatttt 258

<210> 88
<211> 258
<212> DNA
<213> Homo sapiens

<400> 88

gctgtgatca caggggcctg tgagcgggat gtccagtgtg gggcaggcac ctgctgtgcc 60
atcagcctgt ggcttcgagg gctgcggatg tgcaccccgc tggggcggga aggcgaggag 120
tgccaccccg gcagccacaa ggtccccttc ttcaggaaac gcaagcacca cacctgtcct 180
tgcttgccca acctgctgtg ctccaggttc ccggacggca ggtaccgctg ctccatggac 240
ttgaagaaca tcaatttt 258

<210> 89
<211> 315
<212> DNA
<213> Homo sapiens

<400> 89

atgagaggtg ccacgcgagt ctcaatcatg ctcctcctag taactgtgtc tgactgtgct 60
gtgatcacag gggcctgtga gcgggatgtc cagtgtgggg caggcacctg ctgtgccatc 120
agcctgtggc ttcgagggct gcggatgtgc accccgctgg ggcgggaagg cgaggagtgc 180
caccccggca gccacaagat ccccttcttc aggaaacgca agcaccacac ctgtccttgc 240
ttgcccaacc tgctgtgctc caggttcccg gacggcaggt accgctgctc catggacttg 300
aagaacatca atttt 315

<210> 90
<211> 315
<212> DNA
<213> Homo sapiens

<400> 90

atgagaggtg ccacgcgagt ctcaatcatg ctcctcctag taactgtgtc tgactgtgct 60
gtgatcacag gggcctgtga gcgggatgtc cagtgtgggg caggcacctg ctgtgccatc 120
agcctgtggc ttcgagggct gcggatgtgc accccgctgg ggcgggaagg cgaggagtgc 180
caccccggca gccacaaggt ccccttcttc aggaaacgca agcaccacac ctgtccttgc 240
ttgcccaacc tgctgtgctc caggttcccg gacggcaggt accgctgctc catggacttg 300
aagaacatca atttt 315

<210> 91
<211> 382
<212> DNA
<213> Homo sapiens

<400> 91

gaattcgccc ttccaccatg agaggtgcca cgcgagtctc aatcatgctc ctcctagtaa 60
ctgtgtctga ctgtgctgtg atcacagggg cctgtgagcg ggatgtccag tgtggggcag 120

gcacctgctg tgccatcagc ctgtggcttc gagggctgcg gatgtgcacc ccgctggggc 180
gggaaggcga ggagtgccac cccggcagcc acaaggtccc cttcttcagg aaacgcaagc 240
accacacctg tccttgcttg cccaacctgc tgtgctccag gttcccggac ggcaggtacc 300
gctgctccat ggacttgaag aacatcaatt tttaggcgct tgcctggtct caggataccc 360
accatccttt cctgagctcg ag 382

<210> 92
<211> 10
<212> PRT
<213> Homo sapiens

<220>
<221> misc feature
<222> (7)..(7)
<223> Xaa stands for any amino acid

<400> 92

Ala Val Ile Thr Gly Ala Xaa Glu Arg Asp
5 10

<210> 93
<211> 26
<212> DNA
<213> Artificial Sequence

<220>
<223> misc feature

<400> 93

gtcgaccacc atgagaggtg ccacgc 26

<210> 94
<211> 26
<212> DNA
<213> Artificial Sequence

<220>
<223> misc feature

<400> 94

actagtcgca gaactggtag gtatgg 26

<210> 95
<211> 88
<212> DNA
<213> Artificial Sequence

<220>
<223> Primer

<400> 95

tatggcggtg attaccggtg cgtgcgaacg tgatgtgcag tgcggtgcgg gtacctgctg 60
cgcgattagc ctgtggctgc gtggtctg 88

<210> 96
<211> 92
<212> DNA
<213> Artificial Sequence

<220>
<223> Primer

<400> 96

cgtatgtgca ccccgctggg tcgtgaaggt gaagaatgcc atccgggtag ccataaagtg 60
ccgttcttcc gtaaacgtaa acatcatacc tg 92

<210> 97
<211> 86
<212> DNA
<213> Artificial Sequence

<220>
<223> Primer

<400> 97

cccgtgcctg ccgaacctgc tgtgcagccg tttcccggat ggtcgttatc gttgcagcat 60
ggatctgaaa aacattaact tttagg 86

<210> 98
<211> 94
<212> DNA
<213> Artificial Sequence

<220>
<223> Primer

<400> 98

cacatacgca gaccacgcag ccacaggcta atcgcgcagc aggtacccgc accgcactgc 60
acatcacgtt cgcacgcacc ggtaatcacc gcca 94

<210> 99
<211> 93
<212> DNA
<213> Artificial Sequence

<220>
<223> Primer

<400> 99

aggcacgggc aggtatgatg tttacgttta cggaagaacg gcactttatg gctacccgga 60
tggcattctt caccttcacg acccagcggg gtg 93

<210> 100
<211> 81
<212> DNA
<213> Artificial Sequence

<220>
<223> Primer

<400> 100

gatccctaaa agttaatgtt tttcagatcc atgctgcaac gataacgacc atccgggaaa 60
cggctgcaca gcaggttcgg c 81

<210> 101
<211> 258
<212> DNA
<213> Artificial Sequence

<220>
<223> misc feature

<400> 101

gcggtgatta ccggtgcgtg cgaacgtgat gtgcagtgcg gtgcgggtac ctgctgcgcg 60
attagcctgt ggctgcgtgg tctgcgtatg tgcaccccgc tgggtcgtga aggtgaagaa 120
tgccatccgg gtagccataa agtgccgttc ttccgtaaac gtaaacatca tacctgcccg 180

tgcctgccga acctgctgtg cagccgtttc ccggatggtc gttatcgttg cagcatggat 240
ctgaaaaaca ttaacttt 258

<210> 102
<211> 23
<212> DNA
<213> Artificial Sequence

<220>
<223> misc feature

<400> 102

cttggccttc tcggcttgtc tag 23

<210> 103
<211> 25
<212> DNA
<213> Artificial Sequence

<220>
<223> misc feature

<400> 103

ggtgtaaagc aagaggtcac ccagt 25

<210> 104
<211> 22
<212> DNA
<213> Artificial Sequence

<220>
<223> misc feature

<400> 104

acaaggcagc gcagaaggaa gt 22

<210> 105
<211> 24
<212> DNA
<213> Artificial Sequence

<220>
<223> misc feature

<400> 105

ggctccagta cagagtccag acaa 24

<210> 106
<211> 410
<212> DNA
<213> Mus spp

<400> 106

acaaggcagc gcagaaggaa gtgaggggta ccaaagtaga ctgtgtttgt cgtcacctca 60
agtgatcatg agaggcgctg tgcatatctt catcatgctc cttctagcaa cggcgtccga 120
ctgtgcggtc atcacagggg cctgtgaacg agatatccag tgtggggccg gcacctgctg 180
cgctatcagt ctgtggctgc ggggcctgcg gttgtgtacc ccactggggc gtgaaggaga 240
ggagtgccac ccaggaagcc acaagatccc cttcttgagg aaacgccaac accatacctg 300
tccctgctca cccagcctgc tgtgctccag gttcccggac ggcaggtacc gctgcttccg 360
ggacttgaag aatgccaact tttagtttgt ctggactctg tactggagcc 410

<210> 107
<211> 105

<212> PRT
<213> Mus spp

<400> 107

```
Met Arg Gly Ala Val His Ile Phe Ile Met Leu Leu Leu Ala Thr Ala
 1               5                  10                  15
Ser Asp Cys Ala Val Ile Thr Gly Ala Cys Glu Arg Asp Ile Gln Cys
            20                  25                  30
Gly Ala Gly Thr Cys Cys Ala Ile Ser Leu Trp Leu Arg Gly Leu Arg
        35                  40                  45
Leu Cys Thr Pro Leu Gly Arg Glu Gly Glu Glu Cys His Pro Gly Ser
    50                  55                  60
His Lys Ile Pro Phe Leu Arg Lys Arg Gln His His Thr Cys Pro Cys
65                  70                  75                  80
Ser Pro Ser Leu Leu Cys Ser Arg Phe Pro Asp Gly Arg Tyr Arg Cys
                85                  90                  95
Phe Arg Asp Leu Lys Asn Ala Asn Phe
            100                 105
```

<210> 108
<211> 315
<212> DNA
<213> Mus spp

<400> 108

```
atgagaggcg ctgtgcatat cttcatcatg ctccttctag caacggcgtc cgactgtgcg  60
gtcatcacag ggcctgtga acgagatatc cagtgtgggg ccggcacctg ctgcgctatc 120
agtctgtggc tgcggggcct gcggttgtgt accccactgg ggcgtgaagg agaggagtgc 180
cacccaggaa gccacaagat ccccttcttg aggaaacgcc aacaccatac ctgtccctgc 240
tcacccagcc tgctgtgctc caggttcccg gacggcaggt accgctgctt ccgggacttg 300
aagaatgcca acttt                                                  315
```

<210> 109
<211> 86
<212> PRT
<213> Mus spp

<400> 109

```
Ala Val Ile Thr Gly Ala Cys Glu Arg Asp Ile Gln Cys Gly Ala Gly
                5                   10                  15
Thr Cys Cys Ala Ile Ser Leu Trp Leu Arg Gly Leu Arg Leu Cys Thr
            20                  25                  30
Pro Leu Gly Arg Glu Gly Glu Glu Cys His Pro Gly Ser His Lys Ile
        35                  40                  45
Pro Phe Leu Arg Lys Arg Gln His His Thr Cys Pro Cys Ser Pro Ser
    50                  55                  60
Leu Leu Cys Ser Arg Phe Pro Asp Gly Arg Tyr Arg Cys Phe Arg Asp
65                  70                  75                  80
Leu Lys Asn Ala Asn Phe
                85
```

<210> 110
<211> 258
<212> DNA
<213> Mus spp

<400> 110

```
gcggtcatca caggggcctg tgaacgagat atccagtgtg ggccggcac ctgctgcgct  60
atcagtctgt ggctgcgggg cctgcggttg tgtaccccac tggggcgtga aggagaggag 120
tgccacccag gaagccacaa gatccccttc ttgaggaaac gccaacacca tacctgtccc 180
tgctcaccca gcctgctgtg ctccaggttc ccggacggca ggtaccgctg cttccgggac 240
ttgaagaatg ccaacttt                                               258
```

<210> 111

<211> 364
<212> DNA
<213> Mus spp

<400> 111

```
gaggggtacc aaagtagact gtgtttgtcg tcacctcaag tgatcatgag aggcgctgtg  60
catatcttca tcatgctcct tctagcaacg gcgtccgact gtgcggtcat cacaggggcc 120
tgtgaacgag atatccagtg tggggccggc acctgctgcg ctatcagtct gtggctgcgg 180
ggcctgcggt tgtgtacccc actggggcgt gaaggagagg agtgccaccc aggaagccac 240
aagatcccct tcttgaggaa acgccaacac catacctgtc cctgctcacc cagcctgctg 300
tgctccaggt tcccggacgg caggtaccgc tgcttccggg acttgaagaa tgccaacttt 360
tagt                                                              364
```

<210> 112
<211> 23
<212> DNA
<213> Artificial Sequence

<220>
<223> misc feature

<400> 112

tcaccycaag tgaycatgag agg 23

<210> 113
<211> 25
<212> DNA
<213> Artificial Sequence

<220>
<223> misc feature

<400> 113

ctaaaarttg ryrttcttca agtcc 25

<210> 114
<211> 20
<212> DNA
<213> Artificial Sequence

<220>
<223> misc feature

<400> 114

atcacagggg cctgtgarcg 20

<210> 115
<211> 20
<212> DNA
<213> Artificial Sequence

<220>
<223> misc feature

<400> 115

agcagcggta cctgccgtcc 20

<210> 116
<211> 186
<212> DNA
<213> Rattus spp

<400> 116

```
agatgtccag tgtggggctg gcacctgctg tgctatcagc ctgtggctgc ggggcctgag  60
gctgtgtacc cctctggggc gggaaggaga ggagtgccac cctggaagcc acaagatccc 120
tttctttagg aaacgccaac accatacctg tccctgttca cccagcctgc tgtgctccag 180
gttccc                                                            186
```

<210> 117
<211> 186
<212> DNA
<213> Rattus spp

<400> 117

```
agatgtccag tgtggggctg gcacctgctg tgctatcagc ctgtggctgc ggggcctgag  60
gctgtgcacc cctctggggc gggaaggaga ggagtgccac cctggaagcc acaagatccc 120
tttctttagg aaacgccaac accatacctg tccctgttca cccagcctgc tgtgctccag 180
gttccc                                                            186
```

<210> 118
<211> 28
<212> DNA
<213> Artificial Sequence

<220>
<223> misc feature

<400> 118

```
gtggcactcc tctccttccc gccccaga          28
```

<210> 119
<211> 28
<212> DNA
<213> Artificial Sequence

<220>
<223> misc feature

<400> 119

```
caggccccgc agccacaggc tgatagca          28
```

<210> 120
<211> 28
<212> DNA
<213> Artificial Sequence

<220>
<223> misc feature

<400> 120

```
agcaggtgcc agccccacac tggacatc          28
```

<210> 121
<211> 244
<212> DNA
<213> Rattus spp

<400> 121

```
agagagatga ggcatttaga ggcagccctg gatccgacta tataaatctg aaggaggtaa  60
ggtaggacag cttggccttc ttagcttgtc tagtgcaagg cagtgcagaa ggaagtgagg 120
gattccagag tggacagtgt ttgccttcac cccaagtgat catgagaggt gctgtgcaag 180
tcttcatcat gctccttcta gcaactgtct ctgactgtgc ggtgatcaca ggggcctgtg 240
aacg                                                              244
```

<210> 122

<211> 27
<212> DNA
<213> Artificial Sequence

<220>
<223> misc feature

<400> 122

ggaaggagag gagtgccacc ctggaag 27

<210> 123
<211> 28
<212> DNA
<213> Artificial Sequence

<220>
<223> misc feature

<400> 123

accatacctg tccctgttca cccagcct 28

<210> 124
<211> 464
<212> DNA
<213> Rattus spp

<400> 124

ctgttcaccc agcctgctgt gctccaggtt cccagatggc aggtaccgct gctcccagga 60
cttgaagaat gtcaactttt agtttatctg gactctgtct gggtccctac tgggtgacct 120
cttgtgttac atctgtgtga cttagttccg tgcaacttct ccactcccca ccctgtccgt 180
gtgtgtgcag acaagcatat cttccactac ggaacagtcc agcagcgtgc agagaggagt 240
ttgcagcctt gagaagtggg ccagcctggc cttcctggcc agaccgcctg aagttgtgac 300
actgggacct cctcaattgt ctgcccttcc tgcatgtgcc cttctcccta aaccacacct 360
cccaggccct ggcctgtggg tgcgtcacta agtcacgggg tctatggggg gaagatcaac 420
attctcctca ttttctttca ttggctagct ccttgtttta ggag 464

<210> 125
<211> 30
<212> DNA
<213> Artificial Sequence

<220>
<223> misc feature

<400> 125

attccagagt ggacagtgtt tgccttcacc 30

<210> 126
<211> 28
<212> DNA
<213> Artificial Sequence

<220>
<223> misc feature

<400> 126

gatcatgaga ggtgctgtgc aagtcttc 28

<210> 127
<211> 26
<212> DNA
<213> Artificial Sequence

<220>
<223> misc feature

<400> 127

ctctctgcac gctgctggac tgttcc 26

<210> 128
<211> 30
<212> DNA
<213> Artificial Sequence

<220>
<223> misc feature

<400> 128

cagatgtaac acaagaggtc acccagtagg 30

<210> 129
<211> 375
<212> DNA
<213> Rattus spp

<400> 129

gatcatgaga ggtgctgtgc aagtcttcat catgctcctt ctagcaactg tctctgactg 60
tgcggtgatc acaggggcct gtgaacgaga tgtccagtgt ggggctggca cctgctgtgc 120
tatcagcctg tggctgcggg gcctgaggct gtgtacccct ctggggcggg aaggagagga 180
gtgccacccct ggaagccaca agatcccttt ctttaggaaa cgccaacacc atacctgtcc 240
ctgttcaccc agcctgctgt gctccaggtt cccagatggc aggtaccgct gctcccagga 300
cttgaagaat gtcaactttt agtttatctg gactctgtct gggtccctac tgggtgacct 360
cttgtgttac atctg 375

<210> 130
<211> 375
<212> DNA
<213> Rattus spp

<400> 130

gatcatgaga ggtgctgtgc aagtcttcat catgctcctt ctagcaactg tctctgactg 60
tgcggtgatc acaggggcct gtgaacgaga tgtccagtgt ggggctggca cctgctgtgc 120
tatcagcctg tggctgcggg gcctgaggct gtgtacccct ctggggcggg aaggagagga 180
gtgccacccct ggaagctaca agatcccttt ctttaggaaa cgccaacacc atacctgtcc 240
ctgttcaccc agcctgctgt gctccaggtt cccagatggc aggtaccgct gctcccagga 300
cttgaagaat gtcaactttt agtttatctg gactctgtct gggtccctac tgggtgacct 360
cttgtgttac atctg 375

<210> 131
<211> 375
<212> DNA
<213> Rattus spp

<400> 131

gatcatgaga ggtgctgtgc aagtcttcat catgctcctt ctagcaactg tctctgactg 60
tgcggtgatc acaggggcct gtgaacgaga tgtccagtgt ggggctggca cctgctgtgc 120
tatcagcctg tggctgcggg gcctgaggct gtgtacccct ctggggcagg aaggagagga 180
gtgccacccct ggaagccaca agatcccttt ctttaggaaa cgccaacacc atacctgtcc 240
ctgttcaccc agcctgctgt gctccaggtt cccagatggc aggtaccgct gctcccagga 300
cttgaagaat gtcaactttt agtttatctg gactctgtct gggtccctac tgggtgacct 360
cttgtgttac atctg 375

<210> 132
<211> 105
<212> PRT
<213> Rattus spp

<400> 132

```
Met Arg Gly Ala Val Gln Val Phe Ile Met Leu Leu Leu Ala Thr Val
                5                   10                  15
Ser Asp Cys Ala Val Ile Thr Gly Ala Cys Glu Arg Asp Val Gln Cys
            20                  25                  30
Gly Ala Gly Thr Cys Cys Ala Ile Ser Leu Trp Leu Arg Gly Leu Arg
        35                  40                  45
Leu Cys Thr Pro Leu Gly Arg Glu Gly Glu Glu Cys His Pro Gly Ser
    50                  55                  60
His Lys Ile Pro Phe Phe Arg Lys Arg Gln His His Thr Cys Pro Cys
65                  70                  75                  80
Ser Pro Ser Leu Leu Cys Ser Arg Phe Pro Asp Gly Arg Tyr Arg Cys
                85                  90                  95
Ser Gln Asp Leu Lys Asn Val Asn Phe
            100                 105
```

<210> 133
<211> 315
<212> DNA
<213> Rattus spp

<400> 133

```
atgagaggtg ctgtgcaagt cttcatcatg ctccttctag caactgtctc tgactgtgcg  60
gtgatcacag ggcctgtga acgagatgtc cagtgtgggg ctggcacctg ctgtgctatc 120
agcctgtggc tgcggggcct gaggctgtgt acccctctgg ggcgggaagg agaggagtgc 180
caccctggaa gccacaagat ccctttcttt aggaaacgcc aacaccatac ctgtccctgt 240
tcacccagcc tgctgtgctc caggttccca gatggcaggt accgctgctc ccaggacttg 300
aagaatgtca acttt                                                   315
```

<210> 134
<211> 105
<212> PRT
<213> Rattus spp

<400> 134

```
Met Arg Gly Ala Val Gln Val Phe Ile Met Leu Leu Leu Ala Thr Val
                5                   10                  15
Ser Asp Cys Ala Val Ile Thr Gly Ala Cys Glu Arg Asp Val Gln Cys
            20                  25                  30
Gly Ala Gly Thr Cys Cys Ala Ile Ser Leu Trp Leu Arg Gly Leu Arg
        35                  40                  45
Leu Cys Thr Pro Leu Gly Arg Glu Gly Glu Glu Cys His Pro Gly Ser
    50                  55                  60
Tyr Lys Ile Pro Phe Phe Arg Lys Arg Gln His His Thr Cys Pro Cys
65                  70                  75                  80
Ser Pro Ser Leu Leu Cys Ser Arg Phe Pro Asp Gly Arg Tyr Arg Cys
                85                  90                  95
Ser Gln Asp Leu Lys Asn Val Asn Phe
            100                 105
```

<210> 135
<211> 315
<212> DNA
<213> Rattus spp

<400> 135

```
atgagaggtg ctgtgcaagt cttcatcatg ctccttctag caactgtctc tgactgtgcg  60
gtgatcacag ggcctgtga acgagatgtc cagtgtgggg ctggcacctg ctgtgctatc 120
agcctgtggc tgcggggcct gaggctgtgt acccctctgg ggcgggaagg agaggagtgc 180
caccctggaa gctacaagat ccctttcttt aggaaacgcc aacaccatac ctgtccctgt 240
tcacccagcc tgctgtgctc caggttccca gatggcaggt accgctgctc ccaggacttg 300
aagaatgtca acttt                                                   315
```

<210> 136
<211> 105
<212> PRT
<213> Rattus spp

<400> 136

```
Met Arg Gly Ala Val Gln Val Phe Ile Met Leu Leu Leu Ala Thr Val
                5                   10                  15
Ser Asp Cys Ala Val Ile Thr Gly Ala Cys Glu Arg Asp Val Gln Cys
            20                  25                  30
Gly Ala Gly Thr Cys Cys Ala Ile Ser Leu Trp Leu Arg Gly Leu Arg
        35                  40                  45
Leu Cys Thr Pro Leu Gly Gln Glu Gly Glu Glu Cys His Pro Gly Ser
    50                  55                  60
His Lys Ile Pro Phe Phe Arg Lys Arg Gln His His Thr Cys Pro Cys
65                  70                  75                  80
Ser Pro Ser Leu Leu Cys Ser Arg Phe Pro Asp Gly Arg Tyr Arg Cys
                85                  90                  95
Ser Gln Asp Leu Lys Asn Val Asn Phe
            100                 105
```

<210> 137
<211> 315
<212> DNA
<213> Rattus spp

<400> 137

```
atgagaggtg ctgtgcaagt cttcatcatg ctccttctag caactgtctc tgactgtgcg  60
gtgatcacag ggcctgtga acgagatgtc cagtgtgggg ctggcacctg ctgtgctatc 120
agcctgtggc tgcggggcct gaggctgtgt acccctctgg ggcaggaagg agaggagtgc 180
caccctggaa gccacaagat ccctttcttt aggaaacgcc aacaccatac ctgtccctgt 240
tcacccagcc tgctgtgctc caggttccca gatggcaggt accgctgctc ccaggacttg 300
aagaatgtca acttt                                                  315
```

<210> 138
<211> 86
<212> PRT
<213> Rattus spp

<400> 138

```
Ala Val Ile Thr Gly Ala Cys Glu Arg Asp Val Gln Cys Gly Ala Gly
 1              5                   10                  15
Thr Cys Cys Ala Ile Ser Leu Trp Leu Arg Gly Leu Arg Leu Cys Thr
            20                  25                  30
Pro Leu Gly Arg Glu Gly Glu Glu Cys His Pro Gly Ser His Lys Ile
        35                  40                  45
Pro Phe Phe Arg Lys Arg Gln His His Thr Cys Pro Cys Ser Pro Ser
    50                  55                  60
Leu Leu Cys Ser Arg Phe Pro Asp Gly Arg Tyr Arg Cys Ser Gln Asp
65                  70                  75                  80
Leu Lys Asn Val Asn Phe
                85
```

<210> 139
<211> 258
<212> DNA
<213> Rattus spp

<400> 139

```
gcggtgatca caggggcctg tgaacgagat gtccagtgtg gggctggcac ctgctgtgct  60
atcagcctgt ggctgcgggg cctgaggctg tgtacccctc tggggcggga aggagaggag 120
tgccaccctg gaagccacaa gatccctttc tttaggaaac gccaacacca tacctgtccc 180
tgttcaccca gcctgctgtg ctccaggttc ccagatggca ggtaccgctg ctcccaggac 240
ttgaagaatg tcaacttt                                               258
```

<210> 140
<211> 86
<212> PRT
<213> Rattus spp

<400> 140

```
Ala Val Ile Thr Gly Ala Cys Glu Arg Asp Val Gln Cys Gly Ala Gly
 1               5                  10                  15
Thr Cys Cys Ala Ile Ser Leu Trp Leu Arg Gly Leu Arg Leu Cys Thr
            20                  25                  30
Pro Leu Gly Arg Glu Gly Glu Glu Cys His Pro Gly Ser Tyr Lys Ile
        35                  40                  45
Pro Phe Phe Arg Lys Arg Gln His His Thr Cys Pro Cys Ser Pro Ser
    50                  55                  60
Leu Leu Cys Ser Arg Phe Pro Asp Gly Arg Tyr Arg Cys Ser Gln Asp
65                  70                  75                  80
Leu Lys Asn Val Asn Phe
                85
```

<210> 141
<211> 258
<212> DNA
<213> Rattus spp

<400> 141

```
gcggtgatca caggggcctg tgaacgagat gtccagtgtg ggctggcac ctgctgtgct  60
atcagcctgt ggctgcgggg cctgaggctg tgtacccctc tggggcggga aggagaggag 120
tgccaccctg gaagctacaa gatccctttc tttaggaaac gccaacacca tacctgtccc 180
tgttcaccca gcctgctgtg ctccaggttc ccagatggca ggtaccgctg ctcccaggac 240
ttgaagaatg tcaacttt                                               258
```

<210> 142
<211> 86
<212> PRT
<213> Rattus spp

<400> 142

```
Ala Val Ile Thr Gly Ala Cys Glu Arg Asp Val Gln Cys Gly Ala Gly
1               5                   10                  15
Thr Cys Cys Ala Ile Ser Leu Trp Leu Arg Gly Leu Arg Leu Cys Thr
            20                  25                  30
Pro Leu Gly Gln Glu Gly Glu Glu Cys His Pro Gly Ser His Lys Ile
        35                  40                  45
Pro Phe Phe Arg Lys Arg Gln His His Thr Cys Pro Cys Ser Pro Ser
    50                  55                  60
Leu Leu Cys Ser Arg Phe Pro Asp Gly Arg Tyr Arg Cys Ser Gln Asp
65                  70                  75                  80
Leu Lys Asn Val Asn Phe
                85
```

<210> 143
<211> 258
<212> DNA
<213> Rattus spp

<400> 143

```
gcggtgatca caggggcctg tgaacgagat gtccagtgtg ggctggcac ctgctgtgct  60
atcagcctgt ggctgcgggg cctgaggctg tgtacccctc tggggcagga aggagaggag 120
tgccaccctg gaagccacaa gatccctttc tttaggaaac gccaacacca tacctgtccc 180
tgttcaccca gcctgctgtg ctccaggttc ccagatggca ggtaccgctg ctcccaggac 240
ttgaagaatg tcaacttt                                               258
```

<210> 144

<211> 24
<212> DNA
<213> Artificial Sequence

<220>
<223> Primer

<400> 144

atygtstgct gccccttyga gatg 24

<210> 145
<211> 25
<212> DNA
<213> Artificial Sequence

<220>
<223> Primer

<400> 145

tagtagagct gctgrtccac bgrcc 25

<210> 146
<211> 301
<212> DNA
<213> Bos spp

<400> 146

gactactacg tggtgcgcca gctctcctgg gagcacggcc acgtgctgtg cgcctctgtc 60
aactacctgc gcacggtctc tctctacgtc tccaccaacg ccctgctggc catcgccatc 120
gacagatatc tggccattgy ccacccgctg agaccccgga tgaagtacca aacagccacg 180
ggcttgattg ccttggtgtg ggtggtgtcc attcttgttg ccataccgtc agcctacttc 240
accactgaaa cggtcctcgt cattgtcaag agccaggaga agattttctg tggccagatc 300
t 301

<210> 147
<211> 301
<212> DNA
<213> Bos spp

<400> 147

gactactatg tggtgcatca gctctcctgg gagcatggcc atgtgctctg tgcctgtatc 60
aactacctgc gtaccgtctc actctacgtc tccaccaatg ccctgctggc cattgctatt 120
gacagatatc tcgctatcgt tcaccccttg aaaccacgaa tgaattatca aacagcctcc 180
ttcctgatcg ctctggtctg gatggtatcc attctcatct ccatcccatc agcctacttc 240
acaaaggaaa ccgtcctctt cattgtcaaa aaccagaaaa agatcttctg cggccaggtc 300
t 301

<210> 148
<211> 23
<212> DNA
<213> Artificial Sequence

<220>
<223> Primer

<400> 148

ggccagcagg gcgttggtgg aga 23

<210> 149
<211> 28
<212> DNA
<213> Artificial Sequence

<220>
<223> Primer

<400> 149

gtgcgcaggt agttgacaga ggcgcaca 28

<210> 150
<211> 767
<212> DNA
<213> Bos spp

<400> 150

ctaacagcgg ggtcaaaggg ctgaaatgaa gctgaccccc gcgtccagtc gggcttgccc 60
gggggagcga cgtttgagtc gctcccagtg tgtcctgcga gggcggtgtg ggtagaaggg 120
aaggctcctc ccggttcttt gaaatctcgc cggctcatca gcccgaccgc ccactctggc 180
ggtggctcca gggcagacca ggcttaggat gaagcttgga ttctggacgc catggatgtt 240
tgcagggaca cactgactca tgaaggaaaa gctgagtttc agcagaaaag ggagacctca 300
gcctagcaac atcaccccag aggaattccc aagagtgtgg ctcaaggctt caaccagcca 360
gatggagatc accatggggg tcatggatga gaatgccacc aatacgtcaa ccaactattt 420
ccctctgctt gaccccctcg gagcccaagc tgcttctttc cccttcaact tcagctacgg 480
tgactatgat atgcccttgg atgaagatga ggatatgacc aattcccgga ccttctttgc 540
cgccaagatt gtcattggca tggcgctggt gggtattatg ctggtctgtg gtatcggcaa 600
cttcatcttc atcgctgccc tggcccgcta caagaagcta cgcaacctca ccaatctgct 660
catcgccaac ctggccatct ctgatttcct ggtggccatt gtctgctgcc ccttcgagat 720
ggactactac gtggtgcgcc agctctcctg ggagcacggc cacgtgc 767

<210> 151
<211> 720
<212> DNA
<213> Bos spp

<400> 151

gagagcaact cctgcagagc ccagccagga gaaagccccg agcggggacc gtgctcttcc 60
tgcgggcttc tgggagcagt ggctcgtgcc ctccgacccg ggctaaagag cccaggacct 120
cgtctgcctg gctggggact ccgggatgaa tcaggcttag gatgaagctt ggattctgga 180
cgccatggat gtttgcaggg acacactgac tcatgaagga aaagctgagt ttcagcagaa 240
aagggagacc tcagcctagc aacatcaccc cagaggaatt cccaagagtg tggctcaagg 300
cttcaaccag ccagatggag atcaccatgg gggtcatgga tgagaatgcc accaatacgt 360
caaccaacta tttccctctg cttgaccccc tcggagccca agctgcttct ttccccttca 420
acttcagcta cggtgactat gatatgccct tggatgaaga tgaggatatg accaattccc 480
ggaccttctt tgccgccaag attgtcattg gcatggcgct ggtgggtatt atgctggtct 540
gtggtatcgg caacttcatc ttcatcgctg ccctggcccg ctacaagaag ctacgcaacc 600
tcaccaatct gctcatcgcc aacctggcca tctctgattt cctggtggcc attgtctgct 660
gccccttcga gatggactac tacgtggtgc gccagctctc ctgggagcac ggccacgtgc 720

<210> 152
<211> 26
<212> DNA
<213> Artificial Sequence

<220>
<223> Primer

<400> 152

tcatcagccc gaccgcccac tctggc 26

<210> 153
<211> 28
<212> DNA
<213> Artificial Sequence

<220>
<223> Primer

<400> 153

taggatgaag cttggattct ggacgcca 28

<210> 154
<211> 2051
<212> DNA
<213> Bos spp

<400> 154

```
tggatgtttg cagggacaca ctgactcatg aaggaaaagc tgagtttcag cagaaaaggg    60
agacctcagc ctagcaacat caccccagag gaattcccaa gagtgtggct caaggcttca   120
accagccaga tggagatcac catgggggtc atggatgaga atgccaccaa tacgtcaacc   180
aactatttcc ctctgcttga ccccctcgga gcccaagctg cttctttccc cttcaacttc   240
agctacggtg actatgatat gcccttggat gaagatgagg atatgaccaa ttcccggacc   300
ttctttgccg ccaagattgt cattggcatg gcgctggtgg gtattatgct ggtctgtggt   360
atcggcaact tcatcttcat cgctgccctg gcccgctaca agaagctacg caacctcacc   420
aatctgctca tcgccaacct ggccatctct gatttcctgg tggccattgt ctgctgcccc   480
ttcgagatgg actactacgt ggtgcgccag ctctcctggg agcacggcca cgtgctgtgc   540
gcctctgtca actacctgcg cacggtctct ctctacgtct ccaccaacgc cctgctggcc   600
atcgccatcg acagatatct ggccattgtc cacccgctga gaccccggat gaagtaccaa   660
acagccacgg gcttgattgc cttggtgtgg gtggtgtcca ttcttgttgc cataccgtca   720
gcctacttca ccactgaaac ggtcctcgtc attgtcaaga gccaggagaa gattttctgt   780
ggccagatct ggccagtaga ccagcagatc tactacaagt cctacttcct cttcatcttt   840
ggcatcgagt tcgtgggccc agtggtcacc atgaccctat gctatgccag gatctcccga   900
gagctctggt tcaaggccgt ccctggtttc cagacggagc agatccggaa gcgactgcgc   960
tgtcgccgga agaccgtcct ggtgctcatg tgcatcctca cggcgtacgt gctgtgctgg  1020
gcgcccttct acggcttcgc catcgtgcgg gacttcttcc ccacagtgtt tgtgaaggag  1080
aaacactatc tcacggcctt ctacgtggtc gagtgcattg ccatgagcaa cagcatgatc  1140
aacaccgtgt gcttcgtgac tgtcaagaat aacaccatca agtacttcaa gaagattatg  1200
ctgctccact ggaaggcttc ttacaatggc agcaagtcca gtggggacct tgacctcaaa  1260
accacggggg tgcctgccac tgaagaggtg gactgcatcg ggctgaaata actccagaac  1320
aggacctttg ggcccctgac aatgtgctta agcccacact atcaaccaag aactctaggt  1380
ttgccacata gggcaaaaga aatggacaaa tatgtcagtg ctgtgttcag ggagctcaga  1440
atctctaaaa ctggtgtgac cacaccatca ccagtggccg acattcaatg aacatctaat  1500
ttgtgcaaag tgtaggcact ggtgatatct gtatctgttg atgatatttg attggcagaa  1560
agaaataggg attaaacaaa cacaaaaaaa tgatgggaca tagtgtggaa tgatgggttt  1620
tccagaaggg agcctgagta gtcacctaaa actgtggtct atatgtttgt agggtgaagt  1680
tctctctgga tcatctcagc tactcatcag acactgagat aacctgtctc tgggattgtt  1740
cttaggcttg agttcactcc taacacttgc tggcctgggg caagaataca aagcaggttc  1800
ctggctcttt ctgcaatctt cccttctatc cctggttctg actctcaccc tgggcaccca  1860
ggaaagaaag aaacatacaa ataggcggtg gggggtgggg gttggcacca gtgagattca  1920
gagactacag gcttattgag ggcctttta atccacctcc tttgtttggt tttttgaatt  1980
tggaaatttg gaaccagaag atacgaccca gccttggatc tatcacctgt atgggcctgg  2040
gtaaggcctc t                                                       2051
```

<210> 155
<211> 24
<212> DNA
<213> Artificial Sequence

<220>
<223> Primer

<400> 155
tttgagtcgc tcccagtgtg tcct 24

<210> 156
<211> 23
<212> DNA
<213> Artificial Sequence

<220>
<223> Primer

<400> 156

gctcctcccg gttctttgaa atc 23

<210> 157
<211> 26
<212> DNA
<213> Artificial Sequence

<220>
<223> Primer

<400> 157

tgtagtctct gaatctcact ggtgcc 26

<210> 158
<211> 28
<212> DNA
<213> Artificial Sequence

<220>
<223> Primer

<400> 158

ggtggattaa aaaggccctc aataagcc 28

<210> 159
<211> 2038
<212> DNA
<213> Bos spp

<400> 159

gctcctcccg gttctttgaa atctcgccgg ctcatcagcc cgaccgccca ctctggcggt 60
ggctccaggg cagaccaggc ttaggatgaa gcttggattc tggacgccat ggatgtttgc 120
agggacacac tgactcatga aggaaaagct gagtttcagc agaaaaggga gacctcagcc 180
tagcaacatc accccagagg aattcccaag agtgtggctc aaggcttcaa ccagccagat 240
ggagatcacc atgggggtca tggatgagaa tgccaccaat acgtcaacca actatttccc 300
tctgcttgac cccctcggag cccaagctgc ttctttcccc ttcaacttca gctacggtga 360
ctatgatatg cccttggatg aagatgagga tatgaccaat tcccggacct tctttgccgc 420
caagattgtc attggcatgg cgctggtggg tattatgctg gtctgtggta tcggcaactt 480
catcttcatc gctgccctgg cccgctacaa gaagctacgc aacctcacca atctgctcat 540
cgccaacctg gccatctctg atttcctggt ggccattgtc tgctgcccct tcgagatgga 600
ctactacgtg gtgcgccagc tctcctggga gcacggccac gtgctgtgcg cctctgtcaa 660
ctacctgcgc acggtctctc tctacgtctc caccaacgcc ctgctggcca tcgccatcga 720
cagatatctg gccattgtcc acccgctgag accccggatg aagtaccaaa cagccacggg 780
cttgattgcc ttggtgtggg tggtgtccat tcttgttgcc ataccgtcag cctacttcac 840
cactgaaacg gtcctcgtca ttgtcaagag ccaggagaag attttctgtg gccagatctg 900
gccagtagac cagcagatct actacaagtc ctacttcctc ttcatctttg gcatcgagtt 960
cgtgggccca gtggtcacca tgaccctatg ctatgccagg atctcccgag agctctggtt 1020
caaggccgtc cctggtttcc agacggagca gatccggaag cgactgcgct gtcgccggaa 1080
gaccgtcctg gtgctcatgt gcatcctcac ggcgtacgtg ctgtgctggg cgcccttcta 1140
cggcttcgcc atcgtgcggg acttcttccc cacagtgttt gtgaaggaga aacactatct 1200
cacggccttc tacgtggtcg agtgcattgc catgagcaac agcatgatca acaccgtgtg 1260
cttcgtgact gtcaagaata acaccatcaa gtacttcaag aagattatgc tgctccactg 1320
gaaggcttct tacaatggca gcaagtccag tggggacctt gacctcaaaa ccacgggggt 1380
gcctgccact gaagaggtgg actgcatcgg gctgaaataa ctccagaaca ggacctttgg 1440
gcccctgaca atgtgcttaa gcccacacta tcaaccaaga actctaggtt tgccacatag 1500
ggcaaaagaa atggacaaat atgtcagtgc tgtgttcagg gagctcagaa tctctaaaac 1560
tggtgtgacc acaccatcac cagtggccga cattcaatga acatctaatt tgtgcaaagt 1620
gtaggcactg gtgatatctg tatctgttga tgatatttga ttggcagaaa gaaataggga 1680
ttaaacaaac acaaaaaaat gatgggacat agtgtggaat gatgggtttt ccagaaggga 1740
gcctgagtag tcacctaaaa ctgtggtcta tatgtttgta gggtgaagtt ctctctggat 1800
catctcagct actcatcaga cactgagata acctgtctct gggattgttc ttaggcttga 1860
gttcactcct aacacttgct ggcctggggc aagaatacaa agcaggttcc tggctctttc 1920
tgcaatcttc ccttctatcc ctggttctga ctctcaccct gggcacccag gaaagaaaga 1980
aacatacaaa taggcggtgg ggggtggggg ttggcaccag tgagattcag agactaca 2038

<210> 160
<211> 30
<212> DNA
<213> Artificial Sequence

<220>
<223> Primer

<400> 160

gtaggctgat gggatggaga tgagaatgga 30

<210> 161
<211> 30
<212> DNA
<213> Artificial Sequence

<220>
<223> Primer

<400> 161

gcaggtagtt gatacaggca cagagcacat 30

<210> 162
<211> 368
<212> DNA
<213> Bos spp

<220>
<221> misc feature
<222> (196)..(196)
<223> n stands for any base

<220>
<221> misc feature
<222> (229)..(229)
<223> n stands for any base

<220>
<221> misc feature
<222> (234)..(234)
<223> n stands for any base

<220>
<221> misc feature
<222> (297)..(297)
<223> n stands for any base

<220>
<221> misc feature
<222> (350)..(350)
<223> n stands for any base

<400> 162

ccatcctaat acgactcact atagggctcg agcggccgcc cgggcaggtg ggaagggcgc 60
tgggtgagaa ggaataggga cacctccttc gaagcgcaag tccgggttcc cgggaccgct 120
ggtggggaga gcgcggaagc cggatcgagg atggcctgtt gactccccaa ggatgtgggc 180
agggtgctag gatctntgac ctcatgagag caggaggtct ggaccctanc tcanagtctg 240
cggaccctgg aatctcccac acaccgctca ctggaaagct tcaccatggc agtccanaat 300
ggaaatgcta gttttccagc caacttcagt ataccccaag aacatgcctn ctccctcccc 360
ttcaactt 368

<210> 163
<211> 27
<212> DNA

<213> Artificial Sequence

<220>
<223> Primer

<400> 163

agcctccttc ctgatcgctc tggtctg 27

<210> 164
<211> 29
<212> DNA
<213> Artificial Sequence

<220>
<223> Primer

<400> 164

cccatcagcc tacttcacaa aggaaaccg 29

<210> 165
<211> 911
<212> DNA
<213> Bos spp

<400> 165

```
tcctcttcat tgtcaaaaac cagaaaaaga tcttctgcgg ccaggtctgg ccagtggacc    60
agcarctcta ctataaatcc tacttcctct ttgtctttgg catcgagttc ctgggccccg   120
tgttcwccat gaccctgtgc tatgccagga tctcccgaga gctctggttc aaagcggtcc   180
cgggtttcca gactgagcaa atccggaaga ggctgcgctg ccgccggaag acggtactgg   240
tactcatgtg tatcctcacg gcctatgttc tgtgctgggc gcccttctat ggcctcacaa   300
tcgtgcgcga cttcttcccc actgtgttcg tgaaggaaaa acattacctc acagcctttt   360
atgttgtgga gtgcatcgcc atgagcaaca gcatgatcaa caccgtgtgc ttcgtgacag   420
tgaagaacag yaccatgaag tacttcaaga agatgctgct gctccattgg cggccctctc   480
accacgggag taagtccagt gccgacctcg acctcaaaac cagccgcctg ycagccacgg   540
aggaggtgga ttgtatcagg ctgaagtgac ccactggtac ttcacaatgg aaaacccaaa   600
agccagtact tggcgcacag tttatcaata accaagttca caggctgcat ggggaaaggg   660
caacagtttt cacacttcgc tgctgtcaag gagctggatg cttctgcagt cataaatgca   720
gtgtgacttg acacaaacca cagcagccaa catttactga tacgtgctcr acgtactgtg   780
cacaacacca ccagcgagat taaagagcaa caggagctga catttactga ttacctactg   840
tgtgtaaaaa tatttaaata gcaaaatgaa ttggcacctg cccgggcggc cgctcgagcc   900
ctatagtgag t                                                        911
```

<210> 166
<211> 27
<212> DNA
<213> Artificial Sequence

<220>
<223> Primer

<400> 166

ccatcctaat acgactcact atagggc 27

<210> 167
<211> 22
<212> DNA
<213> Artificial Sequence

<220>
<223> Primer

<400> 167

gctgggtgag aaggaatagg ga 22

<210> 168
<211> 21
<212> DNA
<213> Artificial Sequence

<220>
<223> Primer

<400> 168

gcaggtgcca attcattttg c 21

<210> 169
<211> 23
<212> DNA
<213> Artificial Sequence

<220>
<223> Primer

<400> 169

tgctctttaa tctcgctggt ggt 23

<210> 170
<211> 1623
<212> DNA
<213> Bos spp

<400> 170

gctgggtgag aaggaatagg gacacctcct tcgaagcgca agtccgggtt cccgagaccg 60
ctggtgggga gagcgcggaa gccggatcga ggatggcctg ttgactcccc aaggatgtgg 120
gcagggtgct aggatctctg acctcatgag agcagaaggt ctggacccta gctcagagtc 180
tgcagaccct ggaatctccc acacaccgct cactggaaag cttcaccatg gcagcccaga 240
atggaaatgc tagttttcca gccaacttca gtatacccca agaacatgcc tcctccctcc 300
ccttcaactt cagttatgat gattatgacc tccctctgga tgaggatgag gatatgacca 360
agactcagac cttctttgca gccaagattg ttatcggggt ggcacttgtg ggtatcatgc 420
tgacttgtgg tattggcaac tttgtcttta tcactgccct cacccgctat aaaaagctgc 480
gcaacctcac caacctgctc attgctaacc tggccatctc cgacttcctg gtagccatca 540
tctgctgccc ctttgagatg gactactatg tggtgcatca gctctcctgg gagcatggcc 600
atgtgctctg tgcctgtatc aactacctgc gtaccgtctc actctacgtc tccaccaatg 660
ccctgctggc cattgctatt gacagatatc tcgctatcgt tcaccccttg aaaccacgaa 720
tgaattatca aacagcctcc ttcctgatcg ctctggtctg gatggtatcc attctcatct 780
ccatcccatc agcctacttc acaaaggaaa ccgtcctctt cattgtcaaa aaccagaaaa 840
agatcttctg cggccaggtc tggccagtgg accagcagct ctactataaa tcctacttcc 900
tctttgtctt tggcatcgag ttcctgggcc ccgtgttcac catgaccctg tgctatgcca 960
ggatctcccg agagctctgg ttcaaagcgg tcccgggttt ccagactgag caaatccgga 1020
agaggctgcg ctgccgccgg aagacggtac tggtactcat gtgtatcctc acggcctatg 1080
ttctgtgctg ggcgcccttc tatggcttca caatcgtgcg cgacttcttc cccactgtgt 1140
tcgtgaagga aaaacattac ctcacagcct tttatgttgt ggagtgcatc gccatgagca 1200
acagcatgat caacaccgtg tgcttcgtga cagtgaagaa cagcaccatg aagtacttca 1260
agaagatgct gctgctccat tggcggccct ctcaccacgg gagtaagtcc agtgctgacc 1320
tcgacctcaa aaccagccgc ctgccagcca cggaggaggt ggattgtatc aggctgaagt 1380
gacccactgg tacttcacaa tggaaaaccc aaaagccagt acttggcgca cagtttatca 1440
ataaccaagt tcacaggctg catggggaaa gggcaacagt tttcacactt cgctgctgtc 1500
aaggagctgg atgcttctgc agtcataaaa tgcagtgtga cttgacacaa accacagcag 1560
ctaacattta ctgatacgtg ctcaacgtac tgtgcacaac accaccagcg agattaaaga 1620
gca 1623

<210> 171
<211> 393
<212> PRT
<213> Bos spp

<400> 171

Met Glu Ile Thr Met Gly Val Met Asp Glu Asn Ala Thr Asn Thr Ser
5 10 15
Thr Asn Tyr Phe Pro Leu Leu Asp Pro Leu Gly Ala Gln Ala Ala Ser
20 25 30
Phe Pro Phe Asn Phe Ser Tyr Gly Asp Tyr Asp Met Pro Leu Asp Glu
35 40 45
Asp Glu Asp Met Thr Asn Ser Arg Thr Phe Phe Ala Ala Lys Ile Val
50 55 60
Ile Gly Met Ala Leu Val Gly Ile Met Leu Val Cys Gly Ile Gly Asn
65 70 75 80
Phe Ile Phe Ile Ala Ala Leu Ala Arg Tyr Lys Lys Leu Arg Asn Leu
85 90 95
Thr Asn Leu Leu Ile Ala Asn Leu Ala Ile Ser Asp Phe Leu Val Ala
100 105 110
Ile Val Cys Cys Pro Phe Glu Met Asp Tyr Tyr Val Val Arg Gln Leu
115 120 125
Ser Trp Glu His Gly His Val Leu Cys Ala Ser Val Asn Tyr Leu Arg
130 135 140
Thr Val Ser Leu Tyr Val Ser Thr Asn Ala Leu Leu Ala Ile Ala Ile
145 150 155 160
Asp Arg Tyr Leu Ala Ile Val His Pro Leu Arg Pro Arg Met Lys Tyr
165 170 175
Gln Thr Ala Thr Gly Leu Ile Ala Leu Val Trp Val Val Ser Ile Leu
180 185 190
Val Ala Ile Pro Ser Ala Tyr Phe Thr Thr Glu Thr Val Leu Val Ile
195 200 205
Val Lys Ser Gln Glu Lys Ile Phe Cys Gly Gln Ile Trp Pro Val Asp
210 215 220
Gln Gln Ile Tyr Tyr Lys Ser Tyr Phe Leu Phe Ile Phe Gly Ile Glu
225 230 235 240
Phe Val Gly Pro Val Val Thr Met Thr Leu Cys Tyr Ala Arg Ile Ser
245 250 255
Arg Glu Leu Trp Phe Lys Ala Val Pro Gly Phe Gln Thr Glu Gln Ile
260 265 270
Arg Lys Arg Leu Arg Cys Arg Arg Lys Thr Val Leu Val Leu Met Cys
275 280 285
Ile Leu Thr Ala Tyr Val Leu Cys Trp Ala Pro Phe Tyr Gly Phe Ala
290 295 300
Ile Val Arg Asp Phe Phe Pro Thr Val Phe Val Lys Glu Lys His Tyr
305 310 315 320
Leu Thr Ala Phe Tyr Val Val Glu Cys Ile Ala Met Ser Asn Ser Met
325 330 335
Ile Asn Thr Val Cys Phe Val Thr Val Lys Asn Asn Thr Ile Lys Tyr
340 345 350
Phe Lys Lys Ile Met Leu Leu His Trp Lys Ala Ser Tyr Asn Gly Ser
355 360 365
Lys Ser Ser Gly Asp Leu Asp Leu Lys Thr Thr Gly Val Pro Ala Thr
370 375 380
Glu Glu Val Asp Cys Ile Gly Leu Lys
385 390

<210> 172
<211> 1179
<212> PRT
<213> Bos spp

<400> 172

atggagatca ccatgggggt catggatgag aatgccacca atacgtcaac caactatttc 60
cctctgcttg accccctcgg agcccaagct gcttctttcc ccttcaactt cagctacggt 120
gactatgata tgcccttgga tgaagatgag gatatgacca attcccggac cttctttgcc 180
gccaagattg tcattggcat ggcgctggtg ggtattatgc tggtctgtgg tatcggcaac 240
ttcatcttca tcgctgccct ggcccgctac aagaagctac gcaacctcac caatctgctc 300
atcgccaacc tggccatctc tgatttcctg gtggccattg tctgctgccc cttcgagatg 360
gactactacg tggtgcgcca gctctcctgg gagcacggcc acgtgctgtg cgcctctgtc 420
aactacctgc gcacggtctc tctctacgtc tccaccaacg ccctgctggc catcgccatc 480
gacagatatc tggccattgt ccacccgctg agaccccgga tgaagtacca aacagccacg 540
ggcttgattg ccttggtgtg ggtggtgtcc attcttgttg ccataccgtc agcctacttc 600

```
accactgaaa cggtcctcgt cattgtcaag agccaggaga agattttctg tggccagatc    660
tggccagtag accagcagat ctactacaag tcctacttcc tcttcatctt tggcatcgag    720
ttcgtgggcc cagtggtcac catgacccta tgctatgcca ggatctcccg agagctctgg    780
ttcaaggccg tccctggttt ccagacggag cagatccgga agcgactgcg ctgtcgccgg    840
aagaccgtcc tggtgctcat gtgcatcctc acggcgtacg tgctgtgctg ggcgcccttc    900
tacggcttcg ccatcgtgcg ggacttcttc cccacagtgt ttgtgaagga gaaacactat    960
ctcacggcct tctacgtggt cgagtgcatt gccatgagca acagcatgat caacaccgtg   1020
tgcttcgtga ctgtcaagaa taacaccatc aagtacttca agaagattat gctgctccac   1080
tggaaggctt cttacaatgg cagcaagtcc agtggggacc ttgacctcaa aaccacgggg   1140
gtgcctgcca ctgaagaggt ggactgcatc gggctgaaa                          1179
```

<210> 173
<211> 384
<212> PRT
<213> Bos spp

<400> 173

```
Met Ala Ala Gln Asn Gly Asn Ala Ser Phe Pro Ala Asn Phe Ser Ile
1               5                   10                  15
Pro Gln Glu His Ala Ser Ser Leu Pro Phe Asn Phe Ser Tyr Asp Asp
            20                  25                  30
Tyr Asp Leu Pro Leu Asp Glu Asp Glu Asp Met Thr Lys Thr Gln Thr
        35                  40                  45
Phe Phe Ala Ala Lys Ile Val Ile Gly Val Ala Leu Val Gly Ile Met
    50                  55                  60
Leu Thr Cys Gly Ile Gly Asn Phe Val Phe Ile Thr Ala Leu Thr Arg
65                  70                  75                  80
Tyr Lys Lys Leu Arg Asn Leu Thr Asn Leu Leu Ile Ala Asn Leu Ala
                85                  90                  95
Ile Ser Asp Phe Leu Val Ala Ile Ile Cys Cys Pro Phe Glu Met Asp
            100                 105                 110
Tyr Tyr Val Val His Gln Leu Ser Trp Glu His Gly His Val Leu Cys
        115                 120                 125
Ala Cys Ile Asn Tyr Leu Arg Thr Val Ser Leu Tyr Val Ser Thr Asn
    130                 135                 140
Ala Leu Leu Ala Ile Ala Ile Asp Arg Tyr Leu Ala Ile Val His Pro
145                 150                 155                 160
Leu Lys Pro Arg Met Asn Tyr Gln Thr Ala Ser Phe Leu Ile Ala Leu
                165                 170                 175
Val Trp Met Val Ser Ile Leu Ile Ser Ile Pro Ser Ala Tyr Phe Thr
            180                 185                 190
Lys Glu Thr Val Leu Phe Ile Val Lys Asn Gln Lys Lys Ile Phe Cys
        195                 200                 205
Gly Gln Val Trp Pro Val Asp Gln Gln Leu Tyr Tyr Lys Ser Tyr Phe
    210                 215                 220
Leu Phe Val Phe Gly Ile Glu Phe Leu Gly Pro Val Phe Thr Met Thr
225                 230                 235                 240
Leu Cys Tyr Ala Arg Ile Ser Arg Glu Leu Trp Phe Lys Ala Val Pro
                245                 250                 255
Gly Phe Gln Thr Glu Gln Ile Arg Lys Arg Leu Arg Cys Arg Arg Lys
            260                 265                 270
Thr Val Leu Val Leu Met Cys Ile Leu Thr Ala Tyr Val Leu Cys Trp
        275                 280                 285
Ala Pro Phe Tyr Gly Phe Thr Ile Val Arg Asp Phe Phe Pro Thr Val
    290                 295                 300
Phe Val Lys Glu Lys His Tyr Leu Thr Ala Phe Tyr Val Val Glu Cys
305                 310                 315                 320
Ile Ala Met Ser Asn Ser Met Ile Asn Thr Val Cys Phe Val Thr Val
                325                 330                 335
Lys Asn Ser Thr Met Lys Tyr Phe Lys Lys Met Leu Leu Leu His Trp
            340                 345                 350
Arg Pro Ser His His Gly Ser Lys Ser Ser Ala Asp Leu Asp Leu Lys
        355                 360                 365
Thr Ser Arg Leu Pro Ala Thr Glu Glu Val Asp Cys Ile Arg Leu Lys
    370                 375                 380
```

<210> 174

<211> 1152
<212> DNA
<213> Bos spp

<400> 174

```
atggcagccc agaatggaaa tgctagtttt ccagccaact tcagtatacc ccaagaacat      60
gcctcctccc tccccttcaa cttcagttat gatgattatg acctccctct ggatgaggat     120
gaggatatga ccaagactca gaccttcttt gcagccaaga ttgttatcgg ggtggcactt     180
gtgggtatca tgctgacttg tggtattggc aactttgtct ttatcactgc cctcacccgc     240
tataaaaagc tgcgcaacct caccaacctg ctcattgcta acctggccat ctccgacttc     300
ctggtagcca tcatctgctg cccctttgag atggactact atgtggtgca tcagctctcc     360
tgggagcatg gccatgtgct ctgtgcctgt atcaactacc tgcgtaccgt ctcactctac     420
gtctccacca atgccctgct ggccattgct attgacagat atctcgctat cgttcacccc     480
ttgaaaccac gaatgaatta tcaaacagcc tccttcctga tcgctctggt ctggatggta     540
tccattctca tctccatccc atcagcctac ttcacaaagg aaaccgtcct cttcattgtc     600
aaaaaccaga aaaagatctt ctgcggccag gtctggccag tggaccagca gctctactat     660
aaatcctact tcctctttgt ctttggcatc gagttcctgg gccccgtgtt caccatgacc     720
ctgtgctatg ccaggatctc ccgagagctc tggttcaaag cggtcccggg tttccagact     780
gagcaaatcc ggaagaggct gcgctgccgc cggaagacgg tactggtact catgtgtatc     840
ctcacggcct atgttctgtg ctgggcgccc ttctatggct tcacaatcgt gcgcgacttc     900
ttccccactg tgttcgtgaa ggaaaaacat tacctcacag ccttttatgt tgtggagtgc     960
atcgccatga gcaacagcat gatcaacacc gtgtgcttcg tgacagtgaa gaacagcacc    1020
atgaagtact tcaagaagat gctgctgctc cattggcggc cctctcacca cgggagtaag    1080
tccagtgctg acctcgacct caaaaccagc cgcctgccag ccacggagga ggtggattgt    1140
atcaggctga ag                                                        1152
```

<210> 175
<211> 23
<212> DNA
<213> Artificial Sequence

<220>
<223> Primer

<400> 175

```
gctcctcccg gttctttgaa atc                                             23
```

<210> 176
<211> 23
<212> DNA
<213> Artificial Sequence

<220>
<223> Primer

<400> 176

```
tggtgaggtt gcgtagcttc ttg                                             23
```

<210> 177
<211> 25
<212> DNA
<213> Artificial Sequence

<220>
<223> Primer

<400> 177

```
aaccaactat ttccctctgc ttgac                                           25
```

<210> 178
<211> 22
<212> DNA
<213> Artificial Sequence

<220>
<223> Primer

<400> 178

tcaccgtagc tgaagttgaa gg 22

<210> 179
<211> 24
<212> DNA
<213> Artificial Sequence

<220>
<223> Probe

<400> 179

cctcggagcc caagctgctt cttt 24

<210> 180
<211> 22
<212> DNA
<213> Artificial Sequence

<220>
<223> Primer

<400> 180

gctgggtgag aaggaatagg ga 22

<210> 181
<211> 25
<212> DNA
<213> Artificial Sequence

<220>
<223> Primer

<400> 181

ggagagctga tgcaccacat agtag 25

<210> 182
<211> 22
<212> DNA
<213> Artificial Sequence

<220>
<223> Primer

<400> 182

tcatgagagc agaaggtctg ga 22

<210> 183
<211> 22
<212> DNA
<213> Artificial Sequence

<220>
<223> Primer

<400> 183

tggctggaaa actagcattt cc 22

<210> 184

<211> 26
<212> DNA
<213> Artificial Sequence

<220>
<223> Probe

<400> 184

cacacaccgc tcactggaaa gcttca 26